# EUROPEAN PATENT SPECIFICATION

(11) **EP 3 027 615 B1**
(45) Date of publication and mention of the grant of the patent: **21.07.2021**
(21) Application number: 14745158.7
(22) Date of filing: 01.08.2014
(51) Int. Cl.: C07D 471/04

(54) **ANTI-INFECTIVE COMPOUNDS**
ANTIINFEKTIONSVERBINDUNGEN
COMPOSÉS ANTI-INFECTIEUX

(30) Priority: 02.08.2013 US 201361861750 P
(43) Date of publication of application: 08.06.2016
(73) Proprietor: Institut Pasteur Korea, Seongnam-si, Gyeonggi 463-400 (KR); Qurient Co. Ltd., Seongnam-si, Gyeonggi-do 463-400 (KR)
(72) Inventor: KIM, Jaeseung, Seoul 135-903 (KR); KANG, Sunhee, Yongin-si Gyeonggi-do 448-739 (KR); SEO, Min Jung, Incheon 405-761 (KR); SEO, Mooyoung, Yongin-si Gyeonggi-do (KR); SEO, Jeongjea, Seoul 139-916 (KR); LEE, Sumi, Busan, 607-837 (KR); KANG, Juhee, Bucheon-si Gyeonggi-do 420-708 (KR); PARK, Dongsik, Yongin-si Gyeonggi-do 448-504 (KR); KIM, Ryang Yeo, Yongin-si Gyeonggi-do 448-831 (KR); PETHE, Kevin, Seoul 140-809 (KR); NAM, Kiyean, Seoul 138-913 (KR); KIM, Jeongjun, Seoul 137-049 (KR); OH, Soohyun, Seoul (KR); LEE, Saeyeon, Gyeonggi-do (KR); AHN, Jiye, Sungnam-si, Gyeonggi-do (KR)
(74) Representative: Engelhard, Markus
(86) International application number: PCT/EP2014/066614
(87) International publication number: WO 2015/014993

(56) References cited:
- WO-A1-2011/113606
- WO-A2-2009/140101
- WO-A2-2009/140101
- WO-A2-2011/057145
- WO-A2-2012/143796
- US-A1- 2010 298 314
- US-A1- 2010 298 314
- S. EKINS ET. AL.: "Bayesin Models Leveraging Bioactivity and Cytotoxicity Information for Drug Discovery", CHEMISTRY AND BIOLOGY, vol. 20, no. 3, 21 February 2013 (2013-02-21), pages 370-378, XP002768838,
- P. S. SHIRUDE ET. AL.: "Azaindoles. Noncovalent DprE1 Inhibitors from Scaffold Morphing Efforts, Kill Mycobacterium Tuberculosis and are Efficacious in Vivo.", JOURNAL OF MEDICINAL CHEMISTRY, vol. 56, 11 November 2013 (2013-11-11), pages 9701-9708, XP002730637, DOI: 10.1021/jm401382v
- K. PETHE ET. AL.: "Discovery of Q203, a potent climical candidate for the treatment of tuberculosis.", NATURE MEDICINE, vol. 19, no. 9, 4 August 2013 (2013-08-04) , pages 1157-1162, XP002730638,
- S. EKINS ET. AL.: "Bayesin Models Leveraging Bioactivity and Cytotoxicity Information for Drug Discovery", CHEMISTRY AND BIOLOGY, vol. 20, no. 3, 21 February 2013 (2013-02-21), pages 370-378, XP002768838,

## Description

### Anti-infective compounds

The present invention relates to small molecule compounds and their use in the treatment of bacterial infections, in particular Tuberculosis.

### Background of the Invention

Tuberculosis (TB) still claims the life of more than 1.8 million people each year. Inadequate use of chemotherapy has led to an increasing number in multi-drug resistant (MDR) TB, and the situation is likely to worsen with the emergence and spread of exensively drug resistant form of the disease (Chaisson R.E.&Nuermberger E.L., N Engl J Med 2012; Zhao Y. et al., N Engl J Med 2012). The most urgent clinical need is to discover potent agents capable of reducing the time of M-XDR tuberculosis therapy with a success rate comparable to susceptible tuberculosis. The last decade has seen the discovery of promising new agent classes for the management of tuberculosis (Stover C.K. et al. Nature 2000 ; Andreis K. et al. Science 2005; Makarov V. et al. Science 2009), several of which are currently under clinical development (Diacon A.H. et al. Antimicrob Agents Chemother 2010; Diacon A.H. et al. Antimicrob Agents Chemother 2012 ; Gler M.T. et al. N Engl J Med 2012). However, given the high attrition rate during clinical development and emergence of resistance, the discovery of additional clinical candidates is clearly needed.

Current chemotherapy consists of compounds that directly target *Mycobacterium tuberculosis* bacillus, by targeting either the synthesis of macromolecules such as DNA, RNA or protein synthesis, or key components of the cell-wall. The most widely used dedicated anti-tubercular drugs isoniazid, ethionamide and pyrazinamide are pro-drugs that first require activation. As active forms, they demonstrate inhibitory activity on primarily cell-wall synthesis and/or on a wide range of mycobacterial targets, which have not yet been fully characterized.

One of the most challenging obstacle in the discovery of new anti-TB drugs is the lack of predictive *in vitro* screening methods that reproduce critical features found *in vivo.* Although there is still a lack of understanding of the biological mechanisms behind tubercle bacillus persistence, i.e. the location and state of latent bacteria in humans, *M. tuberculosis* is thought to persists in primary granulomas (Lenaerts *et al.,* 2007) and within various cell types (Houben *et al.,* 2006; Neyrolles *et al.,* 2006). The bacillus mainly localizes inside phagocytic cells, such as macrophages and dendritic cells, where it adapts drastically its metabolism to survive the harsh environment found in professional phagocytic cells (Rohde *et al.,* 2007; Schnappinger *et al.,* 2003). Therefore, we developed and used a phenotypic high-content screening technology in infected macrophages to identify novel antitubercular compounds (WO2010003533A2), overcoming many of the numerous and burdensome steps involved with other methodologies (Arain *et al.,* 1996). The technology has several advantages compared to traditional phenotypic screening approaches since it allows i) screening under physiologically relevant conditions, which is notoriously challenging in the field (Pethe K. et al. Nat Commun 2010; Stanley S.A. et al., ACS Chem Biol 2012), ii) selection of non-cytotoxic compounds that penetrate effectively inside macrophages, and iii) selection of compounds that are poor substrates for macrophage-induced efflux mechanisms (Adams K.N. et al. Cell 2011), thereby compressing the discovery and optimization time of new lead molecules.

WO 2011/113606 discloses benzyl aminocarbonyl-imidazo[1,2-a]pyridines and their utility as anti-infective compounds.

WO 2012/143796 discloses piperazine-1-yl-benzyl aminocarbonyl-imidazo[1,2-a]pyridines and their utility as anti-inflammation compounds.

Ekins et al. (Chemistry and Biology, vol. 20, pp. 370-378) discloses Bayesian models to screen for compounds with antitubercular cell activity.

It was an object of the present invention to identify compounds effective against bacterial infections, in particular compounds that would prevent *M. tuberculosis* multiplication inside the host macrophage.

### Description of the Invention

The present invention relates to compounds in accordance with the appended claims.

In one aspect, the present invention relates to compounds having the general formula I: 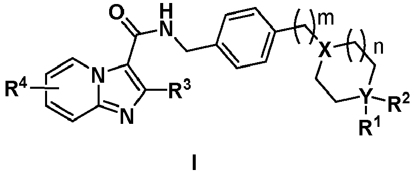 wherein
X is CH or N;
   Y is CH, O or N;
   m is 0 or 1;
   n is 0 or 1;
R¹ is, at each occurrence, independently selected from the group consisting of hydrogen, halogen, methyl, ethyl, t-butyl, phenyl, -NC(O)R⁵, -OR⁵, -C(O)R^{5,} -C(O)OR⁵, any of which is optionally substituted;
R² is, at each occurrence, independently selected from the group consisting of hydrogen and hydroxyl;
R³ is, at each occurrence, independently selected from the group consisting of methyl and ethyl;
R⁴ is, at each occurrence, independently selected from the group consisting of hydrogen, halogen, methyl, -methoxy and -CF₃;
R⁵ is, at each occurrence, independently selected from the group consisting of C₁-C₃ alkylhetorocycle, phenyl and benzyl, any of which is optionally substituted;
and pharmaceutically acceptable salts thereof;
or
a compound which has the general formula VII: 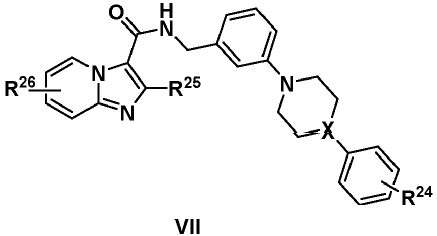 wherein
X is CH or N
R²⁴ is, at each occurrence, independently selected from the group consisting of hydrogen, halogens, C₁-C₂ alkyl, -methoxy, -CF₃ and -OCF₃;
R²⁵ is, at each occurrence, independently selected from the group consisting of methyl and ethyl;
R²⁶ is, at each occurrence, independently selected from the group consisting of hydrogen, halogen, methyl, -methoxy and -CF₃;
and pharmaceutically acceptable salts thereof, or
a compound which has the general formula VIII: 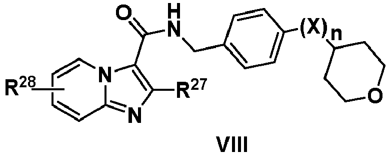 wherein
X is CH₂ or NH
   n is 0 or 1
R²⁷ is, at each occurrence, independently selected from the group consisting of methyl and ethyl;
R²⁸ is, at each occurrence, independently selected from the group consisting of hydrogen, halogen, methyl, -methoxy and -CF₃;
and pharmaceutically acceptable salts thereof,
wherein the compound has one one of the formulae 1-231, 238, 281-285, 289-300, 309-316, 325-328, 333-337, 340-350, as shown further below.

The term "optionally substituted" as used herein is meant to indicate that a hydrogen atom attached to a member atom within a group, or several such hydrogen atoms, is replaced by a group, such as halogen including fluorine, C₁-C₃ alkyl, C₁-C₃ haloalkyl, methylhydroxyl, COOMe, C(O)H, COOH, OMe, or OCF₃;

In one embodiment, the present invention also relates to pharmeceutically acceptable salts of the compounds according to the present invention.

The term "alkyl" refers to a monovalent straight or branched chain, saturated aliphatic hydrocarbon radical having a number of carbon atoms in the specified range. Thus, for example, "C₁-C₆ alkyl" refers to any of the hexyl alkyl and pentyl alkyl isomers as well as n-, iso-, sec-, and t-butyl, n- and isopropyl, ethyl and methyl.

The term "alkenyl" refers to a monovalent straight or branched chain aliphatic hydrocarbon radical containing one carbon-carbon double bond and having a number of carbon atoms in the specified range. Thus, for example, "C₂-C₆ alkenyl" refers to all of the hexenyl and pentenyl isomers as well as 1-butenyl, 2-butenyl, 3-butenyl, isobutenyl, 1-propenyl, 2-propenyl, and ethenyl (or vinyl).

The term "cycloalkyl", alone or in combination with any other term, refers to a group, such as optionally substituted or non-substituted cyclic hydrocarbon, having from three to eight carbon atoms, unless otherwise defined. Thus, for example, "C₃-C₈ cycloalkyl" refers to cyclopropyl, cyclobutyl, cyclopentyl, cyclohexyl, cycloheptyl, and cyclooctyl.

The term "haloalkyl" refers to an alkyl group, as defined herein that is substituted with at least one halogen. Examples of straight or branched chained "haloalkyl" groups useful in the present invention include, but are not limited to, methyl, ethyl, propyl, isopropyl, *n*-butyl, and *t*-butyl substituted independently with one or more halogens. The term "haloalkyl" should be interpreted to include such substituents such as -CHF₂, -CF₃, -CH₂-CH₂-F, -CH₂-CF₃, and the like.

The term "heteroalkyl" refers to an alkyl group where one or more carbon atoms have been replaced with a heteroatom, such as, O, N, or S. For example, if the carbon atom of alkyl group which is attached to the parent molecule is replaced with a heteroatom (*e.g*., O, N, or S) the resulting heteroalkyl groups are, respectively, an alkoxy group (*e.g.,* -OCH₃, etc.), an amine (*e.g.,* -NHCH₃, -N(CH₃)₂, etc.), or thioalkyl group (*e.g.,* -SCH₃, etc.). If a non-terminal carbon atom of the alkyl group which is not attached to the parent molecule is replaced with a heteroatom (*e.g*., O, N, or S) and the resulting heteroalkyl groups are, respectively, an alkyl ether (*e.g.,* -CH₂CH₂-O-CH₃, etc.), alkyl amine (*e.g.,* -CH₂NHCH₃, -CH₂N(CH₃)₂, etc.), or thioalkyl ether (e.g., -CH₂-S-CH₃).

The term "halogen" refers to fluorine, chlorine, bromine, or iodine.

The term "phenyl" as used herein is meant to indicate that optionally substituted or non-substituted phenyl group.

The term "benzyl" as used herein is meant to indicate that optionally substituted or non-substituted benzyl group.

The term "heteroaryl" refers to (i) optionally substituted 5- and 6-membered heteroaromatic rings and (ii) optionally substituted 9- and 10-membered bicyclic, fused ring systems in which at least one ring is aromatic, wherein the heteroaromatic ring or the bicyclic, fused ring system contains from 1 to 4 heteroatoms independently selected from N, O, and S, where each N is optionally in the form of an oxide and each S in a ring which is not aromatic is optionally S(O) or S(O)₂. Suitable 5- and 6-membered heteroaromatic rings include, for example, pyridyl, pyrrolyl, pyrazinyl, pyrimidinyl, pyridazinyl, triazinyl, thienyl, furanyl, imidazolyl, pyrazolyl, triazolyl, tetrazolyl, oxazolyl, isooxazolyl, oxadiazolyl, thiazolyl, isothiazolyl, and thiadiazolyl. Suitable 9-and 10-membered heterobicyclic, fused ring systems include, for example, benzofuranyl, indolyl, indazolyl, naphthyridinyl, isobenzofuranyl, benzopiperidinyl, benzisoxazolyl, benzoxazolyl, chromenyl, quinolinyl, isoquinolinyl, cinnolinyl, quinazolinyl, tetrahydroquinolinyl, tetrahydroisoquinolinyl, isoindolyl, benzodioxolyl, benzofuranyl, imidazo[1,2-a]pyridinyl, benzotriazolyl, dihydroindolyl, dihydroisoindolyl, indazolyl, indolinyl, isoindolinyl, quinoxalinyl, quinazolinyl, 2,3-dihydrobenzofuranyl, and 2,3-dihydrobenzo-1,4-dioxinyl.

The term "heterocyclyl" refers to (i) optionally substituted 4- to 8-membered, saturated and unsaturated but non-aromatic monocyclic rings containing at least one carbon atom and from 1 to 4 heteroatoms, (ii) optionally substituted bicyclic ring systems containing from 1 to 6 heteroatoms, and (iii) optionally substituted tricyclic ring systems, wherein each ring in (ii) or (iii) is independent of fused to, or bridged with the other ring or rings and each ring is saturated or unsaturated but nonaromatic, and wherein each heteroatom in (i), (ii), and (iii) is independently selected from N, O, and S, wherein each N is optionally in the form of an oxide and each S is optionally oxidized to S(O) or S(O)₂. Suitable 4- to 8-membered saturated heterocyclyls include, for example, azetidinyl, piperidinyl, morpholinyl, thiomorpholinyl, thiazolidinyl, isothiazolidinyl, oxazolidinyl, isoxazolidinyl, pyrrolidinyl, imidazolidinyl, piperazinyl, tetrahydrofuranyl, tetrahydrothienyl, pyrazolidinyl, hexahydropyrimidinyl, thiazinanyl, thiazepanyl, azepanyl, diazepanyl, tetrahydropyranyl, tetrahydrothiopyranyl, dioxanyl, and azacyclooctyl. Suitable unsaturated heterocyclic rings include those corresponding to the saturated heterocyclic rings listed in the above sentence in which a single bond is replaced with a double bond. It is understood that the specific rings and ring systems suitable for use in the present invention are not limited to those listed in this and the preceding paragraphs. These rings and ring systems are merely representative.

The term "MIC₈₀" refers to the concentration of compound which inhibits bacterial growth, preferably growth of M. tuberculosis, in comparison to a control without any drug after five days by 80%.

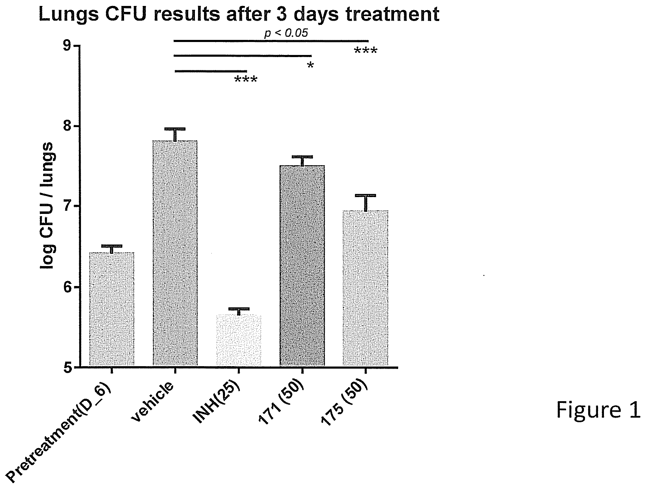

In accordance with the appended claims, the present invention relates to compounds having one of the formulae 1-231, 238, 281-285, 289-300, 309-316, 325-328, 333-337, 340-350, as shown in Tables 1 and 2, preferably one of the formulae 1-21, 23-24, 26, 28-33, 35-57, 59-77, 79-83, 85-87, 90-98, 100-102, 106-111, 113-116 118-124, 126-128, 130-142, 144-150, 153, 155-167, 169-184, 186-188, 190-197, 199, 201, 203-208, 210-211, 213-214, 216, 218-231, 238, 281-285, 289-300, 309-316, 325-328, 333-337, 340-350 as shown in Tables 1 and 2, and pharmaceutically acceptable salts thereof. Particularly preferred compounds are compounds having one of the formulae 55, 171, 175 and 325 as shown in Tables 1 and 2. Their pharmaceutical acitivity is also shown in Figure 1.

Preferably, the compounds as defined above have an inhibitory activity on bacterial growth, preferably on the growth of *M. tuberculosis,* inside a host cell, preferably a macrophage, at a concentration between 1-20 µM, preferably less than 1 µM. Preferably, the compounds as defined above have a MIC₈₀ of less than 1 µM.

In one aspect, the present invention relates to compounds as defined above for use in the treatment of a bacterial infection, e.g. tuberculosis.

In one aspect, the present invention relates to compounds as defined above for use in the treatment of Tuberculosis.

In one aspect, the present invention relates to a pharmaceutical composition comprising a compound as defined above, and a pharmaceutically acceptable carrier.

Also disclosed is a method of treatment of a bacterial infection, in particular Tuberculosis, comprising the application of a suitable amount of a compound as defined above or of a pharmaceutical composition as defined above to a person in need thereof.

In one embodiment, a "suitable amount", as used herein, is meant to refer to an amount in the range of from 0.01 mg/kg body weight to 1 g/kg body weight.

### Pharmaceutical compositions

### Pharmaceutically acceptable salts

Examples of pharmaceutically acceptable addition salts include, without limitation, the nontoxic inorganic and organic acid addition salts such as the acetate derived from acetic acid, the aconate derived from aconitic acid, the ascorbate derived from ascorbic acid, the benzenesulfonate derived from benzensulfonic acid, the benzoate derived from benzoic acid, the cinnamate derived from cinnamic acid, the citrate derived from citric acid, the embonate derived from embonic acid, the enantate derived from enanthic acid, the formate derived from formic acid, the fumarate derived from fumaric acid, the glutamate derived from glutamic acid, the glycolate derived from glycolic acid, the hydrochloride derived from hydrochloric acid, the hydrobromide derived from hydrobromic acid, the lactate derived from lactic acid, the maleate derived from maleic acid, the malonate derived from malonic acid, the mandelate derived from mandelic acid, the methanesulfonate derived from methane sulphonic acid, the naphthalene-2-sulphonate derived from naphtalene-2-sulphonic acid, the nitrate derived from nitric acid, the perchlorate derived from perchloric acid, the phosphate derived from phosphoric acid, the phthalate derived from phthalic acid, the salicylate derived from salicylic acid, the sorbate derived from sorbic acid, the stearate derived from stearic acid, the succinate derived from succinic acid, the sulphate derived from sulphuric acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

In another embodiment, the compounds of the invention are used in their respective free base form according to the present invention.

Metal salts of a chemical compound of the invention include alkali metal salts, such as the sodium salt of a chemical compound of the invention containing a carboxy group.

The chemical compounds of the invention may be provided in unsolvated or solvated forms together with a pharmaceutically acceptable solvent(s) such as water, ethanol, and the like. Solvated forms may also include hydrated forms such as the monohydrate, the dihydrate, the hemihydrate, the trihydrate, the tetrahydrate, and the like. In general, solvated forms are considered equivalent to unsolvated forms for the purposes of this invention.

### Administration and Formulation

The production of medicaments containing the compounds of the invention, its active metabolites or isomers and salts according to the invention and their application can be performed according to well-known pharmaceutical methods.

While the compounds of the invention, useable according to the invention for use in therapy, may be administered in the form of the raw chemical compound, it is preferred to introduce the active ingredient, optionally in the form of a physiologically acceptable salt in a pharmaceutical composition together with one or more adjuvants, excipients, carriers, buffers, diluents, and/or other customary pharmaceutical auxiliaries. Such salts of the compounds of the invention may be anhydrous or solvated.

In a preferred embodiment, the invention provides medicaments comprising a compound useable according to the invention, or a pharmaceutically acceptable salt or derivative thereof, together with one or more pharmaceutically acceptable carriers therefor, and, optionally, other therapeutic and/or prophylactic ingredients. The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The compounds useable according to the invention, together with a conventional adjuvant, carrier, or diluent, may thus be placed into the form of medicament and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such medicament and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The compounds useable according to the invention can be administered in a wide variety of oral and parenteral dosage forms. It will be obvious to those skilled in the art that the following dosage forms may comprise, as the active component, either a compound(s) useable according to the invention or a pharmaceutically acceptable salt of a compound(s) useable according to the invention.

For preparing a medicament from a compound useable according to the invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify. Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The chemical compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

In one embodiment of the present invention, the medicament is applied topically or systemically or via a combination of the two routes.

For administration, the compounds of the present invention may, in one embodiment, be administered in a formulation containing 0,001% to 70% per weight of the compound, preferably between 0,01% to 70% per weight of the compound, even more preferred between 0,1% and 70% per weight of the compound. In one embodiment, a suitable amount of compound administered is in the range of from 0.01 mg/kg body weight to 1 g/kg body weight.

Compositions suitable for administration also include lozenges comprising the active agent in a flavoured base, usually sucrose and acacia or tragacanth; pastilles comprising the active ingredient in an inert base such as gelatin and glycerol or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co. Easton, Pa.).

### Figures and Tables

Reference is now made to the figures and tables, wherein
**Figure 1** shows the in vivo efficacy of compounds 171 and 175 in a murine model of acute tuberculosis infection.
**Table 1** summarizes imidazopyridine derivatives (general scaffolds I-VId) with their respective inhibitory activities.
**Table 2** summarizes compounds 1-350 in terms of their structures and corresponding characteristics.

### Examples

The invention is now further described by reference to the following examples which are intended to illustrate, not to limit the scope of the invention.

### Example 1: Determination the Minimum Inhibitory Concentration 80% (MIC₈₀) of new chemical entities against M. tuberculosis

Cell-based assays are key tools in lead finding and optimization of new chemical entities for *Mycobacterium tuberculosis.* The availability of a robust *in vitro* assay for testing the Minimum inhibitory concentration (MIC) of a new chemical entity is an absolute requirement for the success of a program. The microplate broth dilution assay using a *M. tuberculosis* strain expressing the green-fluorescent protein (GFP) was selected as this method i) delivers highly reproducible results, ii) allows screening of large number of compounds, and iii) can be partially automated if required.

Breiefly, a starting culture of *M. tuberculosis* was prepared by diluting a frozen aliquot in 50mL of 7H9 medium supplemented with glycerol, to an optical density at 600nM (OD₆₀₀) of 0.02. The culture was incubated for 3 days at 37°C to an OD₆₀₀ of 0.2-0.3. The bacteria were the harvested by centrifugation at 3000 rpm, washed once and resuspended to an OD₆₀₀ of 0.1 in 7H9 medium without glycerol. The OD₆₀₀ was finally adjusted to 0.02 and the culture was kept at room temperature before dispensing to the assay plate.

The assay was carried out in 384-well flat bottom microplates in a final volume of 50µl. 25µl of the prepared bacterial working culture was added to the compound test plate containing 0.5µl of serial diluted test compounds.

The plates were incubated at 37°C for 5 days. Bacterial growth was determined after 5 days of incubation by measuring fluorescence intensity at 488nm after 5 days of incubation using the plate reader SPECTRA MAX plus (Molecular Devices®). MIC₈₀, the concentration of the compound that inhibits growth compared to the drug free control after 5 days by 80%, were determined using Graph Pad PRISM® software.

### Example 2: Derivatization of the imidazopyridine general scaffold

The imidazopyridine compounds (scaffolds I - IX; see **Table 1**) underwent derivatization according to the methods outlined below **(Schemes 1-22).** Resulting derivatives were examined for inhibitory activity (MIC) using the assays described above (Example 1) and the results are summarized in **Table 1.** The synthesized compounds 1-350 are shown in **Table 2.**

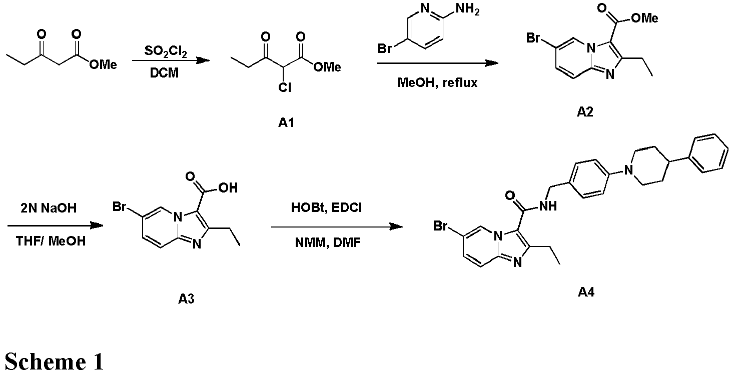

### General procedure for the synthesis of A1

To a solution of methyl 3-oxopentanoate (200 g, 1.55 mol) in anhydrous DCM (500 mL) was added SO₂Cl₂ (220 g, 1.63 mol) dropwise at 0 °C, then the mixture was stirred at 25 °C for 16 hours. The reaction mixture was poured into water (500 mL). The organic layer was separated and washed with water (500 mL×3), brine (500 mL), dried over anhydrous Na₂SO₄ and concentrated under reduced pressure to afford compound **A1** (245 g, yield: 96%) as a colorless oil which was used for next step without further purification.

### General procedure for the synthesis of A2

To a solution of 5-bromopyridin-2-amine (10.0 g, 57.8 mmol) in MeOH (10 mL) was added compound **A1** (10.5 g, 63.6 mmol) dropwise at 25 °C, then the mixture was stirred at reflux for 16 hours. The reaction mixture was concentrated. The residue was partitioned between EtOAc (100 mL) and water (100 mL). The organic layer was separated and washed with water (100 mL x 3), brine (100 mL), dried over anhydrous Na₂SO₄ and concentrated under reduced pressure. The residue was purified by combi flash (PE: EtOAc = 4: 1) to afford compound **A2** (4.00 g, yield: 25%) as a yellow powder.

### General procedure for the synthesis of A3

To a solution of compound **A2** (3.00 g, 10.6 mmol) in THF (40 mL) and MeOH (20 mL) was added 2N NaOH (40 mL) at 25 °C, then the mixture was stirred at 25 °C for 16 hours. Most of the MeOH and THF were evaporated under reduced pressure. The mixture was then washed with DCM (40 mL x 2). The aqueous layer was then acidified with HCl to pH = 6, No solid was precipitated. The aqueous phase was concentrated under reduced pressure and suspended in DCM/ MeOH= 5: 1 (40 mL) under stirring. The mixture was filtered and the filtrate was concentrated under reduced pressure to afford compound **A3** (2.60 g, yield: 91%) as a white powder.

### General procedure for the synthesis of A4

To a solution of compound **A3** (60 mg, 0.224 mmol), HOBt (45 mg, 0.336 mmol), EDCI (86 mg, 0.448 mmol) in 1.5 mL DMF was added NMM (136 mg, 1.34mmol). The mixture was stirred at 20°C for 10 minutes. Then (4-(4-phenylpiperidin-1-yl)phenyl)methanamine (40 mg, 0.212 mmol) was added to the mixture and stirred at 30°C for 18 hours. 15 mL of water was added into the mixture and the solid was formed. The mixture was filtered and the filter cake was dissolved in 20 mL DCM and concentrated under reduced pressure to give the crude product **A4,** which was triturated with 3 mL x 2 of CH₃OH twice and 3 mL of CH₃CN in sequence and then filtered and the filter cake was dried to give the pure product **A4** (12 mg, 12%) as a white solid.

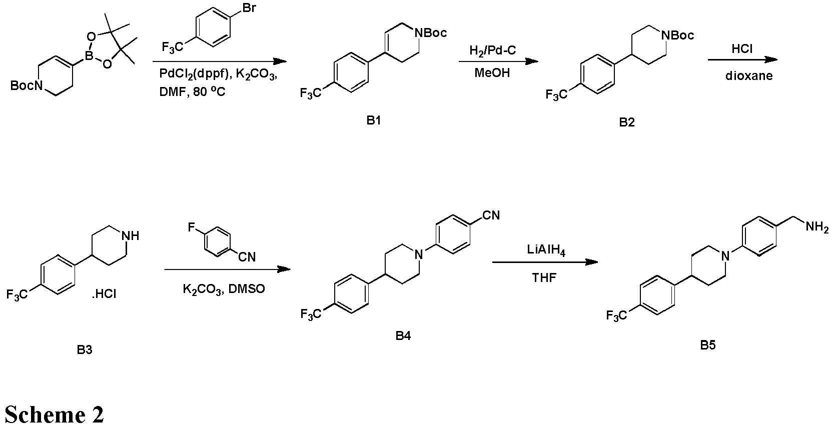

### General procedure for the synthesis of B1

To a suspension of compound 1-bromo-4-(trifluoromethyl)benzene (20.0 g, 89.3 mmol), compound tert-butyl 4-(4,4,5,5-tetramethyl-1,3,2-dioxaborolan-2-yl)-5,6-dihydropyridine-1(2H)-carboxylate (33.1 g, 107 mmol) in DMF (200 mL) was added K₂CO₃ (30.3 g, 223 mmol) and PdCl₂(dppf) (1.33 g, 1.79 mmol) under nitrogen. The reaction mixture was stirred at 80 °C under nitrogen for 16 hours. TLC and LCMS showed the reaction was finished. The mixture was filtered and the filtrate was concentrated under reduced pressure. The residue was partitioned between water (200 mL) and EtOAc (400 mL). The layers were separated and the aqueous layer was extracted with EtOAc (400 mL×2). The combined extracts were washed with water (100 mL×2), dried over anhydrous Na₂SO₄ and concentrated under reduced pressure. The residue pressure was purified by combi flash (Eluents: PE : THF = 19 : 1) to afford compound Y05_1A (16.0 g, 54.6%yield) as a yellow oil.

### General procedure for the synthesis of B2

To a solution of **B1** (16.0 g, 48.8 mmol) in MeOH (250 mL) was added Pd/C (10%, 2.50 g) under Ar atmosphere. The suspension was degassed under vacuum and purged with H₂ for 3 times. The reaction mixture was stirred at 20°C under H₂ atmosphere (40 psi) for 24 hours. LCMS showed that the reaction was finished. The mixture was filtered through a pad of celite and the filter cake was washed with MeOH (50 mL×3). The combined filtrates were concentrated under reduced pressure to dryness to give compound **B2** (14.0 g, 86.9% yield) as a colorless oil.

### General procedure for the synthesis of B3

A solution of compound **B2** (14.0 g, 42.4 mmol) in HCl/dioxane (4N, 140 mL) was stirred at 25 °C for 3 hours. LCMS showed the reaction was finished. The reaction mixture was concentrated to dryness to give compound **B3** (14.0 g 92.1% yield) as a white solid.

### General procedure for the synthesis of B4

To a solution of compound B3 (8.98 g, 39.0 mmol) and 4-fluorobenzonitrile (5.20 g, 43.0 mmol) in anhydrous DMSO (100 mL) was added K₂CO₃ (26.9 g, 195 mmol). The reaction mixture was stirred at 120 °C for 16 hours. LCMS indicated the reaction was finished. The mixture was poured into water (200 mL) and collected by filtration. The solid was dissolved in EtOAc (600 mL) which was washed with water (200 mL×3) and washed with brine (200 mL×3), dried over anhydrous Na₂SO₄ and concentrated under reduced pressure. The residue was triturated with n-hexane (100 mL) to afford compound **B4** (11.0 g, 85.5% yield) as a white solid.

### General procedure for the synthesis of B5

To a suspension of compound **B4** (7.00 g, 21.2 mmol) in anhydrous THF (120 mL) was added LiAlH₄ (4.10 g, 108 mmol) at 0∼10 °C. The reaction mixture was refluxed for 3 hours. TLC and LCMS showed the reaction was finished. The reaction mixture was cooled to 0 °C, and quenched with water (4.1 mL), NaOH (10%, 4.1 mL) and THF (120 mL) carefully. The mixture was filtered and the filtrate was dried over anhydrous Na₂SO₄, concentrated under reduced pressure. The residue was triturated with n-hexane (100 mL) to afford compound Y05 (5.60 g, crude) as white solid. To a solution of **B5** (5.60 g, crude), in MeOH (150 mL) was added Boc₂O (9.42 g, 42.4 mmol). The mixture was stirred at about 18 °C for 2 hours. LCMS showed the reaction was finished. The solution was concentrated in *vacuo* and the residue was purified by combi flash (Eluents: THF/PE = 1/20) to give compound Y05_Boc (5.80 g, crude). A mixture of **B5**_Boc in HCl/dioxane (4N, 80 mL) was stirred at 18 °C for 3 hours. LCMS showed the reaction was finished. The reaction mixture was concentrated under reduced pressure to give compound B5 (5.10 g 72.0% yield) as a white solid.

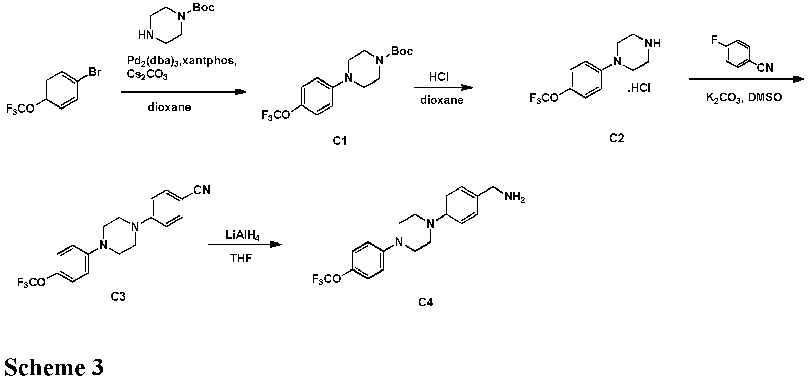

### General procedure for the synthesis of C1

To a suspension of 1-bromo-4-(trifluoromethoxy)benzene (20.0 g, 83.0 mmol), compound tert-butyl piperazine-1-carboxylate (18.6 g, 99.6 mmol) in dioxane (100 mL) was added Cs₂CO₃ (37.8 g, 166 mmol) and Pd₂(dba)₃ (1.20 g), Xantphos (1.20 g) under nitrogen. The reaction mixture was stirred at 120 °C under nitrogen for 16 hours. TLC and LCMS showed the reaction was finished. Water (200 mL) was added and the mixture was extracted with EtOAc (100 mL×3). The combined organic layers were washed with brine (100 mL×2), dried over anhydrous Na₂SO₄ and concentrated under reduced pressure. The residue was triturated with MTBE (50 mL) to afford compound **c1** (18.8 g, 65% yield) as a red solid.

### General procedure for the synthesis of C2

A solution of compound **C1** (18.8 g, 54.0 mmol) in HCl/dioxane (4N, 250 mmol) was stirred at 25 °C for 3 hours. LCMS showed the reaction was finished. The reaction mixture was concentrated to give compound **C2** (13.3 g, crude), which was used to next step directly.

### General procedure for the synthesis of C3

To a solution of compound C2 (13.3 g, 54.0 mmol) and 4-fluorobenzonitrile (7.20 g, 59.4 mmol) in anhydrous DMSO (150 mL) was added K₂CO₃ (30.0 g, 216 mmol). The reaction mixture was stirred at 120 °C for 16 hours. LCMS indicated the reaction was finished. The mixture was poured into water (600 mL) and collected by filtration. The solid was dissolved in EtOAc (500 mL), dried over anhydrous Na₂SO₄ and concentrated under reduced pressure. The residue was triturated with n-Hexane/MTBE to afford compound C3 (11.8 g, 62.9% yield) as brown solid.

### General procedure for the synthesis of C4

To a suspension of compound C3 (11.8 g, 34.0 mmol) in anhydrous THF (150 mL) was added LiAlH₄ (6.50 g, 170 mmol) at 0∼10 °C. The reaction mixture was refluxed for 3 hours. TLC and LCMS showed the reaction was finished. The reaction mixture was cooled to 0 °C, and quenched with water (6.5 mL), NaOH (10%, 6.5 mL), and THF (100 mL) carefully. The mixture was filtered and the filtrate was dried over anhydrous Na₂SO₄, concentrated under reduced pressure. The residue was triturated with n-Hexane/MTBE to afford compound **C4** (5.37 g, 45 %yield) as yellow solid.

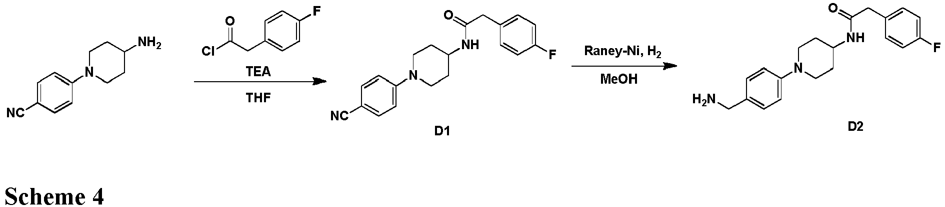

### General procedure for the synthesis of D1

To a solution of compound 4-(4-aminopiperidin-1-yl)benzonitrile (500 mg, 2.48 mmol) in anhydrous THF (5 mL) was added TEA (754 mg, 7.45 mmol) followed by (4-Fluoro-phenyl)-acetyl chloride (514 mg, 2.98 mmol) at 0 °C. After stirring at the temperature for 0.5 hour, the mixture was allowed to warm to 20 °C and stirred for 16 hours. The reaction mixture was diluted with water (100 mL), extracted with EtOAc (50 mL×3). The extracts was washed with brine, dried over Na₂SO₄ and concentrated to give a residue which was purified by silica gel column (eluent: PE/EA = 4/1 to 1/2) to afford 380 mg (yield: 45%) of **D1** as a white solid.

### General procedure for the synthesis of D2

To a solution of compound **D1** (380 mg, 1.13 mmol) in MeOH (10 mL) was added Raney-Ni (50 mg). After stirring at 20 °C for 2 hours, the mixture was filtered and the filtrate was concentrated to give crude product which was purified by silica gel column (eluent: DCM/MeOH = 30/1 to 10/1) to afford 150 mg (yield: 39%) of **D2** as a white solid.

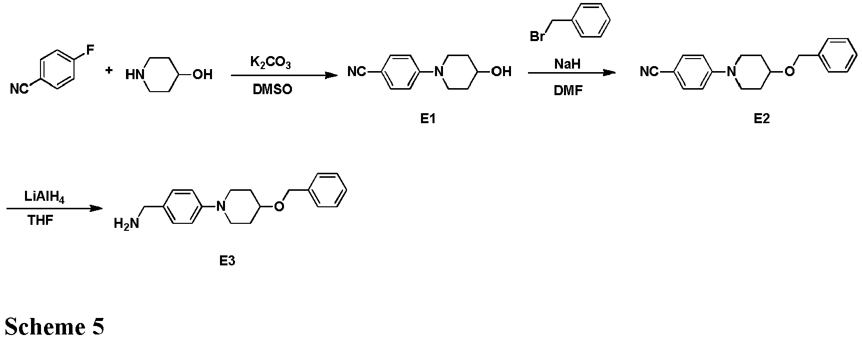

### General procedure for the synthesis of E1

A mixture of 4-fluoro-benzonitrile (5.00 g, 41.3 mmol), piperidin-4-ol (8.35 g, 82.6 mmol) and K₂CO₃ (5.71 g, 41.3 mmol) in DMSO (50 mL) was stirred at 120 °C for 16 hours. The mixture was diluted with water (100 mL), extracted with EtOAc (100 mL×3). The combined extracts were washed with water (100 mL) and brine (100 mL), dried over anhydrous Na₂SO₄ and concentrated to give a residue. The residue was purified by silica gel column (eluent: PE/EtOAc = 5/1 to 1/1) to afford 4.70 g (yield: 57%) of **E1** as a white solid.

### General procedure for the synthesis of E2

To a solution of compound **E1** (1.00 g, 4.94 mmol) in DMF (10 mL) was added NaH (60% dispersion in mineral oil, 237 mg, 5.93 mmol) at 0 °C. After stirring at 0 °C for 0.5 hour, Bromomethyl-benzene (930 mg, 5.4 mmol) was added to the mixture at 0 °C. Then the mixture was allowed to warm to 20°C and stirred for 16 hours. The reaction was quenched with water (100 mL) and extracted with EtOAc (50 mL×3). The combined extracts were washed with water (100 mL) and brine (100 mL), dried over anhydrous Na₂SO₄ and concentrated to give a residue. The residue was purified by silica gel column (eluent: PE/EtOAc = 4/1 to 1/1) to afford 1.10 g (yield: 78%) of **E2** as a white solid.

### General procedure for the synthesis of E3

To a solution of compound **E2** (1.00 g, 3.42 mmol) in anhydrous THF (10 mL) was added LiAlH₄ (390 mg, 10.2 mmol) at 0 °C. After stirring at 0 °C for 0.5 hour, the mixture was allowed to warm to 20 °C and stirred for 0.5 hour. The reaction was quenched with NaOH solution (0.5 mL), diluted with water (50 mL), extracted with EtOAc (50 mL×3). The extracts was washed with brine, dried over Na₂SO₄ and concentrated to give a residue which was purified by silica gel column (eluent: DCM/MeOH = 20/1) to afford 350 mg (yield: 35%) of **E3** as a white solid.

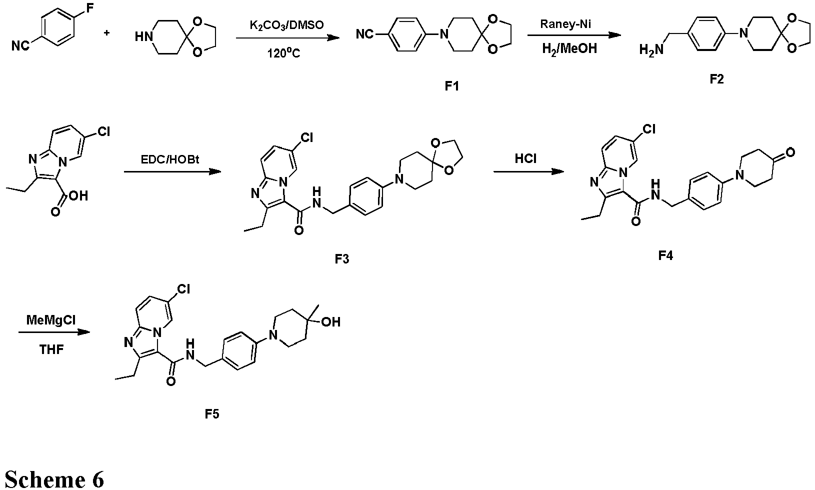

### General procedure for the synthesis of F1

A mixture of 4-fluoro benzonitrile (10.0 g, 82.0 mmol), 1,4-dioxa-8-azaspiro[4.5]decane (11.8 g, 82.0 mmol) and K₂CO₃ (11.4 g, 82.0 mmol) in DMSO (100 mL) was stirred at 100 °C 16 hours. The mixture was diluted with water (200 mL), extracted with EtOAc (250 mL×3). The combined extracts were washed with water (200 mL) and brine (200 mL), dried over anhydrous Na₂SO₄ and concentrated to afford 18.0 g (yield: 90%) of **F1** as a yellow solid.

### General procedure for the synthesis of F2

A solution of compound **F1** (5.00 g, 20.0 mmol) in MeOH (50 mL) was added Raney-Ni (1.0 g). After stirring at the temperature for 4 hours, the mixture was filtered and the filtrate was concentrated to afford **F2** (5.00 g, yield: 98%) as a yellow solid.

### General procedure for the synthesis of F3

A mixture of compound **F2** (1.10 g, 4.50 mmol), 6-chloro-2-ethylimidazo[1,2-a]pyridine-3-carboxylic acid (1.00 g, 4.50 mmol), EDCI (955 mg, 4.90 mmol), HOBt (661 mg, 4.90 mmol) and TEA (1.30 g, 13.3 mmol) in THF (20 mL) was stirred at 20 °C for 16 hours. Then the mixture was diluted with water (50 mL) and extracted with EtOAc (50 mL×3). The extracts were combined, washed with brine, dried over Na₂SO₄ and concentrated to give a residue which was purified by silica gel column (eluent: DCM/MeOH = 20/1 to 15/1) to afford 1.50 g (yield: 75%) of **F3** as a yellow solid.

### General procedure for the synthesis of F4

A solution of compound **F3** (1.40 g, 3.08 mmol) in THF/HCl (5 mL/5 mL, HCl: 2M) was refluxed for 16 hours. The mixture was diluted with water (80 mL) and basified with NaOH aqueous solution (2M, 5 mL) to pH = 8. Then the mixture was extracted with EtOAc (50 mL×3). The extracts were combined, washed with brine, dried over Na₂SO₄ and concentrated to give 1.00 g (yield: 79%) of compound **F4** as a brown solid.

### General procedure for the synthesis of F5

To a solution of compound **F4** (100 mg, 0.24 mmol) in anhydrous THF (5 mL) was added MeMgBr (0.16 mL, 0.48 mmol, 3.0 M in diethyl ether) dropwise at -78°C. The mixture was stirred at the temperature for 0.5 hour. The reaction mixture was quenched with MeOH (1 mL), diluted with water (30 mL), extracted with EtOAc (20 mL×3). The extracts were combined, washed with brine (30 mL), dried over Na₂SO₄ and concentrated to give a residue which was purified by Prep-HPLC (0.1% TFA as additive). Most of MeCN was removed under reduced pressure, the remaining solvent was removed by lyophilization to give 27 mg (as TFA salt, yield: 21%) of compound **F5** as pale yellow oil.

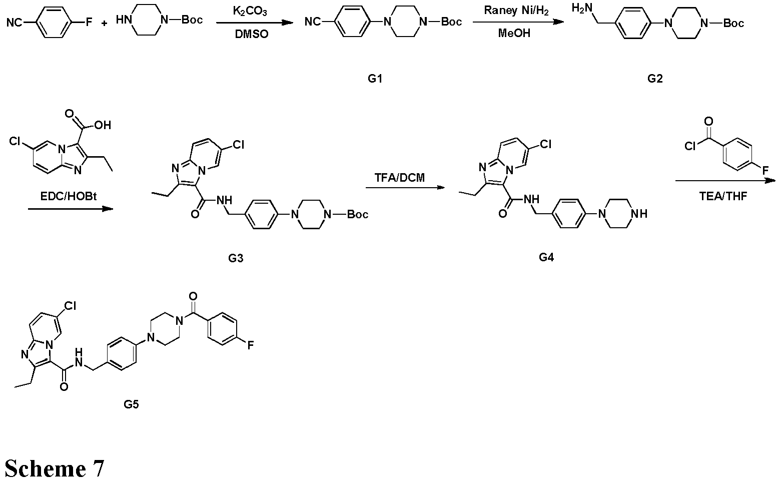

### General procedure for the synthesis of G1

To a solution of 4-fluoro-benzonitrile (7.80 g, 63.9 mmol) and 1-Boc-piperazine (10.0 g, 53.7 mmol) in DMSO (200 mL) was added K₂CO₃ (14.8 g, 107 mmol). The resulting mixture was stirred at 100 °C for 16 hours. TLC and LCMS showed the reaction was finished. The DMSO solvent was removed in vacuum, and the residue was suspended in water (100 mL), extracted with EtOAc (100 mL × 3). The combined extracts were washed with water (100 mL) and brine (100 mL), dried over anhydrous Na₂SO₄ and concentrated to give a residue. The residue was purified by re-crystallization from MeOH (150 mL) to afford 7.08 g (yield: 43%) of compound **G1** as a white powder.

### General procedure for the synthesis of G2

To a solution of **G1** (1.00 g, 3.50 mmol) in MeOH (50 mL) was added Raney-Ni (0.50 g). The suspension was degassed under vacuum and purged with H₂ for three times. The reaction mixture was stirred at 20 °C for 4 hours under H₂ atmosphere (45 psi). LCMS showed the reaction was completed. The reaction mixture was filtrated and the filtrate was concentrated under reduced pressure and purified by silica gel column (eluent: EtOAc/PE = 3/1 to EtOAc, 1% TEA as additive) to afford 1.00 g (yield: 100%) of compound **G2** as a white powder.

### General procedure for the synthesis of G3

A mixture of compound 6-chloro-2-ethylimidazo[1,2-a]pyridine-3-carboxylic acid (140 mg, 0.48 mmol), **G2** (90 mg, 0.40 mmol), EDCI (234 mg, 1.20 mmol), HOBt (162 mg, 1.20 mmol) and TEA (121 mg, 2.00 mmol) in THF (10 mL) was stirred at 20 °C for 16 hours. LCMS showed the reaction was finished. The reaction mixture was poured into water (30 mL), extracted with EtOAc (20 mL × 3). The combined extracts were washed with brine (10 mL), dried over anhydrous Na₂SO₄ and concentrated to give a residue. The residue was purified by silica gel column (eluent: PE/EtOAc = 8/1 to 4/1) to afford 120 mg (yield: 47%) of compound **G3** as a white powder.

### General procedure for the synthesis of G4

To a solution of **G3** (120 mg, 0.24 mmol) in DCM (5mL) was added TFA (1.5 mL). The resulting solution was stirred at 20 °C for 6 hours. LCMS showed the reaction was finished. The solvent was removed by concentration to afford 92 mg TFA Salt (yield: 87%) of compound **G4** as a white powder, without further purification for next step.

### General procedure for the synthesis of G5

To a solution of compound **G4** (45 mg, 0.11 mmol) and TEA (40 mg, 0.55 mmol) in DCM (10 mL) was added dropwise 4-fluorobenzoyl chloride (21 mg, 0.13 mmol). The resulting mixture was stirred at 20 °C for 1.5 hours. LCMS showed the reaction was finished. The reaction mixture was concentrated to give a residue, which was purified by Prep-HPLC (0.1% TFA as additive), most of CH₃CN was removed by evaporation under reduced pressure, and the remaining solvent was removed by lyophilization to afford 35 mg TFA salt (yield: 71%) of **G5** as a white powder.

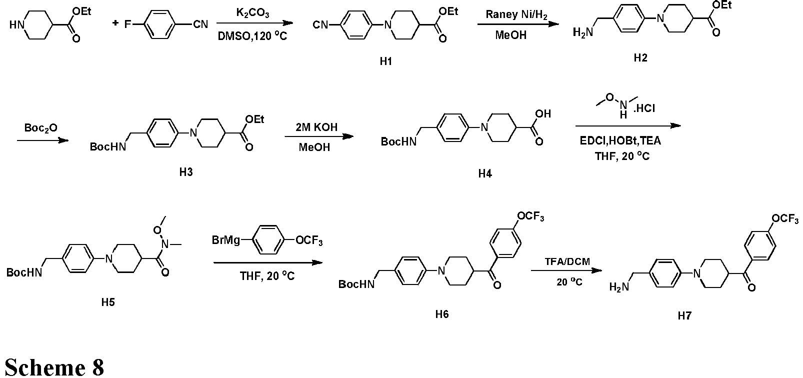

### General procedure for the synthesis of H1

A mixture of compound ethyl piperidine-4-carboxylate (10.0 g, 63.6 mmol), 4-fluorobenzonitrile (8.10 g, 65.5 mmol), K₂CO₃ (14.4 g, 104 mmol) in DMSO (150 mL) were stirred at 120 °C for 16 hours. LCMS showed the reaction was finished. After removal of solvent under vacuum, the residue was poured into water (100 mL), extracted with EtOAc (50 mL × 3), the combined extracts were washed with brine (50 mL), dried over anhydrous Na₂SO₄ and concentrated to give a residue, which was purified by silica gel column (eluent: PE/EtOAc = 4/1) to afford 9.50 g (yield: 51%) of compound **H1** as a dark oil.

### General procedure for the synthesis of H2

A mixture of compound **H1**(8.50 g, 33.0 mmol), Raney Ni (1.00 g) in MeOH (300 mL) was stirred at 20 °C under H₂ ballon for 4 hours. LCMS showed the reaction was finished. After filtration, the filtrate was concentrated to give a residue, which was purified by silica gel column (eluent: EtOAc, 0.5% TEA as additive) to afford 6.08 g (yield: 71%) of compound **H2** as a white solid.

### General procedure for the synthesis of H3

A mixture of compound **H2** (6.08 g, 26.0 mmol), Boc₂O (6.83 g, 32.8 mmol) and TEA (2.55 g, 25.7 mmol) in THF (150 mL) was stirred at 20 °C for 16 hours. LCMS showed the reaction was finished. After removal of the solvent, the mixture was poured into water (100 mL), extracted with EtOAc (50 mL × 3), the combined extracts were washed with brine (50 mL), dried over anhydrous Na₂SO₄ and concentrated to give a residue. The residue was purified by silica gel column (eluent: PE/ EtOAc = 4/1) to afford 6.80 g of crude compound **H3** as a white solid, which was used for the next step without further purification.

### General procedure for the synthesis of H4

A mixture of compound **H3** (crude, 6.80 g) and 2M KOH (20 mL) in MeOH (100 mL) was stirred at 30 °C for 3 hours. LCMS showed the reaction was finished. After removal of the solvent by concentration, the residue was poured into water (100 mL). The aqueous phase was extracted with EtOAc (30 mL × 2) and discarded, the aqueous layer was acidified to pH = 4 with 2M HCl carefully and extracted with EtOAc (50 mL × 3). The combined extracts were washed with brine (40 mL) and dried over anhydrous Na₂SO₄, concentrated to afford 5.50 g of crude compound **H4** as a white solid, which was used for the next step without further purification.

### General procedure for the synthesis of H5

A mixture of compound **H4** (crude, 5.50 g), N,O-dimethylhydroxylamine hydrochloride (4.76 g, 49.0 mmol), EDCI (9.55 g, 49.0 mmol), HOBt (6.62 g, 49.0 mmol) and TEA (10.3 g, 82.0 mmol) in THF (150 mL) was stirred stirred at 20 °C for 12 hours. LCMS showed the reaction was finished. After removal of the solvent under reduced pressure, the mixture was poured into water (100 mL), extracted with EtOAc (70 mL × 3). The combined extracts were washed with brine (50 mL), dried over anhydrous Na₂SO₄ and concentrated to give a residue, which was purified by silica gel column (eluent: PE/EtOAc = 4/1) to afford 5.80 g (3-step yield: 63%) of compound **H5** as a red solid.

### General procedure for the synthesis of H6

A mixture of Mg (99.6 mg, 4.15 mmol) and 4-(trifluoromethoxy)-phenyl bromide (1.00 g, 4.15 mmol) in anhydrous THF (15 mL) was stirred at 50 °C until Mg almost disappeared. Then a solution of compound **H5** (400 mg, 1.06 mmol) in anhydrous THF (10 mL) was added into the above solution at 0 °C dropwise. The resulting mixture was stirred at 20 °C for another 3 hours. LCMS showed the reaction was finished. After the reaction was quenched with saturated NH₄Cl aqueous solution (20 mL), the mixture was extracted with EtOAc (20 mL × 3). The combined extracts were washed with brine (10 mL), dried over anhydrous Na₂SO₄ and concentrated to give a residue, which was purified by silica gel column (eluent: PE/EtOAc = 4/1) to afford 100 mg (yield: 21%) of compound **H6** as a white solid.

### General procedure for the synthesis of H7

To a solution of compound **H6** (100 mg, 0.21 mmol) in DCM (20 mL) was added TFA (4 mL), then the mixture was stirred at 20 °C for 5 hours. After removal of the solvent under vacuum, the mixture was poured into water (20 mL), extracted with EtOAc (10 mL), the extract was discarded. The aqueous layer was basified to pH = 9.0 with 1M NaOH aqueous solution, extracted with EtOAc (20 mL × 3). The combined extracts were washed with brine (10 mL), dried over anhydrous Na₂SO₄ and concentrated to give a residue, which was used directly in next step without further purification.

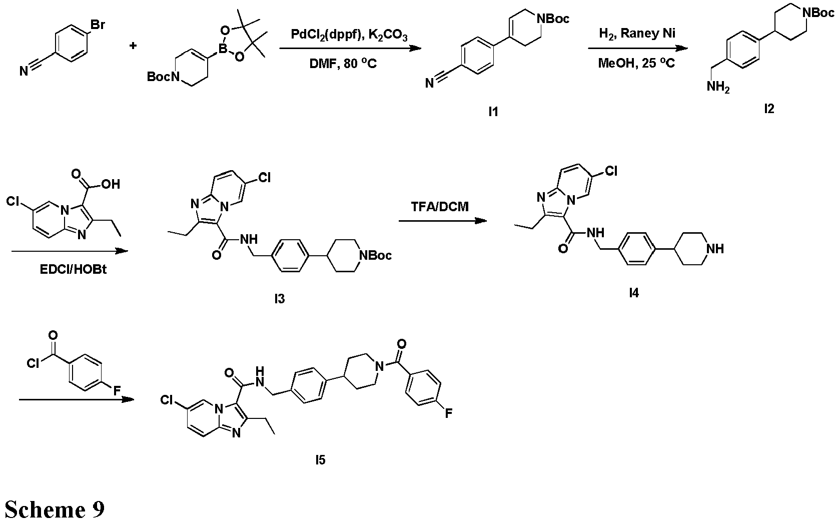

### General procedure for the synthesis of I1

A mixture of 4-bromobenzonitrile (1.40 g, 7.80 mmol), tert-butyl 4-(4,4,5,5-tetramethyl-1,3,2-dioxaborolan-2-yl)-5,6-dihydropyridine-1(2H)-carboxylate (2.00 g, 6.48 mmol), anhydrous potassium carbonate (2.68 g, 19.5mmol) and PdCl₂(dppf) (0.95 g, 1.30 mmol) in anhydrous DMF (30 mL) was stirred at 80 °C under nitrogen atmosphere for 16 hours. The reaction mixture was poured into water (100 mL) and extracted with EtOAc (50 mL × 3). The combined extracts were washed with water (50 mL) and brine (50 mL), dried over anhydrous Na₂SO₄ and concentrated to give a residue. The residue was purified by silica gel column (eluent: PE/EtOAc = 8/1) to afford 1.50 g (yield: 83%) of compound **I1** as a yellow oil.

### General procedure for the synthesis of I2

A mixture of **I1** (1.50 g, 5.00 mmol) and Raney Ni (500 mg) in MeOH (40 mL) was hydrogenated at 25°C under 45 psi of hydrogen pressure for 3 hours. The mixture was filtered and the **filtrate** was concentrated to give crude product. The crude product was purified by silica gel column (elutent: DCM/MeOH = 10/1, 1% TEA as additive) to afford 635 mg (yield: 42%) of **I2** as a yellow powder.

### General procedure for the synthesis of I3

A mixture of compound 6-chloro-2-ethylimidazo[1,2-a]pyridine-3-carboxylic acid (278 mg, 1.24 mmol), **I2** (300 mg, 1.03 mmol), EDCI (242 mg, 3.10 mmol) and HOBT (167 mg, 3.10 mmol) in THF (15 mL) was stirred at 20 °C for 8 hours. The reaction mixture was poured into water (30 mL), extracted with EtOAc (20 mL × 3). The combined extracts were washed with water (20 mL) and brine (20 mL), dried over anhydrous Na₂SO₄ and concentrated to give a residue. The residue was purified by silica gel column (eluent: DCM/MeOH = 10/1, 0.5% TEA as additive) to afford 500 mg (yield: 97%) of **I3** as a yellow power.

### General procedure for the synthesis of I4

To a solution of **I3** (500 mg, 1.00 mmol) in DCM (16 mL) was added TFA (4 mL) and the resulting mixture was stirred at 20 °C for 5 hours. TLC showed the reaction was finished. The reaction mixture was concentrated to afford 300 mg (as TFA salt, yield: 75%) of crude **I4** as yellow oil, which was used for next step without further purification.

### General procedure for the synthesis of I5

To a mixture of **I4** (100 mg, 0.25 mmol) and Et₃N (76 mg, 0.75 mmol) in anhydrous THF (10 mL) was added 4-fluorobenzoyl chloride (48 mg, 0.30 mmol) at 0 °C. The resulting mixture was stirred at 20 °C for 30 minutes. LCMS indicated the reaction was complete. The reaction mixture was poured into H₂O (10 mL) extracted with EtOAc (10 mL×3). The combined extracts were washed with brine (10 mL), dried over anhydrous Na₂SO₄, and concentrated to give a residue. The residue was purified by Prep-HPLC (0.1% TFA as additive), most of MeCN was removed by concentration, then 0.5 mL conc. HCl was added and the water was removed by lyophilization to afford 26 mg (as HCl salt, yield: 20%) of **I5** as a white power.

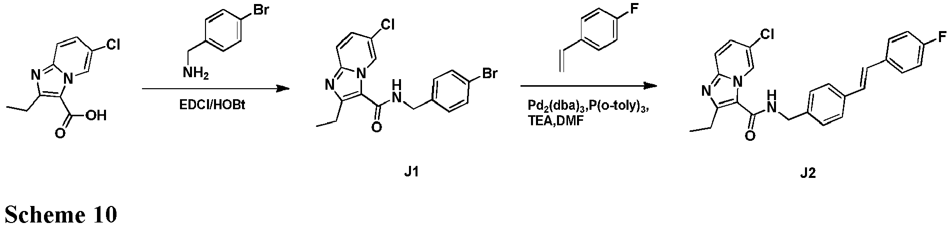

### General procedure for the synthesis of J1

A mixture of compound 6-chloro-2-ethylimidazo[1,2-a]pyridine-3-carboxylic acid (300 mg, 1.34 mmol, 4-Bromo-benzylamine (248 mg, 1.34 mmol), EDCI (286 mg, 1.47 mmol), HOBt (198 mg, 1.47 mmol) and TEA (405 mg, 4.01 mmol) in anhydrous THF (10 mL) was stirred at 20 °C for 16 hours. Then the mixture was diluted with water (50 mL) and extracted with EtOAc (40 mL×3). The combined extracts were washed with brine (100 mL), dried over anhydrous Na₂SO₄ and concentrated to afford 450 mg (yield: 86%) of compound **J1** which was used directly in next step.

### General procedure for the synthesis of J2

A mixture of compound **J1** (100 mg, 0.25 mmol), 1-Fluoro-4-vinyl-benzene (46 mg, 0.38 mmol), Pd₂(dba)₃ (23 mg, 0.025 mmol), P(o-toly)₃ (8 mg, 0.025 mmol) and TEA (129 mg, 1.27 mmol) in DMF (2 mL) was stirred at 100 °C for 16 hours under N₂ atmosphere. The reaction mixture was diluted with water (50 mL) and extracted with EtOAc (40 mL × 3). The combined extracts were washed with water (100 mL) and brine (100 mL), dried over anhydrous Na₂SO₄ and concentrated to give crude product which was purified by prep-HPLC (0.1% NH₃·H₂O as additive). Most of MeCN was removed under reduced pressure, the remaining solvent was removed by lyophilization to afford 14 mg (yield: 13%) of **J2** as a white amorphous.

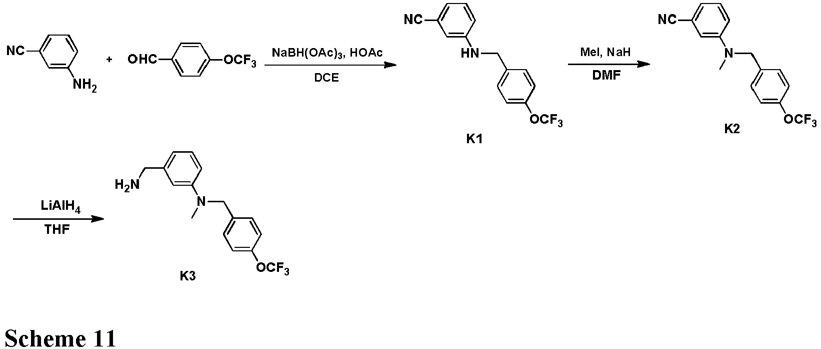

### General procedure for the synthesis of K1

A solution of 3-aminobenzonitrile (4.12 g, 34.9 mmol), 4-(trifluoromethoxy)benzaldehyde (8.38 g, 44.1 mmol) and HOAc (2.43 g, 40.5 mmol) in DCE (100 mL) was stirred at 25 °C for 3 hours, then the NaBH(OAc)₃ (12.7 g, 60.0 mmol) was added into the reaction mixture and the resulting mixture was stirred at 25 °C for 16 hours, TLC showed the reaction was complete. The reaction mixture was basified with aqueous NaHCO₃ till pH = 8, extracted with EtOAc (30 mL × 3), the combined extracts was dried with anhydrous Na₂SO₄ and concentrated under reduced pressure to give 11.4 g (yield: 98%) of compound **K1** as a yellow solid. LCMS purity: 93%, without further purification for next step.

### General procedure for the synthesis of K2

A solution of compound **K1** (2.00 g, 6.85 mmol) in DMF (10 mL) was added dropwise in portions into the suspension of NaH (0.328 g, 8.20 mmol, 60% dispersion in paraffin oil) in anhydrous DMF (5 mL) with syringe during a period of 10 minutes under N₂ while keeping inner temperature between 0 °C to 10 °C. The reaction mixture was allowed to stir at 25 °C for 10 minutes. Then MeI (1.06 g, 7.47 mmol) was added dropwise in portions into the reaction mixture during a period of 10 minutes with syringe while keeping inner temperature between 0 °C and 10 °C and then stirred at 25 °C for 14 hours. the reaction was quenched with saturated aqueous NH₄Cl and extracted with EtOAc (30 mL × 3), The combined extracts was dried with anhydrous Na₂SO₄ and concentrated under reduced pressure, The residue was purified by silica gel column (eluent: PE/EtOAc = 12:1) to afford 350 mg (yield: 17%) of compound **K2** as a yellow oil.

### General procedure for the synthesis of K3

A solution of LiAlH₄ (300 mg, 7.89 mmol) in anhydrous THF (10 mL) was stirred at 0 °C for 5 minutes, then a solution of compound **K2** (350 mg, 1.14 mmol) in anhydrous THF (10 mL) was added dropwise in portions into the mixture during a period of 10 minutes and the resulting mixture was refluxed for 3.5 hours, the reaction was quenched with H₂O (5 mL) and 15% aqueous NaOH (3 mL) and H₂O (10 mL) in turn, the mixture was extracted with EtOAc (15 mL × 3), the combined extracts was dried with anhydrous Na₂SO₄ and concentrated under reduced pressure to give 300 mg (yield: 85%) of compound **K3** as a colorless oil.

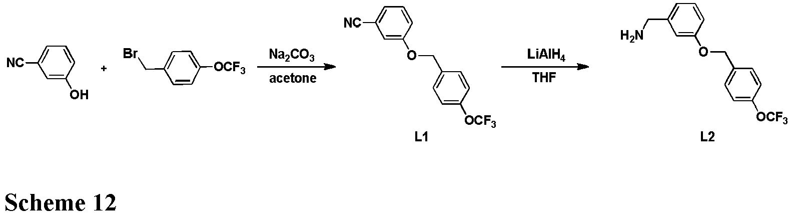

### General procedure for the synthesis of L1

A solution of 3-cyanophenol (1.40 g, 11.8 mmol), 4-(trifluoromethoxy)benzyl bromide (3.29 g , 13.0 mmol) and Na₂CO₃ (3.23 g , 23.4 mmol) in acetone (100 mL) was stirred while maintaining gentle reflux for 15 hours, the TLC showed that the reaction was completed. The reaction mixture was filtered to remove the precipitate. The solution was extracted with EtOAc (20 mL × 3), the combined extracts was dried with anhydrous Na₂SO₄ and concentrated under reduced pressure to dryness, then the crude product was purified by silica gel column (eluent: PE/EtOAc = 12/1) to give 3.01 g (yield: 87%) of compound **L1** as a colourless oil.

### General procedure for the synthesis of L2

A solution of LiAlH₄ (325 mg , 8.55 mmol) in THF (10 mL) was stirred at 0 °C for 5 minutes, then a solution of 3-3 (500 mg, 1.71 mmol) in THF (10 mL) was added dropwise in portions into the mixture during a period of 10 minutes, and the resulting mixture was refluxed for 3.5 hours, the TLC showed that the reaction was completed. The reaction was quenched with H₂O (3 mL), 15% aqueous NaOH (3 mL) and H₂O (9 mL) in turn, extracted with EtOAc (20 mL × 3), the combined extracts was dried with anhydrous Na₂SO₄ and concentrated under reduced pressure to give 480 mg (yield: 96%) of compound **L2** as a colourless oil.

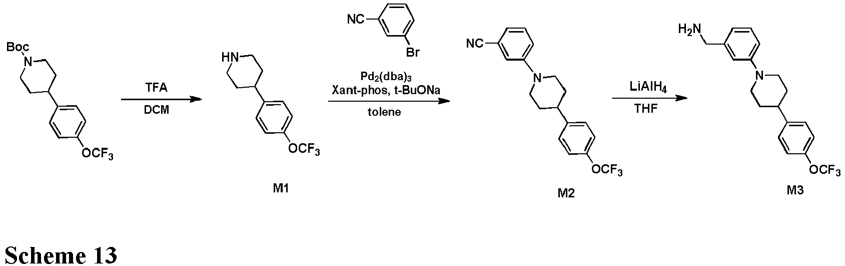

### General procedure for the synthesis of M1

TFA (50 mL) was added dropwise into the solution of compound tert-butyl 4-(4-(trifluoromethoxy)phenyl)piperidine-1-carboxylate (12.5 g, 36.2 mmol) in DCM (100 mL) while keep inner temperature between 0 and 5 °C during a period of 30 minutes, then the reaction mixture was stirred at 25 °C for 17 hours. The reaction mixture was extracted with DCM (20 mL × 3), the combined extracts was dried with anhydrous Na₂SO₄ and concentrated under reduced pressure to give 8.50 g (yield: 96%) of compound **M1** as a yellow power.

### General procedure for the synthesis of M2

A solution of compound **M1** (1.00 g, 4.08 mmol), compound 3-bromophenylisocyanide (890 mg, 4.92 mmol), Pd₂(dba)₃ (750 mg, 0.819 mmol), Xantphos (720 mg, 1.24 mmol) and t-BuONa (1.70 g, 12.3 mmol) in toluene (30 mL) was stirred under N₂ at 110 °C for 18 hours. The reaction mixture was quenched with water (20 mL) at 0 °C, then filtered through celite pad. The mixture was extracted with EtOAc (20 mL × 3). The combined extracts was dried with anhydrous Na₂SO₄ and concentrated under reduced pressure to obtain the crude product. The crude product was purified by silica gel chromatography (eluted: PE: EtOAc = 7 : 1) to give 1.00 g (yield: 69%) of compound **M2** as a yellow solid.

### General procedure for the synthesis of M3

LiAlH₄ (280 mg, 7.36 mmol) was added into the THF (5 mL) and stirred under N₂ at 0 °C for 30 minutes. Then the solution of compound **M2** (500 mg, 1.44 mmol) in THF (5 mL) was added dropwise into the suspension while keep inner temperature between 0 and 5 °C during a period of 30 minutes. Then the reaction mixture was refluxed for 3.5 hours, TLC showed that the reaction was completed. The reaction was quenched with (3 mL) H₂O, 15% aqueous NaOH (3 mL) and H₂O (9 mL) in turn. The mixture was extracted with EtOAc (20 mL × 3), the combined extracts were dried with anhydrous Na₂SO₄ and concentrated under reduced pressure to give 390 mg (yield: 83%) of compound **M3** as a yellow oil.

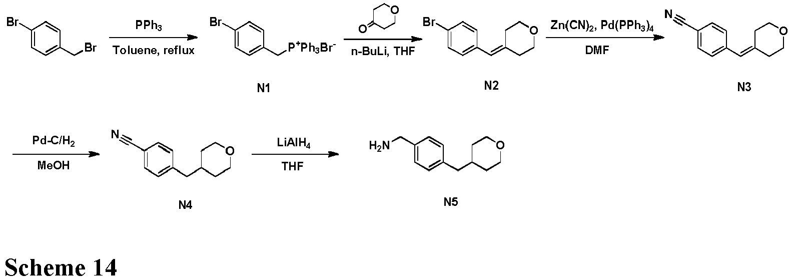

### General procedure for the synthesis of N1

A solution of 4-bromo benzyl bromide (10.0 g, 40.0 mmol) and PPh₃ (10.5 g, 40.0 mmol) in toluene (100 mL) was heated to reflux for 12 hours. After cooled to room temperature, the mixture was filtrated and the filter cake was washed with toluene (200 mL), dried over high vacuum to give compound **N1** (19.5 g, yield: 95%) as a white powder which was used to next step directly.

### General procedure for the synthesis of N2

To a suspension of compound **N1** (14.4 g, 28.1 mmol) in anhydrous THF (120 mL) was dropwise added *n*-BuLi (11.8 mL, 29.5 mmol, 2.5 M in hexane) at -70 °C, the mixture was stirred at -70 °C for 30 minutes. Then the mixture was warmed to 0 °C, and a solution of tetrahydro-4H-pyran-4-one (2.95 g, 29.5 mmol) in anhydrous THF (10 mL) was dropwise added at 0 - 10 °C. Then the reaction mixture was stirred at 20 °C for 12 hours. Saturated NH₄Cl (100 mL) was added at 0 - 10 °C, then diluted with water (200 mL), extracted with EtOAc (100 mL x 2). The combined organic layer was concentrated under reduced pressure to give the residue, which was purified by silica gel column (eluent: PE/EtOAc = 8/1) to give compound **N2** (4.90 g, yield: 69%) as a yellow oil.

### General procedure for the synthesis of N3

A mixture of compound **N2** (4.90 g, 19.3 mmol), Zn(CN)₂ (2.38 g, 20.3 mmol) and Pd(PPh₃)₄ (2.24 g, 1.94 mmol) in DMF (20 mL) was heated to reflux for 1 hour under N₂. Then the reaction mixture was diluted with water (100 mL) and EtOAc (100 mL). After filtration, the organic layer was separated and washed with brine, dried over Na₂SO₄ and concentrated under reduced pressure to give a crude oil, which was purified by silica gel column (eluent: PE/EtOAc = 10/1) to give compound **N3** (5.50 g, yield: 69%) as light yellow oil.

### General procedure for the synthesis of N4

A mixture of compound **N3** (500 mg, 2.51 mmol) and Pd/C (100 mg, 10%) in MeOH (20 mL) was stirred under H₂ (balloon) at 20 °C for 24 hours. The mixture was filtrated and the filtrate was concentrated under reduced pressure to give the crude compound **N4** (420 mg) as a light yellow oil, which was used to next step directly

### General procedure for the synthesis of N5

To a solution of compound **N4** (400 mg, from above) in anhydrous THF (10 mL) was added LiAlH₄ (378 mg, 9.94 mmol) at at 20 °C, the reaction mixture was heated at 70 °C for 12 hours. Water (0.4 mL) and 2M NaOH (0.4 mL) was dropwise added to the reaction mixture at 20 °C to quench the reaction. Then the mixture was filtrated and the cake was washed with THF (20 mL x 2). The combined filtrate was concentrated under reduced pressure to give the crude residue (440 mg) as a light yellow oil. The residue was dissolved in DCM (30 mL) and 1M HCl (30 mL), then extracted with DCM (30 mL x 2). The aqueous layer was adjust to pH = 8 by saturated NaHCO₃, then extracted with DCM (40 mL x 3), the combined organic phase was dried over anhydrous Na₂SO₄ and concentrated to give compound **N5** (310 mg, 2 steps yield: 60%) as gum.

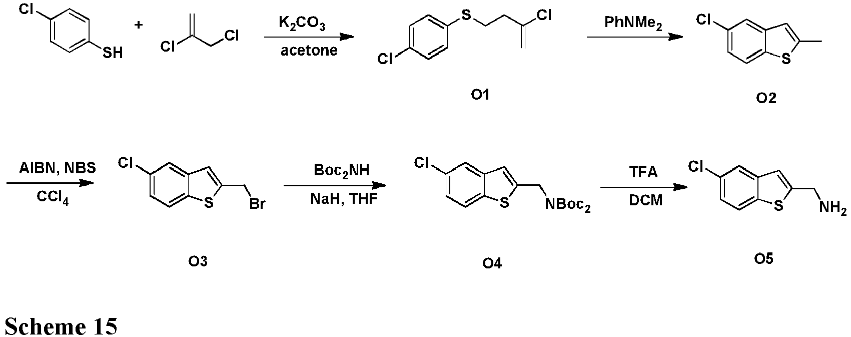

### General procedure for the synthesis of O1

To a mixture of 4-chlorothiophenol (10.0 g, 69.5 mmol) and K₂CO₃ (29.0 g, 210 mmol) in acetone (110 mL) was added 2,3-dichloro-1-propene (9.90 g, 90.0 mmol). The resulting mixture was stirred at 60 °C for 5 hours. After cooled to room temperature, the mixture was filtered and the filtrate was concentrated under reduced pressure to afford 10.0 g (yield: 65%) of compound **O1** as a yellow power.

### General procedure for the synthesis of 02

A solution of compound **O1** (10.0 g, 45.9 mmol) in PhNMe₂ (50 mL) was stirred at 190 °C for 20 hours. After cooled to room temperature, the mixture was extracted with TBME (30 mL×3). The combined extracts was washed by brine (20 mL), dried over anhydrous Na₂SO₄ and concentrated under reduced pressure to give a residue, which was purified by silica gel column (eluent: PE/EtOAc = 20/1) to afford 8.00 g (yield: 96%) of compound **O2** as a white power.

### General procedure for the synthesis of O3

A solution of AIBN (300 mg, 1.83 mmol) and NBS (1.95 g, 11.0 mmol) in CCl₄ (10 mL) was stirred at 80 °C for 10 minutes, then a solution of compound **O2** (2.00 g, 11.0 mmol) in CCl₄ (20 mL) was added into the above solution. The resulting mixture was stirred at 80 °C for 17 hours. After cooling to room temperature, the mixture was filtered and the filtrate was concentrated under reduced pressure to give residue, which was purified by silica gel column (eluent: PE/EtOAc= 15/1) to afford 2.17 g (yield: 76%) of compound **O3** as a yellow power.

### General procedure for the synthesis of 04

To a mixture of NaH (120 mg, 3.00 mmol, 60% dispersion in mineral oil) in anhydrous THF (10 mL) was added a solution of Boc₂NH (454 mg, 1.09 mmol) in anhydrous THF (15 mL) at 0 °C under N₂ dropwise. After stirred at 0 °C for 30 minutes, a solution of compound **O3** (500 mg, 1.93 mmol) in anhydrous THF (10 mL) was added at 0 °C dropwise. The resulting mixture was allowed to stir at 25 °C for 15 hours. The reaction was quenched with water (30 mL) and extracted with EtOAc (30 mL×3). The combined extracts were washed with brine (20 mL), dried over anhydrous Na₂SO₄ and concentrated under reduced pressure to give a yellow power, which was purified by silica gel column (eluent: PE/EtOAc= 12/1) to afford 400 mg (yield: 53%) of compound **O4** as a yellow power.

### General procedure for the synthesis of O5

A solution of compound **O4** (400 mg, 1.05 mmol) and TFA (15 mL) in DCM (30 mL) was stirred at 25 °C for 15 hours. The mixture was concentrated under reduced pressure to give a residue, which was suspended in saturated aqueous Na₂CO₃ solution (20 mL) and extracted with EtOAc (20 mL×3). The combined extracts were washed with brine (20 mL), dried over anhydrous Na₂SO₄ and concentrated under reduced pressure to afford 164 mg (yield: 79%) of compound **O5** as a yellow powder.

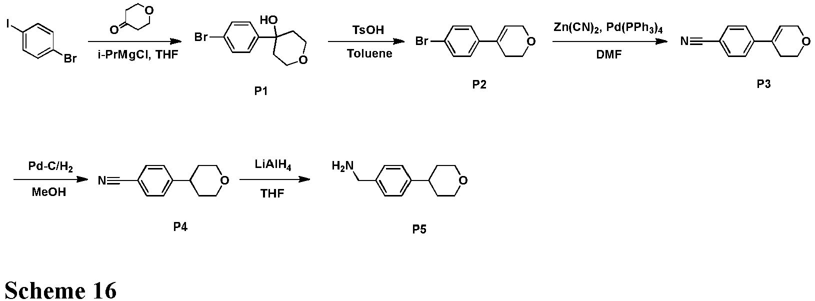

### General procedure for the synthesis of P1

To a solution of compound 1-bromo-4-iodobenzene (5.00 g, 17.7 mmol) in anhydrous THF (20 mL) was dropwise added *i*-PrMgCl (10 mL, 20.0 mmol, 2M in THF) at -40 °C. After being stirred at this temperature for 1 hour, a solution of tetrahydro-4H-pyran-4-one (1.77 g, 17.7 mmol) in anhydrous THF (2 mL) was dropwise added at -40 °C. Then the mixture was allowed to warm to 20 °C and stirred for 2 hours. Saturated NH₄Cl (50 mL) was dropwise added at 10 - 25 °C, to quench the reaction followed by water (50 mL). The mixture was extracted with EtOAc (50 mL x 2). The combined organic layer was concentrated and purified by silica gel column (eluent: PE/EtOAc = 20/1) to give compound **P1**(1.58 g, yield: 35%) as a white powder.

### General procedure for the synthesis of P2

A solution of compound **P1** (1.57 g, 6.11 mmol) and *p*-toluenesulfonic acid monohydrate (5 mg) in toluene (40 mL) was heated to reflux for 8 hours. The reaction solution was concentrated under reduced pressure to give the crude compound **P2** (1.62 g, quant.) which was used to next step directly.

### General procedure for the synthesis of P3

A mixture of compound **P2** (1.62 g, from above), Zn(CN)₂ (835 mg, 7.11 mmol) and Pd(PPh₃)₄ (783 mg, 0.678 mmol) in DMF (15 mL) was heated to reflux for 1 hour under N₂. Then the reaction mixture was diluted with water (50 mL) and EtOAc (30 mL x 3), the EtOAc layer was separated and washed with brine, dried over anhydrous Na₂SO₄ and concentrated under reduced pressure to give a crude residue, which was purified by silica gel column (eluent: PE/EtOAc = 20/1) to give compound **P3** (930 mg, 2 steps yield: 82%) as a light yellow oil.

### General procedure for the synthesis of P4

A mixture of compound **P3** (930 mg, 5.02 mmol) and Pd/C (150 mg, 10%) in MeOH (20 mL) was stirred at 20 °C under H₂ (1 atm) for 48 hours. The reaction mixture was filtrated, and the filtrate was concentrated to give a crude compound **P4** which was used to next step directly.

### General procedure for the synthesis of P5

To a solution of compound **P4** (710 mg, 3.79 mmol) in anhydrous THF (30 mL) was added LiAlH₄ (720 mg, 19.0 mmol) at at 20 °C for 48 hours. Water (0.7 mL) and 2M NaOH (0.7 mL) was dropwise added to the reaction mixture at 20 °C to quench the reaction, then the mixture was filtrated and the filter cake was washed with THF (30 mL x 2). The combined filtrate was concentrated to give a crude residue, which was diluted with DCM (50 mL) and 1M HCl (40 mL), then extracted with DCM (30 mL × 2). The aqueous layer was adjust to pH = 8 by saturated NaHCO₃, then extracted with DCM (50 mL x 3), the combined DCM phase was dried over anhydrous Na₂SO₄ and concentrated to give compound **P5** (210 mg, yield: 29%) as light oil.

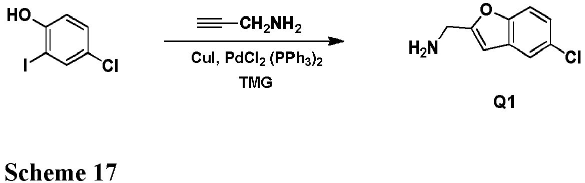

### General procedure for the synthesis of R1

A mixture of 4-chloro-2-iodophenol (1.00 g, 3.94 mmol), propargylamine (1.08 g, 19.6 mmol), CuI (75 mg, 0.40 mmol), PdCl₂(PPh₃)₂ (278 mg, 0.40 mmol) and TMG (4.21 g, 36.6 mmol) in anhydrous DMF (20 mL) was stirred at 50 °C under N₂ for 5 hours. After cooled to room temperature, the mixture was diluted with water (20 mL) and extracted with EtOAc (30 mL × 3). The combined extracts were washed with brine (15 mL), dried over anhydrous Na₂SO₄ and concentrated under reduced pressure to give a residue, which was purified by prep-HPLC (0.1% NH₃.H₂O). Most of CH₃CN was removed by evaporation under reduced pressure, and the remaining solvent was removed by lyophilization to afford 300 mg (yield: 41%) of compound **R1** as a yellow powder.

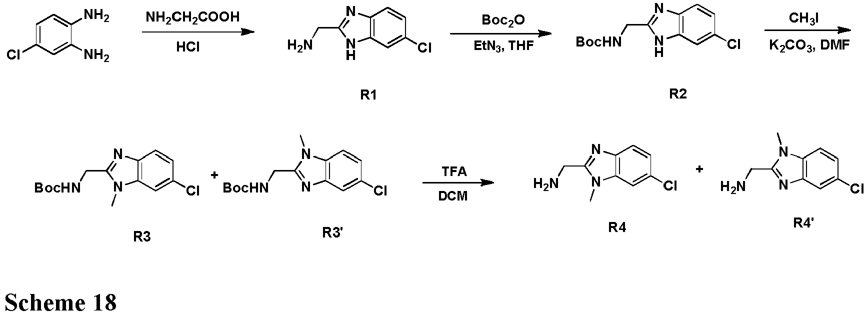

### General procedure for the synthesis of R1

A solution of 4-chloro-1,2-phenylenediamine (3.00 g, 21.1 mmol) and glycine (2.00 g, 26.0 mmol) in 6N HCl (16 mL) was stirred under N₂ at 100 °C for 72 hours. After cooled to room temperature, the mixture was suspended in concentrated NH₃.H₂O solution (18 mL) and extracted with CH₂Cl₂ (30 mL×3). The combined extracts were washed with brine (20 mL), dried over anhydrous Na₂SO₄ and concentrated under reduced pressure to afford 1.21 g (yield: 32%) of compound **R1** as a yellow powder.

### General procedure for the synthesis of R2

To a solution of compound **R1** (3.62 g, 20.0 mmol) and TEA (4.04 g, 40 mmol) in THF (70 mL) was added Boc₂O (4.32 g, 20 mmol) at 0 °C dropwise and the resulting solution was stirred at 25 °C for 15 hours. The mixture was diluted with water (50 mL), extracted with EtOAc (30 mL×3). The combined extracts were dried over anhydrous Na₂SO₄ and concentrated under reduced pressure to give a residue, which was purified by silica gel column (eluent: PE/ EtOAc= 1/5) to afford 900 mg (yield: 16%) of compound **R2** as a yellow powder.

### General procedure for the synthesis of R3 & R3'

To a suspension of compound **R2** (600 mg, 2.14 mmol) and K₂CO₃ (588 mg, 4.26 mmol) in DMF (20 mL) was added CH₃I (420 mg, 2.96 mmol) dropwise at 0 °C. The resulting mixture was stirred at 25 °C for 16 hours. The mixture was diluted with water (50 mL) and extracted with EtOAc (30 mL×3). The combined extracts were washed with brine (20 mL), dried over anhydrous Na₂SO₄ and concentrated under reduced pressure to give residue, which was purified by silica gel column (elutant: PE/EtOAc= 5/1) to afford 350 mg (yield: 56%) of a mixture compound **R3** and compound **R3'** as a yellow power.

### General procedure for the synthesis of R4 & R4'

To a solution of compound **R3** and compound **R3'** (500 mg, 1.69 mmol) in DCM (25 mL) was added TFA (12 mL) dropwise at 0 °C. The resulting solution was stirred at 25 °C for 15 hours, the mixture was concentrated under reduced pressure to give a residue, which was suspended in saturated aqueous Na₂CO₃ (15 mL) and extracted with EtOAc (20 mL×3). The combined extracts were dried over anhydrous Na₂SO₄ and concentrated under reduced pressure to afford 289 mg (yield: 88%) of a mixture of compound **R4** and compound **R4'** as a yellow power, used directly for next step without further purification.

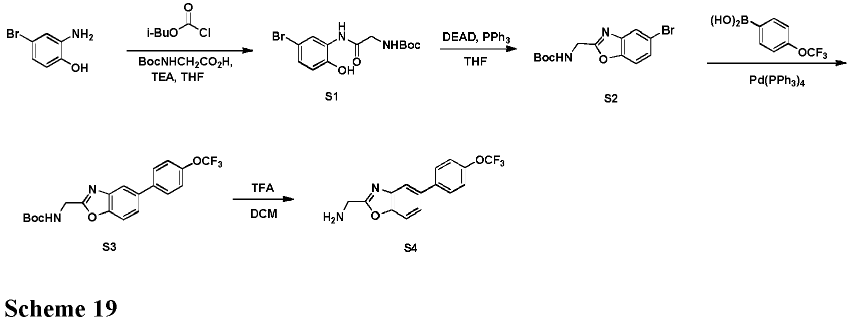

### General procedure for the synthesis of S1

To a solution of Boc-GLY-OH (18.6 g, 106 mmol) and TEA (10.6 g, 105 mmol) in anhydrous THF (200 mL) was added isobutyl chloroformate (12.0 g, 87.9 mmol) at -20 °C dropwise. After the resulting solution was stirred at -20 °C for 1.5 hours, a solution of 2-amino-5-chlorophenol (20.0 g, 106 mmol) in anhydrous THF (50 mL) was added dropwise into above solution and the resulting mixture was stirred at 25 °C for 17 hours. The reaction was quenched with water (50 mL), and the mixture was suspended in saturated aqueous Na₂CO₃ (20 mL), extracted with EtOAc (50 mL×3). The combined extracts were washed with brine (30 mL), dried over anhydrous Na₂SO₄ and concentrated under reduced pressure to give a residue, which was purified by silica gel column (elutent: PE/EtOAc= 4/1) to afford 12.0 g (yield: 32.6%) of compound S1 as a yellow powder.

### General procedure for the synthesis of S2

A solution of compound **S1** (5.00 g , 14.5 mmol) and PPh₃ (8.45 g, 32.2 mmol) in anhydrous THF (70 mL) was stirred at 0 °C for 30 minutes, then DEAD (5.0 mL, 31.7 mmol) was added dropwise. The resulting solution was stirred at 25 °C for 15 hours. The reaction mixture was diluted with water (20 mL) and extracted with EtOAc (30 mL×3). The combined extracts were washed with brine (15 mL), dried over anhydrous Na₂SO₄ and concentrated under reduced pressure to give a residue, which was purified by silica gel column (elutent: PE/EtOAc= 9/1) to afford 2.40 g (yield: 51%) of compound **S2** as a yellow powder

### General procedure for the synthesis of S3

The solution of compound **S2** (1.00 g, 3.06 mmol), 4-(trifluoromethoxy)phenylboronic acid (800 mg, 3.88 mmol), Pd(PPh₃)₄, (600 mg, 0.519 mmol) and aqueous 2M Na₂CO₃ (10 mL) in DME (35 mL)was stirred at 80 °C for 17 hours. The mixture was diluted with water (20 mL), extracted with EtOAc (20 mL × 3), washed with brine (10 mL). The combined extracts was dried over anhydrous Na₂SO₄ and concentrated under reduced pressure to give a residue, which was purified by column (elutent: PE/EtOAc= 9:1) to afford 1.00 g (yield: 80%) of compound **S3** as a white power.

### General procedure for the synthesis of S4

A solution of compound **S3** (400 mg, 0.980 mmol) and TFA (7 mL) in DCM (12 mL) was stirred at 25 °C for 2.5 hours. The mixture was concentrated under reduced pressure to give a residue, the residue was suspended in saturated aqueous Na₂CO₃ (15 mL) and extracted with EtOAc (20 mL × 3). The combined extracts was washed with brine (15 mL), dried over anhydrous Na₂SO₄ and concentrated under reduced pressure to afford 230 mg (yield: 76%) of compound **S4** as a yellow oil.

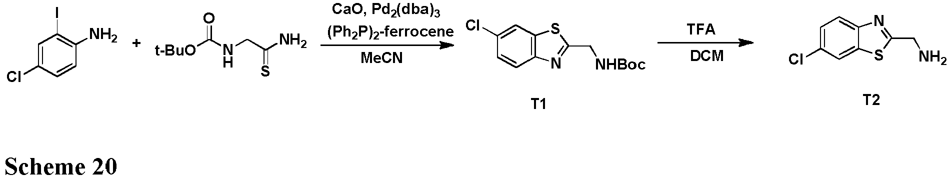

### General procedure for the synthesis of T1

To a mixture of tert-butyl-2-amino-2-thioxoethylcarbamate (450 mg, 2.37 mmol), CaO (165 mg, 2.94 mmol), Pd₂(dba)₃ (365 mg, 0.400 mmol) and dppf (885 mg, 1.60 mmol) in MeCN (7 mL) was added a mixture of 2-chloro-4-iodoaniline (500 mg, 1.97 mmol) in MeCN (3 mL) at 20 °C, the resulting mixture was stirred at 60 °C under N₂ atmosphere for 8 hours. After cooling to room temperature, the mixture was diluted with water (20 mL), extracted with EtOAc (30 mL × 3), washed with brine (10 mL), dried over anhydrous Na₂SO₄ and concentrated under reduced pressure to give a residue, which was purified by column (eluted: PE/EtOAc=6/1) to afford 500 mg (yield: 87%) of compound **T1** as a yellow powder.

### General procedure for the synthesis of T2

A solution of compound **T1** (300 mg, 1.00 mmol) and TFA (5 mL) in DCM (8 mL) was stirred at 25 °C for 3 hours. The mixture was concentrated under reduced pressure to give a residue, which was suspended in saturated aqueous Na₂CO₃ solution (20 mL) and extracted with EtOAc (20 mL × 3). The combined extracts were washed with brine (20 mL), dried over anhydrous Na₂SO₄ and concentrated under reduced pressure to afford 182 mg (yield: 91%) of compound **T2** as a yellow powder.

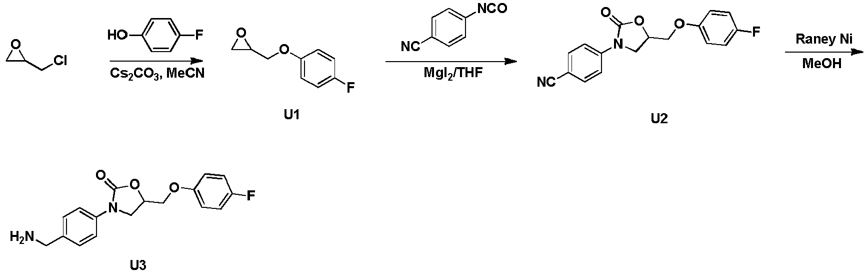

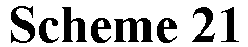

### General procedure for the synthesis of U1

A mixture of epichlorohydrin (4.00 g, 43.2 mmol), 4-fluorophenol (5.34 g, 47.6 mmol) and Cs₂CO₃ (14.1 g, 43.3 mmol) in MeCN (50 mL) was stirred at 80 °C for 17 hours. After cooling to room temperature, the mixture was diluted with water (50 mL), extracted with EtOAc (50 mL x3), washed with brine (30 mL), dried over anhydrous Na₂SO₄ and concentrated under reduced pressure to give a residue, the residue was purified by silica gel column (eluted : EtOAc/PE =1: 10) to afford 2.10 g (yield: 29%) of compound **U1** as a colorless oil.

### General procedure for the synthesis of U2

A mixture of compound **U1** (1.00 g, 5.95 mmol), 4-cyanophenyl isocyanate (1.03 g, 7.15 mmol) and MgI₂ (825 mg, 2.98 mmol) in anhydrous THF (25 mL) was stirred at 60 °C for 17 hours. After cooling to room temperature, the mixture was diluted with water (35 mL), extracted with EtOAc (30 mL x3), washed with brine (30 mL), dried over anhydrous Na₂SO₄ and concentrated under reduced pressure to give a residue, the residue was washed with EtOAc/PE (1/4, 15 mL) to afford 800 mg (yield: 43%) of compound **T2** as a dark powder.

### General procedure for the synthesis of U3

The mixture of compound U2 (400 mg, 1.28 mmol) and Raney Ni (100 mg) in MeOH (20 mL) was stirred under H₂ (50 psi) at 30 °C for 17 hours. The mixture was filtered and the filtration was concentrated under reduced pressure to afford 320 mg (yield: 78%) of compound U3 as a yellow oil.

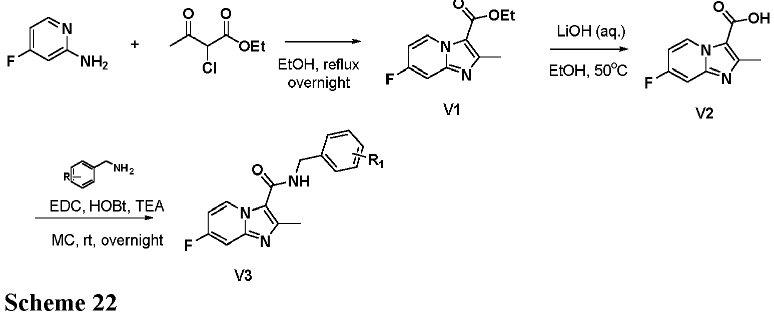

### General procedure for the synthesis of V1

A mixture of 2-amno-4-fluoropyridine (0.41 g, 3.66 mmol) and ethyl-2-chloroacetoacetate (0.66g, 4.02 mmol) in EtOH (7 mL) was stirred at reflux temperature for overnight. The reaction mixture was concentrated under reduced pressure and the resulting residue was purified by column chromatography (n-hexane : ethyl acetate = 3 : 1 ratio) to give **V1.**

### General procedure for the synthesis of V2

To a suspension of **V1** (0.20 g, 0.90 mmol) in MeOH (6 mL) was added aqueous LiOH (0.11 g, 4.5 mmol in 2mL H₂O) and then the resulting mixture was stirred at 50°C. After 2h, the organic solvent was removed under reduced pressure, the resulting aqueous suspension was acidified with 1M HCl (aq.) and then the resulting precipitate was filtered and dried in *vacuo* to give **V2** (0.10 g, 60 %) as a white solid.

### General procedure for the synthesis of V3

To a stirred solution of **V2** (0.030 g, 0.16 mmol), 1-ethyl-3-(3-dimethylaminopropyl)-carbodiimide (0.044 g, 0.23 mmol), 1-hydroxybenzotriazole (0.010 g, 0.078 mmol) and triethylamine (0.043 mL, 0.31 mmol) in anhydrous DMF was added substituted benzylamine (0.17 mmol) and the resulting mixture was stirred for 4h at 80°C. The organic solvent was removed under reduced pressure and the resulting residue was purified by flash column chromatography (n-hexane : ethyl acetate = 2 : 1 ratio) to give **V3.**

### Example 3: In vivo activity in a murine model

The effect of compounds 171 and 175 on the bacterial load of TB-infected mice was compared to that of the reference compound Isoniazid (INH). 8-week old female BalbC mice were infected with 8×10⁶ *M*. *tuberculosis* H37Rv via intranasal inoculation. Mice were sacrificed at day 1 to control the number of CFU in the lungs. In the acute model of infection, mice were treated for 3 days, starting at day 6. Compounds were freshly dissolved in a 20% *d-*α-tocopheryl polyethylene glycol 1000 succinate (ETPGS) solution and administered by oral gavage as single dose per day. Bacterial load was assessed in lungs after homogenizing the organs in 1X PBS. Serial dilutions of organs homogenates were spread on Middlebrook 7H11 plates and CFU were determined after 3 weeks incubation at 37°C under 5% CO2.

In the acute model of infection (after 3 days of treatment; Figure 3), a reduction of CFU compared to untreated mice was observed in the lungs of mice treated with 50 mg/kg of either compound 171 or compound 175 administered orally. Overall both compound 171 and compound 175, demonstrated effect in the acute mouse model of infection.

Investigation of bacillus growth inhibitors within macrophages has long been limited due to cumbersome CFU plating, slow bacillus growth, safety requirements and difficulties in setting-up appropriate infection conditions. As a consequence, this approach was always used as a secondary assay after the initial selection of compounds that are active on *in vitro* extracellular growth. With the advent of automated confocal microscopy, the above mentioned limitations could be readdressed and the methodology employed herein demonstrates the feasibility of large scale compound screening.

Obviously compounds tested to be active against *in vitro M. tuberculosis* growth are the most promising. The best inhibitors isolated from this library have an inhibitory activity. Further structure activity relationship studies will contribute to determine if their activity can be additionally improved. Taken together, the above results show that monitoring *M*. *tuberculosis* growth with automated fluorescence microscopy is highly robust and reliable and that this method enables fast selection of potent anti-TB compounds.

### References

Andries K. et al. A diarylquinoline drug active on the ATP synthase of Mycobacterium tuberculosis (2005). Science 307, 223-227.
Arain, T. M., Resconi, A. E., Singh, D. C., and Stover, C. K. (1996). Reporter gene technology to assess activity of antimycobacterial agents in macrophages. Antimicrob Agents Chemother 40, 1542-1544.
Brodin, P., Christophe, T., No, Z., Kim, J., Genovesio, A., Fenistein, D.P.C., Jeon, H., Ewann, F.A., Kang, S., Lee, S., Seo, M.J., Park, E., Contreras Dominguez, M., Nam, J., Kim, E. Anti-Infective Compounds. WO2010003533A1.
Chaisson, R. E. & Nuermberger, E. L. Confronting multidrug-resistant tuberculosis (2012). N Engl J Med 366, 2223-2224
Diacon A.H. et al. Early bactericidal activity and pharmacokinetics of PA-824 in smear-positive tuberculosis patients (2010). Antimicrob Agents Chemother 54, 3402-3407
Diacon, A. H. et al. Randomized pilot trial of eight weeks of bedaquiline (TMC207) treatment for multidrug-resistant tuberculosis: long-term outcome, tolerability, and effect on emergence of drug resistance (2012). Antimicrob Agents Chemother 56, 3271-3276
Gler, M. T. et al. Delamanid for multidrug-resistant pulmonary tuberculosis (2012). N Engl J Med 366, 2151-2160
Houben, E. N., Nguyen, L., and Pieters, J. (2006). Interaction of pathogenic mycobacteria with the host immune system. Curr Opin Microbiol 9, 76-85.
Lenaerts, A. J., Hoff, D., Aly, S., Ehlers, S., Andries, K., Cantarero, L., Orme, I. M., and Basaraba, R. J. (2007). Location of persisting mycobacteria in a Guinea pig model of tuberculosis revealed by r207910. Antimicrob Agents Chemother 51, 3338-3345.
Makarov, V. et al. Benzothiazinones kill Mycobacterium tuberculosis by blocking arabinan synthesis (2009). Science 324, 801-804.
Neyrolles, O., Hernandez-Pando, R., Pietri-Rouxel, F., Fornes, P., Tailleux, L., Barrios Payan, J. A., Pivert, E., Bordat, Y., Aguilar, D., Prevost, M. C., et al. (2006). Is adipose tissue a place for Mycobacterium tuberculosis persistence? PLoS ONE 1, e43.
Pethe, K. et al. A chemical genetic screen in Mycobacterium tuberculosis identifies carbon-source-dependent growth inhibitors devoid of in vivo efficacy (2010). Nat Commun 1:57. doi: 10.1038/ncomms1060.
Rohde, K. H., Abramovitch, R. B., and Russell, D. G. (2007). Mycobacterium tuberculosis invasion of macrophages: linking bacterial gene expression to environmental cues. Cell Host Microbe 2, 352-364.
Schnappinger, D., Ehrt, S., Voskuil, M. I., Liu, Y., Mangan, J. A., Monahan, I. M., Dolganov, G., Efron, B., Butcher, P. D., Nathan, C., and Schoolnik, G. K. (2003). Transcriptional Adaptation of Mycobacterium tuberculosis within Macrophages: Insights into the Phagosomal Environment. J Exp Med 198, 693-704.
Stanley, S. A. et al. Identification of novel inhibitors of M. tuberculosis growth using whole cell based high-throughput screening (2012). ACS Chem Biol 7, 1377-1384.
Stover, C.K., Arrener, P., VanDevanter, D.R., Sherman, D.R., Arain, T.M., Langhorne, M.H., Anderson, S.W., Towell, J.A., Yuan, Y., McMurray, D.N., Kreiswirth, B.N., Barry, C.E., Baker, W.R. (2000). A small-molecule nitroimidazopyran drug candidate for the treatment of tuberculosis. Naure 405, 962-6.
Zhao, Y. et al. National survey of drug-resistant tuberculosis in China (2012). N Engl J Med 366, 2161-2170.
In the following tables 1 and 2, compounds 1-231, 238, 281-285, 289-300, 309-316, 325-328, 333-337, 340-350 are shown which form part of the present invention, whereas compounds 232-237, 239-280, 286-288, 301-308, 317-324, 329-332, 338-339 do not form part of the present invention.

**Table 1**

| # **cpd** | **QUM (MIC₈₀, uM)** | **# cpd** | **QUM (MIC₈₀, uM)** | **# cpd** | **QUM (MIC₈₀**, **uM)** |
|---|---|---|---|---|---|
| 1 | +++ | 27 | ++ | 53 | +++ |
| 2 | +++ | 28 | +++ | 54 | +++ |
| 3 | +++ | 29 | +++ | 55 | +++ |
| 4 | +++ | 30 | +++ | 56 | +++ |
| 5 | +++ | 31 | +++ | 57 | +++ |
| 6 | +++ | 32 | +++ | 58 | ++ |
| 7 | +++ | 33 | +++ | 59 | +++ |
| 8 | +++ | 34 | ++ | 60 | +++ |
| 9 | +++ | 35 | +++ | 61 | +++ |
| 10 | +++ | 36 | +++ | 62 | +++ |
| 11 | +++ | 37 | +++ | 63 | +++ |
| 12 | +++ | 38 | +++ | 64 | +++ |
| 13 | +++ | 39 | +++ | 65 | +++ |
| 14 | +++ | 40 | +++ | 66 | +++ |
| 15 | +++ | 41 | +++ | 67 | +++ |
| 16 | +++ | 42 | +++ | 68 | +++ |
| 17 | +++ | 43 | +++ | 69 | +++ |
| 18 | +++ | 44 | +++ | 70 | +++ |
| 19 | +++ | 45 | +++ | 71 | +++ |
| 20 | +++ | 46 | +++ | 72 | +++ |
| 21 | +++ | 47 | +++ | 73 | +++ |
| 22 | ++ | 48 | +++ | 74 | +++ |
| 23 | +++ | 49 | +++ | 75 | +++ |
| 24 | +++ | 50 | +++ | 76 | +++ |
| 25 | ++ | 51 | +++ | 77 | +++ |
| 26 | +++ | 52 | +++ | 78 | ++ |
| 79 | +++ | 105 | ++ | 131 | +++ |
| 80 | +++ | 106 | +++ | 132 | +++ |
| 81 | +++ | 107 | +++ | 133 | +++ |
| 82 | +++ | 108 | +++ | 134 | +++ |
| 83 | +++ | 109 | +++ | 135 | +++ |
| 84 | ++ | 110 | +++ | 136 | +++ |
| 85 | +++ | 111 | +++ | 137 | +++ |
| 86 | +++ | 112 | ++ | 138 | +++ |
| 87 | +++ | 113 | +++ | 139 | +++ |
| 88 | ++ | 114 | +++ | 140 | +++ |
| 89 | ++ | 115 | +++ | 141 | +++ |
| 90 | +++ | 116 | +++ | 142 | +++ |
| 91 | +++ | 117 | ++ | 143 | ++ |
| 92 | +++ | 118 | +++ | 144 | +++ |
| 93 | +++ | 119 | +++ | 145 | +++ |
| 94 | +++ | 120 | +++ | 146 | +++ |
| 95 | +++ | 121 | +++ | 147 | +++ |
| 96 | +++ | 122 | +++ | 148 | +++ |
| 97 | +++ | 123 | +++ | 149 | +++ |
| 98 | +++ | 124 | +++ | 150 | +++ |
| 99 | ++ | 125 | ++ | 151 | ++ |
| 100 | +++ | 126 | +++ | 152 | ++ |
| 101 | +++ | 127 | +++ | 153 | +++ |
| 102 | +++ | 128 | +++ | 154 | ++ |
| 103 | ++ | 129 | ++ | 155 | +++ |
| 104 | ++ | 130 | +++ | 156 | +++ |
| 157 | +++ | 183 | +++ | 209 | ++ |
| 158 | +++ | 184 | +++ | 210 | +++ |
| 159 | +++ | 185 | ++ | 211 | +++ |
| 160 | +++ | 186 | +++ | 212 | ++ |
| 161 | +++ | 187 | +++ | 213 | +++ |
| 162 | +++ | 188 | +++ | 214 | +++ |
| 163 | +++ | 189 | ++ | 215 | ++ |
| 164 | +++ | 190 | +++ | 216 | +++ |
| 165 | +++ | 191 | +++ | 217 | ++ |
| 166 | +++ | 192 | +++ | 218 | +++ |
| 167 | +++ | 193 | +++ | 219 | +++ |
| 168 | ++ | 194 | +++ | 220 | +++ |
| 169 | +++ | 195 | +++ | 221 | +++ |
| 170 | +++ | 196 | +++ | 222 | +++ |
| 171 | +++ | 197 | +++ | 223 | +++ |
| 172 | +++ | 198 | ++ | 224 | +++ |
| 173 | +++ | 199 | +++ | 225 | +++ |
| 174 | +++ | 200 | ++ | 226 | +++ |
| 175 | +++ | 201 | +++ | 227 | +++ |
| 176 | +++ | 202 | ++ | 228 | +++ |
| 177 | +++ | 203 | +++ | 229 | +++ |
| 178 | +++ | 204 | +++ | 230 | +++ |
| 179 | +++ | 205 | +++ | 231 | +++ |
| 180 | +++ | 206 | +++ | 232 | + |
| 181 | +++ | 207 | +++ | 233 | +++ |
| 182 | +++ | 208 | +++ | 234 | + |
| 235 | +++ | 261 | +++ | 287 | +++ |
| 236 | +++ | 262 | + | 288 | +++ |
| 237 | +++ | 263 | + | 289 | +++ |
| 238 | +++ | 264 | ++ | 290 | +++ |
| 239 | +++ | 265 | ++ | 291 | +++ |
| 240 | +++ | 266 | ++ | 292 | +++ |
| 241 | +++ | 267 | +++ | 293 | +++ |
| 242 | +++ | 268 | +++ | 294 | +++ |
| 243 | +++ | 269 | +++ | 295 | +++ |
| 244 | +++ | 270 | +++ | 296 | +++ |
| 245 | +++ | 271 | ++ | 297 | +++ |
| 246 | +++ | 272 | ++ | 298 | +++ |
| 247 | ++ | 273 | +++ | 299 | +++ |
| 248 | ++ | 274 | ++ | 300 | +++ |
| 249 | + | 275 | + | 301 | +++ |
| 250 | + | 276 | + | 302 | +++ |
| 251 | ++ | 277 | + | 303 | +++ |
| 252 | +++ | 278 | ++ | 304 | ++ |
| 253 | +++ | 279 | +++ | 305 | ++ |
| 254 | +++ | 280 | +++ | 306 | ++ |
| 255 | + | 281 | ++ | 307 | +++ |
| 256 | +++ | 282 | ++ | 308 | +++ |
| 257 | +++ | 283 | ++ | 309 | +++ |
| 258 | +++ | 284 | +++ | 310 | +++ |
| 259 | +++ | 285 | +++ | 311 | +++ |
| 260 | + | 286 | +++ | 312 | +++ |
| 313 | +++ | 328 | +++ | 343 | +++ |
| 314 | +H- | 329 | ++ | 344 | +++ |
| 315 | +++ | 330 | ++ | 345 | +++ |
| 316 | +++ | 331 | ++ | 346 | +++ |
| 317 | ++ | 332 | ++ | 347 | +++ |
| 318 | ++ | 333 | +++ | 348 | +++ |
| 319 | +++ | 334 | +++ | 349 | +++ |
| 320 | +++ | 335 | +++ | 350 | +++ |
| 321 | +++ | 336 | +++ | | |
| 322 | +++ | 337 | +++ | | |
| 323 | +++ | 338 | +++ | | |
| 324 | +++ | 339 | ++ | | |
| 325 | +++ | 340 | +++ | | |
| 326 | +++ | 341 | +++ | | |
| 327 | +++ | 342 | +++ | | |

| | | | | | |
|---|---|---|---|---|---|
| Activity range: +++ indicates < 1 uM, ++ indicates between 1-20 uM, + indicates > 20 uM | | | | | |

| cpd | Structure | Characterization Data |
|---|---|---|
| 1 | 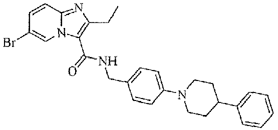 | white solid: ¹H-NMR (CDCl₃): δ 9.66 (1H, d, *J* = 1.2 Hz), 7.21-7.60 (9H, m), 7.02 (2H, d, *J* = 8.4 Hz), 6.05 (1H, brs), 4.64 (2H, d, *J* = 5.2 Hz), 3.79-3.93 (2H, m), 2.99 (2H, q, *J* = 7.6 Hz), 2.80-2.94 (2H, m), 2.61-2.75 (1H, m), 1.87-2.05 (4H, m), 1.42 (3H, t, *J* = 7.6 Hz); LCMS: 100%, MS (ESI): m/z 519.0[M+ H]+. |
| 2 | 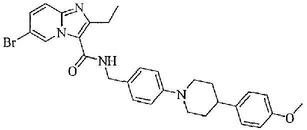 | yellow solid: ¹H-NMR (CDCl₃): δ 9.65 (1H, s), 7.51 (1H, d, *J* = 9.2 Hz), 7.43 (1H, d, *J* = 9.6 Hz), 7.27-7.33 (2H, m), 7.20 (2H, d, *J* = 8.4 Hz), 7.01 (2H, d, *J* = 8.4 Hz), 6.89 (2H. d, *J* = 8.8 Hz), 6.05 (1H, brs), 4.64 (2H, d, *J* = 5.2 Hz), 3.76-3.90 (5H, m), 2.98 (2H, q, *J* = 7.6 Hz), 2.80-2.90 (2H, m), 2.60-2.73 (1H, m), 1.80-2.03 (4H, m), 1.42 (3H, t, *J* = 7.6 Hz); LCMS: 100%, MS (ESI): m/z 549.1[M+ H]+. |
| 3 | 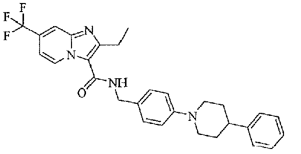 | white solid: ¹H-NMR (CDCl₃): δ 9.55 (1H, d, *J* = 7.6 Hz), 7.93 (1H, s), 7.22-7.37 (10H, m), 7.11-7.13 (1H, m), 7.02 (2H, d, *J* = 8.4 Hz), 6.09 (1H, brs), 4.65 (2H, d, *J* = 5.2 Hz), 3.85 (2H, d, *J* = 12.4 Hz), 3.02 (2H, q, *J* = 7.6 Hz), 2.83-2.91 (2H, m), 2.66-2.70 (1H, m), 1.90-2.02 (4H, m), 1.44 (3H, t, *J* = 7.6 Hz); LCMS: 100%, MS (ESI): m/z 507.1[M+ H]+. |
| 4 | 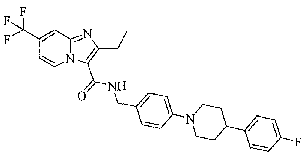 | white solid: ¹H-NMR (CDCl₃): δ 9.55 (1H, d, *J* = 7.2 Hz), 8.04 (1H, s), 7.28-7.30 (3H, m), 7.20-7.24 (2H, m), 7.12 (1H, d, *J* = 1.2 Hz), 7.00-7.10 (4H, m), 6.10 (1H, brs), 4.65 (2H, d, *J* = 5.6 Hz), 3.84 (2H, d, *J* = 12.4 Hz), 2.90-3.05 (2H, m), 2.82-2.89 (2H, m), 2.63-2.71 (1H, m), 1.82-1.99 (4H, m), 1.44 (3H, t, *J* = 7.6 Hz); LCMS: 100%, MS (ESI): m/z 525.0[M+ H]+. |
| 5 | 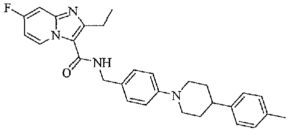 | yellow solid: ¹H-NMR (CDCl₃): δ 9.44(1H, t, *J*= 7.2 Hz, 6.0 Hz), 7.28(2H, d, *J*= 8.8 Hz), 7.21-7.26(1H, m), 7.14-7.16.01(4H, m), 6.98(2H, d, *J*= 8.8 Hz), 6.77-6.81(1H, m), 5.99(1H, brs), 4.61(2H, d, *J*= 5.2 Hz), 3.80-3.87(2H, m), 2.92-2.98(2H, m), 2.80-2.86(2H, m), 2.59-2.67(2H, m), 2.92-2.98(1H, m), 2.34(3H, s), 1.83-1.97(2H, m), 1.39(3H, t, *J*= 7.2 Hz); LCMS: 98.2%, MS (ESI): m/z 493.0[M+ Na]+. |
| 6 | 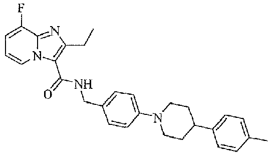 | white solid: ¹H-NMR (CDCl₃): δ 9.20 (1H, d, *J* = 6.8 Hz), 7.30 (2H, d, *J* = 8.8 Hz), 7.20-7.10 (4H, m), 7.09-7.01 (3H, m), 6.86-6.81(1H, m), 6.08 (1H, brs), 4.63 (2H, d, *J* = 5.2 Hz), 3.83-3.80 (2H, m), 3.00 (2H, q, *J* = 7.6 Hz), 2.90-2.83 (2H, m), 2.68-2.60 (1H, m), 2.33 (1H, s), 1.97-1.86 (4H, m), 1.40 (3H, t, *J* = 7.6 Hz); LCMS: 98.4%, MS (ESI): m/z 471.1[M+ H]+. |
| 7 | 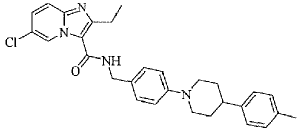 | white solid: ¹H-NMR(CDCl₃): δ 9.54 (1H, d, *J* = 1.2 Hz), 7.54 (1H, d, *J* = 9.6 Hz), 7.26-7.30 (3H, m), 7.12-7.17 (4H, m), 6.99 (2H, d, *J* = 8.8 Hz), 6.02 (1H, brs), 4.62 (2H, d, *J* = 5.2 Hz), 3.82 (2H, d, *J* = 12 Hz), 2.96 (2H, q, *J* = 7.6 Hz), 2.80-2.87 (2H, m), 2.60-2.66 (1H, m), 2.33 (3H, s), 1.83-1.97 (4H, m), 1.40 (3H, t, *J* = 7.6 Hz); LCMS: 98.0%, MS (ESI): m/z 487.1[M+ H]+. |
| 8 | 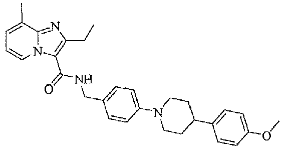 | white solid: ¹H-NMR (CDCl₃): δ 9.24 (1H, d, *J* = 7 Hz), 7.29 (2H, d, *J* = 8.5 Hz), 7.17 (2H, d, *J* = 8.8 Hz), 7.11 (1H, d, *J* = 6.8 Hz), 6.98 (2H, d, *J* = 8.5 Hz), 6.87 (2H, d, *J* = 8.5 Hz), 6.82 (1H, t, *J* = 6.9 Hz), 6.01 (1H, brs), 4.62 (2H, d, *J* = 5.5 Hz), 3.81 (2H, d, *J* = 12.2 Hz), 3.60 (3H, s), 2.99 (2H, q, *J* = 7.5 Hz), 2.82 (2H, td, *J* = 12.2, 2.5 Hz), 2.57-2.66 (1H, m), 2.61 (3H, s), 1.80-2.00 (4H, m), 1.37 (3H, t, *J* = 7.7 Hz); LCMS: 100%, MS (ESI): m/z 483.1 [M+ H]+. |
| 9 | 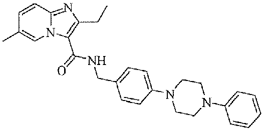 | white solid: ¹H-NMR (CDCl₃): δ 9.16 - 9.29 (1 H, m), 7.46 - 7.56 (1 H, m), 7.28 - 7.37 (4 H, m), 7.12 - 7.21 (1 H, m), 6.96 - 7.05 (4 H, m), 6.84 - 6.95 (1 H, m), 5.93 - 6.09 (1 H, m), 4.56 - 4.69 (2 H, m), 3.35 (8 H, s), 2.90 - 3.02 (2 H, m), 2.37 (3 H, s), 1.32 - 1.45 (3 H, m); LCMS:100%, MS (ESI): m/z 453.2[M+ H]+. |
| 10 | 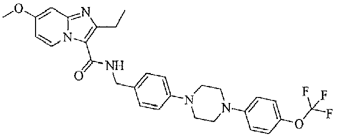 | pink solid : ¹H-NMR (CDCl₃): δ 9.24 (1H, d, *J* = 7.6 Hz), 7.31 (2H, d, *J* = 8.4 Hz), 7.13 (2H, d, *J* = 8.8 Hz), 6.91-7.01 (4H, m), 6.88 (1H, d, *J=* 2.0 Hz), 6.61 (1H, dd, *J=* 7.6 Hz, 2.4 Hz), 5.95 (1H, brs), 4.61 (2H, d, *J=* 5.2 Hz), 3.86 (3H, s), 3.33 (8H, s), 2.91 (2H, q, *J=* 7.6 Hz), 1.38 (3H, t, *J* = 7.6 Hz); LCMS: 98.3%, MS (ESI): m/z 554.1 [M+ H]+. |
| 11 | 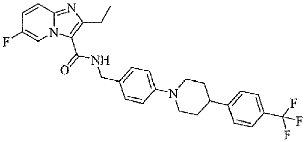 | white solid: ¹H-NMR (CDCl₃): δ 9.47 (1H, dd, *J* = 5.2, 2.4 Hz), 7.50-7.65 (3H, m), 7.37 (2H, d, *J* = 8.4 Hz), 7.20-7.35 (3H, m overlap with CDCl₃ signal), 7.00 (2H, d, *J* = 8.4 Hz), 6.04 (1H, bra), 4.63 (2H, d, *J* = 5.6 Hz), 3.84 (2H, d, *J* = 12.4 Hz), 2.98 (2H, q, *J* = 7.6 Hz), 2.80-2.90 (2H, m), 2.65-2.80 (1H, m), 1.85-2.05 (4H, m), 1.41 (3H, t, *J* = 7.6 Hz); LCMS: 98.3%, MS (ESI): m/z 525.1[M+ H]+. |
| 12 | 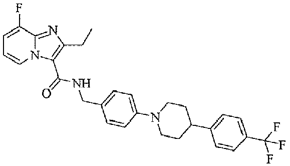 | white solid: ¹H-NMR (CDCl₃): δ 9.21 (1H, d, *J* = 6.8 Hz), 7.59 (2H, d, *J* = 8.0 Hz), 7.37 (2H, d, *J* = 8.0 Hz), 7.30 (2H, d, *J* = 8.4 Hz), 7.06-6.99 (3H, m), 6.85-6.83 (1H, m), 6.07 (1H, brs), 4.63 (2H, d, *J* = 5.6 Hz), 3.86-3.82 (2H, m), 3.01 (2H, q, *J* = 7.6 Hz), 2.90-2.83 (2H, m), 2.80-2.67 (1H, m), 2.02-1.85 (4H, m), 1.41 (3H, t, *J* = 7.6 Hz); LCMS: 98.4%, MS (ESI): m/z 525.1[M+ H]+. |
| 13 | 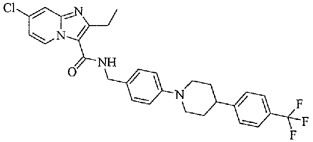 | white solid: ¹H-NMR (CDCl₃): δ 9.37 (1H, d, *J* = 7.6 Hz), 7.58 (2H, d, *J* = 8.0 Hz), 7.37 (2H, d, *J* = 8.0 Hz), 7.25-7.35 (3H, m), 7.00 (2H, d, *J* = 8.4 Hz), 6.91 (1H, dd, *J*₁ = 2.0 Hz, *J*₂ = 7.2 Hz), 6.03 (1H, brs), 4.62 (2H, d, *J* = 5.2 Hz), 3.83 (2H, d, *J* = 12 Hz), 2.96 (2H, q, *J* = 7.6 Hz), 2.70-2.90 (2H, m), 2.65-2.79 (1H, m), 1.85-2.02 (4H, m), 1.39 (3H, t, *J* = 7.6 Hz); LCMS: 98.4%, MS (ESI): m/z 541.0 [M+ H]+. |
| 14 | 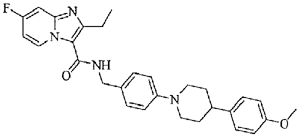 | white solid: ¹H-NMR (CDCl₃): δ 9.44(1H, t, *J*= 6.4 Hz), 7.17-7.30(6H, m), 6.99(2H, d, *J* = 8.8 Hz), 6.87(2H, d, J= 8.8 Hz), 6.87(1H, m), 6.00(1H, brs), 4.61(2H, d, *J*= 5.2 Hz), 3.81-3.87(5H, m), 2.93-2.98(2H, m), 2.80-2.86(2H, m), 2.54-2.57(1H, m), 1.18-1.97(4H, m), 1.40(3H, t, *J*= 7.2 Hz); LCMS: 98.2%, MS (ESI): m/z 487.1[M+ H]+. |
| 15 | 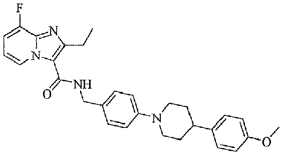 | white solid: ¹H-NMR (CDCl₃): δ 9.21 (1H, d, *J* = 6.8 Hz), 7.29 (2H, d, *J* = 8.4 Hz), 7.18 (2H, d, *J* = 8.4 Hz), 7.04-6.98 (3H, m), 6.89-6.84 (3H, m), 6.07 (1H, brs), 4.63 (2H, d, *J* = 5.2 Hz), 3.84-3.80 (5H, m), 3.00 (2H, q, *J* = 7.6 Hz), 2.86-2.80 (2H, m), 2.64-2.56 (1H, m), 1.97-1.84 (4H, m), 1.41 (3H, t, *J* = 7.6 Hz); LCMS: 98.7%, MS (ESI): m/z 487.1[M+H]+. |
| 16 | 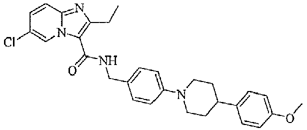 | white solid: ¹H-NMR(CDCl₃): δ 9.54 (1H, d, *J* = 1.2 Hz), 7.54 (1H, d, *J* = 9.2 Hz), 7.27-7.31 (4H, m), 7.18 (2H, d, *J* = 8.8 Hz), 6.99 (2H, d, *J* = 8.8 Hz), 6.87 (2H, *J* = 8.8 Hz), 6.03 (1H, brs), 4.62 (2H, d, *J* = 5.6 Hz), 3.79-3.85 (5H, m), 2.97 (2H, q, *J* = 7.6 Hz), 2.80-2.88 (2H, m), 2.58-2.68 (1H, m), 1.84-1.97 (4H, m), 1.40 (3H, t, *J* = 7.6 Hz); LCMS: 100%, MS (ESI): m/z 525.1[M+ Na]+. |
| 17 | 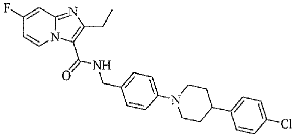 | yellow solid: ¹H-NMR (CDCl₃): δ 9.44(1H, t, *J*= 6.4 Hz, *J*₂= 6.8 Hz), 7.18-7.30(7H, m), 6.98(2H, d, *J*= 8 Hz), 6.78-6.80(1H, m), 6.01(1H, brs), 4.62(2H, d, *J*= 5.2 Hz), 3.81-3.84(2H, m), 2.93-2.98(2H, m), 2.80-2.86(2H, m), 2.52-2.55(1H, m), 1.84-1.96(4H, m), 1.40(3H, t, *J*=7.6 Hz); LCMS: 99.8%, MS (ESI): m/z 491.1[M+ H]+. |
| 18 | 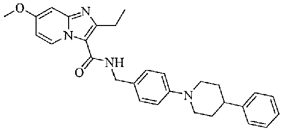 | white solid: ¹H-NMR (CDCl₃): δ 9.25 (1H, d, *J* = 7.6 Hz), 7.15-7.36 (9H, m), 6.98 (2H, d, *J* = 8.8 Hz), 6.89 (1H, d, *J* = 2.4 Hz), 6.61 (1H, dd, *J* = 7.6 Hz, 2.4 Hz), 5.93 (1H, brs), 4.60 (2H, d, *J* = 5.2 Hz), 3.87 (3H, s), 3.82 (2H, d, *J* = 12.4 Hz), 2.75-2.95 (4H, m), 2.60-2.72 (1H, m), 1.80-2.02 (4H, m), 1.39 (3H, t, *J* = 7.6 Hz); LCMS: 100.0%, MS (ESI): m/z 469.1[M+ H]+. |
| 19 | 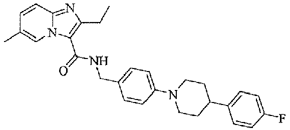 | pink solid: ¹H-NMR (CDCl₃): δ 9.19 - 9.30 (1 H, m), 7.53 (1 H, d, *J*=9.03 Hz), 7.28 - 7.33 (2 H, t), 7.18 - 7.24 (3 H, m), 6.95 - 7.07 (4 H, m), 5.96 - 6.10 (1 H, m), 4.65 (2 H, d, J=5.52 Hz), 3.84 (2 H, d, *J*=12.30 Hz), 2.98 (2 H, q, *J*=7.57 Hz), 2.77 - 2.93 (2 H, m), 2.67 (1 H, s), 2.39 (3 H, s), 1.94 - 2.01 (2 H, m), 1.82 - 1.94 (2 H, m), 1.42 (3 H, t, *J*=7.59 Hz); LCMS: 98.0%, MS (ESI): m/z 471.1[M+H]+. |
| 20 | 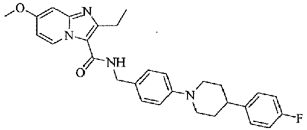 | yellow solid: ¹H-NMR (CDCl₃): δ 9.24 (1H, d, *J* = 7.6 Hz), 7.15-7.35 (4H, m), 6.93-7.05 (4H, m), 6.89 (1H, d, *J* = 2.4 Hz), 6.61 (1H, dd, *J* = 7.6 Hz, 2.4 Hz),5.94 (1H, brs), 4.60 (2H, d, *J* = 5.2 Hz), 3.87 (3H, s), 3.81 (2H, d, *J* = 12.4 Hz), 2.92 (2H, q, *J* = 7.6 Hz), 2.82 (2H, td, *J* = 12.4 Hz, 2.4 Hz), 2.55-2.70 (1H, m), 1.75-2.02 (4H, m), 1.39 (3H, t, *J* = 7.6 Hz); LCMS: 99.0%, MS (ESI): m/z 487.1[M+ H]+. |
| 21 | 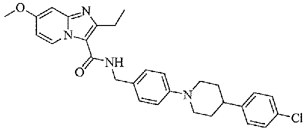 | off-white solid: ¹H-NMR (CDCl₃): δ 9.24 (1H, d, *J* = 7.6 Hz), 7.22-7.35 (5H, m), 7.18 (2H, 2H, d, *J* = 8.4 Hz), 6.98 (2H, d, *J* = 8.4 Hz), 6.88 (1H, d, *J* = 2.4 Hz), 6.61 (1H, dd, *J* = 7.6 Hz, 2.4 Hz), 5.94 (1H, m), 4.60 (2H, d, *J* = 5.2 Hz), 3.87 (3H, s), 3.81 (2H, d, *J* = 12.4 Hz), 2.92 (2H, q, *J* = 7.6 Hz), 2.82 (2H, td, *J* = 12.0 Hz, 2.4 Hz), 2.58-2.68 (1H, m), 1.80-2.00 (4H, m), 1.38 (3H, t, *J* = 7.6 Hz); LCMS: 100.0%, MS (ESI): m/z 503.1[M+ H]+. |
| 22 | 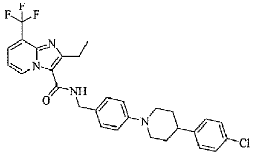 | pink solid : ¹H-NMR (CDCl₃): δ 9.60 (1H, d, *J* = 7.2 Hz), 7.68 (1H, d, *J* = 7.2 Hz), 7.20-7.40 (4H, m), 7.20 (2H, d, *J* = 8.4 Hz), 6.95-7.05 (3H, m), 6.12 (1H, brs), 4.65 (2H, d, *J* = 5.2 Hz), 3.80-3.90 (2H, m), 3.05 (2H, q, *J* = 7.6 Hz), 2.80-2.90 (2H, m), 2.60-2.70 (1H, m), 1.80-2.00 (4H, m), 1.40 (3H, t, *J* = 7.6 Hz); LCMS: 100%, MS (ESI): m/z 541.0[M + H]+. |
| 23 | 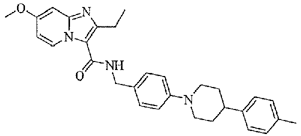 | white solid : ¹H-NMR (CDCl₃): δ 9.25 (1H, d, *J* = 7.6 Hz), 7.28 (2H, d, *J* = 8.8 Hz), 7.08-7.20 (4H, m), 6.98 (2H, d, *J* = 8.8 Hz), 6.88 (1H, d, *J* = 2.4 Hz), 6.61 (1H, dd, *J* = 7.6 Hz, 2.4 Hz), 5.93 (1H, brs), 4.60 (2H, d, *J* = 5.2 Hz), 3.87 (3H, s), 3.80 (2H, d, *J* = 12.4 Hz), 2.92 (2H, q, *J* = 7.6 Hz), 2.82 (2H, td, *J* = 12.0 Hz, 2.8 Hz), 2.55-2.68 (1H, m), 2.33 (3H, s), 1.80-2.00 (4H, m), 1.38 (3H, t, *J* = 7.6 Hz); LCMS: 98.7%, MS (ESI): m/z. 483.2[M+ H]+. |
| 24 | 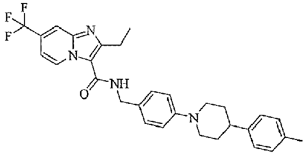 | pink solid : ¹H-NMR (CDCl₃): δ 9.52 (1H, d, *J* = 7.2 Hz), 7.90 (1H, s), 7.26-7.29 (2H, m), 7.10-7.15 (4H, m), 7.07 (1H, d, *J* = 2.0 Hz), 6.99 (2H, d, *J* = 8.8 Hz), 6.07 (1H, brs), 4.62 (2H, d, *J* = 5.2 Hz), 3.81 (2H, d, *J* = 12.0 Hz), 2.99 (2H, q, *J* = 7.6 Hz), 2.79-2.87 (2H, m), 2.60-2.68 (1H, m), 2.33 (3H, s), 1.85-1.96 (4H, m), 1.41 (3H, t, *J* = 7.6 Hz); LCMS: 98.6%, MS (ESI): m/z 521.1[M+ H]+. |
| 25 | 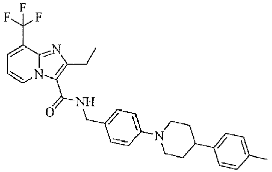 | white solid : ¹H-NMR (CDCl₃): δ 9.61 (1H, d, *J* = 6.8 Hz), 7.68 (1H, d, *J* = 7.2 Hz), 7.20-7.40 (2H, m), 7.10-7.20 (4H, m), 6.95-7.05 (3H, m), 6.12 (1H, brs), 4.65 (2H, d, *J* = 5.2 Hz), 3.80-3.90 (2H, m), 3.05 (2H, q, *J* = 7.2 Hz), 2.80-2.90 (2H, m), 2.55-2.70 (1H, m), 2.36 (3H, s), 1.80-2.00 (4H, m), 1.40 (3H, t, *J* = 7.2 Hz); LCMS: 98.4%, MS (ESI): m/z 521.0[M + H]+. |
| 26 | 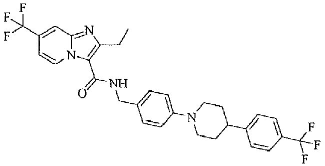 | white solid: ¹H-NMR (CDCl₃): δ 9.55 (1H, d, *J* = 7.2 Hz), 7.93 (1H, s), 7.60 (2H, d, *J* = 8.0 Hz), 7.38 (2H, d, *J* = 8.0 Hz), 7.28-7.34 (2H, m), 7.11 (1H, d, *J* = 7.2 Hz), 7.01 (2H, d, *J* = 8.4 Hz), 6.10 (1H, brs), 4.66 (2H, d, *J* = 5.2 Hz), 3.86 (2H, d, *J* = 12.4 Hz), 3.02 (2H, q, *J* = 7.6 Hz), 2.85-2.91 (2H, m), 2.72-2.79 (1H, m), 1.88-2.04 (4H, m), 1.44 (3H, t, *J* = 7.6Hz); LCMS: 99.2%, MS (ESI): m/z 575.0[M+ H]+. |
| 27 | 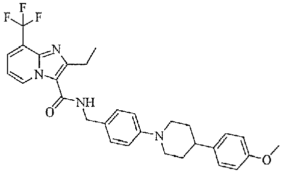 | yellow solid: ¹H-NMR (CDCl₃): δ 9.58 (1H, d, *J* = 6.8 Hz), 7.65 (1H, d, *J* = 7.2 Hz), 7.28 (2H, d, *J* = 8.8 Hz), 7.17 (2H, d, *J* = 8.8 Hz), 6.90-7.00 (3H, m), 6.86 (2H, d, *J* = 8.8 Hz), , 6.08 (1H, brs), 4.62 (2H, d, *J* = 5.6 Hz), 3.75-3.85 (2H, m), 3.80 (3H, s), 3.03 (2H, q, *J* = 7.6 Hz), 2.80-2.90 (2H, m), 2.55-2.65 (1H, m), 1.80-2.00 (4H, m), 1.38 (3H, t, *J* = 7.6 Hz); LCMS: 99.7%, MS (ESI): m/z 537.0[M + H]+. |
| 28 | 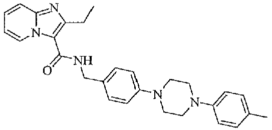 | yellow solid : ¹H-NMR (CDCl₃): δ 9.41 (1H, d, *J* = 7.2 Hz), 7.60 (1H, d, *J* = 8.8 Hz), 7.28-7.40 (3H, m), 7.10 (2H, d, *J* = 8.4 Hz), 6.98 (2H, d, *J* = 8.4 Hz), 6.80-6.95 (3H, m), 6.01 (1H, brs), 4.62 (2H, d, *J* = 5.2 Hz), 3.20-3.41 (8H, m), 2.97 (2H, q, *J* = 7.6 Hz), 2.28 (3H, s), 1.40 (3H, t, *J* = 7.6 Hz); LCMS: 99.7%, MS (ESI): m/z 454.0[M+ H]+. |
| 29 | 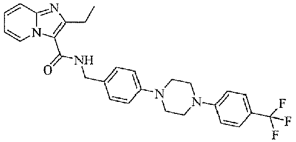 | white solid: ¹H-NMR (CDCl₃): δ 9.41 (1H, d, *J* = 6.8 Hz), 7.61 (1H, d, *J* = 9.2 Hz), 7.51 (2H, d, *J* = 8.8 Hz), 7.27-7.38 (3H, m), 6.95-7.04 (4H, m), 6.92 (1H, t, *J* = 6.8 Hz), 6.02 (1H, brs), 4.63 (2H, d, *J* = 5.6 Hz), 3.39-3.50 (4H, m), 3.29-3.39 (4H, m), 2.98 (2H, q, *J* = 7.6 Hz), 1.40 (3H, t, *J* = 7.6 Hz); LCMS: 100%, MS (ESI): m/z 508.1 [M+ H]+. |
| 30 | 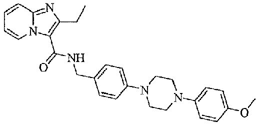 | white solid: ¹H-NMR (CDCl₃): δ 9.44 (1H, d, *J* = 6.8 Hz), 7.63 (1H, d, *J* = 9.2 Hz), 7.31-7.42 (2H, m, overlap with CDCl₃ signal), 6.85-7.08 (8H, m), 6.04 (1H, brs), 4.66 (2H, d, *J* = 5.2 Hz), 3.81 (3H, s), 3.38 (4H, t, *J* = 4.4 Hz), 3.26 (4H, t, *J* = 4.4 Hz), 3.00 (2H, q, *J* = 7.6 Hz), 1.43 (3H, t, *J* = 7.6 Hz); LCMS: 98.8%, MS (ESI): m/z 492.3 [M+ Na]+. |
| 31 | 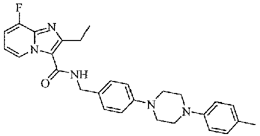 | off-white solid: ¹H-NMR (CDCl₃): δ 9.23 (1H, d, *J* = 7.2 Hz), 7.33 (2H, d, *J* = 8.8 Hz), 7.13 (2H, d, *J* = 8.4 Hz), 7.08-6.99 (3H, m), 6.93 (2H, d, *J* = 8.4 Hz), 6.88-6.83 (1H, m), 6.08 (1H, brs), 4.65 (2H, d, *J* = 5.6 Hz), 3.39-3.30 (8H, m), 3.02 (2H, q, *J* = 7.6 Hz), 1.43 (3H, t, *J* = 7.6 Hz); LCMS: 99.5%, MS (ESI): m/z 494.1[M+ Na]+. |
| 32 | 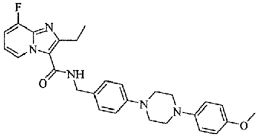 | white solid: ¹H-NMR (CDCl₃): δ 9.23 (1H, d, *J* = 7.2 Hz), 7.33 (2H, d, *J* = 8.8 Hz), 7.08-6.96 (5H, m), 6.91-6.83 (3H, m), 6.08 (1H, brs), 4.65 (2H, d, *J* = 5.6 Hz), 3.81 (3H, s), 3.39-3.36 (4H, m), 3.27-3.24 (4H, m), 3.02 (2H, q, *J* = 7.6 Hz), 1.43 (3H, t, *J* = 7.6 Hz); LCMS: 98.8%, MS (ESI): m/z 488.1 [M+ H]+. |
| 33 | 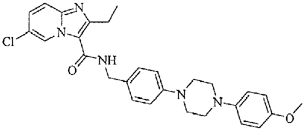 | white solid : ¹H-NMR(CDCl₃): δ 9.56 (1H, d, *J* = 1.2 Hz), 7.56 (1H, d, *J* = 9.6 Hz), 7.30-7.35 (3H, m), 6.97-7.05 (4H, m), 6.89 (2H, d, *J* = 8.8 Hz), 6.05 (1H, brs), 4.65 (2H, d, *J* = 5.2 Hz), 3.81 (3H, s), 3.35-3.40 (4H, m), 3.23-3.29 (4H, m), 2.98 (2H, q, *J* = 7.6 Hz), 1.42 (3H, t, *J* = 7.6 Hz); LCMS: 98.4%, MS (ESI): m/z 526.1[M+ Na]+. |
| 34 | 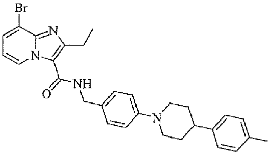 | white solid: ¹H-NMR (CDCl₃): δ 9.42 (1H, d, *J* = 6.8 Hz), 7.60 (1H, d, *J* = 6.8 Hz), 7.25-7.35 (2H, m), 7.12-7.20 (4H, m), 7.01 (2H, d, *J* = 8.8 Hz), 6.82 (1H, t, *J* = 7.2 Hz), 6.08 (1H, brs), 4.64 (2H, d, *J* = 5.6 Hz), 3.84 (2H, d, *J* = 12.0 Hz), 3.04 (2H, q, *J* = 7.6 Hz), 2.85 (2H, t, *J* = 12.0 Hz), 2.60-2.70 (1H, m), 1.85-2.00 (4H, m), 1.40 (3H, t, *J* = 7.6 Hz); LCMS: 100%, MS(ESI):m/z 531.1/533.1 [M+H]. |
| 35 | 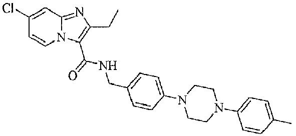 | white solid: ¹H-NMR (CDCl₃): δ 9.39 (1H, d, *J* = 7.2 Hz), 7.61 (1H, s), 7.25-7.32 (2H, m), 7.13 (2H, d, *J* = 8.0 Hz), 6.90-7.05 (5H, m), 6.04 (1H, brs), 4.63 (2H, d, *J* = 5.2 Hz), 3.30-3.40 (8H, m), 2.97 (2H, q, *J* = 7.6 Hz), 2.31(1H, s), 1.41 (3H, t, *J* = 7.6 Hz); LCMS: 100%, MS (ESI): m/z 488.1 [M+H]+. |
| 36 | 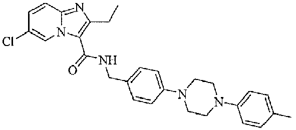 | off-white solid: ¹H-NMR(CDCl₃): δ 9.56 (1H, d, *J* = 1.2 Hz), 7.56 (1H, d, *J* = 9.6 Hz), 7.27-7.35 (3H, m), 7.13 (2H, d, *J* = 8.4 Hz), 7.01 (2H, d, *J* = 8.8 Hz), 6.93 (2H, d, *J* = 8.4 Hz), 6.06 (1H, brs), 4.65 (2H, d, *J* = 5.6 Hz), 3.29-3.40 (8H, m), 2.98 (2H, q, *J* = 7.6 Hz), 1.42 (3H, t, *J* = 7.6 Hz); LCMS: 100%, MS (ESI): m/z 510.1[M+Na]+. |
| 37 | 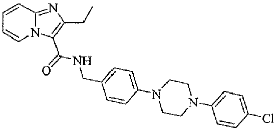 | white solid: ¹H-NMR (CDCl₃): δ 9.43 (1H, d, *J* = 7.2 Hz), 7.63 (1H, d, *J* = 8.8 Hz), 7.31-7.42 (3H, m), 7.20-7.30 (2H, m, overlap with CDCl₃ signal), 7.00 (2H, d, *J* = 8.4 Hz), 6.85-6.98 (3H, m), 6.05 (1H, brs), 4.65 (2H, d, *J* = 5.2 Hz), 3.20-3.45 (8H, m), 3.01 (2H, q, *J* = 7.6 Hz), 1.43 (3H, t, *J* = 7.6 Hz); LCMS: 100%, MS (ESI): m/z 474.1[M+ H]+. |
| 38 | 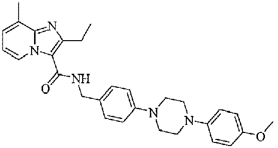 | white solid: ¹H-NMR (CDCl₃): δ 9.24 (1H, d, *J* = 6.8 Hz), 7.31 (2H, d, *J* = 8.5 Hz), 7.11 (1H, d, *J* = 6.9 Hz), 6.97 (4H, t, *J* = 8.7 Hz), 6.84-6.90 (2H, m), 6.82 (1H, t, *J* = 7.0 Hz), 6.02 (1H, brs), 4.63 (2H, d, *J* = 5.4 Hz), 3.78 (3H, s), 3.18-3.38 (8H, m), 3.00 (2H, q, *J* = 7.6 Hz), 2.61 (3H, s), 1.37 (3H, t, *J* = 7.7 Hz); LCMS: 100%, MS (ESI): m/z 484.1[M+ H]+. |
| 39 | 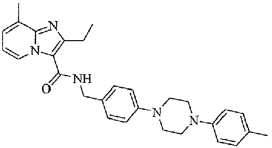 | white solid: ¹H-NMR (CDCl₃): δ 9.24 (1H, d, *J* = 6.9 Hz), 7.31 (2H, d, *J* = 8.7 Hz), 7.07-7.15 (3H, m), 6.98 (2H, d, *J* = 8.7 Hz), 6.90 (2H, d, *J* = 8.5 Hz), 6.82 (1H, t, *J* = 7 Hz), 6.03 (1H, brs), 4.63 (2H, d, *J* = 5.5 Hz), 3.25-3.38 (8H, m), 3.00 (2H, q, *J* = 7.6 Hz), 2.61 (3H, s), 2.29 (3H, s), 1.37 (3H, t, *J* = 7.6 Hz); LCMS: 100%, MS (ESI): m/z 490.1[M+ H]+. |
| 40 | 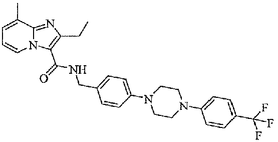 | white solid: ¹H-NMR (CDCl₃): δ 9.25 (1H, d, *J* = 7 Hz), 7.51 (2H, d, *J* = 8.7 Hz), 7.32 (2H, d, *J* = 8.5 Hz), 7.12 (1H, d, *J* = 7.5 Hz), 6.98 (4H, d, *J* = 8.5 Hz), 6.82 (1H, t, *J* = 6.9 Hz), 6.03 (1H, brs), 4.63 (2H, d, *J* = 5.5 Hz), 3.30-3.48 (8H, m), 3.00 (2H, q, *J* = 7.7 Hz), 2.62 (3H, s), 1.37 (3H, t, *J* = 7.7 Hz); LCMS: 98.5%, MS (ESI): m/z 522.1[M+ H]+. |
| 41 | 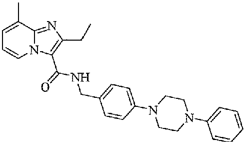 | white solid: ¹H-NMR (CDCl₃): δ 9.25 (1H, d, *J* = 7 Hz), 7.26-7.35 (4H, m), 7.11 (1H, d, *J* = 6.9 Hz), 6.96-7.03 (4H, m), 6.90 (1H, t, *J* = 7.3 Hz), 6.82 (1H, t, *J* = 7.0 Hz), 6.02 (1H, brs), 4.63 (2H, d, *J* = 5.4 Hz), 3.35 (8H, s), 3.00 (2H, q, *J* = 7.5 Hz), 2.61 (3H, s), 1.37 (3H, t, *J* = 7.6 Hz); LCMS: 100%, MS (ESI): m/z 476.1[M+H]+. |
| 42 | 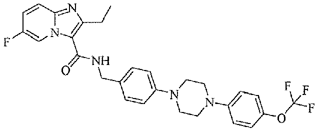 | white solid: ¹H-NMR (CDCl₃): δ 9.48 (1H, dd, *J* = 4.8, 2.0 Hz), 7.59 (1H, dd, *J* = 9.6, 5.2 Hz), 7.34 (2H, d, *J* = 8.4 Hz), 7.24-7.30 (1H, m, overlap with CDCl₃ signal), 7.17 (2H, d, *J* = 8.8 Hz), 6.90-7.08 (4H, m), 6.06 (1H, brs), 4.65 (2H, d, *J* = 5.6 Hz), 3.36 (8H, s), 2.99 (2H, q, *J* = 7.6 Hz), 1.43 (3H, t, *J* = 7.6 Hz); LCMS: 98.2%, MS(ESI): m/z 542.1 [M+H]+. |
| 43 | 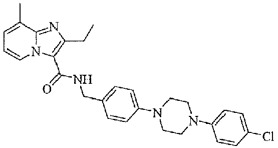 | white solid: ¹H-NMR (CDCl₃): δ 9.24 (1H, d, *J* = 6.9 Hz), 7.31 (2H, d, *J* = 8.5 Hz), 7.23 (2H, d, *J* = 8.9 Hz),7.12 (1H, d, *J* = 6.7 Hz), 6.98 (2H, d, *J* = 8.7 Hz), 6.90 (2H, d, *J* = 8.9 Hz), 6.82 (1H, t, *J* = 6.8 Hz), 6.03 (1H, brs), 4.63 (2H, d, *J* = 5.5 Hz), 3.28-3.38 (8H, m), 3.00 (2H, q, *J* = 7.7 Hz), 2.62 (3H, s), 1.37 (3H, t, *J* = 7.7 Hz); LCMS: 100%, MS (ESI): m/z 488.1[M+ H]+. |
| 44 | 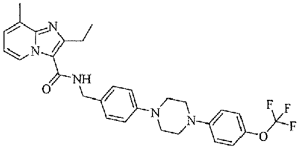 | white solid : ¹H-NMR (CDCl₃): δ 9.24 (1H, d, *J* = 6.9 Hz), 7.32 (2H, d, *J* = 8.7 Hz), 7.08-7.17 (3H, m), 6.91-7.03 (4H, m), 6.82 (1H, t, *J* = 6.9 Hz), 6.03 (1H, brs), 4.63 (2H, d, *J* = 5.5 Hz), 3.30-3.38 (8H, m), 3.00 (2H, q, *J* = 7.5 Hz), 2.62 (3H, s), 1.37 (3H, t, *J* = 7.7 Hz); LCMS: 98.3%, MS (ESI): m/z 538.1[M+ H]+. |
| 45 | 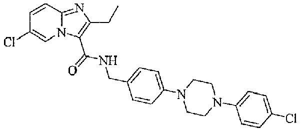 | white solid : ¹H-NMR(CDCl₃): δ 9.56 (1H, d, *J* = 1.2 Hz), 7.56 (1H, d, *J* = 9.2 Hz), 7.30-7.40 (3H, m), 7.23-7.29 (2H, m), 7.00 (2H, d, *J* = 8.4 Hz), 6.92 (2H, d, *J* = 8.8 Hz), 6.06 (1H, brs), 4.65 (2H, d, *J* = 5.6 Hz), 3.25-3.40 (8H, m), 2.98 (2H, q, *J* = 7.6 Hz), 1.42 (3H, t, *J* = 7.6 Hz); LCMS: 100%, MS (ESI): m/z 530.0[M+Na]+. |
| 46 | 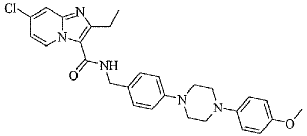 | white solid : ¹H-NMR (CDCl₃): δ 9.38 (1H, d, *J* = 7.2 Hz), 7.61 (1H, d, *J* = 1.6 Hz), 7.32 (2H, d, *J* = 8.4 Hz), 6.99 (4H, t, *J* = 8.8 Hz), 6.85-6.95 (3H, m), 6.03 (1H, brs), 4.64 (2H, d, *J* = 5.6 Hz), 3.81 (3H, s), 3.20-3. 30 (4H, m), 3.32-3.40 (4H, m), 2.97 (2H, q, *J=* 7.6 Hz), 1.41 (3H, t, *J* = 7.2 Hz); LCMS: 100%, MS (ESI): m/z 526.0 [M+ Na]+. |
| 47 | 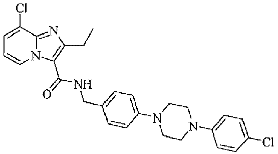 | white solid : ¹H-NMR (CDCl₃): δ 9.37 (1H, d, *J* = 6.4 Hz) , 7.42 (1H, d, *J* = 7.2 Hz), 7.33 (2H, d, *J* = 8.8 Hz), 7.25 (2H, d, *J* = 8.8 Hz), 7.00 (2H, d, *J* = 8.4 Hz), 6.82-6.93 (3H, m), 6.09 (1H, brs), 4.65 (2H, d, *J*= 5.2 Hz), 3.34 (8H, d, *J* = 6.8 Hz), 3.03 (2H, q, *J* = 7.6 Hz), 1.40 (3H, t, *J* = 7.6 Hz); LCMS: 100.0%, MS (ESI): m/z 508.0[M+ H]+. |
| 48 | 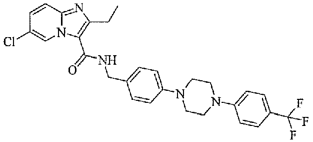 | white solid : ¹H-NMR(CDCl₃): δ 9.56 (1H, d, *J* = 1.6 Hz), 7.53-7.59 (3H, m), 7.29-7.35 (3H, m), 7.00 (4H, d, *J* = 8.8 Hz), 6.07 (1H, brs), 4.65 (2H, d, *J* = 5.6 Hz), 3.43-3.49 (4H, m), 3.35-3.40 (4H, m), 2.98 (2H, q, *J* = 7.6 Hz), 1.42 (3H, t, *J* = 7.6 Hz); LCMS: 98.4%, MS (ESI): m/z 542.0[M+ H]+. |
| 49 | 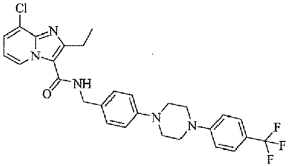 | white solid : ¹H-NMR (CDCl₃): δ 9.37 (1H, dd, *J1* = 0.8 Hz, *J2* = 7.2 Hz), 7.54 (2H, d, *J* = 8.8 Hz), 7.42 (1H, dd, *J1* = 0.8 Hz, *J2* = 7.6 Hz), 7.34 (2H, d, *J* = 8.8 Hz), 7.00 (4H, d, *J* = 8.4 Hz), 6.88 (1H, t, *J* = 7.2 Hz), 6.10 (1H, brs), 4.66 (2H, d, *J* = 5.6 Hz), 3.47 (4H, dd, *J1* = 4.4 Hz, *J2* =7.2 Hz), 3.38 (4H, dd, *J1* = 7.2 Hz, *J2* = 10 Hz), 3.04 (2H, q, *J* = 7.6 Hz), 1.41 (3H, t, *J* = 7.6 Hz); LCMS: 98.7%, MS (ESI): m/z 542.0[M+ H]+. |
| 50 | 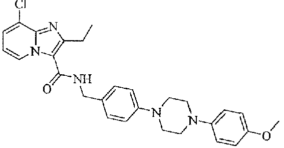 | yellow solid: ¹H-NMR (CDCl₃): δ 9.37 (1H, d, *J* = 6.8 Hz), 7.42 (1H, d, *J* = 6.4 Hz), 7.33 (2H, d, *J* = 8.4 Hz), 7.00 (4H, t, *J* = 9.2 Hz), 6.86-6.90 (3H, m), 6.10 (1H, brs), 4.65 (2H, d, *J* = 5.6 Hz), 3.81 (3H, s), 3.38 (4H, t, *J* = 5 Hz), 3.25 (4H, t, *J* = 5 Hz), 3.04 (2H, q, *J* = 7.6 Hz), 1.41 (3H, t, *J* = 7.6 Hz); LCMS: 100.0%, MS (ESI): m/z 526.1[M+ Na]+. |
| 51 | 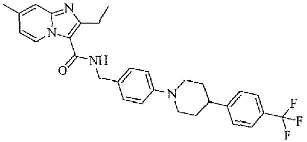 | white solid: ¹H-NMR (CDCl₃): δ 9.31 (1H, d, *J* = 7.2 Hz), 7.60 (2H, d, *J* = 8.4 Hz), 7.38 (3H, d, *J* = 8.0 Hz), 7.33-7.28 (2H, m), 7.01 (2H, d, *J* = 8.8 Hz), 6.78 (1H, d, *J* = 7.0 Hz), 6.01 (1H, brs), 4.64 (2H, d, *J=* 5.2 Hz), 3.84 (2H, d, *J* = 12.4 Hz), 2.96 (2H, q, *J* = 7.2 Hz), 2.83 (2H, td, *J* = 12 Hz, 2.4 Hz), 2.78-2.72 (1H, m), 2.45 (3H, s), 2.02-1.91 (4H, m), 1.28 (3H, t, *J=* 7.2 Hz); LCMS: 100%, MS (ESI): m/z 521.1[M+ H]+. |
| 52 | 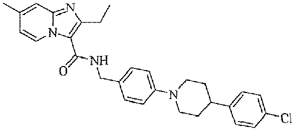 | yellow solid : ¹H-NMR (CDCl₃): δ 9.30 (1H, d, *J* = 7.2 Hz), 7.38 (1H, s), 7.32-7.29 (4H, m), 7.20 (2H, d, *J* = 8.4 Hz), 7.00 (2H, d, *J* = 8.4 Hz), 6.77 (1H, dd, *J* = 7.2 Hz, 1.6 Hz), 6.0 (1H, brs), 4.64 (2H, d, *J* = 5.6 Hz), 3.81 (2H, d, *J* = 12.4 Hz), 2.97 (2H, q, *J* = 7.2 Hz), 2.84 (2H, td, *J* = 12 Hz, 2.4 Hz), 2.70-2.60 (1H, m), 2.42 (3H, s), 2.01-1.82 (4H, m), 1.39 (3H, t, *J* = 7.2 Hz); LCMS: 100%, MS (ESI): m/z 487.1[M+ H]+. |
| 53 | 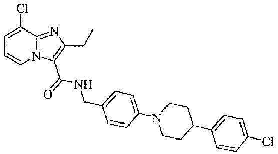 | white solid: ¹H-NMR (CDCl₃): δ 9.37 (1H, dd, *J* = 0.8 Hz, *J* = 6.8 Hz), 7.42 (1H, dd, *J* = 0.8 Hz, *J* = 7.2 Hz), 7.31 (4H, d, *J* = 8.4 Hz), 7.20 (2H, d, *J* = 8.4 Hz), 7.00 (2H, d, *J* = 8.8 Hz), 6.88 (1H, t, *J* = 7.2 Hz), 6.09 (1H, brs), 4.64 (2H, d, *J* = 5.2 Hz), 3.84 (2H, d, *J* = 12.0 Hz), 3.04 (2H, q, *J* = 7.6 Hz), 2.85 (2H, td, *J* = 2.4, 12.4 Hz), 2.62-2.70 (1H, m), 1.83-1.98 (4H, m), 1.41 (3H, t, *J* = 7.6 Hz); LCMS: 100.0%, MS (ESI): m/z 507.0[M+ H]+. |
| 54 | 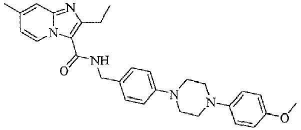 | white solid : ¹H-NMR (CDCl₃): δ 9.28 (1H, d, *J* = 7.2 Hz), 7.35-7.29 (3H, m), 6.99-6.95 (4H, m), 6.86 (2H, d, *J* = 8.8 Hz), 6.74 (1H, d, *J* = 7.2 Hz), 5.99 (1H, brs), 4.62 (2H, d, *J* = 5.6 Hz), 3.78 (3H, s), 3.36-3.22 (8H, m), 2.95 (2H, q, *J* = 7.6 Hz), 2.17 (3H, s), 1.39 (3H, t, *J* = 7.6 Hz); LCMS: 100%, MS (ESI): m/z 484.1[M+ H]+. |
| 55 | 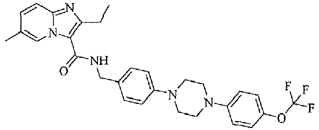 | white solid: ¹H-NMR (CDCl₃): δ 9.14 - 9.31 (1H, m), 7.45 - 7.56 (1 H, m), 7.29 - 7.38 (2H, m), 7.08 - 7.22 (3H, m), 6.96 (4H, s), 5.95 - 6.09 (1H, m), 4.57 - 4.73 (2H, m), 3.34 (8H, s), 2.89 - 3.04 (2H, m), 2.37 (3H, s), 1.40 (3H, s); LCMS: 98.2%, MS (ESI): m/z 538.1[M+ H]+. |
| 56 | 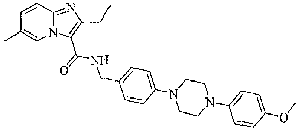 | white solid: ¹H-NMR (CDCl₃): δ 9.13 - 9.25 (1H, m), 7.46 - 7.54 (1H, m), 7.27 - 7.36 (2H, m), 7.09 - 7.21 (1H, m), 6.96 (4H, d, *J*=9.54 Hz), 6.88 (2H, s), 5.96 - 6.06 (1H, m), 4.59 - 4.67 (2H, m), 3.78 (3H, s), 3.35 (4H, d, *J*=5.27 Hz), 3.23 (4H, brs), 2.90 - 3.01 (2H, m), 2.36 (3H, s), 1.39 (3H, t, *J*=7.53 Hz); LCMS: 98.8%, MS (ESI): m/z 484.0[M+ H]+. |
| 57 | 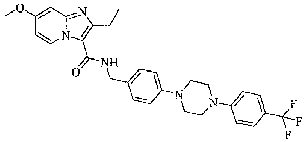 | yellow solid: ¹H-NMR (CDCl₃): δ 9.25 (1H, d, *J* = 7.6 Hz), 7.51 (2H, d, *J* = 8.8 Hz), 7.31 (2H, d, *J* = 8.4 Hz), 6.92-7.03 (4H, m),6.89 (1H, d, *J* = 2.0 Hz) 6.61 (1H, dd, *J* = 7.6 Hz, 2.4 Hz), 5.96 (1H, brs), 4.61 (2H, d, *J* = 5.6 Hz), 3.87 (3H, s), 3.25-3.49 (8H, m), 2.92 (2H, q, *J* = 7.6 Hz), 1.39 (3H, t , *J* = 7.6 Hz); LCMS: 98.5%, MS (ESI): m/z 538.1[M+ H]+, 560.0 [M+Na]+. |
| 58 | 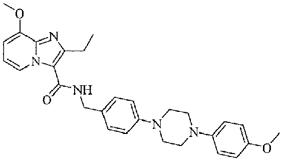 | white solid : ¹H-NMR (CDCl₃): δ 8.99 (1H, d, *J*= 6.8 Hz), 7.31 (2H, d, *J* = 8.4 Hz), 6.97 (4H, t, *J*= 8.8 Hz), 6.87 (2H, d, *J* = 8.8 Hz), 6.81 (1H, t, = 7.2 Hz), 6.62 (1H, d, *J* = 8.0 Hz), 6.05 (1H, brs), 4.62 (2H, d, *J* = 5.6 Hz), 4.02 (3H, s). 3.78 (3H, s), 3.35 (4H, t, *J* = 4.8 Hz), 3.23 (4H, t, *J* = 4.8 Hz), 2.98 (2H, q, *J* = 7.6 Hz), 1.38 (3H, t, *J* = 7.6 Hz); LCMS: 99.4%, MS (ESI): m/z 522.1 [M+Na] +. |
| 59 | 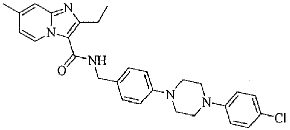 | white solid : ¹H-NMR (CDCl₃): δ 9.28 (1H, d, *J* = 7.2 Hz), 7.36 (1H, s), 7.31 (2H, d, *J* = 8.4 Hz), 7.23 (2H, d, *J* = 8.8 Hz), 6.98 (2H, d, *J* = 8.4 Hz), 6.89 (2H, d, *J* = 8.8 Hz), 6.75 (1H, d, *J* = 6.4 Hz), 6.00 (1H, brs), 4.62 (2H, d, *J* = 5.6 Hz), 3.33-3.31 (8H, m), 2.95 (2H, q, *J* = 7.6 Hz), 2.42 (3H, s), 1.38 (3H, t, *J* = 7.6 Hz); LCMS: 100%, MS (ESI): m/z 488.1[M+ H]+. |
| 60 | 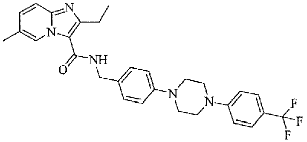 | white solid : ¹H-NMR (CDCl₃): δ 9.13 - 9.25 (1H, m), 7.46 - 7.54 (1H, m), 7.27 - 7.36 (2H, m), 7.09 - 7.21 (1H, m), 6.96 (4H, d, J=9.54 Hz), 6.88 (2H, s), 5.96 - 6.06 (1H, m), 4.59 - 4.67 (2H, m) ,3.78 (3H, s), 3.35 (4H, d, *J*=5.27 Hz), 3.23 (4H, brs), 2.90 - 3.01 (2H, m), 2.36 (3H, s), 1.39 (3H, t, *J*=7.53 Hz); LCMS: 99.0%, MS (ESI): m/z 522.1[M+ H]+. |
| 61 | 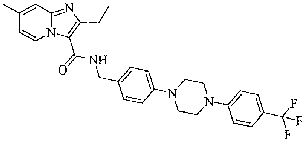 | white solid : ¹H-NMR (CDCl₃): δ 9.28 (1H, d, *J* = 7.2 Hz), 7.51 (2H, d, *J* = 8.8 Hz), 7.36-7.30 (3H, m), 6.98 (4H, d, *J* = 8.4 Hz), 6.76 (1H, d, *J* = 7.2 Hz), 6.00 (1H, brs), 4.63 (2H, d, *J* = 5.2 Hz), 3.45-3.34 (8H, m), 2.94 (2H, q, *J* = 7.6 Hz), 2.42 (3H, s), 1.39 (3H, t, *J* = 7.6 Hz); LCMS: 99.3%, MS (ESI): m/z 522.1 [M+ H]+. |
| 62 | 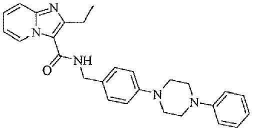 | white solid : ¹H-NMR (CDCl₃): δ 9.41 (1H, d, *J* = 7.2 Hz), 7.61 (1H, d, *J* = 8.8 Hz), 7.25-7.36 (5H, m, overlap with CDCl₃ signal), 6.95-7.04 (m, 4H), 6.86-6.94 (2H, m), 6.03 (1H, brs), 4.63 (2H, d, *J =* 5.2 Hz), 3.35 (8H, s), 2.98 (2H, q, *J* = 7.6 Hz), 1.41 (3H, t, *J* = 7.6 Hz); LCMS: 100%, MS (ESI): m/z 440.1 [M+ H]+. |
| 63 | 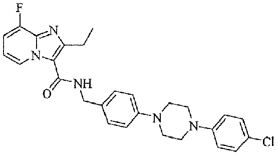 | white solid : ¹H-NMR (CDCl₃): δ 9.21 (1H, d, *J* = 6.8 Hz), 7.31 (2H, d, *J* = 8.4 Hz), 7.24 (2H, d, *J* = 9.2 Hz), 7.06-6.97 (3H, m), 6.90 (2H, d, *J* = 9.2 Hz), 6.86-6.81 (1H, m), 6.07 (1H, brs), 4.63 (2H, d, *J* = 5.6 Hz), 3.35-3.30 (8H, m), 3.00 (2H, q, *J=* 7.6 Hz), 1.40 (3H, t, *J=* 7.6 Hz); LCMS: 100%, MS (ESI): m/z 491.9[M+ Na]+. |
| 64 | 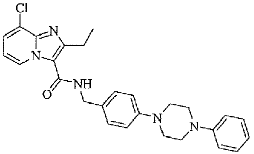 | white solid : ¹H-NMR (CDCl₃): δ 9.35 (1H, d, *J*=6.8 Hz) , 7.39 (1H, d, *J* = 7.2 Hz), 7.28-7.32 (4H, m), 6.99 (4H, d, *J* = 8 Hz), 6.83-6.92 (2H, m), 6.07 (1H, brs), 4.63 (2H, d, *J=* 5.2 Hz), 3.35 (8H, s), 3.01 (2H, q, *J=* 7.6 Hz), 1.39 (3H, t, *J=* 7.8 Hz); LCMS: 100.0%, MS (ESI): m/z474.0[M+ H]+. |
| 65 | 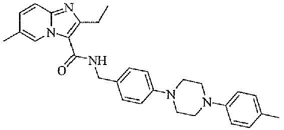 | yellow solid : ¹H-NMR (CDCl₃): δ 9.12 - 9.29 (1 H, m), 7.48 - 7.53 (1 H, m), 7.29 - 7.34 (2 H, m), 7.15 - 7.20 (1 H, m), 7.07 - 7.14 (2 H, m), 6.94 - 7.01 (2 H, m,), 6.85 - 6.94 (2 H, m), 5.95 - 6.04 (1 H, m), 4.63 (2 H, d, *J*=5.52 Hz,), 3.34 (4 H, d, *J*=5.77 Hz), 3.30 (4 H, d, *J*=5.77 Hz), 2.96 (2 H, d, *J*=7.53 Hz), 2.36 (3 H, s), 2.29 (3 H, s), 1.39 (3 H, t, *J*=7.53 Hz); LCMS: 100%, MS (ESI): m/z 467.2[M+ H]+. |
| 66 | 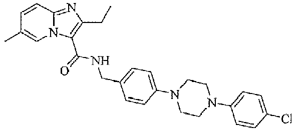 | white solid : ¹H-NMR (CDCl₃): δ 9.14 - 9.31 (1 H, m), 7.45 - 7.56 (1 H, m), 7.28 - 7.40 (2 H, m), 7.19 - 7.30 (2 H, m), 7.15 - 7.20 (1 H, m), 6.94 - 7.02 (2 H, m), 6.85 - 6.94 (2 H, m), 5.97 - 6.06 (1 H, m), 4.63 (2 H, d, *J*=5.27 Hz), 3.32 (8 H, q, *J*=5.94 Hz), 2.96 (2 H, d, *J*=7.53 Hz), 2.36 (3 H, s), 1.39 (3 H, t, *J*=7.53 Hz); LCMS: 100%, MS (ESI): m/z 488.0[M+ H]+. |
| 67 | 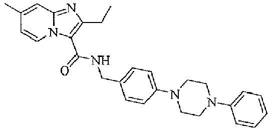 | white solid : ¹H-NMR (CDCl₃): δ 9.30 (1H, d, *J* = 7.2 Hz), 7.38-7.28 (5H, m), 7.01 (4H, dd, *J* = 8.0 Hz, 1.6 Hz), 6.92 (1H, t, *J* = 7.2 Hz), 6.76 (1H, dd, *J* = 7.2 Hz, 1.6 Hz), 6.02 (1H, brs), 4.64 (2H, d, *J* = 5.6 Hz), 3.37 (8H, s), 2.97 (2H, q, *J* = 7.6 Hz), 2.44 (3H, s), 1.41 (3H, t, *J* = 7.6 Hz); LCMS: 100%, MS (ESI): m/z476.1[M+ H]+. |
| 68 | 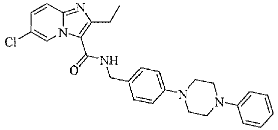 | white solid : ¹H-NMR(CDCl₃): δ 9.56 (1H, d, *J* = 1.2 Hz), 7.57 (1H, d, *J* = 9.2 Hz), 7.29-7.35 (5H, m), 7.01 (4H, d, *J=* 8.4 Hz), 6.93 (1H, t, *J=* 7.2 Hz), 6.05 (1H, brs), 4.65 (2H, d, *J* = 5.6 Hz), 3.38 (8H, s), 2.98 (2H, q, *J* = 7.6 Hz), 1.42 (3H, t, *J* = 7.6 Hz); LCMS: 100%, MS (ESI): m/z 496.0[M+ Na]+. |
| 69 | 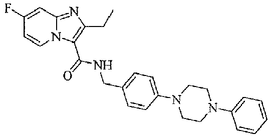 | white solid : ¹H-NMR (CDCl₃): δ 9.44-9.48(1H, m), 7.33-7.28(3H, m), 7.25-7.23(1H, m), 7.01(4H, d, *J* = 8.4 Hz), 6.92(4H, d, J=7.2Hz), 6.83-6.78(1H, m), 6.02(1H, brs), 4.64(2H, d, *J*=5.6Hz), 3.37(8H, s), 2.99-2.94(2H, m), 1.41(3H, t, *J* = 7.6 Hz); LCMS: 99.9%, MS (ESI): m/z 480.1[M+ Na]+. |
| 70 | 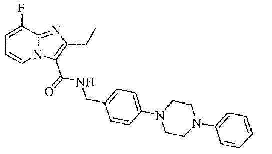 | yellow solid : ¹H-NMR (CDCl₃): δ 9.23 (1H, d, *J* = 6.8 Hz), 7.34-7.28 (4H, m), 7.08-7.00 (5H, m), 6.92 (1H, t, *J* = 7.2 Hz), 6.88-6.83 (1H, m), 6.10 (1H, brs), 4.65 (2H, d, *J* = 5.6 Hz), 3.38 (8H, s), 3.02 (2H, q, *J* = 7.6 Hz), 1.42 (3H, t, *J* = 7.6 Hz); LCMS: 98.8%, MS (ESI): m/z 480.1 [M+ Na]+. |
| 71 | 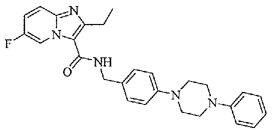 | white solid : ¹H-NMR (CDCl₃): δ 9.48 (1H, dd, *J* = 4.8, 2.0 Hz), 7.59 (1H, dd, *J* = 9.6, 5.2 Hz), 7.24-7.35 (5H, m, overlap with CDCl₃ signal), 7.01 (4H, d, *J* = 8.8 Hz), 6.93 (1H, t, *J* = 7.6 Hz), 6.06 (1H, brs), 4.65 (2H, d, *J* = 5.6 Hz), 3.38 (8H, s), 2.99 (2H, q, *J* = 7.6 Hz), 1.42 (3H, t, *J* = 7.6 Hz); LCMS: 100%, MS (ESI): m/z 480.1[M+ Na]+. |
| 72 | 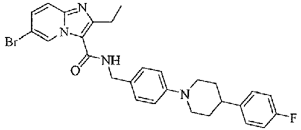 | white solid : ¹H-NMR (CDCl₃): δ 9.56 (1H, d, *J* = 1.6 Hz), 7.40-7.49 (1H, m), 7.29-7.37 (1H, m), 7.05-7.29 (4H. m), 6.84-7.01 (4H, m), 5.95 (1H, brs), 4.48-4.62 (2H, m), 3.65-3.81 (2H, m), 2.89 (2H, q, *J* = 7.6 Hz), 2.69-2.81 (2H, m), 2.50-2.61 (1H, m), 1.71-1.95 (4H, m), 1.33 (3H, t, *J* = 7.6 Hz); LCMS: 100%, MS (ESI): m/z 535.0[M+ H]+. |
| 73 | 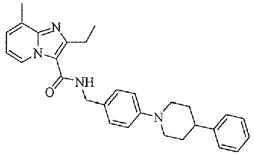 | white solid : ¹H-NMR (CDCl₃): δ 9.24 (1H, d, *J* = 6.8 Hz), 7.18-7.36 (7H, m), 7.1 (1H, d, *J* = 6.8 Hz), 6.98 (2H, d, *J* = 8.4 Hz), 6.81 (1H, t, *J* = 6.9 Hz), 6.03 (1H, brs), 4.62(2H, d, *J* = 5.5 Hz), 3.82 (2H, d, *J* = 12.3 Hz), 2.99 (2H, q, *J* = 7.7 Hz), 2.83 (2H, td, *J* = 12, 2.8 Hz), 2.62-2.71 (1H, m), 2.61 (3H, s), 1.83-2.01 (4H, m), 1.36 (3H, t, *J* = 7.5 Hz); LCMS: 100%, MS (ESI): m/z 475.2[M+ H]+. |
| 74 | 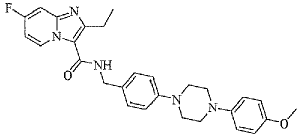 | yellow solid : ¹H-NMR (CDCl₃): δ 9.41-9.45(1H, m), 7.29(2H, d, J= 8.4 Hz), 7.20-7.22(1H, m), 6.93-6.99(4H, m), 6.85-6.88(2H, m), 6.75-6.80(1H, m), 5.99(1H, brs), 4.61(2H, d, *J*=5.2Hz), 3.78(3H, s), 3.33-3.36(4H, m), 3.21-3.24(4H, m), 2.91-2.97(2H, m), 1.38(3H, t, *J*= 7.2 Hz); LCMS: 98.0%, MS (ESI): m/z 488.1 [M+ H]+, 510.1 [M+ H]+ |
| 75 | 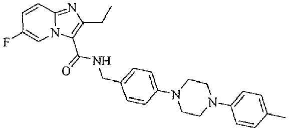 | white solid :¹H-NMR (CDCl₃): δ 9.40-9.48 (1H, m), 7.52-7.60 (1H, m), 7.30 (2H, d, *J* = 8.8 Hz), 7.20-7.28 (1H, m, overlapped with CDCl₃), 7.10 (2H, d, *J* = 8.4 Hz), 6.98 (2H, d, *J* = 8.4 Hz), 6.90 (2H, d, *J* = 8.8 Hz), 6.02 (1H, brs), 4.62 (2H, d, *J* = 5.6 Hz), 3.20-3.40 (8H, m), 2.96 (2H, q, *J* = 7.6 Hz), 2.28 (3H, s), 1.39 (3H, t, *J* = 7.6 Hz); LCMS: 100%, MS (ESI): m/z 494.1 [M+ Na]+. |
| 76 | 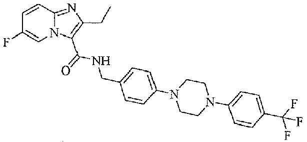 | white solid : ¹H-NMR (DMSO-*d₆*): δ 9.40-9.51 (1H, m), 7.42-7.62 (3H, m), 7.26-7.40 (3H, m, overlap with CDCl₃ signal), 6.89-7.10 (4H, m), 6.04 (1H, brs), 4.63 (1H, d, *J* = 5.6 Hz), 3.20-3.50 (8H, m), 2.97 (2H, q, *J* = 7.6 Hz), 1.40 (3H, t, *J* = 7.6 Hz); LCMS: 100%, MS (ESI): m/z 526.0[M+ H]+. |
| 77 | 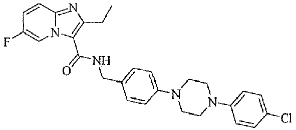 | white solid : ¹H-NMR (CDCl₃): δ 9.40-9.52 (1H, m), 7.52-7.62 (1H, m), 7.31 (2H, d, *J* = 8.0 Hz), 7.15-7.28 (3H, m, overlapped with CDCl₃), 6.98 (2H, d, *J* = 8.0 Hz), 6.90 (2H, d, *J* = 8.4 Hz), 6.03 (1H, brs), 4.63 (2H, d, *J* = 5.2 Hz), 3.20-3.45 (8H, m), 2.97 (2H, q, *J* = 7.2 Hz), 1.40 (3H, t, *J* = 7.2 Hz); LCMS: 100%, MS (ESI): m/z 492.0[M+ H]+. |
| 78 | 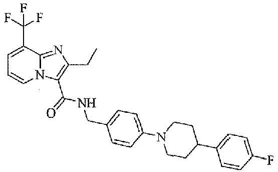 | white solid : ¹H-NMR (CDCl₃): δ 9.60 (1H, d, *J* = 6.8 Hz), 7.65 (1H, d, *J* = 6.8 Hz), 7.25-7.40 (2H, m), 7.10-7.25 (2H, m), 6.90-7.10 (5H, m), 6.09 (1H, brs), 4.62 (2H, d, *J* = 5.2 Hz), 3.70-3.90 (2H, m), 3.02 (2H, q, *J* = 7.6 Hz), 2.75-2.90 (2H, m), 2.60-2.70 (1H, m), 1.80-2.05 (4H, m), 1.38 (3H, t, *J* = 7.2 Hz); LCMS: 100%, MS (ESI): m/z 525.0[M + H]+. |
| 79 | 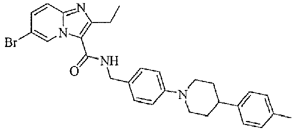 | white solid : ¹H-NMR (CDCl₃): δ 9.63 (1H, s), 7.49 (1H, d, *J* = 9.6 Hz), 7.39 (1H, d, *J* = 9.6 Hz), 7.29-7.35 (2H, m), 7.05-7.20 (4H, m), 6.90-7.05 (2H, m), 6.03 (1H, brs), 4.62 (2H, d, *J* = 5.2 Hz), 3.71-3.91 (2H, m), 2.96 (2H, q, *J* = 7.6 Hz), 2.75-2.90 (2H, m), 2.55-2.70 (1H, m), 2.33 (3H, s), 1.80-2.05 (4H, m), 1.40 (3H, t, *J* = 7.6 Hz); LCMS: 98.1%, MS (ESI): m/z 531.1 [M+ H]+. |
| 80 | 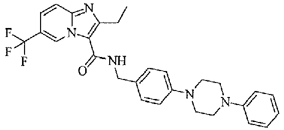 | white solid : ¹H-NMR (CDCl₃): δ 9.86 (1H, s), 7.69 (1H, d, *J* = 8.8 Hz), 7.47 (1H, d, *J* = 9.2 Hz), 7.28-7.40 (4H, m), 6.95-7.10 (4H, m), 6.87-6.95 (1H, m), 6.07 (1H, s), 4.64 (2H, d, *J* = 5.2 Hz), 3.36 (8H, s), 2.99 (2H, q, *J* = 7.6 Hz), 1.42 (3H, t, *J* = 7.2 Hz); LCMS: 100%, MS (ESI): m/z 530.1 [M+ H]+. |
| 81 | 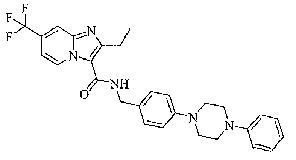 | white solid : ¹H-NMR (CDCl₃): δ 9.53 (1H, d, *J* = 7.2 Hz), 7.91 (1H, s), 7.26-7.33 (4H, m), 7.09 (2H, d, *J* = 7.6 Hz), 6.96-7.05 (4H,m), 6.89-6.92 (1H, m), 6.08 (1H, brs), 4.64 (2H, d, *J* = 5.2 Hz), 3.39-3.41 (8H, m), 3.00 (2H, q, *J* = 7.2 Hz), 1.42 (3H, t, *J* = 7.2 Hz); LCMS: 98.7%, MS (ESI): m/z 508.1 [M+ H]+. |
| 82 | 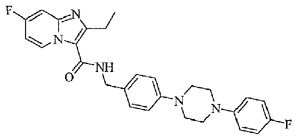 | yellow solid : ¹H-NMR (CDCl₃): δ 9.42-9.46(1H, m), 7.31-7.13(3H, m), 7.01-6.86(6H, m), 6.80-6.77(1H, m), 6.00(1H, brs), 4.62(2H, d, *J*=5.2Hz), 3.35(4H, d, *J*=4.4Hz), 3.27(4H, d, *J*=4.4Hz),2.97-2.92(2H, m), 1.39(3H, t, *J*=7.6 Hz); LCMS: 99.4%, MS (ESI): m/z 476.1[M+ H]+. |
| 83 | 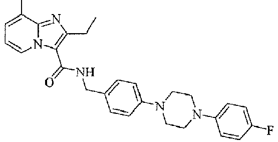 | white solid : ¹H-NMR (CDCl₃): δ 9.25 (1H, d, *J* = 7 Hz), 7.31 (2H, d, *J* = 8.3 Hz), 7.12 (1H, d, *J* = 6.3 Hz), 6.89-7.04 (6H, m), 6.82 (1H, t, *J* = 6.8 Hz), 6.03 (1H, brs), 4.63 (2H, d, *J* = 5.3 Hz), 3.20-3.44 (8H, m), 3.00 (2H, q, *J* = 7.4 Hz), 2.62 (3H, s,), 1.37 (3H, t, *J=* 7.7 Hz); LCMS: 99.5%, MS (ESI): m/z 494.1 [M+ H]+. |
| 84 | 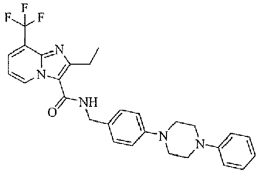 | white solid : ¹H-NMR (CDCl₃): δ 9.58 (1H, d, *J* = 6.8 Hz), 7.66 (1H, d, *J* = 6.8 Hz), 7.25-7.35 (4H, m), 6.95-7.05 (5H, m), 6.91 (1H, t, *J* = 6.8 Hz), 6.10 (1H, brs), 4.64 (2H, d, *J* = 5.2 Hz), 3.36 (8H, s), 3.03 (2H, q, *J* = 7.6 Hz), 1.38 (3H, t, *J* = 7.6 Hz); LCMS: 100%, MS (ESI): m/z 508.0[M + H]+. |
| 85 | 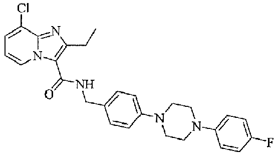 | yellow solid : ¹H-NMR (CDCl₃): δ 9.35 (1H, d, J=6.8 Hz), 7.39 (1H, d, *J* = 7.6 Hz), 7.30 (2H, d, *J* = 8 Hz), 6.89-7.19 (6H, m), 6.85 (1H, t, *J* = 7.2 Hz), 6.08 (1H, brs), 4.63 (2H, d, *J* = 5.6 Hz), 3.35 (2H, d, *J* = 5.6 Hz), 3.27 (2H, d, *J* = 5.6 Hz), 3.01 (2H, q, *J* = 7.6 Hz), 1.38 (3H, t, *J* = 7.6 Hz): LCMS:100.0%, MS (ESI): m/z 492.0[M+ H]+. |
| 86 | 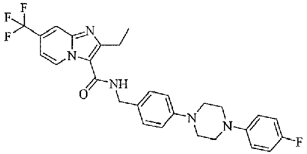 | yellow solid : ¹H-NMR (CDCl₃): δ 9.53 (1H, d, *J* = 7.2 Hz), 7.91 (1H, s), 7.45 (2H, d, *J* = 10.0 Hz), 7.09 (1H, d, *J* = 7.2 Hz), 6.94-7.00 (6H, m), 6.08 (1H, brs), 4.63 (2H, d, *J* = 4.8 Hz), 3.32-3.40 (4H, m), 3.27-3.30 (4H, m), 3.00 (2H, q, *J* = 7.6 Hz), 1.42 (3H, t, *J* = 7.6 Hz); LCMS: 98.8%, MS (ESI): m/z 526.0[M+ H]+. |
| 87 | 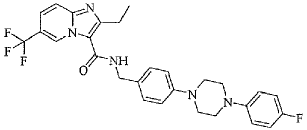 | white solid : ¹H-NMR (CDCl₃): δ 9.86 (1H, s), 7.69 (1H, d, *J* = 9.6 Hz), 7.48 (1H, d, *J* = 9.2 Hz), 7.28-7.41 (2H, m), 6.83-7.12 (6H, m), 6.08 (1H, s), 4.64 (2H, d, *J* = 5.2 Hz), 3.36 (4H, s), 3.26 (4H, s), 2.99 (2H, q, *J* = 7.6 Hz), 1.42 (3H, t, *J* = 7.6 Hz); LCMS: 99.0%, MS (ESI): m/z 526.0[M+ H]+. |
| 88 | 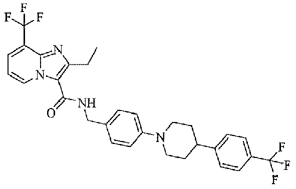 | yellow solid : ¹H-NMR (CDCl₃): δ 9.60 (1H, d, *J* = 7.2 Hz), 7.66 (1H, d, *J* = 7.2 Hz), 7.58 (2H, d, *J* = 8.0 Hz), 7.36 (2H, d, *J* = 7.6 Hz), 7.29 (2H, d, *J* = 8.0 Hz), 6.90-7.05 (3H, m), 6.10 (1H, brs), 4.63 (2H, d, *J* = 5.2 Hz), 3.75-3.90 (2H, m), 3.05 (2H, q, *J* = 7.6 Hz), 2.80-2.90 (2H, m), 2.65-2.75 (1H, m), 1.80-2.05 (4H, m), 1.38 (3H, t, *J* = 7.6 Hz); LCMS: 100%, MS (ESI): m/z 575.0[M + H]+. |
| 89 | 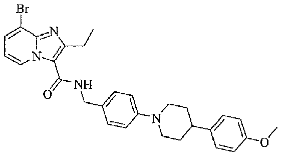 | yellow solid : ¹H-NMR (CDC13): δ 9.39 (1H, d, *J* = 7.2 Hz), 7.58 (1H, d, *J* = 7.6 Hz), 7.20-7.40 (2H, m), 7.17 (2H, d, *J* = 8.4 Hz), 6.98 (2H, d, *J* =8.0 Hz), 6.87 (2H, d, *J* = 7.2 Hz), 6.79 (1H, t, *J* = 7.2 Hz), 6.06 (1H, brs), 4.62 (2H, d, *J* = 5.2 Hz), 3.70-3.90 (5H, m), 3.01 (2H, q, *J* = 7.6 Hz), 2.83 (2H, t, *J* = 11.6 Hz), 2.61 (2H, t, *J* = 12.0 Hz), 1.80-2.00 (4H, m), 1.38 (3H, t, *J* = 7.6 Hz); LCMS: 100%, MS(ESI):m/z 569.0/571.0 [M+23] |
| 90 | 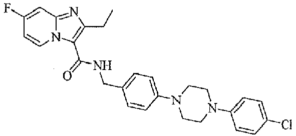 | white solid : ¹H-NMR (CDCl₃): δ 9.43(1H, t, *J*= 7.6 Hz), 7.22-7.63 (5H, m), 6.97(2H, d, *J*= 8.4 Hz), 6.89(2H, d, *J*= 8.4 Hz), 6.61-6.81(1H, m), 6.00(1H, brs), 4.61(2H, d, *J*= 4.8 Hz), 3.48-3.55(8H, m), 2.91-2.95(2H, m), 1.39(3H, d, *J*= 7.6 Hz); LCMS: 99.9%, MS (ESI): m/z 492.0[M+ H]+. |
| 91 | 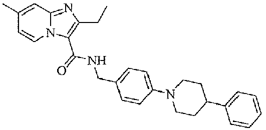 | white solid : ¹H-NMR (CDCl₃): δ 9.28 (1H, d, *J* = 6.8 Hz), 7.35-7.22 (9H, m), 6.99 (2H, d, *J* = 7.6 Hz), 6.75 (1H, d, *J* = 6.8 Hz), 5.98 (1H, brs), 4.62 (2H, d, *J* = 4.4 Hz), 3.82 (2H, d, *J* = 11.2 Hz), 2.66 (1H, brs), 2.42 (3H, s), 1.95-1.83 (4H, m), 1.40-1.30 (3H, m); LCMS: 100%, MS (ESI): m/z 453.1[M+ H]+. |
| 92 | 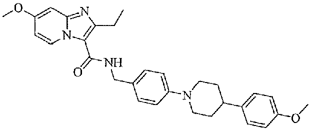 | white solid : ¹H-NMR (CDCl₃): δ 9.25 (1H, d, *J* = 7.6 Hz), 7.28 (2H, d, *J* = 8.4 Hz), 7.17 (2H, d, *J* = 8.0 Hz), 6.98 (2H, d, *J* = 8.0 Hz), 6.80-6.90 (3H, m), 6.60 (1H, d, *J* = 6.4 Hz), 5.94 (1H, brs), 4.60 (2H, d, *J* = 5.2 Hz), 3.86 (3H, s), 3.70-3.82 (5H, m), 2.92 (2H, q, *J* = 7.6 Hz), 2.82 (2H, t, *J* = 12.0 Hz), 2.55-2.70 (1H, m), 1.85-2.02 (4H, m), 1.39 (3H, t, *J* = 7.6 Hz); LCMS: 98.7%, MS (ESI): m/z 499.1 [M+H]+, 521.1 [M+Na]+. |
| 93 | 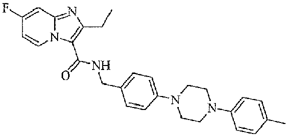 | yellow solid : ¹H-NMR (CDCl₃): δ 9.44(1H, t, *J* = 7.2 Hz), 7.30(2H, d, *J* = 8 Hz), 7.21-7.24(1H, m), 7.10(2H, d, *J*= 7.6 Hz), 6.98(2H, d, *J*= 8.4 Hz), 6.90(2H, d, *J*= 8.0 Hz), 6.77-6.81(1H, m), 5.97(1H, brs), 4.61(2H, d, *J*= 5.2 Hz), 3.19-3.46(8H, m), 2.92-2.97(2H, m), 2.89(3H, s), 1.39(3H, t, *J*= 7.2 Hz); LCMS: 99.9%, MS (ESI): m/z 494.0[M+ Na]+. |
| 94 | 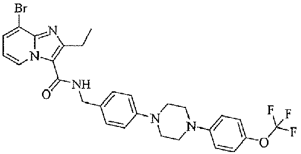 | white solid : ¹H-NMR (CDCl₃): δ 9.40 (1H, d, *J* = 6.8 Hz), 7.58 (1H, d, *J* = 7.6 Hz), 7.31 (2H, d, *J* = 8.0 Hz), 7.14 (2H, d, *J* = 8.4 Hz), 7.05 (2H, d, *J* = 8.4 Hz), 6.85-7.05 (4H, m), 6.80 (1H, t, *J* = 7.2 Hz), 6.08 (1H, brs), 4.63 (2H, d, *J* = 5.2 Hz), 3.18-3.44 (8H, m), 3.02 (2H, q, *J* = 7.6 Hz), 1.38 (3H, t, *J* = 7.6 Hz); LCMS: 98.2%, MS(ESI):m/z 601.8/603.8 [M+H] + |
| 95 | 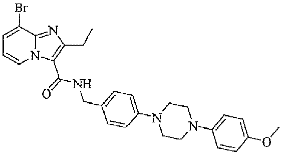 | white solid : ¹H-NMR (CDC13): δ 9.40 (1H, d, *J* = 6.8 Hz), 7.58 (1H, d, *J* = 6.8 Hz), 7.30 (2H, d, *J* = 8.4 Hz), 6.90-7.05 (4H, m), 6.87 (2H, d, *J* = 9.2 Hz), 6.79 (1H, t, *J* = 7.2 Hz), 6.07 (1H, brs), 4.63 (2H, d, *J* = 5.6 Hz), 3.96 (3H, s), 3.28-3.50 (4H, m), 3.15-3.28 (4H, m), 3.02 (2H, q, *J* = 7.6 Hz), 1.38 (3H, t, *J* = 7.6 Hz); LCMS: 100%, MS(ESI):m/z 571.6/573.6 [M+23] |
| 96 | 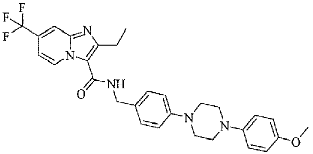 | white solid : ¹H-NMR (CDCl₃): δ 9.53 (1H, d, *J* = 7.2 Hz), 7.91 (1H, s), 7.31 (2H, d, *J* = 8.4 Hz), 7.09 (1H, d, *J* = 6.8 Hz), 6.81-7.01 (4H, m), 6.87 (2H, d, *J* = 9.2 Hz), 6.11 (1H, brs), 4.63 (2H, d, *J=* 5.6 Hz), 3.79 (3H, s), 3.35-3.40 (4H, m), 3.28-3.32 (4H, m), 3.00 (2H, q, *J* = 7.6 Hz), 1.42 (3H, t, *J* = 7.6 Hz); LCMS: 100%, MS (ESI): m/z 538.1[M+ H]+. |
| 97 | 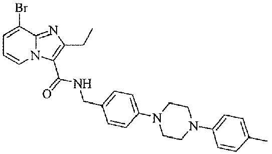 | white solid : ¹H-NMR (CDC13): δ 9.40 (1H, d, *J* = 6.8 Hz), 7.58 (1H, d, *J* = 7.2 Hz), 7.31 (2H, d, *J* = 8.8 Hz), 7.11 (2H, d, *J* = 8.8 Hz), 6.99 (2H, d, *J* = 8.8 Hz), 6.90 (2H, d, *J* = 8.8 Hz), 6.80 (1H, t, *J* = 7.2 Hz), 6.07 (1H, brs), 4.63 (2H, d, *J* = 5.2 Hz), 3.20-3.40 (8H, m), 3.02 (2H, q, *J* = 7.6 Hz), 2.29 (3H, s), 1.38 (3H, t, *J* = 7.6 Hz); LCMS: 100%, MS(ESI):m/z 532.1/534.1 [M+H]+ |
| 98 | 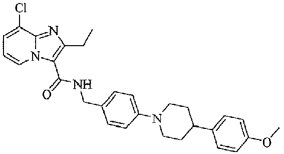 | grey solid : ¹H-NMR (CDCl₃): δ 9.34 (1H, d, *J* = 6.8 Hz) , 7.39 (1H, d, *J* = 7.2 Hz), 7.27 (2H, d, *J* = 8.4 Hz), 7.17 (2H, d, *J* = 8.4 Hz), 6.99 (2H, d, *J* = 8.4 Hz), 6.83-6.87 (3H, m), 6.07 (1H, brs), 4.62 (2H, d, *J* = 5.2 Hz), 3.80-3.82 (5H, m), 3.02 (2H, q, *J* = 7.2 Hz), 2.82 (2H, t, *J* = 11.2 Hz), 2.61 (1H, t, *J* = 11.6 Hz), 1.81-1.95 (4H, m), 1.38 (3H, t, *J* = 7.2 Hz); LCMS: 100.0%, MS (ESI): m/z 525.1[M+Na]+. |
| 99 | 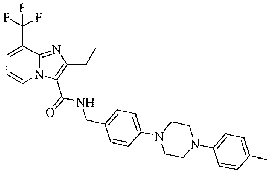 | white solid : ¹H-NMR (CDCl₃): δ 9.61 (1H, d, *J* = 6.8 Hz), 7.68 (1H, d, *J* = 6.8 Hz), 7.32 (2H, d, *J* = 8.8 Hz), 7.13 (2H, d, *J* = 8.4 Hz), 6.98-7.03 (3H, m), 6.92 (2H, d, *J* = 8.4 Hz), 6.12 (1H, brs), 4.66 (2H, d, *J* = 5.2 Hz), 3.31-3.38 (8H, m), 3.05 (2H, q, *J* = 7.6 Hz), 2.31 (3H, s), 1.40 (3H, t, *J* = 7.6 Hz); LCMS: 100%, MS (ESI): m/z 522.1[M + H]+. |
| 100 | 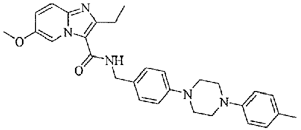 | white solid : ¹H-NMR (CDCl₃): δ 9.13 (1H, d, *J* = 2.4 Hz), 7.52 (1H, d, *J* = 9.6 Hz), 7.33 (2H, d, *J* = 8.8 Hz), 7.14 (2H, d, *J* =8.8 Hz), 7.01 (2H, d, *J* =8.4 Hz), 6.93 (2H, d, *J* = 8.4 Hz), 6.05 (1H, brs), 4.65 (2H, d, *J* = 5.6 Hz), 3.90 (3H, s), 3.25-3.40 (8H, m), 2.97 (2H, q, *J* = 7.6 Hz), 2.31 (3H, s), 1.41 (3H, t, *J* = 7.6 Hz); LCMS: 100%, MS(ESI):m/z 484.1 [M+H]+ |
| 101 | 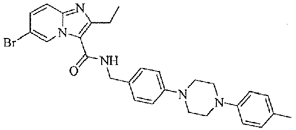 | white solid : ¹H-NMR (CDCl₃): δ 9.65 (1H, s), 7.51 (1H, d, *J* = 9.2 Hz), 7.41 (1H, d, *J* = 9.6 Hz), 7.33 (2H, d, *J* = 8.4 Hz), 7.13 (2H, d, *J* = 8.4 Hz), 7.01 (2H, d, *J* = 8.4 Hz), 6.93 (2H, d, *J* = 8.4 Hz), 6.07 (1H, brs), 4.64 (2H, d, *J* = 5.2 Hz), 3.26-3.42 (8H, m), 2.98 (2H, q, *J* = 7.6 Hz), 1.42 (3H, t, *J* = 7.6 Hz); LCMS: 100%, MS (ESI): m/z 534.0[M+ H]+. |
| 102 | 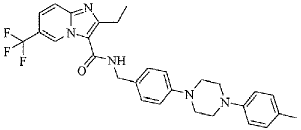 | yellow solid : ¹H-NMR (CDCl₃): δ 9.88 (1H, s), 7.71 (1H, d, *J* = 9.2 Hz), 7.49 (1H, dd, *J* = 7.6, 2.0 Hz), 7.33 (2H, d, *J* = 8.4 Hz), 7.13 (2H, d, *J* = 8.0 Hz), 7.01 (2H, d, *J* = 8.4 Hz), 6.93 (2H, d, *J* = 4.8 Hz), 6.09 (1H, s), 4.66 (2H, d, *J* = 5.2 Hz), 3.35-3.45 (4H, m), 3.25-3.35 (4H, m), 3.01 (2H, q, *J* = 7.6 Hz), 2.31 (3H, s), 1.43 (3H, t, *J* = 7.6 Hz); LCMS: 100%, MS (ESI): m/z 522.1 [M+ H]+. |
| 103 | 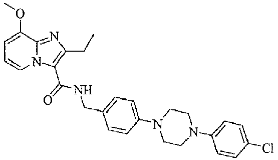 | white solid : ¹H-NMR (CDCl₃): δ 9.01 (1H, d, *J* = 6.8 Hz), 7.34 (2H, d, *J* = 8.4 Hz), 7.26 (2H, d, *J* = 8.8 Hz), 7.00 (2H, d, *J* = 8.4 Hz), 6.92 (2H, d, *J* = 9.2 Hz), 6.83 (1H, t, *J* = 7.4 Hz), 6.64 (1H, d, *J* = 7.6 Hz), 6.07 (1H, brs), 4.65 (2H, d, *J=* 5.6 Hz), 4.04 (3H, s). 3.36 (4H, d, *J* = 5.6 Hz), 3.33 (4H, d, *J* = 6.0 Hz), 3.00 (2H, q, *J* = 7.6 Hz), 1.40 (3H, t, *J* = 7.6 Hz); LCMS: 100%, MS (ESI): m/z 526.1[M+ Na]+. |
| 104 | 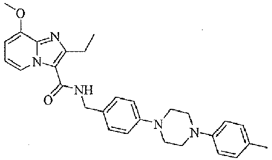 | yellow solid : ¹H-NMR (CDC13): δ 8.99 (1H, dd, *J* = 0.8 Hz, *J* = 7.2 Hz), 7.31 (2H, d, *J* = 8.8 Hz), 7.11 (2H, d, *J* = 8.0 Hz), 6.98 (2H, d, *J* = 8.8 Hz), 6.93 (2H, d, *J* = 10.0 Hz), 6.81 (1H, t, *J* = 7.4 Hz), 6.62 (1H, d, *J* = 7.2 Hz), 6.04 (1H, brs), 4.62 (2H, d, *J* = 5.2 Hz), 4.02 (3H, s). 3.28-3.84 (8H, m), 2.98 (2H, q, *J* = 7.6 Hz), 2.29 (3H, s), 1.38 (3H, t, *J* = 7.6 Hz); LCMS: 100%, MS (ESI): m/z 484.1 [M+ H]+. |
| 105 | 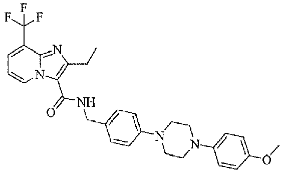 | white solid : ¹H-NMR (CDCl₃): δ 9.59 (1H, d, *J* = 7.2 Hz), 7.66 (1H, d, *J* = 6.8 Hz), 7.30 (2H, d, *J* = 8.4 Hz), 6.94-7.06 (5H, m), 6.85-6.90 (2H, m), 6.13 (1H, brs), 4.63 (2H, d, *J* = 5.6 Hz), 3.79 (3H, s), 3.34-3.37 (4H, m), 3.19-3.30 (4H, m), 3.03 (2H, q, *J* = 7.6 Hz), 1.38 (3H, t, *J* = 7.6 Hz); LCMS: 99.4%, MS (ESI): m/z 538.1 [M + H]+. |
| 106 | 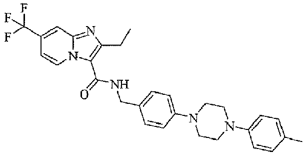 | white solid : ¹H-NMR (CDCl₃): δ 9.55 (1H, d, *J* = 7.2 Hz), 7.93 (1H, s), 7.33 (2H, d, *J* = 8.4 Hz), 7.10-7.15 (3H, m), 7.01 (2H, d, *J* = 8.4 Hz), 6.92 (2H, d, *J* = 8.4 Hz), 6.10 (1H, brs), 4.65 (2H, d, *J* = 5.6 Hz), 3.36-3.40 (4H, m), 3.27-3.38 (4H, m), 3.01 (2H, q, *J* = 7.6 Hz), 2.31 (3H, s), 1.44 (3H, t, *J* = 7.6 Hz); LCMS: 100%, MS (ESI): m/z 522.0[M+ H]+. |
| 107 | 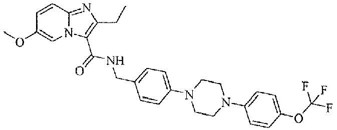 | white solid : ¹H-NMR (CDCl3): δ 9.13 (1H, d, *J* = 2.4 Hz), 7.52 (1H, d, *J* =9.6 Hz), 7.34 (2H, d, *J* = 8.8 Hz), 7.10-7.20 (3H, m), 6.92-7.04 (4H, m), 6.06 (1H, brs), 4.66 (2H, d, *J* = 5.6 Hz), 3.90 (3H, s), 3.26-3.40 (8H, m), 2.98 (2H, q, *J* = 7.6 Hz), 1.42 (3H, t, *J* = 7.6 Hz); LCMS: 98.0%, MS(ESI):m/z 554.1 [M+H]⁺ |
| 108 | 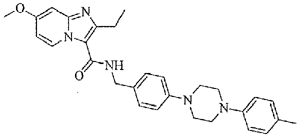 | yellow solid : ¹H-NMR (CDCl₃): δ 9.25 (1H, d, *J* = 7.6 Hz), 7.31 (2H, d, *J* = 8.4 Hz), 7.11 (2H, d, *J* = 8.4 Hz),6.99 (2H, d, *J* = 8.4 Hz), 6.89-6.96 (3H, m), 6.61 (1H, dd, *J* = 7.6 Hz,2.4 Hz), 5.95 (1H, brs), 4.62 (2H, d, *J* = 5.6 Hz), 3.87 (3H, s), 3.52-3.71 (8H, m), 2.92 (2H, q, *J* = 7.6 Hz), 2.29 (3H, s), 1.39 (3H, t, *J* = 7.6 Hz); LCMS: 100.0%, MS (ESI): m/z 484.1[M+H]+, 506.1 [M+Na]+. |
| 109 | 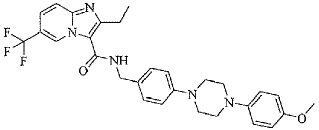 | white solid : ¹H-NMR (CDCl₃): δ 9.88 (1H, s), 7.72 (1H, d, *J* = 9.6 Hz), 7.50 (1H, dd, *J* = 8.0, 1.6 Hz), 7.33 (2H, d, *J* = 8.4 Hz), 6.95-7.08 (4H, m), 6.89 (2H, d, *J* = 9.2 Hz), 6.10 (1H, s), 4.66 (2H, d, *J =* 5.6 Hz), 3.81 (3H, s), 3.35-3.52 (4H, m), 3.21-3.30 (4H, m), 3.01 (2H, q, *J* = 7.6 Hz), 1.44 (3H, t, *J* = 7.6 Hz); LCMS: 100%, MS (ESI): m/z 538.1[M+ H]+. |
| 110 | 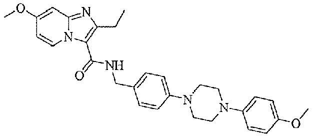 | white solid : ¹H-NMR (CDCl₃): δ 9.27 (1H, d, *J* = 7.6 Hz), 7.32 (2H, d, *J* = 8.4 Hz), 6.95-7.04 (4H, m), 6.85-6.94 (3H, m), 6.32 (1H, dd, *J* = 7.6, 2.4 Hz), 5.97 (1H, t, *J* = 4.4 Hz), 4.63 (2H, d, *J* = 5.6 Hz), 3.89 (3H, s), 3.81 (3H, s), 3.30-3.40 (4H, m), 3.20-3.29 (4H, m), 2.94 (2H, q, *J* = 7.6 Hz), 1.41 (3H, t, *J* = 7.6 Hz); LCMS: 99.0%, MS (ESI): m/z 522.1[M+ Na]+. |
| 111 | 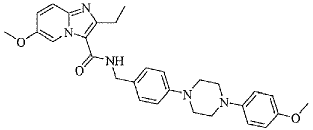 | white solid : ¹H-NMR (CDCl₃): δ 9.13 (1H, d, *J* = 2.0 Hz), 7.51 (1H, d, *J* =9.6 Hz), 7.33 (2H, d, *J* = 8.8 Hz), 7.12 (2H, dd, *J* =9.6, 2.4 Hz), 6.92-7.08 (4H, m), 6.89 (2H, d, *J* =9.2 Hz), 6.05 (1H, brs), 4.65 (2H, d, *J=* 5.2 Hz), 3.89 (3H, s), 3.30-3.40 (4H, m), 3.20-3.30 (4H, m), 2.97 (2H, q, *J* = 7.6 Hz), 1.41 (3H, t, *J* = 7.6 Hz); LCMS: 100%, MS(ESI):m/z 522.1 [M+23]. |
| 112 | 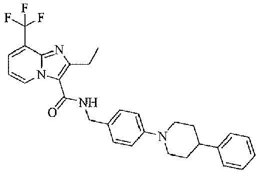 | white solid : ¹H-NMR (CDCl₃): δ 9.61 (1H, d, *J* = 6.8 Hz), 7.68 (1H, d, *J* = 7.2 Hz), 7.20-7.40 (7H, m), 6.95-7.10 (3H, m), 6.12 (1H, brs), 4.65 (2H, d, *J* = 5.6 Hz), 3.80-3.90 (2H, m), 3.06 (2H, q, *J* = 7.6 Hz), 2.80-2.90 (2H, m), 2.60-2.75 (1H, m), 1.85-2.05 (4H, m), 1.40 (3H, t, *J* = 7.6 Hz); LCMS: 100%, MS (ESI): m/z 507.0[M + H]+. |
| 113 | 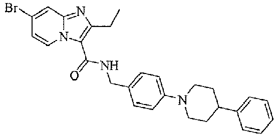 | yellow solid : H-NMR (CDCl₃): δ 9.29 (1H, d, *J* = 7.6 Hz), 7.76 (1H, d, *J* = 2.0 Hz ), 7.15-7.36 (7H, m), 6.93-7.05 (3H, m), 6.05 (1H, brs), 4.60 (2H, d, *J* = 5.6 Hz), 4.52-4.63 (2H. m), 2.94 (2H, q, *J* = 7.6 Hz), 2.77-2.89 (2H, m), 2.60-2.71 (1H. m), 1.81-2.02 (4H. m), 1.38 (3H, t, *J* = 7.6 Hz): LCMS: 98.3%, MS (ESI): m/z 517.0[M+ H]+. |
| 114 | 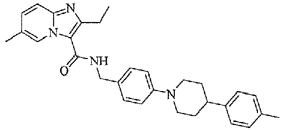 | white solid : ¹H-NMR (CDCl₃): δ 9.16 - 9.27 (1 H, m), 7.44 - 7.54 (1 H, m), 7.23 - 7.31 (3 H, m), 7.09 - 7.20 (4 H, m), 6.98 (2 H, d, *J*=8.60 Hz), 5.98 (1 H, br. s.), 4.61 (2 H, d, *J*=5.51 Hz), 3.80 (2 H, d, *J*=12.35 Hz), 2.95 (2 H, d, *J*=7.72 Hz), 2.77 - 2.86 (2 H, m), 2.62 (1 H, s), 2.34 (6 H, d, *J*=12.35 Hz), 1.81 - 1.98 (4 H, m), 1.38 (3 H, t, *J*=7.50 Hz,); LCMS: 98.0%, MS (ESI): m/z 453.0[M+ H]+. |
| 115 | 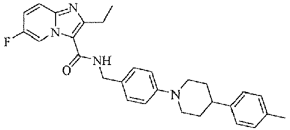 | yellow solid : ¹H-NMR (CDCl₃): δ 9.45 (1H, dd, *J* = 5.2, 2.4 Hz), 7.55 (1H, dd, *J* = 10.0, 5.2 Hz), 7.20-7.30 (3H, m, overlap with CDCl₃ signa), 7.10-7.20 (4H, m), 6.98 (2H, d, *J* = 8.8 Hz), 6.01 (1H, brs), 4.55-4.65 (2H, m), 3.81 (2H, d, *J* = 12.0 Hz), 2.96 (2H, q, *J* = 7.6 Hz), 2.82 (2H, td, *J* = 12.0, 2.8 Hz), 2.60-2.70 (1H, m), 2.32 (3H, s), 1.80-2.00 (4H, m), 1.39 (3H, t, *J* = 7.6 Hz); LCMS: 98.7%, MS (ESI): m/z 493.0[M+Na]+. |
| 116 | 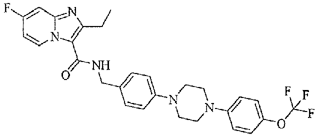 | white solid : ¹H-NMR (CDCl₃): δ 9.41-9.45(1H, m), 7.29(2H, d, *J*= 8.4 Hz), 7.19-7.22(1H, m), 7.12(2H, d, *J*= 8.4 Hz), 6.92-6.98(5H, m), 6.75-6.80(1H, m), 5.99(1H, brs), 4.60(2H, d, *J*=5.2Hz), 3.30-3.35(8H, m), 2.91-2.97(2H, m), 1.38(3H, t, *J*= 7.6 Hz); LCMS: 98.1%, MS (ESI): m/z 542.1[M+ H]+. |
| 117 | 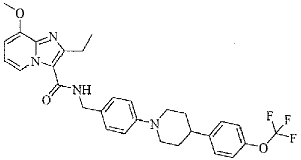 | red solid: ¹H-NMR (CDCl₃): δ 8.98 (1H, dd, *J*= 0.8 Hz, *J* = 6.8 Hz), 7.24-7.29 (4H, m), 7.15 (2H, d, *J* = 8.0 Hz), 6.97 (2H, d, *J* = 8.4 Hz), 6.80 (1H, t, *J* = 6.2 Hz), 6.60 (1H, d, *J* = 7.6 Hz), 6.03 (1H, brs), 4.61 (2H, d, *J* = 5.2 Hz), 4.01 (3H, s). 3.81 (2H, d, *J* = 12.4 Hz), 2.97 (2H, q, *J* = 7.6 Hz), 2.82 (2H, t, *J* = 11.4 Hz), 2.62-2.69 (1H, m), 1.82-1.96 (4H, m), 1.37 (3H, t, *J* = 7.6 Hz); LCMS: 100%, MS (ESI): m/z 553.1[M+ H]+. |
| 118 | 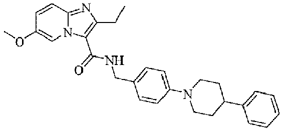 | pink solid: ¹H-NMR (CDCl₃): δ 9.10 (1H, d, *J* = 1.6 Hz), 7.48 (1H, d, *J* = 9.6 Hz), 7.17-7.35 (7H, m), 7.08 (1H, d, *J* = 7.2 Hz), 6.98 (2H, d, *J* = 8.4 Hz), 6.02 (1H, brs), 4.62 (2H, d, *J* = 5.2 Hz), 3.86 (3H, s), 3.82 (2H, d, *J* = 12.4 Hz), 2.94 (2H, q, *J* = 7.6 Hz), 2.83 (2H, t, *J* = 12.0 Hz), 2.60-2.70 (1H, m), 1.80-2.00 (4H, m), 1.38 (3H, t, *J* = 7.6 Hz); LCMS: 100%, MS(ESI):m/z 469.1 [M+H]⁺ |
| 119 | 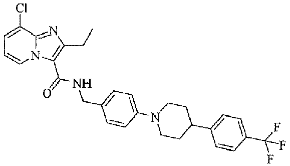 | red solid: ¹H-NMR (CDCl₃): δ 9.34 (1H, d, *J* = 6.8 Hz), 7.57 (2H, d, *J* = 8 Hz), 7.34-7.39 (3H, m), 7.28 (2H, d, *J* = 8. 8 Hz), 6.98 (2H, d, *J* = 8.4 Hz), 6.85 (1H, t, *J* = 7.2 Hz), 6.06 (1H, brs), 4.62 (2H, d, *J* = 5.6 Hz), 3.82 (2H, d, *J* = 12.0 Hz), 3.01 (2H, q, *J* = 7.6 Hz), 2.84 (2H, td, *J* = 2.8, *J* = 12.4 Hz), 2.68-2.76 (1H, m), 1.84-2.00 (4H, m), 1.38 (3H, t, *J* = 7.6 Hz); LCMS: 98.5%, MS (ESI): m/z 541.1 [M+ H]+. |
| 120 | 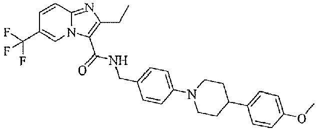 | yellow solid: ¹H-NMR (CDCl₃): δ 9.70-9.80 (1H, m), 7.68 (1H, d, *J* = 8.8 Hz), 7.46 (1H, dd, *J* = 7.6, 2.0 Hz), 7.26 (2H, d, *J* = 8.8 Hz), 7.16 (2H, dd, *J* = 4.8 2.0 Hz), 6.98 (2H, d, *J* = 8.8 Hz), 6.86 (2H, dd, *J* = 4.8 2.0 Hz), 6.06 (1H, m), 4.62 (2H, d, *J* = 5.2 Hz), 3.82 (2H, s), 3.79 (3H, s), 2.99 (2H, q, *J* = 7.6 Hz), 2.75-2.89 (2H, m), 2.53-2.69 (1H, m), 1.78-2.01 (4H, m), 1.40 (3H, t, *J* = 7.6 Hz); LCMS: 100%, MS (ESI): m/z 537.0[M+ H]+. |
| 121 | 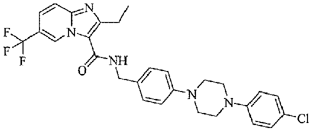 | white solid: ¹H-NMR (CDCl₃): δ 9.85 (1H, s), 7.69 (1H, d, *J* = 5.2 Hz), 7.47 (1H, dd, *J* = 7.6, 2.0 Hz), 7.30 (2H, d, *J* = 8.0 Hz), 7.18-7.24 (2H, m), 6.98 (2H, d, *J* = 8.4 Hz), 6.88 (2H, d, *J* = 5.2 Hz), 6.02-6.11 (1H, m), 4.63 (2H, d, *J* = 5.6 Hz), 3.30-3.35 (8H, m), 2.99 (2H, q, *J* = 8.4 Hz), 1.41 (3H, t, *J* = 7.6 Hz); LCMS: 100%, MS (ESI): m/z 541.8[M+ H]+. |
| 122 | 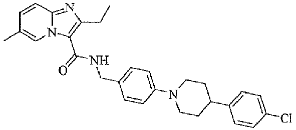 | red solid : ¹H-NMR (CDCl₃): δ 9.15 - 9.27 (1 H, m), 7.49 (1 H, d, *J*=9.04 Hz), 7.22 - 7.33 (4 H, m), 7.17 (3 H, d, *J*=8.38 Hz), 6.97 (2 H, d, *J*=8.60 Hz), 5.94 - 6.06 (1 H, m), 4.61 (2 H, d, *J*=5.51 Hz), 3.80 (2 H, d, *J*=12.35 Hz), 2.95 (2 H, d, *J*=7.50 Hz), 2.81 (2 H, d, *J*=2.43 Hz), 2.57 - 2.69 (1 H, m), 2.35 (3 H, s), 1.92 (2 H, br. s.), 1.85 (2 H, dd, *J*=12.46, 3.42 Hz), 1.38 (3 H, t, *J*=7.50 Hz); LCMS: 100%, MS (ESI): m/z 487.0[M+ H]+. |
| 123 | 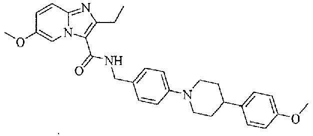 | pink solid : ¹H-NMR (CDCl3): δ 9.13 (1H, d, *J* = 2.0 Hz), 7.52 (1H, d, *J* = 9.6 Hz), 7.31 (2H, d, *J* = 8.4 Hz), 7.20 (2H, d, *J* = 8.4 Hz), 7.13 (1H, dd, *J* = 9.6, 2.4 Hz), 7.01 (2H, d, *J* = 8.8 Hz), 6.89 (2H, d, *J* = 8.8 Hz), 6.05 (1H, brs), 4.65 (2H, d, *J* = 5.2 Hz), 3.90 (3H, s), 3.76-3.88 (5H, m), 2.97 (2H, q, *J* = 7.6 Hz), 2.87 (2H, td, *J* = 12.0, 2.4 Hz), 2.60-2.70 (1H, m), 1.80-2.00 (4H, m), 1.41 (3H, t, *J* = 7.6 Hz); LCMS: 100%, MS(ESI):m/z 521.1 [M+23] |
| 124 | 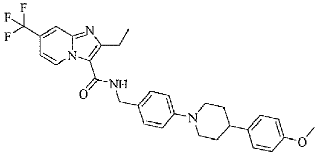 | white solid : ¹H-NMR (CDCl₃): δ 9.55 (1H, d, *J* = 7.2 Hz), 7.93 (1H, s), 7.30-7.32 (2H, m), 7.19 (2H, d, *J* = 8.4 Hz), 7.10 (1H, dd, *J* = 7.2, 1.6 Hz), 7.01 (2H, d, *J* = 8.8 Hz), 6.89 (2H, d, *J* = 8.4 Hz), 6.09 (1H, brs), 4.65 (2H, d, *J* = 5.6 Hz), 3.86 (2H, s), 3.82 (3H, s), 3.01 (2H, q, *J* = 7.6 Hz), 2.81-2.89 (2H, m), 2.61-2.67 (1H, m), 1.86-2.00 (4H, m), 1.44 (3H, t, *J* = 7.6 Hz); LCMS: 99.1%, MS (ESI): m/z 537.0[M+ H]+. |
| 125 | 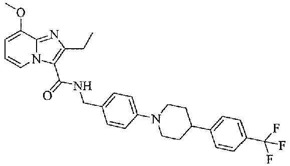 | pink solid : ¹H-NMR (CDCl3): δ 9.02 (1H, d, *J* = 6.8 Hz), 7.61 (2H, d, *J* = 8.0 Hz), 7.38 (2H, d, *J* = 8.0 Hz), 7.31 (2H, d, *J* = 8.8 Hz), 7.00 (2H, d, *J* = 8.8 Hz), 6.83 (1 H, t, *J* = 7.6 Hz), 6.63 (1H, d, *J* = 7.6 Hz), 6.07 (1H, brs), 4.64 (2H, d, *J* = 5.6 Hz), 4.04 (3H, s), 3.85 (2H, d, *J* = 12.4 Hz), 3.00 (2H, q, *J* = 7.6 Hz), 2.86 (2H, dt, *J* = 2.8 Hz, *J* = 12 Hz), 2.73 (1H, m), 1.87-2.00 (4H, m), 1.41 (3H, t, *J*= 7.6 Hz); LCMS: 99.1%, MS (ESI): m/z 537.1[M+ H]+. |
| 126 | 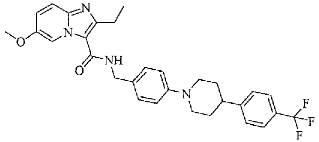 | red solid : ¹H-NMR (CDC13): δ 9.14 (1H, d, *J* = 2.0 Hz), 7.60 (1H, d, *J* = 8.4 Hz), 7.53 (2H, d, *J* = 9.6 Hz), 7.39 (2H, d, *J* = 8.4 Hz), 7.32 (2H, d, *J* = 8.4 Hz), 7.15 (2H, d, *J* = 9.6 Hz), 7.02 (2H, d, *J* = 8.8 Hz), 6.05 (1H, brs), 4.65 (2H, d, *J* = 5.6 Hz), 3.90 (3H, s), 3.86 (2H, d, *J* = 12.4 Hz), 2.98 (2H, q, *J* = 7.6 Hz), 2.87 (2H, td, *J* = 12.0, 2.4 Hz), 2.70-2.80 (1H, m), 1.88-2.05 (4H, m), 1.42 (3H, t, *J* = 7.6 Hz); LCMS: 98.40%, MS(ESI):m/z 537.1 [M+H]⁺ |
| 127 | 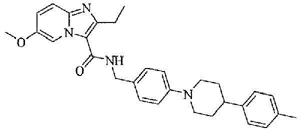 | white solid : ¹H-NMR (CDCl3): δ 9.13 (1H, d, *J* = 2.4 Hz), 7.52 (1H, d, *J* = 9.6 Hz), 7.31 (2H, d, *J* = 8.8 Hz),7.08-7.21 (5H, m), 7.02 (2H, d, *J* = 8.8 Hz), 6.04 (1H, brs), 4.65 (2H, d, *J* = 5.2 Hz), 3.90 (3H, s), 3.85 (2H, d, *J* = 12.0 Hz), 2.98 (2H, q, *J* = 7.6 Hz), 2.85 (2H, td, *J* = 12.0, 2.8 Hz), 2.61-2.70 (1H, m), 2.36 (3H, m), 1.80-2.00 (4H, m), 1.39 (3H, t, *J* = 7.6 Hz); LCMS: 98.40%, MS(ESI):m/z 483.1 [M+H]⁺ |
| 128 | 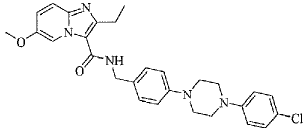 | gray solid: ¹H-NMR (CDCl₃): δ 9.13 (1H, d, *J* = 2.4 Hz), 7.51 (1H, d, *J* = 9.6 Hz), 7.33 (2H, d, *J=* 8.8 Hz), 7.26 (2H, d, *J* = 9.2 Hz), 7.13 (1H, dd, *J* = 9.6, 2.4 Hz), 7.01 (2H, d, *J* = 7.6 Hz), 6.92 (2H, d, *J* = 8.8 Hz), 6.05 (1H, brs), 4.66 (2H, d, *J=* 5.6 Hz), 3.90 (3H, s), 3.25-3.40 (8H, m), 2.98 (2H, q, *J* = 7.6 Hz), 1.41 (3H, t, *J* = 7.6 Hz); LCMS: 100%, MS(ESI):m/z 504.1 [M+H]⁺ |
| 129 | 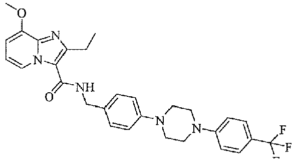 | yellow solid: ¹H-NMR (CDCl3): δ 9.01 (1H, d, *J* = 6.8 Hz), 7.53 (2H, d, *J* = 8.8 Hz), 7.34 (2H, d, *J* = 8.4 Hz), 7.00 (4H, d, *J* = 8.4 Hz), 6.83 (1H, t, *J* = 7.4 Hz), 6.64 (1H, d, *J* = 7.6 Hz), 6.08 (1H, brs), 4.65 (2H, d, *J* = 5.6 Hz), 4.04 (3H, s). 3.36-3.47 (8H, m), 3.01 (2H, q, *J* = 7.6 Hz), 1.41 (3H, t, *J* = 7.6 Hz); LCMS: 99.0%, MS (ESI): m/z 538.0[M+ H]+. |
| 130 | 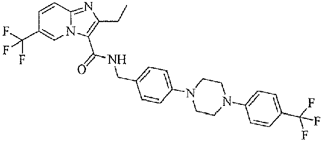 | white solid: ¹H-NMR (CDCl₃): δ 9.88 (1H, s), 7.72 (1H, d, *J* = 9.2 Hz), 7.48-7.59 (3H, m), 7.34 (2H, d, *J* = 8.4 Hz), 6.95-7.08 (4H, m), 6.10 (1H, s), 4.67 (2H, d, *J* = 5.6 Hz), 3.41-3.52 (4H, m), 3.32-3.51 (4H, m), 3.02 (2H, q, *J* = 7.2 Hz), 1.44 (3H, t, *J* = 7.6 Hz); LCMS: 98.8%, MS (ESI): m/z 576.0[M+ H]+. |
| 131 | 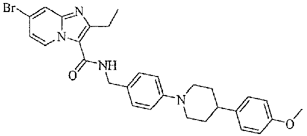 | white solid: ¹H-NMR (CDCl₃): δ 9.32 (1H, d, *J* = 7.2 Hz), 7.79 (1H, d, *J* = 1.2 Hz ), 7.23-7.34 (2H, m), 6.95-7.08 (3H, m), 6.89 (2H, d, *J* = 8.8 Hz), 6.04 (1H, brs), 4.63 (2H, d, *J* = 5.2 Hz), 3.78-3.89 (5H. m), 2.97 (2H, q, *J* = 7.6 Hz), 2.79-2.90 (2H, m), 2.56-2.69 (1H. m), 1.80-2.02 (4H. m), 1.41 (3H, t, *J* = 7.6 Hz); LCMS: 98.5%, MS (ESI): m/z 517.0[M+ H]+. |
| 132 | 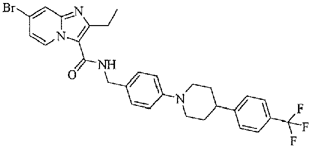 | red solid : ¹H-NMR (CDCl₃): δ 9.33 (1H, d, *J* = 7.6 Hz), 7.80 (1H, d, *J* = 1.2 Hz), 7.60 (2H, d, *J* = 8.0 Hz), 7.39 (2H, d, *J* = 8.0 Hz), 7.29-7.34 (2H, m), 6.97-7.09 (3H, m), 6.04 (1H, brs), 4.64 (2H, d, *J* = 5.2 Hz), 4.56-4.68 (2H. m), 2.98 (2H, q, *J* = 7.6 Hz), 2.80-2.93 (2H, m), 2.68-2.80 (1H. m), 1.83-2.02 (4H. m), 1.41 (3H, t, *J* = 7.6 Hz); LCMS: 98.2%, MS (ESI): m/z 585.0[M+ H]+. |
| 133 | 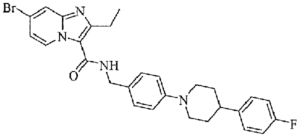 | yellow solid: ¹H-NMR (CDCl₃): δ 9.32 (1H, d, *J* = 7.2 Hz), 7.80 (1H, d, *J* = 1.6 Hz), 7.28-7.37 (2H, m), 7.20-7.26 (2H, m), 6.94-7.09 (5H. m), 6.04 (1H, brs), 4.63 (2H, d, *J* = 5.6 Hz), 4.57-4.66 (2H. m), 2.98 (2H, q, *J* = 7.6 Hz), 2.79-2.90 (2H, m), 2.59-2.73 (1H. m), 1.80-2.01 (4H. m), 1.41 (3H, t, *J* = 7.6 Hz) |
| 134 | 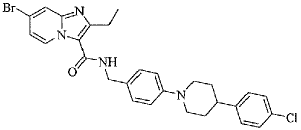 | gray solid: ¹H-NMR (CDCl₃): δ 9.33 (1H, d, *J* = 7.6 Hz), 7.80 (1H, d, *J* = 1.6 Hz), 7.28-7.35 (6H, m), 7.17-7.25 (2H, m), 6.97-7.08 (3H. m), 6.04 (1H, brs), 4.63 (2H, d, *J* = 5.6 Hz), 4.57-4.68 (2H. m), 2.98 (2H, q, *J* = 7.6 Hz), 2.78-2.89 (2H, m), 2.60-2.71 (1H. m), 1.81-2.00 (4H. m), 1.41 (3H, t, *J* = 7.6 Hz); LCMS: 98.4%, MS (ESI): m/z 552.6[M+ H]+. |
| 135 | 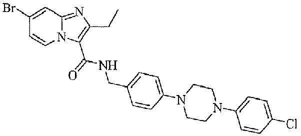 | white solid: ¹H-NMR (CDCl₃): δ 9.33 (1H, d, *J* = 7.6 Hz), 7.80 (1H, d, *J* = 1.2 Hz), 7.33 (2H, d, *J* = 8.8 Hz), 7.21-7.31 (2H, m), 6.96-7.08 (3H, m), 6.88-6.95 (2H, m), 6.05 (1H, brs), 4.64 (2H, d, *J* = 5.2 Hz), 3.27-3.41 (8H, m), 2.98 (2H, q, *J* = 7.6 Hz), 1.41 (3H, t, *J* = 7.6 Hz); LCMS: 99.0%, MS (ESI): m/z 553.6[M+ H]+. |
| 136 | 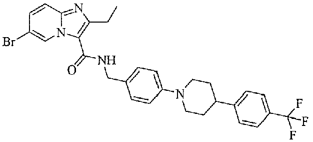 | yellow solid : ¹H-NMR (CDCl₃): δ 9.66 (1H, d, *J* = 1.2 Hz), 7.60 (2H, d, *J* = 8.0 Hz), 7.52 (1H, d, *J* = 9.2 Hz), 7.35-7.45 (3H, m), 7.29-7.35 (2H, m), 7.02 (2H, d, *J* = 8.4 Hz), 6.06 (1H, brs), 4.65 (2H, d, *J* = 5.6 Hz), 3.80-3.91 (2H, m), 2.99 (2H, q, *J* = 7.6 Hz), 2.81-2.91 (2H, m), 2.70-2.80 (1H, m), 1.86-2.06 (4H, m), 1.42 (3H, t, *J* = 7.6 Hz); LCMS: 98.9%, MS (ESI): m/z 585.0[M+ H]+. |
| 137 | 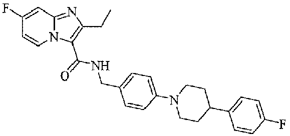 | yellow solid : ¹H-NMR (CDCl₃): δ 9.43(1H, t, *J*= 6.4 Hz), 7.19-7.30(5H, m), 6.99-7.02(4H, m), 6.75-6.81(1H, m), 5.99(1H, brs), 4.61(2H, d, *J*= 5.6 Hz), 3.73-3.83(2H, m), 2.92-2.98(2H, m), 2.80-2.86(2H, m), 2.62-2.68(1H, m), 1.78-1.96(4H, m), 1.39(3H, t, *J*=7.6 Hz); LCMS: 99.9%, MS (ESI): m/z 475.0[M+ H]+. |
| 138 | 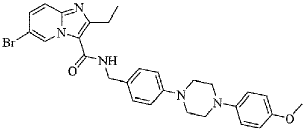 | gray solid : ¹H-NMR (CDCl₃): δ 9.63 (1H, s), 7.49 (1H, d, *J* = 9.2 Hz ), 7.39 (1H, d, *J* = 9.6 Hz), 7.31 (2H, d, *J* = 8.4 Hz), 6.90-7.17 (4H, m), 6.87 (2H, d, *J* = 8.8 Hz), 6.03 (1H, brs), 4.62 (2H, d, *J=* 5.2 Hz), 3.78 (3H, s), 3.14-3.45 (8H. m), 2.96 (2H, q, *J* = 7.6 Hz), 1.40 (3H, t, *J* = 7.6 Hz); LCMS: 100%, MS (ESI): m/z 548.0[M+ H]+. |
| 139 | 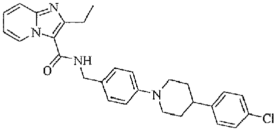 | yellow solid : ¹H-NMR (CDCl₃): δ 9.41 (1H, d, *J* = 7.2 Hz), 7.61 (1H, d, *J* = 8.8 Hz), 7.20-7.40 (5H, m, overlap with CDCl₃ signal), 7.18 (2H, d, *J* = 8.4 Hz), 6.82-7.05 (3H, m), 6.01 (1H, brs), 4.63 (2H, d, *J* = 5.6 Hz), 3.82 (2H, d, *J* = 12.0 Hz), 2.98 (2H, q, *J* = 7.6 Hz), 2.83 (2H, t, *J* = 11.2 Hz), 2.53-2.70 (1H, m), 1.72-2.03 (4H, m), 1.41 (3H, t, *J* = 7.6 Hz); LCMS: 100%, MS (ESI): m/z 473.0[M+ H]+. |
| 140 | 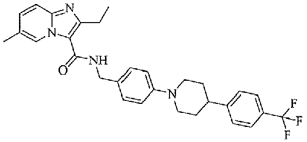 | yellow solid : ¹H-NMR (CDCl₃): δ 9.16 - 9.29 (m, 1 H), 7.58 (br. s., 3 H), 7.12 - 7.41 (m, 6 H) 6.99 (br. s, 2 H), 5.94 - 6.10 (m, 1 H), 4.62 (br. s, 2 H), 3.83 (d, *J*=10.54 Hz, 2 H), 2.95 (d, *J*=6.53 Hz, 2 H), 2.84 (br. s, 2 H), 2.72 (br. s, 1 H), 2.36 (d, *J*=4.27 Hz, 3 H), 1.85 - 2.04 (m, 4 H), 1.31 - 1.47 (m, 3 H); LCMS: 99.1%, MS (ESI): m/z 521.0[M+ H]+. |
| 141 | 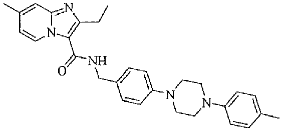 | white solid : ¹H-NMR (CDCl₃): δ 9.28 (1H, d, *J* = 7.2 Hz), 7.35-7.29 (3H, m), 7.10 (2H, d, *J* = 8.0 Hz), 6.98 (2H, d, *J* = 8.4 Hz), 6.75 (2H, d, *J* = 6.8 Hz), 5.99 (1H, brs), 4.62 (2H, d, *J* = 5.6 Hz), 3.35-3.29 (8H, m), 2.95 (2H, q, *J* = 7.6 Hz), 2.42 (3H, s), 2.29 (3H, s), 1.39 (3H, t, *J* = 7.6 Hz); LCMS: 100%, MS (ESI): m/z 490.1 [M+ H]+. |
| 142 | 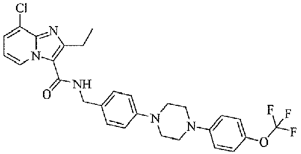 | white solid : ¹H-NMR (CDCl₃): δ 9.35 (1H, d, *J* = 6.4 Hz), 7.40 (1H, d, *J* = 7.2 Hz), 7.31 (2H, d, *J* = 6.8 Hz), 7.14 (2H, d, *J* = 7.2 Hz), 6.94-7.00 (4H, m), 6.86-6.88 (1H, m), 6.08 (1H, brs), 4.64 (2H, s), 3.34 (8H, s), 3.02 (2H, d, *J* = 7.2 Hz), 1.39 (3H, t, *J* = 7.6 Hz); LCMS: 97.9%, MS (ESI): m/z 474.0[M+ H]+. |
| 143 | 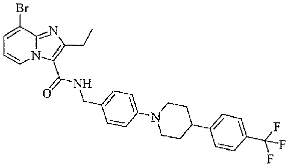 | yellow solid : ¹H-NMR (CDCl₃): δ 9.40 (1H, d, *J* = 6.8 Hz), 7.45-7.65 (3H, m), 7.30-7.45 (2H, m), 7.20-7.30 (2H, m), 7.00 (2H, d, *J* = *7.6* Hz), 6.80 (1H, t, *J* = 7.2 Hz), 6.07 (1H, brs), 4.62 (2H, d, *J* = 5.2 Hz), 3.83 (2H, d, *J* = 12.0 Hz), 3.02 (2H, q, *J* = 7.6 Hz), 2.85 (2H, t, *J* = 12.0 Hz), 2.73 (2H, t, *J* = 7.6 Hz), 1.75-2.00 (4H, m), 1.38 (3H, t, *J* = 7.6 Hz); LCMS: 98.6%, MS(ESI):m/z 584.9/586.9 [M+H] |
| 144 | 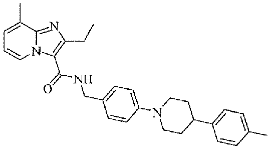 | yellow solid : ¹H-NMR (CDCl₃): δ 9.25 (1H, d, *J* = 7 Hz), 7.29 (2H, d, *J* = 8.5 Hz), 7.07-7.20 (5H, m), 6.98 (2H, d, *J* = 8.5 Hz), 6.82 (1H, t, *J* = 6.9 Hz), 6.01 (1H, brs), 4.62 (2H, d, *J* = 5.3 Hz), 3.81 (2H, d, *J* = 12.0 Hz), 2.99 (2H, q, *J* = 7.5 Hz), 2.83 (2H, td, *J* = 11.7, 2.0 Hz), 2.55-2.68 (1H, m), 2.61 (3H, s), 2.33 (3H, s), 1.82-1.98 (4H, m), 1.37 (3H, t, *J* = 7.5 Hz); LCMS: 98.7%, MS (ESI): m/z 489.1[M+ H]+. |
| 145 | 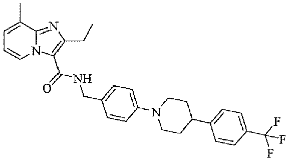 | yellow solid : ¹H-NMR (CDCl₃): δ 9.25 (1H, d, *J* = 6.8 Hz), 7.58 (2H, d, *J* = 8.2 Hz), 7.36 (2H, d, *J* = 8.2 Hz), 7.30 (2H, d, *J* = 8.6 Hz), 7.11 (1H, d, = 6.6 Hz), 6.99 (2H, d, *J* = 8.6 Hz) 6.82 (1H, t, *J* = 6.9 Hz), 6.02 (1H, brs), 4.63 (2H, d, *J* = 5.5 Hz), 3.83 (2H, d, *J* = 12.3 Hz), 3.00 (2H, q, *J* = 7.6 Hz), 2.84 (2H, td, *J* = 12, 2.5 Hz), 2.68-2.78 (1H, m), 2.61 (3H, s), 1.84-2.02 (4H, m), 1.37 (3H, t, *J* = 7.5 Hz); LCMS: 98.6%, MS (ESI): m/z 521.1[M+ H]+. |
| 146 | 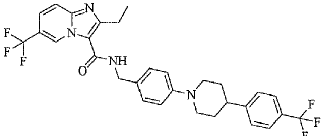 | yellow solid : ¹H-NMR (CDCl₃): δ 9.86 (1H, d, *J* = 0.8 Hz), 7.68 (1H, d, *J* = 9.6 Hz), 7.56 (2H, d, *J* = 8.0 Hz), 7.46 (1H, d, *J* = 9.2 Hz), 7.35 (2H, d, *J* = 8.0 Hz), 7.29 (2H, d, *J* = 8.4 Hz), 6.98 (2H, d, *J* = 8.8 Hz), 6.06 (1H, m), 4.63 (2H, d, *J* = 5.2 Hz), 3.83 (2H, d, *J* = 12.4 Hz), 2.99 (2H, q, *J* = 7.6 Hz), 2.80-2.91 (2H, m), 2.57-2.79 (1H, m), 1.82-2.03 (4H, m), 1.41 (3H, t, *J* = 7.6 Hz); LCMS: 99.3%, MS (ESI): m/z 575.0[M+ H]+. |
| 147 | 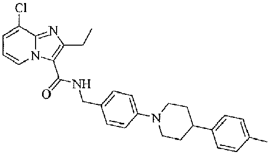 | red solid : ¹H-NMR (CDCl₃): δ 9.34 (1H, dd, *J* = 8 Hz), 7.38 (1H, dd, *J* = 8 Hz), 7.27 (2H, m) 7.07-7.17 (4H, m), 6.98 (2H, d, *J* = 8.40 Hz), 6.84 (1H, t, *J* = 7.2 Hz), 6.05 (1H, brs), 4.61 (2H, d, *J* = 5.6 Hz), 3.81 (2H, d, *J* = 12.4 Hz), 3.01 (2H, q, *J* = 7.6 Hz), 2.82 (2H, td, *J* = 2.8, *J* = 12.0 Hz), 2.62 (1H, m), 2.32 (3H, s), 1.85-1.96 (4H, m), 1.37 (3H, t, *J* = 8 Hz); LCMS: 100.0%, MS (ESI): m/z 487.1[M+ H]+. |
| 148 | 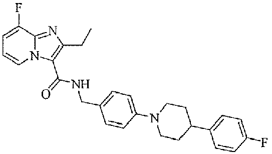 | yellow solid : ¹H-NMR (CDCl₃): δ 9.20 (1H, d, *J* = 6.8 Hz), 7.29-7.17 (4H, m), 7.04-6.96 (5H, m), 6.85-6.80 (1H, m), 6.04 (1H, brs), 4.61 (2H, d, *J* = 5.6 Hz), 3.82-3.79 (2H, m), 2.98 (2H, q, *J* = 7.6 Hz), 2.85-2.78 (2H, m), 2.67-2.60 (1H, m), 1.95-1.79 (4H, m), 1.39 (3H, t, *J* = 7.6 Hz); LCMS: 98.7%, MS (ESI): m/z 475.0[M+ H]+. |
| 149 | 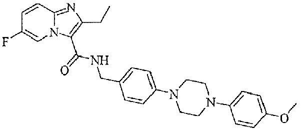 | white solid : ¹H-NMR (CDCl₃): δ 9.40-9.49 (1H, m), 7.55 (1H, dd, *J* = 9.6, 4.8 Hz), 7.29 (2H, d, *J* = 8.4 Hz), 7.19-7.28 (2H, m, overlap with CDCl₃ signal), 6.90-7.00 (4H, m), 6.84 (2H, d, *J* = 8.4 Hz), 6.03 (1H, t, *J* = 4.4 Hz), 4.61 (2H, d, *J* = 5.6 Hz), 3.77 (3H, s), 3.30-3.40 (4H, m), 3.15-3.25 (4H, m), 2.96 (2H, q, *J* = 7.6 Hz), 1.39 (3H, t, *J* = 7.6 Hz); LCMS: 100%, MS (ESI): m/z 488.1[M+ H]+. |
| 150 | 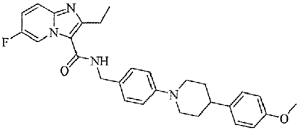 | pink solid : ¹H-NMR (CDCl₃): δ 9.45 (1H, dd, *J* = 4.8, 2.0 Hz), 7.56 (1H, dd, *J* = 10.0, 5.6 Hz), 7.20-7.31 (3H, m, overlap with CDCl₃ signal), 7.16 (2H, d, *J* = 8.8 Hz), 6.98 (2H, d, *J* = 8.8 Hz), 6.86 (2H, d, *J* = 8.8 Hz), 5.95-6.05 (1H, m), 4.61 (2H, d, *J* = 5.6 Hz), 3.76-3.88 (5H, m), 2.96 (2H, q, *J* = 7.2 Hz), 2.77-2.87 (2H, m), 2.53-2.66 (1H, m), 1.78-1.99 (4H, m), δ 1.39 (3H, t, *J* = 7.2 Hz); LCMS: 100%, MS(ESI): m/z 487.0 [M+H]+. |
| 151 | 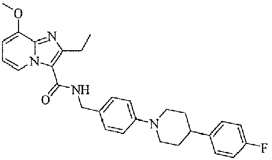 | pink solid : ¹H-NMR (CDCl₃): δ 8.97 (1H, dd, *J* = 0.4 Hz, *J* = 6.8 Hz) 7.25-7.29 (2H, m), 7.17-7.20 (2H, m), 6.96-7.01 (4H, m), 6.80 (1H, t, *J* = 7.6 Hz), 6.60 (1H, d, *J* = 7.6 Hz), 6.02 (1H, brs), 4.60 (2H, d, *J* = 5.6 Hz), 4.00 (3H, s), 3.80 (2H, d, *J* = 12.4 Hz), 2.95 (2H, q, *J* = 8.0 Hz), 2.82 (2H, td, *J* = 2.4 Hz, *J* = 12.4 Hz), 2.66-2.60 (1H, m), 1.95-1.82 (4H, m), 1.37 (3H, *J* = 7.6 Hz); LCMS: 100%, MS (ESI): m/z 487.1[M+ H]+. |
| 152 | 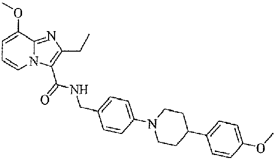 | pink solid : ¹H-NMR (CDCl₃): δ 8.97 (1H, dd, *J* = 0.8 Hz, *J* = 7.2 Hz), 7.25-7.28 (2H, m), 7.17 (2H, d, *J* = 2.0 Hz), 7.15 (2H, d, *J* = 1.6 Hz), 6.96-6.98 (2H, m), 6.84-6.87 (2H, m), 6.80 (1H, t, *J* = 7.2 Hz), 6.60 (1H, d, *J* = 7.2 Hz), 6.02 (1H, brs), 4.60 (2H, d, *J* = 5.6 Hz), 4.0 (3H, s), 3.78-3.81 (5H, m), 2.96 (2H, q, *J* = 7.6 Hz), 2.80 (2H, td, *J* =2.4 Hz, *J* = 12Hz) 2.60 (1H, m), 1.82-1.99 (4H, m), 1.37 (3H, t, *J* = 7.6 Hz); LCMS: 100%, MS (ESI): m/z 521.1[M+ Na]+. |
| 153 | 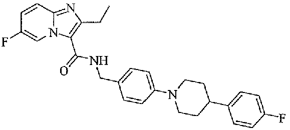 | yellow solid : ¹H-NMR (CDCl₃): δ 9.45 (1H, dd, *J* = 5.2, 2.4 Hz) , 7.56 (1H, dd, *J* = 9.6, 5.2 Hz), 7.15-7.32 (5H, m, overlap with CDCl₃ signal), 6.90-7.05 (4H, m) , 6.01 (1H, brs) , 4.60 (2H, d, *J* = 5.6 Hz) , 3.80 (2H, d, *J* = 12.4 Hz) , 2.95 (2H, q, *J* = 7.6 Hz) , 2.78-2.86 (2H, m) , 2.60-2.70 (1H, m), 1.78-2.00 (4H, m), 1.38 (3H, t, *J* = 7.6 Hz); LCMS: 100%, MS (ESI): m/z 457.1 [M+ H]+. |
| 154 | 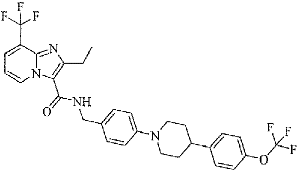 | yellow solid : ¹H-NMR (CDCl₃): δ 9.57 (1H, d, *J* = 7.6 Hz), 7.64 (1H, d, *J* = 7.2 Hz), 7.20-7.30 (4H, m), 7.15 (2H, d, *J* = 8.0 Hz), 6.95-7.00 (3H, m), , 6.08 (1H, brs), 4.62 (2H, d, *J* = 6.0 Hz), 3.68-3.85 (2H, m), 3.03 (2H, q, *J* = 7.6 Hz), 2.78-2.88 (2H, m), 2.65-2.72 (1H, m), 1.80-2.00 (4H, m), 1.37 (3H, t, *J* = 7.6 Hz); LCMS: 100%, MS (ESI): m/z 591.0[M + H]+. |
| 155 | 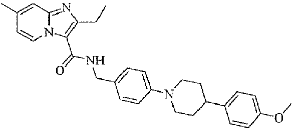 | yellow solid : ¹H-NMR (CDCl₃): δ 9.27 (1H, d, *J* = 7.2 Hz), 7.35 (1H, s), 7.28 (2H, d, *J* = 8.4 Hz), 7.16 (2H, dd, *J* = 6.4 Hz, 2.0 Hz), 6.97 (2H, dd, *J* = 8.8 Hz, 2.0 Hz), 6.86 (2H, dd, *J* = 6.4 Hz, 2.0 Hz), 6.73 (1H, dd, *J* = 6.4 Hz, 2.0 Hz), 5.96 (1H, brs), 4.60 (2H, d, *J* = 2.8 Hz), 3.81-3.78 (5H, m), 2.93 (2H, q, *J* = 8.4 Hz), 2.83 (2H, td, *J* = 12 Hz, 2.4 Hz), 2.66-2.56 (1H, m), 2.41 (3H, d, *J* = 0.4 Hz), 1.98-1.82 (4H, m), 1.37 (3H, t, *J* = 7.2 Hz); LCMS: 100%, MS (ESI): m/z 483.1 [M+ H]+. |
| 156 | 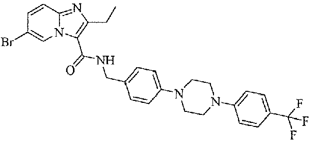 | white solid : ¹H-NMR (CDCl₃): δ 9.56 (1H, s), 7.40-7.50 (3H, m), 7.29-7.37 (1H. m), 7.21-7.29 (2H, m), 6.81-6.97 (4H, m), 5.98 (1H, brs), 4.56 (2H, d, *J* = 5.6 Hz), 3.41-3.51 (4H, m), 3.22-3.41 (4H, m), 2.89 (2H, q, *J* = 7.6 Hz), 1.33 (3H, t, *J* = 7.6 Hz); LCMS: 100%, MS (ESI): m/z 586.0[M+ H]+. |
| 157 | 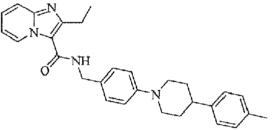 | yellow solid : ¹H-NMR (CDCl₃): δ 9.39 (1H, d, *J* = 5.6 Hz), 7.59 (1H, d, *J* = 8.8 Hz), 7.26-7.35 (3H, m, overlap with CDCl₃ signal), 7.10-7.17 (4H, m), 6.95-7.03 (2H, m), 6.91 (1H, t, *J* = 6.8 Hz), 5.96-6.05 (1H, m), 4.61 (2H, d, *J* = 5.6 Hz), 3.75-3.85 (2H, m), 2.97 (2H, q, *J* = 7.6 Hz), 2.76-2.88 (2H, m), 2.56-2.68 (1H, m), 2.32 (3H, s), 1.80-1.99 (4H, m), 1.39 (3H, t, *J* = 7.6 Hz); LCMS: 98.4%, MS (ESI): m/z 475.1[M+ Na]+. |
| 158 | 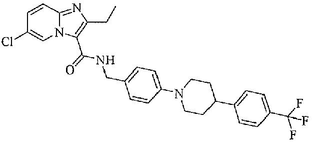 | yellow solid : ¹H-NMR(CDCl₃): δ 9.54 (1H, dd, *J* = 2.0 Hz, *J* = 0.8 Hz), 7.50-7.60 (3H, m), 7.36-7.40 (2H, m), 7.27-7.32 (3H, m), 6.93-7.00 (2H, m), 6.04 (1H, brs), 4.63 (2H, d, *J* = 5.6 Hz), 3.78-3.86 (2H, m), 2.98 (2H, q, *J* = 7.6 Hz), 2.82-2.89 (2H, m), 2.69-2.80 (1H, m), 1.82-2.00 (4H, m), 1.40 (3H, t, *J* = 7.2 Hz); LCMS: 100%, MS (ESI): m/z 541.1[M+ H]+. |
| 159 | 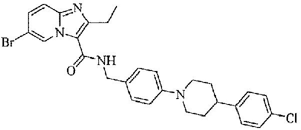 | yellow solid: ¹H-NMR (CDCl₃): δ 9.65 (1H, d, *J* = 1.2 Hz), 7.51 (1H, d, *J* = 9.2 Hz), 7.41 (1H, dd, *J* = 9.2, 2.0 Hz), 7.28-7.35 (4H, m), 7.20 (2H, d, *J* = 8.4 Hz), 7.01 (2H, d, *J* = 8.4 Hz), 6.05 (1H, brs), 4.64 (2H, d, *J* = 5.2 Hz), 3.76-3.90 (2H, m), 2.98 (2H, q, *J* = 7.6 Hz), 2.80-2.90 (2H, m), 2.60-2.73 (1H, m), 1.80-2.03 (4H, m), 1.42 (3H, t, *J* = 7.6 Hz); LCMS: 100%, MS (ESI): m/z 553.0[M+ H]+. |
| 160 | 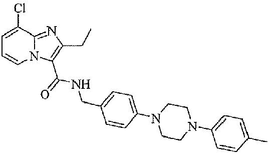 | yellow solid : ¹H-NMR (CDCl₃): δ 9.37 (1H, dd, *J1* = 0.8 Hz, *J2* = 6.8 Hz), 7.42 (1H, dd, *J1* = 0.8 Hz, *J2* = 7.6 Hz), 7.33 (2H, d, *J* = 8.4 Hz), 7.13 (2H, d, *J* = 8.0 Hz), 7.01 (2H, d, *J* = 8.8 Hz), 6.86-6.94 (3H, m), 6.09 (1H, brs), 4.65 (2H, d, *J* = 5.2 Hz), 3.37 (4H, dd, *J1* = 3.6 Hz, *J2* = 7.2 Hz), 3.31 (4H, dd, *J1* = 2.4 Hz, *J2* = 5.6 Hz), 3.04 (2H, q, *J* = 7.6 Hz), 1.41 (3H, t, *J* = 7.6 Hz); LCMS: 98.4%, MS (ESI): m/z 488.1 [M+ H]+. |
| 161 | 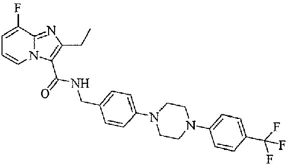 | white solid : ¹H-NMR (CDCl₃): δ 9.23 (1H, d, *J* = 6.8 Hz), 7.53 (2H, d, *J* = 8.8 Hz), 7.34 (2H, d, *J* = 8.4 Hz), 7.08-6.99 (5H, m), 6.88-6.83 (1H, m), 6.10 (1H, brs), 4.66 (2H, d, *J* = 5.6 Hz), 3.48-3.36 (8H, m), 3.01 (2H, q, *J* = 7.6 Hz), 1.43 (3H, t, *J* = 7.6 Hz); LCMS: 99.5%, MS (ESI): m/z 526.1[M+ H]+. |
| 162 | 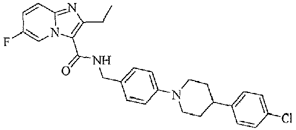 | yellow solid : ¹H-NMR (CDCl₃): δ 9.48 (1H, dd, *J* = 4.8, 2.4 Hz), 7.59 (1H, dd, *J* = 9.6, 5.2 Hz), 7.25-7.35 (5H, m, overlap with CDCl₃ signa), 7.20 (2H, d, *J* = 8.4 Hz), 7.00 (2H, d, *J* = 8.8 Hz), 6.05 (1H, brs), 4.64 (2H, d, *J* = 5.6 Hz), 3.84 (2H, d, *J* = 12.4 Hz), 2.99 (2H, q, *J* = 7.6 Hz), 2.85 (2H, td, *J* = 12.0, 2.4 Hz), 2.60-2.70 (1H, m), 1.80-2.00 (4H, m), 1.42 (3H, t, *J* = 7.6 Hz); LCMS: 98.7%, MS (ESI): m/z 491.0[M+ H]+. |
| 163 | 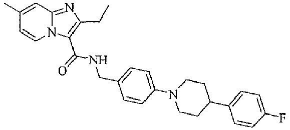 | yellow solid : ¹H-NMR (CDCl₃): δ 9.28 (1H, d, *J* = 7.2 Hz), 7.35 (1H, s), 7.30-7.26 (2H, m), 7.21-7.18 (2H, m), 7.02-6.97 (4H, m), 6.75 (1H, dd, *J* = 7.2 Hz, 1.6 Hz), 5.98 (1H, brs), 4.61 (2H, d, *J*= 5.6 Hz), 3.80 (2H, d, *J* = 12.4 Hz), 2.94 (2H, q, *J* = 7.2 Hz), 2.82 (2H, td, *J* = 12 Hz, 2.4 Hz), 2.68-2.61 (1H, m), 2.42 (3H, s), 1.98-1.80 (4H, m), 1.38 (3H, t, *J* = 7.2 Hz); LCMS: 100%, MS (ESI): m/z 471.1 [M+ H]+. |
| 164 | 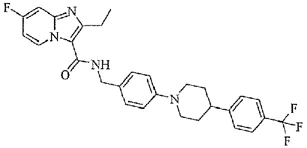 | white solid : ¹H-NMR (CDCl₃): δ 9.44(1H, t, *J*₁= 7.6 Hz, 6.0 Hz), 7.53(2H, d, *J*= 7.6 Hz), 7.34(2H, d, *J*= 8.8 Hz), 7.27(2H, d, *J*= 7.6 Hz), 7.21-7.23(1H, m), 7.95(2H, d, *J*= 8.8 Hz), 6.75-6.81(1H, m), 6.76-6.81(1H, m), 6.03(1H, brs), 4.61(2H, d, *J*= 5.6 Hz), 3.49-3.88(2H, m), 2.93-2.98(2H, m), 2.82-2.88(2H, m), 2.70-2.77(1H, m), 1.63-1.99(4H, m), 1.23(3H, t, *J*= 7.6 Hz); LCMS: 99.1%, MS (ESI): m/z 525.0[M+ H]+.490.1 [M+ Na]+ |
| 165 | 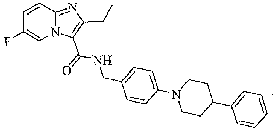 | white solid : ¹H-NMR (CDCl₃): δ 9.45 (1H, dd, *J* = 5.2, 2.4 Hz), 7.56 (1H, dd, *J* = 10.0, 5.2 Hz), 7.18-7.40 (8H, m, overlap with CDCl₃ signal), 7.00 (2H, d, *J* = 8.4 Hz) , 6.02 (1H, brs), 4.62 (2H, d, *J* = 5.2 Hz), 3.82 (2H, d, *J* = 12.4 Hz), 2.97 (2H, q, *J* = 7.6 Hz), 2.80-2.90 (2H, m) , 2.60-2.71 (1H, m) , 1.80-2.05 (4H, m), 1.40 (3H, t, *J* = 7.6 Hz); LCMS: 100%, MS (ESI): m/z 457.0[M+ H]+ |
| 166 | 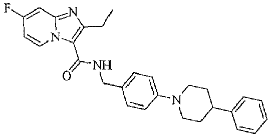 | white solid : ¹H-NMR (CDCl₃): δ 9.44(1H, t, *J*= 7.2 Hz), 7.21-7.35(7H, m), 7.00(2H, d, J= 8.4 Hz), 6.77-6.81(1H, m), 5.60(1H, brs), 4.61(2H, d, *J*= 5.6 Hz), 3.81-3.84(2H, m), 2.92-2.98(1H, m), 2.81-2.88(2H, m), 2.62-2.70(1H, m), 1.86-2.01(4H, m), 1.39(3H, t, *J*=7.6 Hz); LCMS: 99.9%, MS (ESI): m/z 457.0[M+ H]+. |
| 167 | 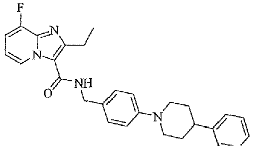 | red solid : ¹H-NMR (CDCl₃): δ 9.20 (1H, d, *J* = 6.8 Hz), 7.35-7.20 (6H, m), 7.05-6.98 (3H, m), 6.86-6.81 (1H, m), 6.06 (1H, brs), 4.62 (2H, d, *J* = 5.6 Hz), 3.84-3.81 (2H, m), 3.00 (2H, q, *J* = 7.6 Hz), 2.87-2.81 (2H, m), 2.71-2.63 (1H, m), 2.01-1.86 (4H, m), 1.40 (3H, t, *J* = 7.6 Hz); LCMS: 100%, MS (ESI): m/z 457.0[M+ H]+. |
| 168 | 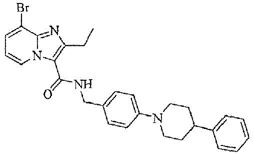 | white solid : ¹H-NMR (CDC13): δ 9.39 (1H, d, *J* = 6.8 Hz), 7.58 (1H, d, *J* = 7.2 Hz), 7.18-7.38 (8H, m), 6.99 (2H, d, *J* = 7.6 Hz), 6.80 (1H, t, *J* = 7.2 Hz), 6.07 (1H, brs), 4.62 (2H, d, *J* = 5.6 Hz), 3.82 (2H, d, *J* = 12.4 Hz), 3.02 (2H, q, *J* = 7.6 Hz), 2.84 (2H, t, *J* = 11.6 Hz), 2.60-2.75 (1H, m), 1.85-2.00 (4H, m), 1.38 (3H, t, *J* = 7.6 Hz); LCMS: 100%, MS(ESI):m/z 516.8/518.8 [M+H]. |
| 169 | 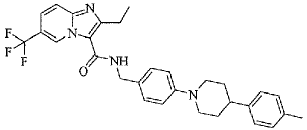 | white solid : ¹H-NMR (CDCl₃): δ 9.86 (1H, s), 7.69 (1H, d, *J* = 9.2 Hz), 7.47 (1H, dd, *J* = 7.6, 1.6 Hz), 7.28 (2H, t, *J* = 8.4 Hz), 7.12 (4H, s), 6.99 (2H, d, *J* = 8.0 Hz), 6.06 (1H, m), 4.63 (2H, d, *J* = 5.2 Hz), 3.82 (2H, d, *J* = 12.4 Hz), 2.99 (2H, q, *J* = 7.2 Hz), 2.81-2.86 (2H, m), 2.58-2.70 (1H, m), 2.33 (3H, s), 1.82-2.02 (4H, m), 1.41 (3H, t, *J* = 7.6 Hz); LCMS: 100%, MS (ESI): m/z 521.0[M+ H]+. |
| 170 | 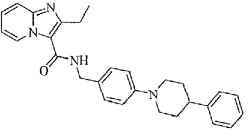 | white solid : ¹H-NMR (CDCl₃): δ 9.41 (1H, d, *J* = 7.2 Hz), 7.61 (1H, d, *J* = 8.8 Hz), 7.15-7.36 (8H, m, overlap with CDCl₃ signal), 6.99 (2H, d, *J* = 8.8 Hz), 6.92 (1H, t, *J* = 7.2 Hz), 6.02 (1H, brs), 4.63 (2H, d, *J* = 5.6 Hz), 3.83 (2H, d, *J* = 12.0 Hz), 2.98 (2H, q, *J* = 7.6 Hz), 2.72-2.90 (2H, m), 2.60-2.70 (1H, m), 1.81-2.04 (4H, m), 1.40 (3H, t, *J* = 7.6 Hz); LCMS: 99.3%, MS (ESI): m/z439.1[M+ H]+. |
| 171 | 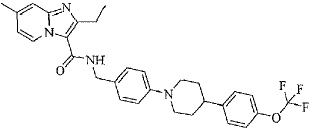 | white solid : ¹H-NMR (CDCl₃): δ 9.28 (1H, d, *J* = 7.2 Hz), 7.38 (1H, s), 7.30-7.25 (3H, m), 7.17-7.15 (2H, m), 6.98 (2H, d, *J* = 8.4 Hz), 6.77 (1H, d, *J* = 6.0 Hz), 5.98 (1H, brs), 4.62 (2H, d, *J* = 5.6 Hz), 2.95 (2H, q, *J* = 7.6 Hz), 2.83 (2H, td, *J* = 10.8 Hz, 2.4 Hz), 2.70-2.62 (1H, m), 2.43 (3H, s), 2.03-1.75 (4H, m), 1.39 (3H, t, *J* = 8.4 Hz); LCMS: 100%, MS (ESI): m/z 537.1[M+ H]+. |
| 172 | 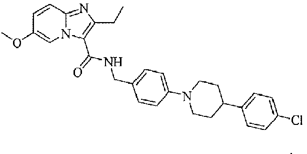 | white solid : ¹H-NMR (CDCl₃): δ 9.11 (1H, d, *J* = 2.4 Hz), 7.49 (1H, d, *J* = 9.6 Hz), 7.26-7.35 (4H, m), 7.18 (2H, d, *J* = 8.4 Hz), 7.11 (1H, dd, *J* = 9.6, 2.4 Hz), 6.98 (2H, d, *J* = 8.4 Hz), 6.02 (1H, brs), 4.63 (2H, d, *J* = 5.2 Hz), 3.88 (3H, s), 3.82 (2H, d, *J* = 12.4 Hz), 2.95 (2H, q, *J* = 7.6 Hz), 2.85 (2H, td, *J* = 12.0, 2.4 Hz), 2.58-2.70 (1H, m), 1.80-2.00 (4H, m), 1.39 (3H, t, *J* = 7.6 Hz); LCMS: 100%, MS(ESI):m/z 503.0 [M+H]+ |
| 173 | 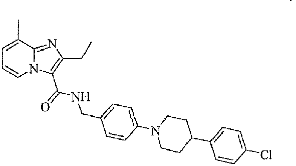 | white solid : ¹H-NMR (CDCl₃): δ 9.24 (1H, d, *J* = 7 Hz), 7.26-7.31 (4H, m), 7.15-7.20 (2H, m), 7.11 (1H, d, *J* = 7 Hz), 6.98(2H, d, *J* = 8.5 Hz), 6.82 (1H, t, *J* = 6.9 Hz), 6.03 (1H, brs), 4.62(2H, d, *J* = 5.3 Hz), 3.81 (2H, d, *J* = 12.5 Hz), 2.99 (2H, q, *J* = 7.5 Hz), 2.83 (2H, td, *J* = 12.2, 2.5 Hz), 2.62-2.69 (1H, m), 2.61 (3H, s), 1.79-1.98 (4H, m), 1.37 (3H, t, *J* = 7.5 Hz); LCMS: 100%, MS (ESI): m/z 487.0[M+ H]+. |
| 174 | 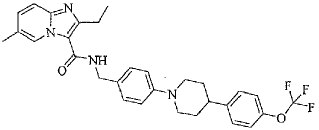 | white solid : ¹H-NMR (CDCl₃): δ 9.19 - 9.27 (m, 1 H) 7.46 - 7.55 (m, 1 H), 7.25 - 7.32 (m, 4 H), 7.14 - 7.21 (m, 3 H), 6.92 - 7.07 (m, 2 H), 5.96 - 6.12 (m, 1 H), 4.59 - 4.65 (m, 2 H), 3.77 - 3.89 (m, 2 H), 2.91 - 3.00 (m, 2 H), 2.77 - 2.90 (m, 2 H), 2.61 - 2.75 (m, 1 H), 2.37 (s, 3 H), 1.80 - 2.00 (m, 4 H), 1.40 (s, 3 H); LCMS: 100%, MS (ESI): m/z 537.0[M+ H]+. |
| 175 | 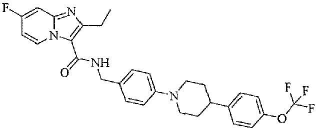 | white solid : ¹H-NMR (CDCl₃): δ 9.42-9.46 (1H, m), 7.30-7.21 (3H, m), 7.17-7.15 (2H, m), 6.99 (2H, d, *J*= 7.6 Hz), 6.81-6.77 (1H, m), 5.99 (1H, brs), 4.61 (2H, d, *J*= 5.2 Hz), 2.97-2.92 (2H, m), 2.86-2.80 (2H, m), 2.67 (1H, m), 1.97-1.89 (4H, m), 1.41-1.37 (3H, t, *J* = 7.6 Hz); LCMS: 99.9%, MS (ESI): m/z 541.0[M+ H]+. |
| 176 | 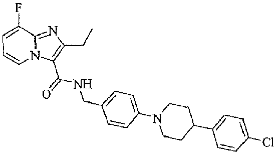 | white solid : ¹H-NMR (CDCl₃): δ 9.20 (1H, d, *J* = 6.8 Hz), 7.30-7.26 (4H, m), 7.18 (2H, d, *J* = 8.4 Hz), 7.06-6.97 (3H, m), 6.86-6.81(1H, m), 6.06 (1H, brs), 4.62 (2H, d, *J* = 5.2 Hz), 3.83-3.80 (2H, m), 3.00 (2H, q, *J* = 7.6 Hz), 2.86-2.80 (2H, m), 2.68-2.60 (1H, m), 1.96-1.83 (4H, m), 1.40 (3H, t, *J* = 7.6 Hz); LCMS: 100%, MS (ESI): m/z 491.0[M+ H]+. |
| 177 | 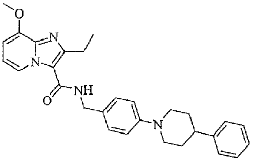 | yellow solid : ¹H-NMR (CDC13): δ 8.99 (1H, d, *J* = 6.8 Hz) 7.20-7.35 (7H, m), 6.99 (2H, d, *J* = 8.0 Hz), 6.81 (1H, t, *J* = 7.6 Hz), 6.61 (1H, d, *J* = 7.6 Hz), 6.04 (1H, brs), 4.61 (2H, d, *J* = 5.6 Hz), 4.01 (3H, s), 3.82 (2H, d, *J* = 12 Hz), 2.98 (2H, q, *J* = 7.6 Hz), 2.83 (2H, t, *J* = 9.6 Hz), 2.63-2.69 (1H, m), 1.89-1.98 (4H, m), 1.38 (3H, t, *J* = 7.6 Hz); LCMS: 100%, MS (ESI): m/z 469.0[M+ H]+. |
| 178 | 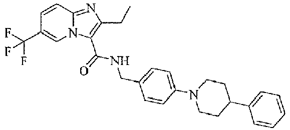 | white solid : ¹H-NMR (CDCl₃): δ 9.86 (1H, s), 7.69 (1H, d, *J* = 9.2 Hz), 7.47 (1H, dd, *J* = 7.6, 1.6 Hz), 7.18-7.36 (7H, m), 7.00 (2H, d, *J* = 8.0 Hz), 6.07 (1H, m), 4.63 (1H, d, *J* = 5.2 Hz), 3.83 (2H, d, *J* = 12 Hz), 2.97 (2H, q, *J* = 7.6 Hz), 2.71-2.88 (2 H, m), 2.57-2.63 (1H, m), 1.81-2.03 (4H, m), 1.41 (3H, t, *J* = 7.6 Hz); LCMS: 100%, MS (ESI): m/z 507.0[M+ H]+. |
| 179 | 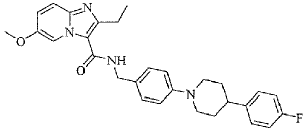 | yellow solid : ¹H-NMR (CDCl₃): δ 9.10 (1H, d, *J* = 2.4 Hz), 7.49 (1H, d, *J* = 9.6 Hz), 7.29 (2H, d, *J* = 8.4 Hz), 7.15-7.25 (2H, m), 7.11 (1H, dd, *J* = 8.4, 2.4 Hz), 7.00 (1H, t, *J* = 8.8 Hz), 6.03 (1H, brs), 4.62 (2H, d, *J* = 5.6 Hz), 3.87 (3H, s), 3.81 (2H, d, *J =* 12.4 Hz), 2.95 (2H, q, *J* = 7.6 Hz), 2.82 (2H, td, *J* = 12.0, 2.4 Hz), 2.55-2.70 (1H, m), 1.80-2.00 (4H, m), 1.39 (3H, t, *J* = 7.6 Hz); LCMS: 100%, MS(ESI):m/z 487.0 [M+H]+. |
| 180 | 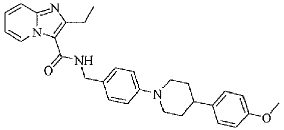 | red solid : ¹H-NMR (CDCl₃): δ 9.40 (1H, d, *J* = 6.8 Hz), 7.60 (1H, d, *J* = 8.8 Hz), 7.25-7.35 (3H, m, overlap with CDCl₃ signal), 7.17 (2H, d, *J* = 8.4 Hz), 6.99 (2H, d, *J =* 8.8 Hz), 6.89-6.91 (1H, m), 6.87 (2H, d, *J =* 8.8 Hz), 6.02 (1H, brs), 4.62 (2H, d, *J* = 5.2 Hz), 3.72-3.84 (5H, m), 2.98 (2H, q, *J =* 7.6 Hz), 2.77-2.87 (2H, m), 2.56-2.67 (1H, m), 1.63-1.98 (4H, m), 1.40 (3H, t, *J* = 7.2 Hz); LCMS: 99.2%, MS (ESI): m/z 491.1 [M+ Na]+. |
| 181 | 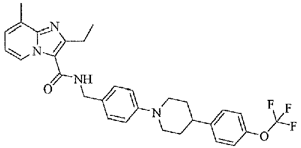 | white solid : ¹H-NMR (CDCl₃): δ 9.24 (1H, d, *J* = 6.8 Hz), 7.23-7.32 (4H, m), 7.16 (2H, d, *J* = 8.3 Hz), 7.11 (1H, d, *J* = 6.8 Hz), 6.98 (2H, d, *J* = 8.5 Hz), 6.82 (1H, t, *J* = 6.9 Hz), 6.02 (1H, brs), 4.62 (2H, d, *J* = 5.3 Hz), 3.82 (2H, d, *J* = 12.0 Hz), 2.99 (2H, q, *J* = 7.6 Hz), 2.84 (2H, td, *J* = 12.1, 2.4 Hz), 2.627-2.72 (1H, m), 2.61 (3H, s), 1.81-2.00 (4H, m), 1.37 (3H, t, *J* = 7.7 Hz); LCMS: 100%, MS (ESI): m/z 537.1[M+ H]+. |
| 182 | 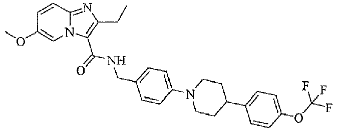 | white solid : ¹H-NMR (CDCl₃): δ 9.11 (1H, d, *J* = 2.4 Hz), 7.50 (1H, d, *J* = 9.6 Hz), 7.22-7.32 (4H, m), 7.17 (2H, d, *J* = 8.4 Hz), 7.10 (1H, dd, *J* = 9.6, 2.4 Hz), 6.99 (2H, d, *J* = 8.8 Hz), 6.02 (1H, brs), 4.62 (2H, d, *J* = 5.6 Hz), 3.88 (3H, s), 3.82 (2H, q, *J* = 12.4 Hz), 2.95 (2H, q, *J* = 7.6 Hz), 2.83 (2H, td, *J* = 12.0, 2.4 Hz), 2.62-2.74 (1H, m), 1.80-2.00 (4H, m), 1.39 (3H, t, *J* = 7.6 Hz); LCMS: 100%, MS(ESI):m/z 553.1 [M+H]+. |
| 183 | 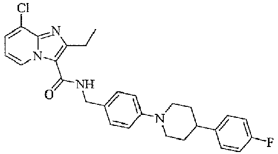 | white solid : ¹H-NMR (CDCl₃): δ 9.35 (1H, d, *J* = 6.8 Hz), 7.40 (1H, d, *J* = 7.2 Hz), 7.29 (2H, d, *J* = 8.4 Hz), 7.20 (2H, dd, *J* = 8.8, *J* = 5.6 Hz), 6.98-7.03 (4H, m), 6.86 (1H, t, *J* = 7.2 Hz), 6.07 (1H, brs), 4.62 (2H, d, *J* = 5.6 Hz), 3.82 (2H, d, *J* = 12.4 Hz), 3.02 (2H, q, *J* = 7.6 Hz), 2.83 (2H, t, *J* = 11. 2 Hz), 2.64-2.65 (1H, m), 1.84-1.96 (4H, m), 1.39 (3H, t, *J* = 7.65 Hz); LCMS: 100.0%, MS (ESI): m/z491.0[M+ H]+. |
| 184 | 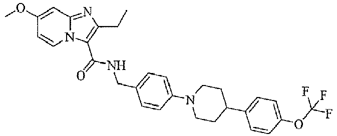 | white solid : ¹H-NMR (CDCl₃): δ 9.25 (1H, d, *J* = 7.6 Hz), 7.22-7.32 (4H, m), 7.16 (2H, d, *J* = 8.4 Hz), 6.98 (2H, d, *J* = 8.4 Hz), 6.89 (1H, d, *J* = 2.4 Hz), 6.60 (1H, dd, *J* = 6.4 Hz, 2.8 Hz), 5.94 (1H, brs), 4.60 (2H, d, *J* = 5.6 Hz), 3.87 (3H, s), 3.82 (2H, d, *J* = 12.4 Hz), 2.92 (2H, q, *J* = 7.6 Hz), 2.82 (2H, td, *J* = 12.0 Hz, 2.4 Hz), 2.60-2.72 (1H, m), 1.70-2.02 (4H, m), 1.39 (3H, t, *J* = 7.6 Hz); LCMS: 100.0%, MS (ESI): m/z 553.1 [M+H]+. |
| 185 | 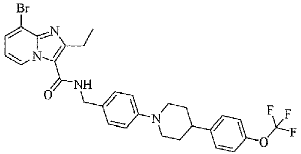 | yellow solid : ¹H-NMR (CDCl₃): δ 9.39 (1H, d, *J* = 6.8 Hz), 7.58 (1H, d, *J* = 7.2 Hz), 7.20-7.32 (4H, m), 7.19 (2H, d, *J* = 8.0 Hz), 6.99 (2H, d, *J* = 8.8 Hz), 6.79 (1H, t, *J* = 7.2 Hz), 6.08 (1H, brs), 4.62 (2H, d, *J* = 5.6 Hz), 3.83 (2H, d, *J* = 12.4 Hz), 3.04 (2H, q, *J* = 7.6 Hz), 2.83 (2H, td, *J* = 12.0, 2.4 Hz), 2.60-2.72 (1H, m), 1.80-2.00 (4H, m), 1.38 (3H, t, *J* = 7.6 Hz); LCMS: 100%, MS(ESI):m/z 601.0/603.0 [M+H]+ |
| 186 | 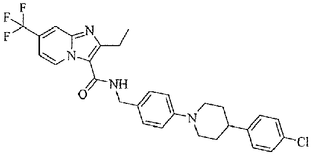 | yellow solid : ¹H-NMR (CDCl₃): δ 9.53 (1H, d, *J* = 7.2 Hz), 7.91 (1H, s), 7.24-7.38 (4H, m), 7.18 (2H, d, *J* = 8.0 Hz), 7.09 (1H, dd, *J* = 7.2, 1.6 Hz), 6.98 (2H, d, *J* = 8.4 Hz), 6.11 (1H, brs), 4.63 (2H, d, *J* = 5.2 Hz), 3.81-3.86 (2H, m), 3.10 (2H, q, *J* = 7.6 Hz), 2.80-2.86 (2H, m), 2.61-2.68 (1H, m), 1.80-2.08 (4H, m), 1.44 (3H, t, *J* = 7.6Hz); LCMS: 100%, MS (ESI): m/z 541.1 [M+ H]+. |
| 187 | 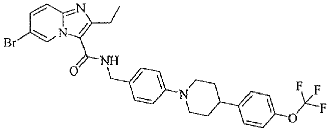 | white solid : ¹H-NMR (CDCl₃): δ 9.63 (1H, s), 7.49 (1H, d, *J* = 9.2 Hz), 7.43 (1H, d, *J* = 7.6 Hz), 7.21-7.32 (3H, m), 7.13-7.20 (2H, m), 6.92-7.04 (2H, m), 6.04 (1H, brs), 4.54-4.67 (2H, m), 3.76-3.89 (2H, m), 2.94 (2H, q, *J* = 7.6 Hz), 2.76-2.89 (2H, m), 2.59-2.71 (1H, m), 1.77-2.00 (4H, m), 1.42 (3H, t, *J* = 7.6 Hz); LCMS: 100%, MS (ESI): m/z 602.8[M+ H]+. |
| 188 | 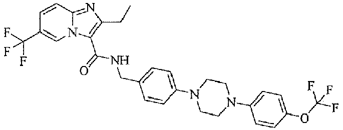 | white solid : ¹H-NMR (CDCl₃): δ 9.82 (1H, d, *J* = 15.2 Hz), 7.70 (1H, d, *J* = 9.2 Hz), 7.45-7.52 (2H, m), 7.32 (2H, d, *J* = 8.4 Hz), 7.14 (2H, d, *J* = 8.8 Hz), 6.88-7.08 (4H, m), 6.02-6.15 (1H, m), 4.64 (2H, d, *J* = 5.6 Hz), 3.23-3.42 (8H, m), 3.00 (2H, q, *J* = 7.6 Hz), 1.44 (3H, t, *J* = 7.2 Hz); LCMS: 98.0%, MS (ESI): m/z 592.0[M+ H]+. |
| 189 | 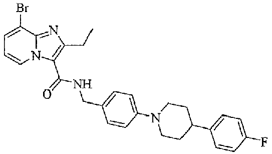 | yellow solid : ¹H-NMR (CDCl₃): δ 9.39 (1H, d, *J* = 6.4 Hz), 7.58 (1H, d, *J* = 6.8 Hz), 7.22-7.30 (2H, m), 7.15-7.21 (2H, m), 6.90-7.03 (4H, m), 6.80 (1H, t, *J* = 7.2 Hz), 6.07 (1H, brs), 4.62 (2H, d, *J* = 5.6 Hz), 3.82 (2H, d, *J* = 12.4 Hz), 3.12 (2H, q, *J* = 7.6 Hz), 2.83 (2H, td, *J* = 12.4, 2.8 Hz), 2.60-2.70 (1H, m), 1.80-2.00 (4H, m), 1.38 (3H, t, *J* = 7.6 Hz); LCMS: 99.0%, MS(ESI):m/z 535.0/537.0 [M+H] |
| 190 | 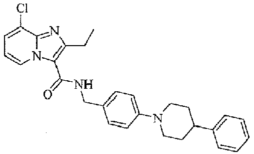 | yellow solid : ¹H-NMR (CDCl₃): δ 9.35 (1H, d, *J* = 6.8 Hz), 7.40 (1H, d, *J* = 6.8 Hz), 7.28-7.37 (4H, m), 7.21-7.25 (3H, m), 7.00 (2H, d, *J* = 8.4 Hz), 6.86 (1H, t, *J* = 7.2 Hz), 6.07 (1H, brs), 4.63 (2H, d, *J* = 5.6 Hz), 3.83 (2H, d, *J* = 12 Hz), 3.02 (2H, q, *J* = 7.6 Hz), 2.82-2.87 (2H, m), 2.63-2.70 (1H, m), 1.86-2.01 (4H, m), 1.39 (3H, t, *J* = 7.6 Hz); LCMS: 99.6%, MS (ESI): m/z 473.0[M+ H]+. |
| 191 | 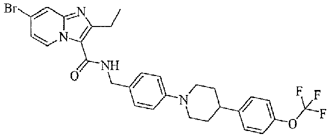 | white solid : ¹H-NMR (CDCl₃): δ 9.33 (1H, d, *J* = 7.6 Hz), 7.80 (1H, d, *J* = 1.6 Hz), 7.28-7.36 (3H, m), 7.15-7.26 (2H, m), 6.97-7.12 (3H, m), 6.04 (1H, brs), 4.63 (2H, d, *J* = 5.6 Hz), 3.73-3.92 (2H. m), 2.98 (2H, q, *J* = 7.6 Hz), 2.79-2.91 (2H, m), 2.63-2.78 (1H. m), 1.81-2.03 (4H. m), 1.41 (3H, t, *J* = 7.6 Hz); LCMS: 100%, MS (ESI): m/z 603.0[M+ H]+. |
| 192 | 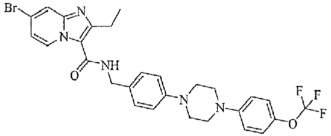 | yellow solid : ¹H-NMR (CDCl₃): δ 9.30 (1H, d, *J* = 7.6 Hz), 7.78 (1H, d, *J* = 1.2 Hz), 7.31 (2H, d, *J* = 8.4 Hz), 7.14 (2H, d, *J* = 8.4 Hz), 6.87-7.05 (5H, m), 6.04 (1H, brs), 4.62 (2H, d, *J* = 5.6 Hz), 3.22-3.40 (8H, m), 2.98 (2H, q, *J* = 7.6 Hz), 1.41 (3H, t, *J* = 7.6 Hz); LCMS: 98.3%, MS (ESI): m/z 602.0[M+ H]+. |
| 193 | 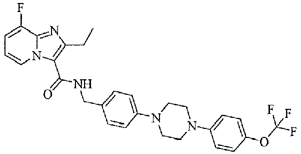 | white solid: ¹H-NMR (CDCl₃): δ 9.23 (1H, d, *J* = 7.2 Hz), 7.33 (2H, d, *J* = 8.4 Hz), 7.17 (2H, d, *J* = 8.8 Hz), 7.08-6.96 (5H, m), 6.88-6.83 (1H, m), 6.09 (1H, brs), 4.66 (2H, d, *J* = 5.2 Hz), 3.40-3.30 (8H, m), 3.02 (2H, q, *J* = 7.6 Hz), 1.43 (3H, t, *J* = 7.6 Hz); LCMS: 96.5%, MS (ESI): m/z 542.1[M+ H]+. |
| 194 | 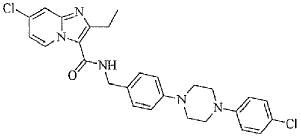 | white solid: ¹H-NMR (CDCl₃): δ 9.39 (1H, d, *J* = 7.6 Hz), 7.61 (1H, d, *J* = 1.6 Hz), 7.32 (2H, d, *J* = 8.8 Hz), 7.26 (2H, d, *J* = 9.2 Hz), 7.00 (2H, d, *J* = 8.4 Hz), 6.88-9.95 (3H, m), 6.05 (1H, brs), 4.64 (2H, d, *J* = 5.2 Hz), 3.34 (8H, d, *J* = 6.8 Hz), 2.98 (2H, q, *J* = 7.6 Hz), 1.41 (3H, t, *J* = 7.6 Hz); LCMS: 97.6%, MS (ESI): m/z 507.9 [M+ H]+. |
| 195 | 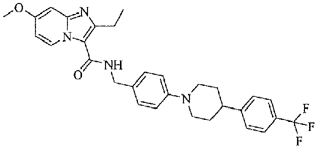 | white solid: ¹H-NMR (CDCl₃): δ 9.25 (1H, d, *J* = 7.6 Hz), 7.57 (2H, d, *J* = 8.0 Hz), 7.37 (2H, d, *J* = 8.0 Hz), 7.29 (2H, d, *J* = 8.8 Hz), 6.99 (2H, d, *J* = 8.4 Hz), 6.89 (1H, d, *J* = 2.0 Hz), 6.61 (1H, dd, *J* = 7.6 Hz, 2.4 Hz), 5.95 (1H, brs), 4.61 (2H, d, *J* = 5.2 Hz), 3.87(3H, s),3.83 (2H, d, *J* = 12.4 Hz), 2.92 (2H, q, *J* = 7.6 Hz), 2.84 (2H, td, *J* = 12.0 Hz, 2.4 Hz), 2.55-2.70 (1H, m), 1.85-2.02 (4H, m), 1.39 (3H, t, *J* = 7.6 Hz); LCMS: 98.7%, MS (ESI): m/z 537.1 [M+H]+, 559.1 [M+Na]+. |
| 196 | 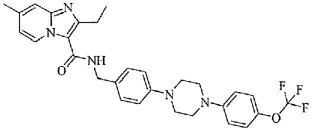 | white solid: ¹H-NMR (CDCl₃): δ 9.28 (1H, d, *J* = 6.8 Hz), 7.36-7.31 (3H, m), 7.14 (2H, d, *J* = 8.8 Hz), 6.99-6.94 (4H, m), 6.75 (1H, d, *J* = 6.8 Hz), 5.30 (1H, brs), 4.62 (2H, d, *J* = 5.6 Hz), 3.34 (8H, brs), 2.95 (1H, q, *J* = 7.2 Hz), 2.42 (3H, s), 1.39 (3H, t, *J* = 7.6 Hz); LCMS: 97.9%, MS (ESI): m/z 454.1 [M+ H]+. |
| 197 | 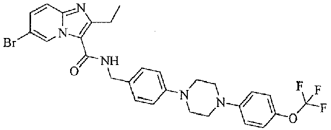 | white solid: ¹H-NMR (CDCl₃): δ 9.63 (1H, s), 7.49 (1H, d, *J* = 9.6 Hz), 7.39 (1H, d, *J* = 9.2 Hz), 7.31 (2H, d, *J* = 8.4 Hz), 7.14 (2H. d. *J* = 8.8 Hz), 6.87-7.05 (4H, m), 6.05 (1H, brs), 4.62 (2H, d, *J* = 5.6 Hz), 3.21-3.45 (8H. m), 2.96 (2H, q, *J* = 7.6 Hz), 1.40 (3H, t, *J* = 7.6 Hz); LCMS: 95.9%, MS (ESI): m/z 603.8[M+ H]+. |
| 198 | 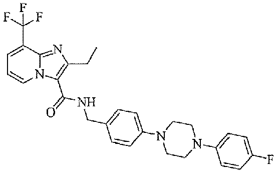 | white solid : ¹H-NMR (CDCl₃): δ 9.58 (1H, d, *J* = 6.8 Hz), 7.66 (1H, d, *J* = 6.8 Hz), 7.31 (2H, d, *J* = 8.4 Hz), 6.90-7.10 (7H, m), 6.10 (1H, brs), 4.64 (2H, d, *J =* 5.6 Hz), 3.30-3.42 (4H, m), 3.20-3.30 (4H, m), 3.03 (2H, q, *J* = 7.2 Hz), 1.38 (3H, t, *J* = 7.2 Hz); LCMS: 97.5%, MS (ESI): m/z 526.0[M + H]+. |
| 199 | 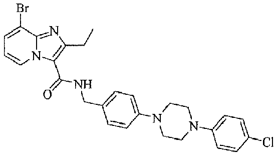 | white solid : ¹H-NMR (CDC13): δ 9.40 (1H, d, *J* = 7.2 Hz), 7.58 (1H, d, *J* = 7.2 Hz), 7.42 (2H, d, *J* = 7.2 Hz), 7.24 (2H, d, *J* = 8.8 Hz), 6.98 (2H, d, *J* = 8.0 Hz), 6.90 (2H, d, *J* = 8.8 Hz), 6.80 (1H, t, *J* = 6.8 Hz), 6.08 (1H, brs), 4.63 (2H, d, *J* = 5.2 Hz), 3.20-3.40 (8H, m), 3.02 (2H, q, *J* = 7.6 Hz), 1.38 (3H, t, *J* = 7.6 Hz); LCMS: 97.5%, MS(ESI):m/z 552.0/554.0 [M+H]+ |
| 200 | 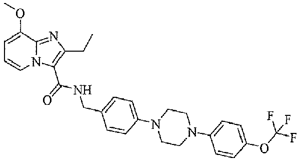 | yellow solid : ¹H-NMR (CDCl₃): δ 8.99 (1H, d, *J* = 6.8 Hz), 7.32 (2H, d, *J* = 8.0 Hz), 7.14 (2H, d, *J* = 8.8 Hz), 6.94-6.99 (4H, m), 6.82 (1H, t, *J* = 7.2 Hz), 6.62 (1H, d, *J* = 7.6 Hz), 6.05 (1H, brs), 4.63 (2H, d, *J* = 5.6 Hz), 4.02 (3H, s). 3.34 (8H, s), 2.98 (2H, q, *J* = 7.6 Hz), 1.38 (3H, t, *J* = 7.6 Hz); LCMS: 96.2%, MS (ESI): m/z 554.1 [M+ H]+. |
| 201 | 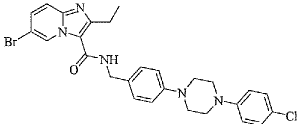 | white solid : ¹H-NMR (CDCl₃): δ 9.63 (1H, s), 7.49 (1H, d, *J* = 9.6 Hz), 7.39 (1H, d, *J* = 9.6 Hz), 7.31 (2H, d, *J* = 8.4 Hz), 7.19-7.26 (2H, m), 6.98 (2H, d, *J* = 8.4 Hz), 6.90 (2H, d, *J* = 8.8 Hz), 6.04 (1H, brs), 4.62 (2H, d, *J* = 5.6 Hz), 3.15-3.40 (8H, m), 2.96 (2H, q, *J* = 7.6 Hz), 1.42 (3H, t, *J* = 7.6 Hz); LCMS: 97.8%, MS (ESI): m/z 552.0[M+ H]+. |
| 202 | 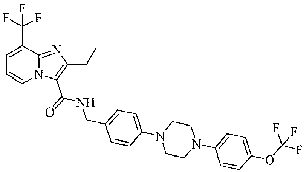 | white solid : ¹H-NMR (CDCl₃): δ 9.59 (1H, d, *J* = 6.8 Hz), 7.66 (1H, d, *J* = 7.2 Hz), 7.31 (2H, d, *J* = 8.4 Hz), 7.14 (2H, d, *J* = 8.4 Hz), 6.92-7.01 (5H, m), 6.12 (1H, brs), 4.64 (2H, d, *J* = 5.6 Hz), 3.17-3.54 (8H, m), 3.03 (2H, q, *J* = 7.6 Hz), 1.37 (3H, t, *J* = 7.6 Hz); LCMS: 97.9%, MS (ESI): m/z 592.0[M + H]+. |
| 203 | 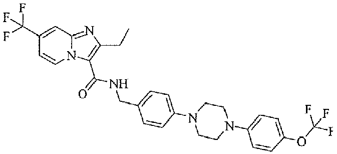 | white solid : ¹H-NMR (CDCl₃): δ 9.55 (1H, d, *J* = 7.2 Hz), 7.93 (1H, s), 7.34 (2H, d, *J =* 8.4 Hz), 7.16 (2H, d, *J* = 8.4 Hz), 7.11 (1H, dd, *J* = 7.6, 2.0 Hz), 6.96-7.03 (4H, m), 6.11 (1H, brs), 4.66 (2H, d, *J* = 5.6 Hz), 3.29-3.38 (8H, m), 3.01 (2H, q, *J* = 7.6 Hz), 1.44 (3H, t, *J =* 7.6 Hz); LCMS: 97.7%, MS (ESI): m/z 592.0[M+ H]+. |
| 204 | 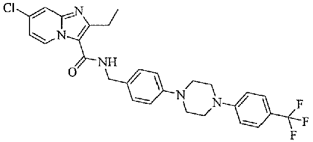 | white solid : ¹H-NMR (CDCl₃): δ 9.39 (1H, d, *J* = 7.2 Hz), 7.62 (1H, s), 7.53 (2H, d, *J* = 8.4 Hz), 7.33 (2H, d, *J* = 8.0 Hz), 7.00 (4H, d, *J* = 8.8 Hz), 6.93 (1H, dd, *J*₁ = 2.0 Hz, *J*₂ = 7.6 Hz), 6.04 (1H, brs), 4.64 (2H, d, *J* = 5.6 Hz), 3.30-3.50 (8H, m), 2.98 (2H, q, *J* = 7.6 Hz), 1.41 (3H, t, *J* = 7.6 Hz); LCMS: 95.6%, MS (ESI): m/z 542.1 [M+ H]+. |
| 205 | 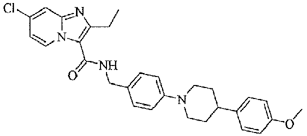 | white solid: ¹H-NMR (CDCl₃): δ 9.39 (1H, d, *J* = 7.6 Hz), 7.61 (2H, d, *J* = 1.6 Hz), 7.25-7.33 (2H, m), 7.20 (2H, d, *J* = 8.8 Hz), 7.00 (2H, d, *J* = 8.4 Hz), 6.85-6.95 (3H, m), 6.03 (1H, brs), 4.63 (2H, d, *J* = 5.6 Hz), 3.75-3.90 (5H, m), 2.98 (2H, q, *J* = 7.2 Hz), 2.80-2.90 (2H, m), 2.60-2.70 (1H, m), 1.83-2.02 (4H, m), 1.40 (3H, t, *J* = 7.6 Hz); LCMS: 97.3%, MS (ESI): m/z 525.1 [M+ Na]+. |
| 206 | 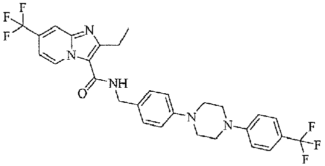 | white solid: ¹H-NMR (CDCl₃): δ 9.55 (1H, d, *J* = 7.2 Hz), 7.93 (1H, s), 7.53 (2H, d, *J* = 8.8 Hz), 7.34 (2H, d, *J* = 8.4 Hz), 7.11 (1H, dd, *J* = 7.2, 1.6 Hz), 6.97-7.04 (4H, m), 6.11 (1H, brs), 4.66 (2H, d, *J* = 5.2 Hz), 3.46-3.50 (4H, m), 3.31-3.40 (4H, m), 3.02 (2H, q, *J* = 7.6 Hz), 1.44 (3H, t, *J* = 7.6 Hz); LCMS: 97.2%, MS (ESI): m/z 576.0[M+ H]+. |
| 207 | 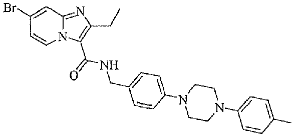 | white solid : ¹H-NMR (CDCl₃): δ 9.32 (1H, d, *J* = 7.2 Hz), 7.79 (1H, d, *J* = 2.0 Hz), 7.32 (2H, d, *J* = 8.8 Hz), 7.13 (2H. d. *J* = 8.0 Hz), 6.97-7.10 (3H, m), 6.89-7.00 (2H. m), 6.05 (1H, brs), 4.64 (2H, d, *J* = 5.2 Hz), 3.25-3.43 (8H, m), 2.98 (2H, q, *J* = 7.6 Hz), 1.41 (3H, t, *J* = 7.6 Hz); LCMS: 97.1%, MS (ESI): m/z 532.0[M+ H]+. |
| 208 | 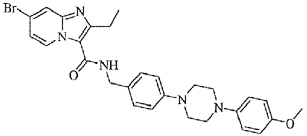 | white solid: ¹H-NMR (CDCl₃): δ 9.33 (1H, d, *J* = 7.2 Hz), 7.80 (1H, d, *J* = 1.6 Hz), 7.32 (2H, d, *J* = 8.8 Hz), 6.94-7.07 (5H, m), 6.90 (2H, d, *J* = 8.8 Hz), 6.04 (1H, brs), 4.64 (2H, d, *J* = 5.2 Hz), 3.90 (3H, s), 3.31-3.42 (4H, m), 3.20-3.30 (4H, m), 2.98 (2H, q, *J* = 7.6 Hz), 1.41 (3H, t, *J* = 7.6 Hz); LCMS: 95.4%, MS (ESI): m/z 548.0[M+ H]+. |
| 209 | 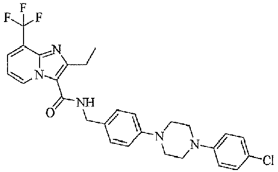 | yellow solid: ¹H-NMR (CDCl₃): δ 9.57 (1H, d, *J* = 7.2 Hz), 7.64 (1H, d, *J* = 7.2 Hz), 7.29 (2H, d, *J* = 8.8 Hz), 7.20-7.26 (2H, m), 6.94-6.98 (3H, m), 6.86-6.91 (2H, m), 6.08 (1H, brs), 4.62 (2H, d, *J* = 5.2 Hz), 3.28-3.35 (8H, m), 3.01 (2H, q, *J* = 7.6 Hz), 1.37 (3H, t, *J* = 7.6 Hz); LCMS: 97.4%, MS (ESI): m/z 542.0[M + H]+. |
| 210 | 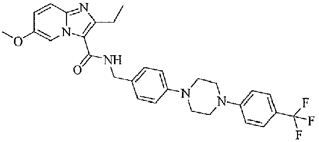 | white solid: ¹H-NMR (CDCl₃): δ 9.13 (1H, s), 7.45-7.60 (3H, m), 7.34 (2H, d, *J* = 8.4 Hz), 7.14 (2H, d, *J* = 8.8 Hz), 7.00 (4H, d, *J* = 8.0 Hz), 6.06 (1H, brs), 4.66 (2H, d, *J* = 5.2 Hz), 3.90 (3H, s), 3.40-3.50 (4H, m), 3.30-3.40 (4H, m), 2.98 (2H, q, *J* = 7.6 Hz), 1.41 (3H, t, *J* = 7.6 Hz); LCMS: 98.2%, MS(ESI):m/z 538.0 [M+H]+. |
| 211 | 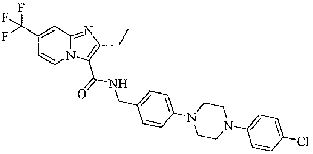 | gray solid: ¹H-NMR (CDCl₃): δ 9.51 (1H, d, *J* = 6.8 Hz), 7.89 (1H, s), 7.29 (2H, d, *J* = 15.2 Hz), 7.24-7.30 (2H, m), 7.07 (1H, d, *J* = 7.6 Hz), 7.00 (2H, dd, *J* = 12.0, 11.6 Hz), 6.89 (2H, m), 6.04 (1H, brs), 4.62 (2H, d, *J* = 5.6 Hz), 3.31-3.48 (4H, m), 3.25-3.30 (4H, m), 2.98 (2H, q, *J* = 7.6 Hz), 1.40 (3H, t, *J* = 7.6 Hz); LCMS: 97.4%, MS (ESI): m/z 541.8[M+ H]+. |
| 212 | 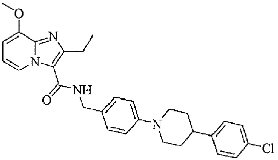 | white solid: ¹H-NMR (CDCl₃): δ 8.99 (1H, d, *J* = 6.8 Hz), 7.26-7.30 (4H, m), 7.17 (2H, d, *J* = 8.4 Hz), 6.98 (2H, d, *J* = 8.4 Hz), 6.81 (1H, t, *J* = 7.2 Hz), 6.61 (1H, d, *J* = 7.6 Hz), 6.04 (1H, brs), 4.61 (2H, d, *J* = *5*.2 Hz), 4.02 (3H, s), 3.80 (2H, d, *J* = 12 Hz), 2.99 (2H ,q, *J* = 7.6 Hz), 2.83 (2H, td, *J* = 2.4 Hz, *J* = 12 Hz), 2.67-2.60 (1H, m), 2.01-1.80 (4H, m), 1.38 (3H, t, *J* = 7.6 Hz); LCMS: 99.3%, MS (ESI): m/z 503.0[M+ H]+. |
| 213 | 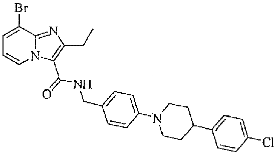 | yellow solid: ¹H-NMR (CDCl₃): δ 9.38 (1H, d, *J* = 6.8 Hz), 7.56 (1H, d, *J* = 7.2 Hz), 7.20-7.35 (4H, m), 7.16 (2H, d, *J* = 7.6 Hz), 6.97 (2H, d, *J* = 7.6 Hz), 6.78 (1H, t, *J* = 7.2 Hz), 6.07 (1H, brs), 4.60 (2H, d, *J* = 5.6 Hz), 3.80 (2H, d, *J* = 12.4 Hz), 3.02 (2H, q, *J* = 7.6 Hz), 2.81 (2H, td, *J* = 12.0, 2.4 Hz), 2.55-2.70 (1H, m), 1.75-2.00 (4H, m), 1.36 (3H, t, *J* = 7.6 Hz); LCMS: 100%, MS(ESI):m/z 550.8/552.8 [M+H] |
| 214 | 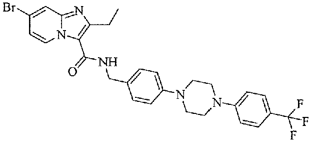 | white solid: ¹H-NMR (CDCl₃): δ 9.31 (1H, d, *J* = 7.6 Hz), 7.78 (1H, s), 7.12 (2H, d, *J* = 6.8 Hz), 7.31 (2H, d, *J* = 8.4 Hz), 6.81-7.09 (5H, m), 6.03 (1H, brs), 4.62 (2H, d, *J* = 5.2 Hz), 3.26-3.53 (8H, m), 2.87-3.05 (2H, m), 1.41 (3H, t, *J =* 7.6 Hz); LCMS: 98.2%, MS (ESI): m/z 588.0[M+ H]+. |
| 215 | 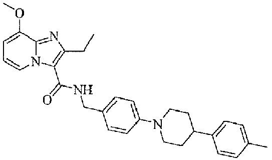 | yellow solid: ¹H-NMR (CDCl₃): δ 9.01 (1H, d, *J* = 6.8 Hz), 7.28-7.32 (2H, m), 7.14-7.18 (4H, m), 7.00 (2H, d, *J* = 8.8 Hz), 6.83 (1H, t, *J* = 7.2 Hz), 6.63 (1H, d, *J* = 7.6 Hz), 6.06 (1H, brs), 4.62 (2H, d, *J* = 5.6 Hz), 4.04 (3H, s), 3.82 (2H, d, *J*= 12.4 Hz), 3.01 (2H ,q, *J* = 7.6 Hz), 2.85 (2H, td, *J* = 2.8 Hz, *J* = 12 Hz), 2.61-2.68 (1H, m), 2.35 (3H, s), 1.85-1.98 (4H, m), 1.40 (3H, t, *J* = 7.6 Hz); LCMS: 97.8%, MS (ESI): m/z 483.1[M+ H]+. |
| 216 | 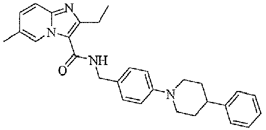 | yellow solid: ¹H-NMR (CDCl₃): δ 9.19 - 9.29 (1 H, m), 7.52 (1 H, d, *J*=9.16 Hz), 7.14 - 7.41 (8 H, m), 7.01 (2 H, d, *J*=8.66 Hz), 5.97 - 6.11 (1 H, m), 4.64 (2 H, d, *J*=5.40 Hz), 3.85 (2 H, d, *J*=12.30 Hz), 2.91 - 3.04 (2 H, m), 2.86 (2 H, d, *J*=2.76 Hz), 2.60 - 2.73 (1 H, m), 2.39 (3 H, s), 1.98 (4 H, br. s.), 1.41 (3 H, t, *J*=7.53 Hz); LCMS: 100%, MS (ESI): m/z 453.1 [M+ H]+. |
| 217 | 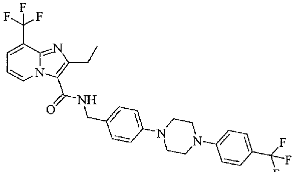 | yellow solid: ¹H-NMR (CDCl₃): δ 9.61 (1H, d, *J* = 6.8 Hz), 7.68 (1H, d, *J* = 7.2 Hz), 7.53 (2H, d, *J* = 8.4 Hz), 7.33 (2H, d, *J* = 8.4 Hz), 6.99-7.02 (5H, m), 6.13 (1H, brs), 4.66 (2H, d, *J* = 5.6 Hz), 3.31-3.49 (8H, m), 3.05 (2H, q, *J* = 7.6 Hz), 1.41 (3H, t, *J* = 7.6 Hz); LCMS: 98.6%, MS (ESI): m/z 576.0[M + H]+. |
| 218 | 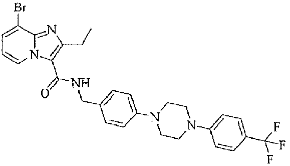 | white solid: ¹H-NMR (CDCl₃): δ 9.42 (1H, d, *J* = 6.0 Hz), 7.61 (1H, d, *J* = 7.6 Hz), 7.53 (2H, d, *J* = 8.4 Hz), 7.34 (2H, d, *J* = 8.8 Hz), 7.00 (4H, d, *J* = 8.8 Hz), 6.82 (1H, t, *J* = 7.2 Hz), 6.10 (1H, brs), 4.65 (2H, d, *J* = 5.6 Hz), 3.30-3.50 (8H, m), 3.04 (2H, q, *J* = 7.6 Hz), 1.38 (3H, t, *J* = 7.6 Hz); LCMS: 100%, MS(ESI):m/z 585.8/587.8 [M+H]⁺ |
| 219 | 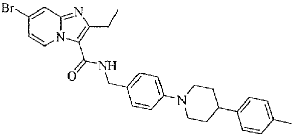 | white solid: ¹H-NMR (CDCl₃): δ 9.32 (1H, d, *J* = 7.6 Hz), 7.79 (1H, d, *J* = 1.6 Hz), 7.28-7.33 (2H, m), 7.13-7.20 (4H, m), 6.93-7.06 (3H. m), 6.04 (1H, brs), 4.63 (2H, d, *J* = 5.6 Hz), 4.56-4.67 (2H. m), 2.98 (2H, q, *J* = 7.6 Hz), 2.76-2.92 (2H, m), 2.60-2.70 (1H. m), 1.83-2.02 (4H. m), 1.41 (3H, t, *J* = 7.6 Hz); LCMS: 95.0%, MS (ESI): m/z 533.0[M+ H]⁺. |
| 220 | 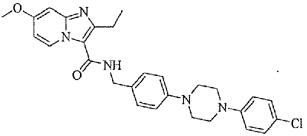 | yellow solid: ¹H-NMR (CDCl₃): δ 9.27 (1H, d, *J* = 7.6 Hz), 7.33 (2H, d, *J* = 8.8 Hz), 7.25 (2H, d, *J* = 8.8 Hz), 6.99 (2H, d, *J* = 8.8 Hz), 6.91 (3H, d, *J* = 8.8 Hz), 6.63 (1H, dd, *J* = 7.6 Hz, 2.4 Hz), 5.97 (1H, m), 4.63 (2H, d, *J* = 5.6 Hz), 3.89 (3H, s), 3.20-3.40 (8H, m), 2.94 (2H, q, *J* = 7.6 Hz), 1.41 (3H, t, *J* = 7.6 Hz); LCMS: 100.0%, MS (ESI): m/z 504.0[M+ H]+. |
| 221 | 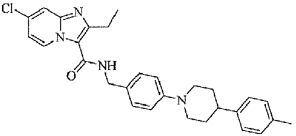 | white solid: ¹H-NMR (CDCl₃): δ 9.38 (1H, d, *J* = 7.6 Hz), 7.61 (1H, d, *J* = 1.6 Hz), 7.24-7.33 (2H, m), 7.13-7.20 (4H, m), 7.00 (2H, d, *J=* 8.4 Hz), 6.92 (1H, dd, *J*₁ = 2.4 Hz, *J*₂ = 7.6 Hz), 6.03 (1H, brs), 4.63 (2H, d, *J* = 5.6 Hz), 3.83 (2H, d, *J* = 12.4 Hz), 2.97 (2H, q, *J* = 7.6 Hz), 2.80-2.90 (2H, m), 2.59-2.72 (1H, m), 2.35 (3H, s), 1.85-2.02 (4H, m), 1.41 (3H, t, *J* = 7.6 Hz); LCMS: 97.2%, MS (ESI): m/z 486.8 [M+ H]+. |
| 222 | 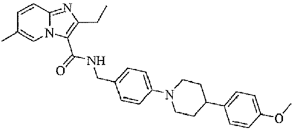 | yellow solid: ¹H-NMR (CDCl₃): δ 9.18 - 9.34 (m, 1 H), 7.48 - 7.58 (m, 1 H), 7.28 (s, 2 H) 7.17 - 7.23 (m, 3 H), 6.98 - 7.03 (m, 2 H), 6.90 (s, 2 H), 5.96 - 6.06 (m, 1 H), 4.64 (d, *J*=5.52 Hz, 2 H), 3.83 - 3.88 (m, 1 H), 3.82 (s, 4 H), 2.94 - 3.03 (m, 2 H), 2.79 - 2.90 (m, 2 H), 2.59 - 2.69 (m, 1 H), 2.39 (s, 3 H), 1.82 - 2.01 (m, 4 H), 1.42 (t, *J*=7.53 Hz, 3 H); LCMS: 100%, MS (ESI): m/z 483.0[M+ H]⁺. |
| 223 | 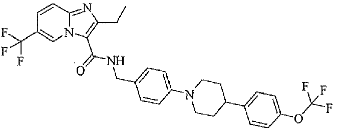 | white solid: ¹H-NMR (CDCl₃): δ 9.86 (1H, s), 7.69 (1H, d, *J* = 9.2 Hz), 7.46 (1H, dd, *J* = 7.6, 1.6 Hz), 7.25-7.32 (4H, m), 7.16 (2H, d, *J* = 8.0 Hz), 7.00 (2H, d, *J* = 8.8 Hz), 6.00-6.16 (1H, m), 4.63 (2H, d, *J* = 5.2 Hz), 3.83 (2H, d, *J* = 12.4 Hz), 2.99 (2H, q, *J* = 7.6 Hz), 2.79-2.89 (2H, m), 2.51-2.73 (1H, m), 1.78-2.02 (4H, m), 1.42 (3H, t, *J* = 7.6 Hz); LCMS: 100%, MS (ESI): m/z 591.0[M+ H]+. |
| 224 | 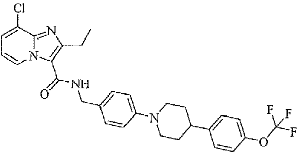 | yellow solid: ¹H-NMR (CDCl₃): δ 9.37 (1H, dd, *J₁* = 0.8 Hz, *J₂* = 6.8 Hz) , 7.41 (1H, dd, *J₁ =* 1.2 Hz, *J₂* = 7.6 Hz), 7.27-7.32 (4H, m), 7.18(2H, d, *J* = 8 Hz), 7.01 (2H, d, *J* = 8.4 Hz), 6.87 (1H, t, *J* = 7.2 Hz), 6.09 (1H, brs), 4.64 (2H, d, *J* = 5.2 Hz), 3.84 (2H, d, *J* = 12.4 Hz), 3.05 (2H, q, *J* = 7.6 Hz,), 2.86 (2H, t, *J* = 11.6 Hz), 2.70 (1H, t, *J* = 12 Hz), 1.88-1.99 (4H, m), 1.41 (3H, t, *J* = 7.6 Hz); LCMS: 100.00%, MS (ESI): m/z 556.9[M+ H]+. |
| 225 | 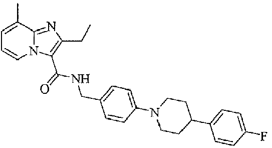 | yellow solid: ¹H-NMR (CDCl₃): δ 9.24 (1H, d, *J* = 7 Hz), 7.29 (2H, d, *J* = 8.5 Hz), 7.16-7.23 (2H, m), 7.11 (1H, d, *J* = 7 Hz), 6.95-7.03 (4H, m), 6.81 (1H, t, *J* = 6.9 Hz), 6.03 (1H, brs), 4.62 (2H, d, *J* = 5.3 Hz), 3.81 (2H, d, *J* = 12.3 Hz), 2.99 (2H, q, *J* = 7.5 Hz), 2.83 (2H, td, *J* = 12.2, 2.6 Hz), 2.62-2.69 (1H, m), 2.61 (3H, s), 1.79-1.98 (4H, m), 1.36 (3H, t, *J* = 7.5 Hz); LCMS: 100%, MS (ESI): m/z 471.1[M+ H]+. |
| 226 | 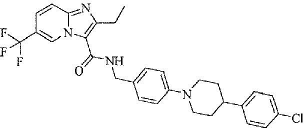 | white solid: ¹H-NMR (CDCl₃): δ 9.86 (1H, s), 7.69 (1H, d, *J* = 9.2 Hz), 7.47 (1H, dd, *J* = 7.6, 2.0 Hz), 7.28-7.33 (4H, m), 7.18 (2H, d, *J* = 8.4 Hz), 6.99 (2H, d, *J* = 8.4 Hz), 6.07 (1H, m), 4.62 (2H, d, *J* = 5.6 Hz), 3.81 (2H, m), 2.99 (2H, q, *J* = 7.6 Hz), 2.77-2.90 (2H, m), 2.58-2.70 (1H, m), 1.79-2.00 (4H, m), 1.41 (3H, t, *J* = 7.6 Hz); LCMS: 100%, MS (ESI): m/z 541.0[M+ H]⁺. |
| 227 | 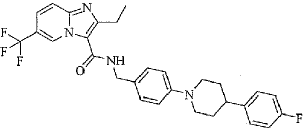 | yellow solid: ¹H-NMR (CDCl₃): δ 9.86 (1H, s), 7.69 (1H, d, *J* = 9.2 Hz), 7.47 (1H, dd, *J* = 7.6, 2.0 Hz), 7.25-7.38 (2H, m), 7.15-7.25 (2H, m), 6.91-7.09 (4H, m), 6.13 (1H, m), 4.63 (1H, d, *J* = 5.2 Hz), 3.82 (2H, d, *J* = 12.4 Hz), 3.00 (2H, q, *J* = 7.8 Hz), 2.78-2.90 (2H, m), 2.49-2.73 (1H, m), 1.78-2.03 (4H, m), 1.43 (3H, t, *J* = 7.6 Hz); LCMS: 100%, MS (ESI): m/z 525.0[M+ H]⁺. |
| 228 | 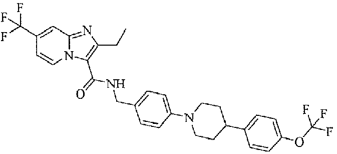 | white solid: ¹H-NMR (CDCl₃): δ 9.53 (1H, d, *J* = 7.6 Hz), 7.91 (1H, s), 7.29-7.52 (3H, m), 7.28 (1H, s), 7.16 (2H, d, *J* = 8.0 Hz), 7.10 (1H, dd, *J* = 7.6, 1.6 Hz), 6.96 (2H, d, *J* = 22.0 Hz), 6.08 (1H, brs), 4.63 (2H, d, *J* = 5.6 Hz), 3.82 (2H, d, *J* = 12.4 Hz), 3.00 (2H, q, *J* = 7.6 Hz), 2.79-2.82 (2H, m), 2.63-2.70 (1H, m), 1.84-1.89 (4H, m), 1.35 (3H, t, *J* = 7.6 Hz); LCMS: 100%, MS (ESI): m/z 590.8[M+ H]⁺. |
| 229 | 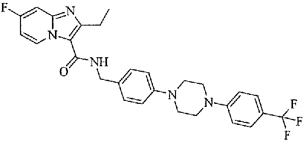 | white solid : ¹H-NMR (CDCl₃): δ 9.46(1H, t, *J* = 7.2 Hz), 7.53(2H, d, *J*= 8.8 Hz), 7.33(2H, d, *J* = 8.4Hz), 7.23-7.25(1H, m), 6.99-7.01(4H, m), 6.79-6.83(1H, m), 6.04(1H, brs), 4.64(2H, d, *J* = 5.6 Hz), 3.36-3.48(8H, m), 2.94-3.00(2H, m), 1.41(3H, t, *J* = 7.6 Hz); LCMS: 99.9%, MS (ESI): m/z 526.0[M+ H]+. |
| 230 | 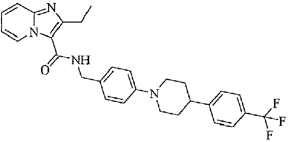 | white solid : ¹H-NMR (CDCl₃): δ 9.41 (1H, d, *J* = 7.2 Hz), 7.53-7.65 (3H, m), 7.28-7.40 (5H, m), 6.99 (2H, d, *J* = 8.4 Hz), 6.89-6.96 (1H, m), 5.97-6.09 (1H, m), 4.63 (2H, d, *J* = 5.6 Hz), 3.75-3.90 (2H, m), 2.98 (2H, q, *J* = 7.6 Hz), 2.80-2.90 (2H, m), 2.65-2.79 (1H, m), 1.85-2.01 (4H, m), 1.41 (3H, t, *J* = 7.6 Hz); LCMS: 100%, MS (ESI): m/z 507.0[M+ H]⁺. |
| 231 | 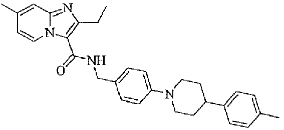 | white solid : ¹H-NMR (CDCl₃): δ 9.28 (1H, d, *J* = 7.2 Hz), 7.36 (1H, s), 7.30-7.26 (2H, m), 7.17-7.12 (4H, m), 6.98 (2H, d, *J* = 8.4 Hz), 6.75 (1H, dd, *J* = 7.2 Hz, 1.6 Hz), 4.61 (2H, d, *J* = 5.6 Hz), 3.73 (2H, d, *J* = 12.4 Hz), 2.94 (2H, q, *J* = 7.2 Hz), 2.81 (2H, td, *J* = 12 Hz, 2.4 Hz), 2.67-2.57 (1H, m), 2.42 (3H, s), 2.01-1.81 (4H, m), 1.39 (3H, t, *J* = 7.2 Hz). |
| 232 | 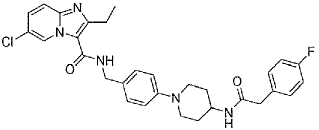 | white solid; ¹H-NMR (DMSO-d6, 400 MHz): δ 1.26 (3H, t, *J* = 7.6 Hz), 1.65-1.63 (2H, m), 1.92-1.89 (2H, m), 3.01 (2H, q, *J* = 7.6 Hz), 3.17 (1H, brs), 3.39 (2H, s), 3.62-3.59 (2H, m), 3.82 (1H, m), 4.49 (2H, d, *J* = 5.6 Hz), 7.10 (2H, t, *J* = 8.8 Hz), 7.29-7.25 (4H, m), 7.38-7.36 (2H, m), 7.65 (1H, dd, *J* = 9.2, 1.6 Hz), 7.78 (1H, d, *J* = 9.6 Hz), 8.19 (1H, d, *J* = 7.2 Hz), 8.70 (1H, t, *J* = 5.6 Hz), 9.11 (1H, s); LCMS: 99.7%, MS (ESI): m/z 548.2[M+ H]+. |
| 233 | 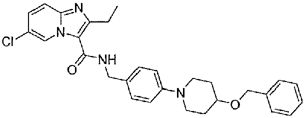 | yellow solid; ¹H-NMR (MeOD, 300 MHz): δ1.37 (3H, t, *J* = 7.5 Hz), 2.15-2.23 (4H, m), 3.10 (2H, q, *J* = 7.5 Hz), 3.51-3.57 (2H, m), 3.77-3.87 (3H, m), 4.63 (2H, s), 4.68 (2H, s), 7.29-7.41 (5H, m), 7.57-7.64 (4H, m), 7.76-7.81 (2H, m), 9.22 (1H, d, *J* = 9.0 Hz); LCMS: 98.9%, MS (ESI): m/z 503.2[M+ H]+. |
| 234 | 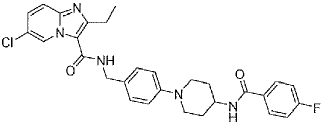 | white solid: ¹H-NMR (DMSO-d6, 400 MHz): δ1.29 (3H, t, *J* = 7.6 Hz), 1.79-1.81 (2H, m), 1.97-1.99 (2H, m), 3.02 (2H, q, *J* = 7.6 Hz), 3.15 (1H, m), 3.70-3.73 (2H, m), 4.05 (1H, m), 4.50 (2H, d, *J* = 5.6 Hz), 7.25-7.33 (4H, m), 7.36-7.38 (2H, m), 7.64 (1H, dd, *J* = 1.6 Hz, 9.6 Hz), 7.78 (1H, d, *J* = 9.6 Hz), 7.92-7.96 (2H, m), 8.42 (1H, d, *J* = 7.2 Hz), 8.65 (1H, t, *J* = 5.6 Hz), 9.12 (1H, d, *J* = 1.6 Hz); LCMS: 100%, MS (ESI): m/z 534.1 [M+ H]+. |
| 235 | 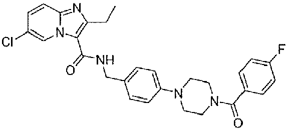 | white solid: ¹H-NMR (DMSO-d6, Bruker Avance 300 MHz): δ 1.25 (3H, t, *J* = 7.5Hz), 3.00 (2H, q, *J* = 7.5Hz), 3.08-3.28 (4H, m), 3.31-3.91 (4H, m), 4.43 (2H, d, *J* = 5.7 Hz), 6.95 (2H, d, *J* = 8.7 Hz), 7.20-7.33 (4H, m), 7.49 (2H, dd, *J* = 8.4, 5.4 Hz), 7.70 (1H, dd, *J* = 9.2, 1.8 Hz), 7.80 (1H, d, *J* = 9.2 Hz), 8.70 (1H, t, *J* = 5.7 Hz), 9.10 (1H, s); LCMS: 100%, MS (ESI): m/z 520.0 [M+ H]⁺. |
| 236 | 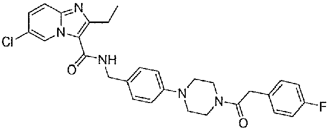 | white solid: ¹H-NMR (DMSO-d6, Bruker Advance 300 MHz): δ 1.24 (3H, t, J= 7.5Hz), 2.91-3.12 (6H, m), 3.51-3.65 (4H, m), 3.74 (2H, s), 4.42-4.44 (2H, m), 6.92 (2H, d, *J* = 8.7 Hz), 7.10 (2H, t, *J* = 8.8 Hz), 7.19-7.31 (4H, m), 7.69 (1H, dd, *J* = 9.6, 1.8 Hz), 7.78 (1H, d, *J* = 9.6 Hz), 8.66 (1H, t, *J* = 5.7 Hz), 9.10 (1H, s). LCMS: 100%, MS (ESI): m/z 534.0 [M+ H]⁺. |
| 237 | 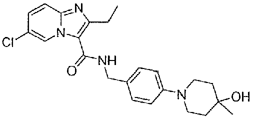 | pale yellow oil; ¹H-NMR (CD3OD, 300 MHz): δ1.32-1.41 (6H, m), 1.91-1.96 (2H, m), 2.02-2.13 (2H, m), 3.12 (2H, q, *J* = 7.5 Hz), 3.51-3.55 (2H, m), 3.82-3.91 (2H, m), 4.70 (2H, s), 7.66 (4H, s), 7.80-7.90 (2H, m), 9.24 (1H, s); LCMS: 98.4%, MS (ESI): m/z 427.1[M+ H]+. |
| 238 | 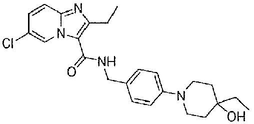 | pale yellow oil; ¹H-NMR (CD3OD, 300 MHz): δ0.99 (3H, t, *J* = 7.5 Hz), 1.39 (3H, t, *J* = 7.5 Hz), 1.62 (2H, q, *J* = 7.5 Hz), 1.90-2.08 (4H, m), 3.12 (2H, q, *J* = 7.5 Hz), 3.53-3.59 (2H, m), 3.83-3.91 (2H, m), 4.70 (2H, s), 7.66 (4H, s), 7.80-7.90 (2H, m), 9.25 (1H, s); LCMS: 99.0%, MS (ESI): m/z 440.2[M+ H]+. |
| 239 | 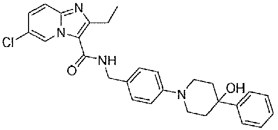 | white solid; ¹H-NMR (CD3OD, 400 MHz): δ1.40 (2H, t, *J* = 7.6 Hz), 2.11 (2H, d, *J* = 13.6 Hz), 2.50-2.61 (2H, m), 3.13 (2H, q, *J* = 7.6 Hz), 3.67 (2H, d, *J* = 12.4 Hz), 4.02-4.09 (2H, m), 4.72 (2H, s), 7.29 (1H, d, *J* = 7.6 Hz), 7.39 (2H, t, *J* = 8.0 Hz), 7.57 (2H, d, *J* = 7.2 Hz), 7.67-7.73 (4H, m), 7.81-7.89 (2H, m), 9.26 (1H, d, *J* = 0.8 Hz); LCMS: 99.9%, MS (ESI): m/z 489.2[M+ H]+. |
| 240 | 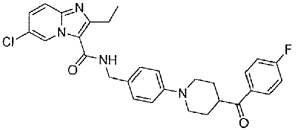 | white amorphous (powder); ¹H-NMR (DMSO-d6, Bruker Avance 400 MHz): δ 1.33 (3H, t, *J* = 7.2 Hz), 2.00-2.12 (2H, m), 2.13-2.30 (2H, m), 3.11 (2H, q, *J* = 7.6 Hz), 3.55-3.70 (5H, m), 4.59 (2H, d, *J* = 5.6 Hz), 7.43 (2H, t, *J* = 8.8 Hz), 7.57 (2H, d, *J* = 7.6 Hz), 7.65-7.78 (2H, m), 7.88-7.95 (2H, m), 8.12 (2H, dd, *J* = 8.8, 5.6 Hz), 9.11 (1H, brs), 9.19 (1H, s); LCMS: 100%, MS (ESI): m/z 519 [M+H]⁺. |
| 241 | 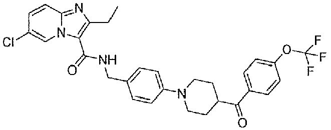 | white amorphous (powder); ¹H-NMR (MeOD, Bruker Avance 400 MHz): δ 1.43 (3H, t, *J* = 7.6 Hz), 2.16-2.38 (4H, m), 3.17 (2H, q, *J* = 7.6 Hz), 3.75-3.88 (4H, m), 3.90-4.01 (1H, m), 4.74 (2H, d, *J* = 4.4 Hz), 7.48 (2H, d, *J* = 8.4 Hz), 7.70 (4H, s), 7.89 (1H, d, *J* = 9.6 Hz), 8.00 (1H, dd, *J* = 9.6, 2.0 Hz), 8.22 (2H, d, *J* = 8.8 Hz), 8.95 (1H, t, *J* = 5.6 Hz), 9.31 (1H, s); LCMS: 100%, MS (ESI): m/z 584.8 [M+H]+. |
| 242 | 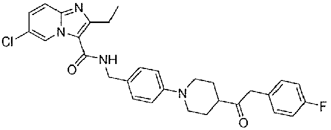 | white amorphous (powder); ¹H-NMR (MeOD, Bruker Avance 400 MHz): δ 1.42 (3H, t, *J* = 7.6 Hz), 2.03-2.18 (2H, m), 2.25-2.35 (2H, m), 3.04-3.12 (1H, m), 3.18 (2H, q, *J* = 7.6 Hz), 3.68-3.70 (4H, m), 3.93 (2H, s), 4.72 (2H, d, *J* = 2.8 Hz), 7.06 (2H, t, *J* = 8.8 Hz), 7.23-7.30 (2H, dd, *J* = 8.4, 5.2 Hz), 7.67 (4H, s), 7.93 (1H, d, *J* = 9.6 Hz), 8.08 (1H, dd, *J* = 9.6, 2.0 Hz), 9.05 (1H, t, *J* = 6.0 Hz), 9.32 (1H, d, *J* = 1.2 Hz); LCMS: 100%, MS (ESI): m/z 533.0 [M+H]+. |
| 243 | 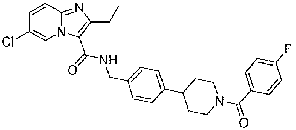 | white amorphous (gum); ¹H-NMR (DMSO-d6, Bruker Avance 400 MHz): δ 1.32 (3H, t, *J* = 7.6 Hz), 1.52-1.91 (4H, m), 2.75-2.92 (2H, m), 3.08 (2H, q, *J* = 7.6 Hz), 3.10-3.25 (1H, m), 3.58-3.72 (1H, m), 4.53 (2H, d, *J* = 1.6 Hz), 7.26-7.38 (6H, m), 7.51 (2H, dd, *J* = 8.4, 5.6 Hz), 7.95 (2H, s), 9.09 (1H, t, *J* = 5.6 Hz), 9.19 (1H, s); LC-MS purity: 100%. MS (ESI): m/z 519.1 [M+H]+. |
| 244 | 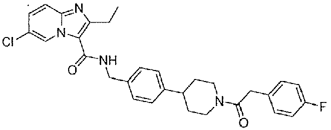 | white solid(sticky powder); mp = 216.2-220.7°C: ¹H-NMR (DMSO-d6, Bruker Avance 400 MHz): δ 1.32 (3H, t, *J* = 7.2 Hz), 1.37-1.50 (2H, m), 1.68-1.81 (2H, m), 2.58-2.82 (2H, m), 3.01-3.15 (3H, m), 3.74 (2H, s), 4.03-4.08 (1H, m), 4.51 (2H, d, *J* = 5.6 Hz), 4.54-4.58 (1H, m), 7.11-7.20 (4H, m), 7.24-7.40 (4H, m), 7.950 (2H, s), 9.06 (1H, brs), 9.19 (1H, s); LC-MS purity: 100%. MS (ESI): m/z 533.0 [M+H]⁺. |
| 245 | 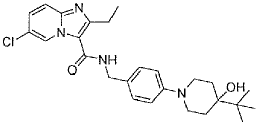 | white solid (powder); mp = 221.5-221.8°C: ¹H-NMR (DMSO-d6, 400 MHz): δ 0.85 (9H, s). 1.23 (3H, t, *J* = 7.6 Hz), 1.50 (2H, d, *J* = 12.4 Hz), 1.61-1.69 (2H, m), 2.86-2.98 (4H, m), 3.45 (2H, d, *J* = 9.6 Hz), 3.92 (1H, s), 4.40 (2H, d, *J* = 5.6 Hz), 6.89 (2H, d, *J* = 8.8 Hz), 7.19 (2H, d, *J* = 8.4 Hz), 7.44 (1H, dd, *J* = 2.0 Hz, 9.2 Hz), 7.65 (1H, d, *J* = 9.6 Hz), 8.39 (1H, t, *J* = 5.6 Hz), 9.05 (1H, s); LCMS: 97.4%, MS (ESI): m/z 440.2[M+ H]+. |
| 246 | 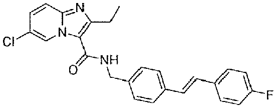 | white amorphous (powder); ¹H-NMR (DMSO-d6, 300 MHz): δ 1.23 (3H, t, *J* = 7.5 Hz), 2.97 (2H, q, *J* = 7.5 Hz), 4.50 (2H, d, *J* = 5.7 Hz), 7.10-7.23 (4H, m), 7.34 (2H, d, *J* = 8.1 Hz), 7.43 (1H, dd, *J* = 9.6, 1.5 Hz), 7.50-7.70 (5H, m), 8.48 (1H, t, *J* = 5.7 Hz), 9.04 (1H, s); LCMS: 98.7%, MS (ESI): m/z 433.9 [M+ H]⁺. |
| 247 | 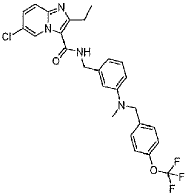 | white power; mp >142.3 °C: decomposed; ¹H-NMR (DMSO-d6, 400 MHz): δ 1.23 (3H, t, *J* = 7.6 Hz), 2.94 (2H, q, *J* = 7.6 Hz), 2.97 (3H, s), 4.45 (2H, d, *J* = 6.0 Hz), 4.59 (2H, s), 6.60-6.70 (2H, m), 6.76 (1H, s), 7.12 (1H, t, *J* = 8.0 Hz), 7.24 (2H, d, *J* = 8.4 Hz), 7.29 (2H, d, *J* = 8.8 Hz), 7.45 (1H, dd, *J* = 9.2, 2.0 Hz), 7.66 (1H, d, *J* = 9.6 Hz), 8.43 (1H, t, *J* = 6.0 Hz), 9.04 (1H, d, *J* = 1.2 Hz); LCMS: 98.6%, MS (ESI): m/z 517.0 [M+H]+. °C |
| 248 | 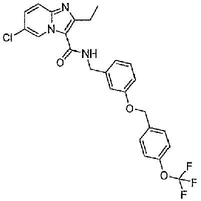 | white powder; mp >142.7°C: decomposed; ¹H-NMR (DMSO-d6, 400 MHz): δ 1.26 (3H, t, *J* = 7.6 Hz), 2.99 (2H, q, *J* = 7.6 Hz), 4.51 (2H, d, *J* = 5.6 Hz), 5.14 (2H, s), 6.90 (1H, dd, *J* = 8.4, 2.0 Hz), 6.94 (1H, d, *J* = 7.6 Hz), 7.03 (1H, s), 7.27 (1H, t, *J* = 8.0 Hz), 7.35 (2 H, d, *J* = 8.0 Hz), 7.45 (1H, dd, *J* = 9.2, 2.0 H, 7.56 (2H, d, *J* = 8.8 Hz), 7.67 (1H, d, *J* = 9.6 Hz), 8.47 (1H, t, *J* = 5.6 Hz), 9.07 (1H, d, *J* = 1.2 Hz); LCMS: 96.1%, MS (ESI): m/z 504.1[M+ H]+. |
| 249 | 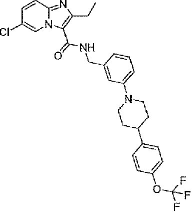 | white solid; mp = 135.5-136.4°C: ¹H-NMR (DMSO-d6, 400 MHz): δ 1.26 (3H, t, *J* = 7.6 Hz), 1.69-1.79 (2H, m), 1.80-1.90 (2H, m), 2.70-2.80 (3H, m), 2.99 (2H, q, *J* = 7.6 Hz), 3.75-3.85 (2H, m), 4.49 (2H, d, *J* = 6.0 Hz), 6.78 (1H, d, *J* = 7.6 Hz), 6.88 (1H, d, *J* = 8.0 Hz), 7.01 (1H, s), 7.19 (1H, t, *J* = 8.0 Hz), 7.28 (2H, d, *J* = 8.0 Hz), 7.39-7.47 (3H, m), 7.66 (1H, d, *J* = 9.6 Hz), 8.51 (1H, t, *J* = 6.0 Hz), 9.03 (1H, d, *J* = 1.2 Hz); LCMS: 100%, MS (ESI): m/z 557.0 [M+ H]+. |
| 250 | 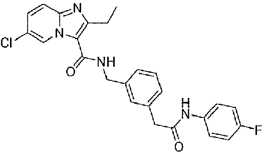 | white amorophous; mp >198.8°C: decomposed; ¹H-NMR (DMSO-d6, 400 MHz): δ 1.24 (3 H, t-*J* = 7.2 Hz), 2.98 (2H, q, *J* = 7.6 Hz), 3.62 (2H, s), 4.53 (2H, d, *J* = 6.0 Hz), 7.09 (2H, t, *J* = 9.2 Hz), 7.10-7.35 (4H, m), 7.46 (1H, dd, J= 9.2, 2.0 Hz), 7.58 (2H, dd *, J* = 9.5, 5.2 Hz), 7.66 (1H, d, *J* = 9.2 Hz), 8.50 (1H, brs, *J* = 6.0 Hz), 9.07(1H, d, *J* = 1.6 Hz), 10.21 (1H, brs); LCMS: 99%, MS (ESI): m/z 465.1 [M+ H]+. |
| 251 | 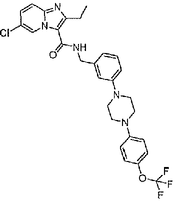 | white amorophous; mp >168.0°C: decomposed; ¹H-NMR (DMSO-d6, 400 MHz): δ 1.27 (3 H, t-*J* = 7.6 Hz), 3.00 (2H, q, *J* = 7.2 Hz), 3.20-3.30 (8H, m), 4.50 (2H, d, *J* = 6.0 Hz), 4.50 (2H, d, *J* = 6.0 Hz), 6.83 (1H, d, *J* = 7.6 Hz), 6.91 (1H, d, *J* = 8.4 Hz), 7.02 (1 H, s), 7.07 (2H, d, *J* = 8.8 Hz), 7.20-7.26 (3H, m), 7.45 (1H, dd, *J* = 9.6, 2.0 Hz), 8.50 (1H, t, *J* = 6.0 Hz), 9.04 (1H, d, *J* = 1.6 Hz); LCMS: 95.2%, MS (ESI): m/z 580.1 [M+ Na]+. |
| 252 | 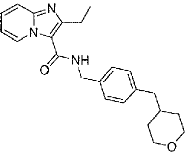 | Gum; ¹H-NMR (CDCl3, 400 MHz): δ 1.20-1.40 (2H, m), 1.41 (3H, t, *J* = 7.2 Hz), 1.50-1.59 (2H, m), 1.68-1.82 (1H, m), 2.55 (2 H, d, *J* = 6.8 Hz), 2.99 (2H, q, *J* = 7.6 Hz), 3.28-3.38 (2H, m), 3.90-4.00 (2H, m), 4.68 (2 H, d, *J* = 5.2 Hz), 6.08 (1H, brs), 6.92 (1H, t, *J* = 6.8 Hz), 7.15 (2 H, d, *J* = 8.0 Hz), 7.26-7.40 (3H, m), 7.61 (1H, d, *J* = 8.8 Hz), 9.41 (1H, d, *J* = 6.8 Hz); LCMS: 98.96%, MS (ESI): m/z 377.8 [M+ H]⁺. |
| 253 | 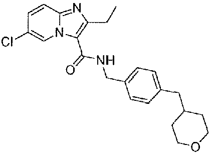 | white solid; mp = 132.2-133.0°C: ¹H-NMR (CDCl3, 400 MHz): δ 1.25-1.40 (2H, m), 1.44 (3H, t, *J* = 7.6 Hz), 1.52-1.59 (2H, m), 1.70-1.85 (1H, m), 2.57 (2H, d, *J* = 7.2 Hz), 3.00 (2H, q, *J* = 7.6 Hz), 3.30-3.40 (2H, m), 3.90-4.00 (2H, m), 4.69 (2H, d, *J* = 5.6 Hz), 6.11 (1H, brs), 7.18 (2H, d, *J* = 8.0 Hz), 7.30-7.40 (3H, m), 7.57 (1H, d, *J* = 9.6 Hz), 9.56 (1H, d, *J* = 1.6 Hz); LCMS: 100%, MS (ESI): m/z 411.8 [M+ H]⁺. |
| 254 | 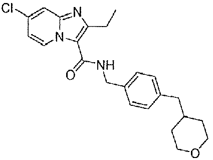 | white amorphous; mp >125.9°C: decomposed; ¹H-NMR (CDCl3, 400 MHz): δ 1.20-1.40 (2H, m), 1.40 (3H, t, *J* = 7.2 Hz), 1.50-1.60 (2H, m), 1.68-1.83 (1H, m), 2.56 (2H, d, *J* = 7.2 Hz), 2.97 (2H, q, *J* = 7.6 Hz), 3.28-3.40 (2H, m), 3.90-4.00 (2H, m), 4.67 (2H, d, *J* = 5.6 Hz), 6.08 (1H, brs), 6.91 (1H, dd, *J* = 7.6, 2.0 Hz), 7.15 (2H, d, *J* = 8.0 Hz), 7.26-7.38 (2H, m), 7.59 (1H, d, *J* = 1.6 Hz), 9.36 (1H, d, *J* = 7.2 Hz); LCMS: 99.86%, MS (ESI): m/z 411.7 [M+ H]+. |
| 255 | 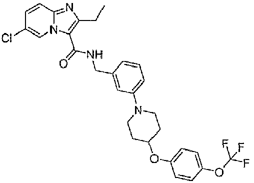 | yellow solid; mp = 126.3-127.2°C: ¹H-NMR (DMSO-d6, 400 MHz): δ 1.24 (3H, t, *J* = 7.6 Hz), 1.65-1.73 (2H, m), 1.95-2.05 (2H, m), 2.97 (2H, t, *J* = 7.2 Hz), 3.00-3.07 (2H, m), 3.45-3.55 (2H, m), 4.47 (2H, d, *J* = 6.0 Hz), 4.55-4.59 (1H, m), 6.76 (1H, d, *J* = 7.2 Hz), 6.84-6.86 (1H, m), 6.97 (1H, s), 7.06 (2H, d, *J* = 9.2 Hz), 7.17 (1H, t, *J* = 8.0 Hz), 7.26 (2H, d, *J* = 8.8 Hz), 7.41-7.44 (1H, dd, *J* = 9.6, 2.0 Hz), 7.64 (1H, d, *J* = 9.2 Hz), 8.49 (1H, t, *J* = 5.6 Hz), 9.01 (1H, d, *J* = 1.6 Hz); LCMS: 96.4%, MS (ESI): m/z 573.1[M+ H]+. |
| 256 | 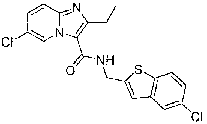 | white solid; mp >220°C: ¹H-NMR (DMSO-d6, 400 MHz): δ 1.28 (3H, t, *J* = 7.6 Hz), 3.00 (2H, q, *J* = 7.6 Hz), 4.79 (2H, d, *J* = 5.6 Hz), 7.33 (1H, dd, *J* = 8.4, 2.0 Hz), 7.37 (1H, s), 7.48 (1H, dd, *J* = 9.6, 2.0 Hz), 7.68 (1H, d, *J* = 9.2 Hz), 7.90 (1H, d, *J* = 2.0 Hz), 7.96(1 H, d, *J* = 8.4 Hz), 8.70 (1H, t, *J* = 5.6 Hz), 9.12 (1H, d, *J* = 1.6 Hz); LCMS: 95.4%, MS (ESI): m/z 404.0 [M+ H]+. |
| 257 | 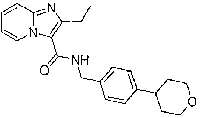 | White amorphous; mp >133.7°C: decomposed; ¹H-NMR (CDCl3, 400 MHz): δ 1.42 (3H, t, *J* = 7.6 Hz), 1.72-1.90 (4H, m), 2.70-2.82 (1H, m), 3.00 (2H, q, *J* = 7.6 Hz), 3.53 (2H, td, *J* = 11.6, 2.8 Hz), 3.93 (2H, dd, *J* = 10.8, 2.8 Hz), 4.68 (2H, d, *J* = 6.0 Hz), 6.09 (1H, brs), 6.90-6.95 (1H, m), 7.21-7.30 (2H, m), 7.30-7.40 (3H, m),7.61 (1H, d, *J* = 9.2 Hz), 9.41 (1H, d, *J* = 7.2 Hz); LCMS: 99.27%, MS (ESI): m/z 364.1 [M+ H]+. |
| 258 | 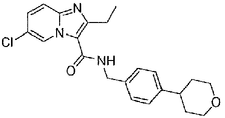 | White amorphous; mp >195.5°C: decomposed; ¹H-NMR (CDCl3, 400 MHz): δ 1.41 (3H, t, *J* = 7.6 Hz), 1.71-1.90 (4H, m), 2.72-2.84 (1H, m), 2.98 (2H, q, *J* = 7.2 Hz), 3.53 (2H, td, *J* = 11.6, 2.8 Hz), 4.08 (2H, dd, *J* = 11.2, 3.6 Hz), 4.68 (2 H, d, *J* = 5.6 Hz), 6.10 (1H, brs), 7.20-7.28 (2H, m), 7.28-7.49 (3H, m), 7.54 (1H, d, *J* = 9.6 Hz), 9.54 (1H, d, *J* = 1.6 Hz); LCMS: 100%, MS (ESI): m/z 398.1 [M+ H]+. |
| 259 | 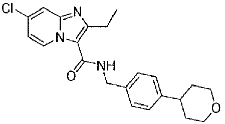 | White amorphous; mp >156.6°C: decomposed; ¹H-NMR (CDC13, 400 MHz): δ 1.40 (3H, t, *J* = 7.6 Hz), 1.71-1.90 (4H, m), 2.72-2.84 (1H, m), 2.97 (2H, q, *J* = 7.6 Hz), 3.53 (2H, td, *J* = 11.6, 2.8 Hz), 4.05-4.15 (2H, m), 4.67 (2H, d, *J* = 6.0 Hz), 6.09 (1H, brs), 6.91 (1H, dd, *J* = 7.6, 2.4 Hz), 7.23-7.26 (2H, m), 7.33 (2H, ,d, *J* = 8.0 Hz), 7.59 (1H, d, *J* = 2.0 Hz), 9.36 (1H, d, *J* = 7.2 Hz); LCMS: 100%, MS (ESI): m/z 398.1 [M+ H]+. |
| 260 | 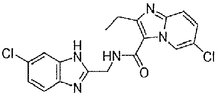 | yellow amorphous; ¹H-NMR (DMSO-d6, 400 MH°Cz): δ 1.31 (3H, t, *J =* 7.6 Hz), 3.03 (2H, q, *J* = 7.6 Hz), 4.76 (2H, s), 7.18 (1H, dd, *J* = 8.4, 2.0 Hz), 7.45-7.55 (2H, m), 7.56 (1H, d, *J* = 2.0 Hz), 7.68-7.70 (1H, m), 8.57 (1H, brs), 9.24 (1H, d, *J* = 1.6 Hz); LCMS: 100%, MS (ESI): m/z 388.1[M+H]+. |
| 261 | 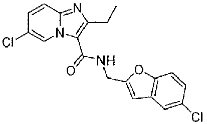 | white solid; mp = 223.5-225.6°C: ¹H-NMR (DMSO-d6, 400 MHz): δ 1.27 (3H, t, *J* = 7.6 Hz), 3.00 (2H, q, *J* = 7.2 Hz), 4.70 (2H, d, *J* = 5.6 Hz), 6.81 (1H, s), 7.29 (1H, dd, *J* = 8.8, 2.4 Hz), 7.47 (1H, dd, *J* = 9.6, 2.0 Hz), 7.58 (1H, d, *J* = 8.4 Hz), 7.68 (2H, dd, *J* = 5.6, 3.6 Hz), 8.59 (1H, t, *J* = 5.2 Hz), 9.08 (1H, d, *J* = 1.6 Hz); LCMS: 99.3%, MS (ESI): m/z 387.8[M+ H]+. |
| 262 | 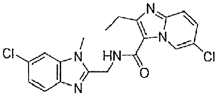 | white solid; mp >220°C: ¹H-NMR (DMSO-d6, 400 MHz): δ 1.29 (3H, t, *J* = 7.6 Hz), 3.00 (2H, q, *J* = 7.6 Hz), 3.85 (3H, s), 4.84 (2H, d, *J* = 5.6 Hz), 7.21 (1H, dd, *J* = 8.4, 2.0 Hz), 7.47 (1H, dd, *J* = 9.2, 2.0 Hz), 7.59 (1H, d, *J* = 8.8 Hz), 7.68 (1H, d, *J* = 9.6 Hz), 7.74 (1H, d, *J* = 2.0 Hz), 8.65 (1H, t, *J* = 5.6 Hz), 9.26 (1H, d, *J* = 1.6 Hz); LCMS: 100%, MS (ESI): m/z 402.0[M+ H]+. |
| 263 | 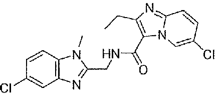 | white solid; mp >220°C: ¹H-NMR (DMSO-d6, 400 MHz): δ 1.28 (3H, t, *J* = 7.6 Hz), 3.01 (2H, q, *J* = 7.6 Hz), 3.86 (3H, s), 4.84 (2H, d, *J* = 5.6 Hz), 7.28 (1H, dd, *J* = 8.4, 2.0 Hz), 7.47 (1H, dd, *J* = 9.2, 2.0 Hz), 7.59-7.71 (3H, m), 8.66 (1H, t, *J* = 5.6 Hz), 9.24 (1H, d, *J* = 1.6 Hz); LCMS: 98.7%, MS (ESI): m/z 401.9[M+ H]+. |
| 264 | 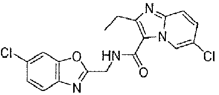 | white solid; mp = 201.1-201.8°C: ¹H-NMR (DMSO-d6, 400 MHz): δ 1.31 (3H, t, *J* = 7.2 Hz), 3.05 (2H, q, *J* = 7.2 Hz), 4.84 (2H, d, *J* = 5.6 Hz), 7.43 (1H, dd, *J* = 8.4, 2.0 Hz), 7.49 (1H, dd, *J* = 9.6, 2.0 Hz), 7.69 (1H, d, *J* = 5.6 Hz), 7.71 (1H, d, *J* = 9.6 Hz), 7.74 (1H, d, *J* = 8.8 Hz), 7.95 (1H, d, *J* = 2.0 Hz), 8.68 (1H, t, *J* = 5.6 Hz), 9.13 (1H, d, *J* = 1.6 Hz); LCMS: 98.6%, MS (ESI): m/z 389.0[M+ H]+. |
| 265 | 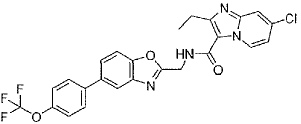 | white solid; mp >220°C: ¹H-NMR (DMSO-d6, 400 MHz): δ 1.32 (3H, t, *J* = 7.6 Hz), 3.06 (2H, q, *J* = 7.6 Hz), 4.86 (2H, d, *J* = 4.4 Hz), 7.12 (1H, dd, *J* = 7.6, 2.4 Hz), 7.46 (2H, d, *J* = 8.4 Hz), 7.69 (1H, dd, *J* = 8.4, 1.6 Hz), 7.79-7.86 (4H, m), 8.02 (1H, d, *J* = 1.2 Hz), 8.70 (1H, brs), 9.01 (1H, d, *J* = 7.6 Hz); LCMS: 98.2%, MS (ESI): m/z 515.1[M+ H]+. |
| 266 | 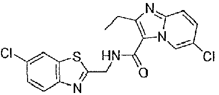 | white solid; mp >220°C: ¹H-NMR (DMSO-d6, 400 MHz): δ 1.33 (3H, t, *J* = 7.6 Hz), 3.07 (2H, q, *J* = 7.6 Hz), 4.93 (2H, d, *J* = 6.0 Hz), 7.50 (1H, dd, *J* = 9.6, 2.0 Hz), 7.55 (1H, dd, *J* = 8.8, 2.4 Hz), 7.70 (1H, dd, *J* = 9.6, 0.8 Hz), 7.97 (1H, d, *J* = 8.8 Hz), 8.24 (1H, d, *J* = 2.0 Hz), 8.88 (1H, t, *J* = 6.0 Hz), 9.15 (1H, dd, *J* = 2.4, 0.8 Hz); LCMS: 100%, MS (ESI): m/z 405.0 [M+H]+. |
| 267 | 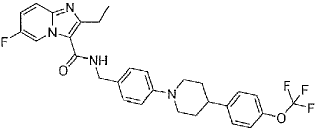 | yellow amorphous; mp >167.9°C: decomposed; ¹H-NMR (CDC13, 400 MHz): δ 1.40 (3H, t, *J* = 7.6 Hz), 1.80-1.99 (4H, m), 2.65-2.72 (1H, m), 2.75-2.87 (2H, m), 2.97 (2H, q, *J* = 7.6 Hz), 3.75-3.85 (2H, m), 4.62 (2H, d, *J* = 5.2 Hz), 6.03 (1H, brs), 6.99 (2H, d, *J* = 8.8 Hz), 7.16 (2H, d, *J* = 8.4 Hz), 7.25-7.32 (5H, m), 7.56 (1H, dd, *J* = 10.0, 5.2 Hz), 9.46 (1H, dd, *J* = 5.2, 2.4 Hz); LCMS: 98.7%, MS (ESI): m/z 541.3 [M+ H]+. |
| 268 | 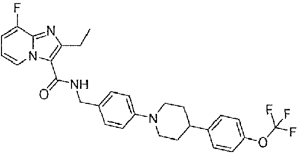 | white amorphous; mp >177.7°C: decomposed; ¹H-NMR (CDCl3, 400 MHz): δ 1.39 (3H, *t, J* = 7.6 Hz), 1.83-1.96 (4H, m), 2.63-2.69 (1H, m), 2.70-2.90 (2H, m), 2.97 (2H, q, *J* = 7.6 Hz), 3.75-3.85 (2H, m), 4.60 (2H, d, *J* = 5.2 Hz), 6.04 (1H, brs), 6.78-6.84 (1H, m), 6.95-6.99 (3H, m), 7.14 (2H, d, *J* = 8.8 Hz), 7.23-7.29 (4H, m), 9.18 (1H, dd, *J* = 6.8, 0.8 Hz); LCMS: 99.5%, MS (ESI): m/z 541.3 [M+ H]+. |
| 269 | 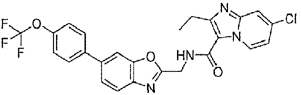 | white solid; mp >220°C: ¹H-NMR (DMSO-d6, 400 MHz): δ 1.32 (3H, t, *J* = 7.2 Hz), 3.07 (2H, q, *J* = 7.2 Hz), 4.86 (2H, d, *J* = 5.6 Hz), 7.12 (1H, dd, *J* = 7.6, 2.4 Hz), 7.46-7.50 (2H, m), 7.69 (1H, dd, *J* = 8.4, 0.8 Hz), 7.79-7.80 (4H, m), 8.06(1H, d, *J* = 1.2 Hz ), 8.69 (1H, t, *J* = 6.0 Hz ), 9.01 (1H, dd, *J* = 7.6, 0.8 Hz); LCMS: 99.4%, MS (ESI): m/z 515.2 [M+ H]+. |
| 270 | 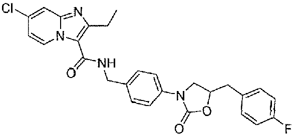 | white amorphous; mp > 96.9°C: decomposed; ¹H-NMR (CDCl3, 400 MHz): δ 1.38 (3H, t, *J* = 7.6 Hz), 2.95 (2H, q, *J* = 7.6 Hz), 3.02 (1H, dd, *J* = 14.4, 6.4 Hz), 3.15 (1H, dd, *J* = 14.0, 6.0 Hz), 3.70 (1H, dd, *J* = 8.8, 6.8 Hz), 4.02 (1H, t, *J* = 8.8 Hz), 4.65 (2H, d, *J* = 5.6 Hz), 4.82-4.90 (1H, m), 6.05 (1H, t, *J* = 2.8 Hz), 6.89 (1H, dd, *J* = 7.6, 2.4 Hz), 6.99-7.05 (2H, m), 7.21-7.26 (2H, m), 7.36 (2H, dd, *J* = 6.8, 2.0 Hz), 7.46 (2H, dd, *J* = 6.4, 2.0 Hz), 7.58 (1H, dd, *J* = 2.0, 0.8 Hz), 9.36 (1H, d, *J* = 0.8 Hz); LCMS: 100%, MS (ESI): m/z 507.0 [M+ H]+. |
| 271 | 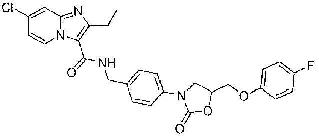 | white amorphous; mp > 97.10°C: ¹H-NMR (CDCl3, 400 MHz): δ 1.27 (3H, t, *J* = 7.6 Hz), 3.00 (2H, q, *J* = 7.6 Hz), 3.89-3.94 (1H, m), 4.19-4.30 (3H, m), 4.51 (2H, d, *J* = 6.0 Hz), 5.01-.06 (1H, m), 6.95-7.00 (2H, m), 7.08-7.22 (3H, m), 7.40 (2H, d, *J* = 8.8 Hz), 7.56 (2H, d, *J* = 8.8 Hz), 7.85 (1H, s), 8.58 (1H, t, *J* = 7.2 Hz), 8.97 (1H, dd, *J* = 7.2, 0.4 Hz); LCMS: 97.1%, MS (ESI): m/z 523.3 [M+ H]+. |
| 272 | 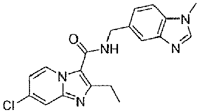 | Brown solid: ¹H-NMR (400 MHz, DMSO): δ 8.96 (d, *J* = 7.6 Hz, 1H), 8.56 - 8.50 (m, 1H), 8.15 (s, 1H), 7.78 (s, 1H), 7.62 (t, *J* = 5.2 Hz, 1H), 7.53 (d, *J* = 8.0 Hz, 1H), 7.33 - 7.24 (m, 1H), 7.10 - 7.07 (m, 1H), 4.65 (dd, *J* = 13.2, 6.4 Hz, 2H), 3.82 (s, 3H), 3.02 - 2.94 (m, 2H), 1.28 - 1.23 (m, 3H); LCMS (electrospray) m/z (M+H)+ |
| 273 | 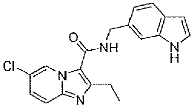 | Brown solid: ¹H-NMR (400 MHz, DMSO): δ 11.03 (s, 1H), 9.08 (d, *J* = 2.0 Hz, 1H), 8.50 (t, *J* = 6.0 Hz, 1H), 7.67 (d, *J* = 9.2 Hz, 1H), 7.50 (d, *J* = 8.0 Hz, 1H), 7.45 (dd, *J* = 9.2, 2.0 Hz, 1H), 7.39 (s, 1H), 7.30 (t, *J* = 2.8 Hz, 1H), 7.03 (dd, *J* = 8.0, 1.2 Hz, 1H), 6.38 (t, *J* = 2.4 Hz, 1H), 4.61 (d, *J* = 6.0 Hz, 2H), 2.99 (q, *J* = 14.4, 7.6 Hz, 2H), 1.26 (t, *J* = 7.6 Hz, 3H); LCMS (electrospray) m/z (M+H)+ |
| 274 | 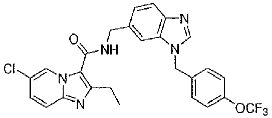 | White solid: ¹H-NMR (400 MHz, DMSO): δ 9.06 (d, *J* = 2.0 Hz, 1H), 8.51 -8.43 (m, 1H), 8.40 (s, 1H), 7.68 - 7.63 (m, 2H), 7.50 -7.43 (m, 2H), 7.40 (d, *J* = 8.8 Hz, 2H), 7.34 (t, *J* = 8.8 Hz, 2H), 7.27 (d, *J* = 8.4 Hz, 1H), 5.34 (s, 2H), 4.62 (d, *J* = 5.6 Hz, 2H), 3.00 -2.93 (m, 2H), 1.26 -1.19 (m, 3H); LCMS (electrospray) m/z (M+H)+ 528.26 |
| 275 | 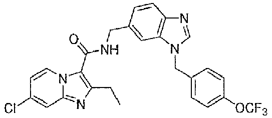 | White solid: ¹H-NMR (400 MHz, DMSO): δ 8.95 (d, *J* = 7.2 Hz, 1H), 8.60 -8.41 (m, 1H), 8.40 (s, 1H), 7.78 (t, *J* = 5.2 Hz, 1H), 7.66 (s, 1H), 7.49 (d, *J* = 8.4 Hz, 1H), 7.40 (d, *J* = 8.8 Hz, 2H), 7.37 -7.31 (m, 1H), 7.29 -7.25 (m, 2H), 7.09 -7.06 (m, 1H), 5.34 (s, 2H), 4.61 (d, *J* = 5.6 Hz, 2H), 2.99 -2.91 (m, 2H), 1.25 -1.09 (m, 3H); LCMS (electrospray) m/z (M+H)+ 528.26 |
| 276 | 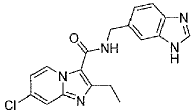 | Brown solid: ¹H-NMR (400 MHz, DMSO): δ 12.40 (s, 1H), 8.96 (d, *J* = 7.2 Hz, 1H), 8.52 (t, *J* = 5.3 Hz, 1H), 8.17 (s, 1H), 7.78 (d, *J* = 1.6 Hz, 1H), 7.57 (brs, 2H), 7.23 (d, *J* = 8.4 Hz, 1H), 7.09 (dd, *J* = 7.6, 2.0 Hz, 1H), 4.63 (d, *J* = 5.6 Hz, 2H), 2.98 (q, *J* = 15.2, 7.6 Hz, 2H), 1.25 (t, *J* = 7.6 Hz, 3H), ; LCMS (electrospray) m/z (M+H)+ 354.16. |
| 277 | 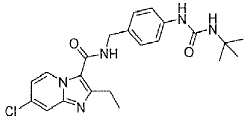 | White solid: ¹H-NMR (400 MHz, DMSO): δ 8.94 (d, *J* = 7.2, 1H), 8.41 (t, *J* = 6.0 Hz, 1H), 8.30 (brs, 1H), 7.78 (d, *J* = 2.0 Hz, 1H), 7.32 (d, *J* = 8.4 Hz, 2H), 7.20 (d, *J* = 8.4 Hz, 2H), 7.08 (dd, *J* = 7.2, 2.0 Hz, 1H), 6.03 (brs, 1H), 4.43 (d, *J* = 6.0 Hz, 2H), 2.96 (q, *J* = 14.8, 7.2 Hz, 2H), 1.25 (t, *J* = 7.6 Hz, 3H), ; LCMS (electrospray) m/z (M+H)+ 428.15 |
| 278 | 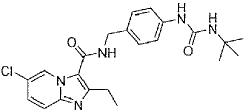 | White solid: ¹H-NMR (400 MHz, DMSO): δ 9.06 (d, *J* = 2.0, 1H), 8.42 (t, *J* = 6.0 Hz, 1H), 8.20 (s, 1H), 7.66 (d, *J* = 9.6 Hz, 1H), 7.45 (dd, *J* = 9.6, 2.0 Hz, 1H), 7.31 (d, *J* = 8.4 Hz, 2H), 7.21 (d, *J* = 8.8 Hz, 2H), 5.95 (s, 1H), 4.44 (d, *J* = 6.0 Hz, 2H), 2.97 (q, *J* = 15.2, 7.6 Hz, 2H), 1.24 (t, *J* = 7.6 Hz, 3H), ; LCMS (electrospray) m/z (M+H)+ 428.24 |
| 279 | 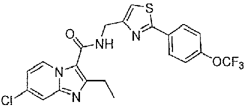 | Brown solid: ¹H-NMR (400 MHz, DMSO): δ 8.98 (d, *J* = 7.2, 1H), 8.55 (t, *J* = 5.6 Hz, 1H), 8.07 (d, *J* = 8.8 Hz, 2H), 7.80 (d, *J* = 2.4 Hz, 1H), 7.58 (s, 1H), 7.49 (d, *J* = 8.0 Hz, 2H), 7.11 (dd, *J* = 7.2, 2.0 Hz, 1H), 4.69 (d, *J* = 5.6 Hz, 2H), 3.03 (q, *J* = 15.2, 7.6 Hz, 2H), 1.28 (t, *J* = 7.2 Hz, 3H); LCMS (electrospray) m/z (M+H)+ 481.09 |
| 280 | 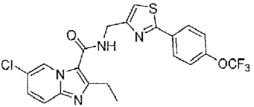 | Brown solid: ¹H-NMR (400 MHz, DMSO): δ 9.09 (d, *J* = 7.2 Hz, 1H), 8.58 (t, *J* = 5.6 Hz, 1H), 8.07 (d, *J* = 9.6 Hz, 2H), 7.68 (d, *J* = 9.2 Hz, 1H), 7.61 (s, 1H), 7.50 (d, *J* = 8.0 Hz, 2H), 7.46 (dd, *J* = 9.6, 2.0 Hz, 1H), 4.69 (d, *J* = 5.6 Hz, 2H), 3.03 (q, *J* = 14.8, 7.2 Hz, 2H), 1.29 (t, *J* = 7.6 Hz, 3H); LCMS (electrospray) m/z (M+H)+ 481.08 |
| 281 | 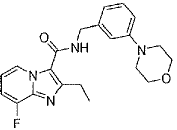 | White solid: ¹H-NMR (400 MHz, DMSO): δ 8.74 (d, *J* = 6.8 Hz, 1H), 8.57 (t, *J* = 6.0 Hz, 1H), 7.28 (dd, *J* = 10.8, 7.6 Hz, 1H), 7.20 (t, *J* = 8.0 Hz, 1H), 7.00 -6.97 (m, 1H), 6.95 (s, 1H), 6.83 (t, *J* = 8.0 Hz, 2H), 4.49 (d, *J* = 6.0 Hz, 2H), 3.73 (t, *J* = 4.4 Hz,, 4H), 3.09 (t, *J* = 4.7 Hz,, 4H), 3.00 (q, *J* = 14.8, 7.6 Hz, 2H), 1.27 (t, *J* = 7.6 Hz, 3H); LCMS (electrospray) m/z (M+H)+ 383.31 |
| 282 | 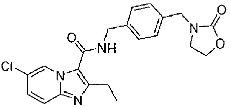 | White solid; mp = 179°C: ¹H-NMR (400 MHz, DMSO-d6): δ 1.25 (t, *J* = 7.6 Hz, 3H), 2.98 (q, *J* = 7.6 Hz, 2H), 3.39 (t, *J* = 8.0 Hz, 2H), 4.24 (t, *J* = 8.0 Hz, 2H), 4.31 (s, 2H), 4.51 (d, *J* = 5.6 Hz, 2H), 7.25 (d, *J* = 8.0 Hz, 2H), 7.36 (d, *J* = 8.0 Hz, 2H), 7.43 (d, *J* = 9.2 Hz, 1H), 7.65 (d, *J* = 9.2 Hz, 1H), 8.46 (t, *J* = 5.6 Hz, 1H, NH), 9.06 (s, 1H); LCMS (electrospray) m/z (M+H) + 413. |
| 283 | 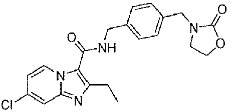 | White solid; mp = 172°C: ¹H-NMR (400 MHz, DMSO-d6): δ 1.25 (t, *J* = 7.6 Hz, 3H), 2.97 (q, *J* = 7.6 Hz, 2H), 3.39 (t, *J* = 8.0 Hz, 2H), 4.24 (t, *J* = 8.0 Hz, 2H), 4.32 (s, 2H), 4.51 (d, *J* = 5.6 Hz, 2H), 7.08 (d, *J* = 7.6 Hz, 1H), 7.25 (d, *J* = 8.0 Hz, 2H), 7.36 (d, *J* = 8.0 Hz, 2H), 7.77 (s, 1H), 8.46 (t, *J* = 5.6 Hz, 1H, NH), 8.95 (d, *J* = 7.6 Hz, 1H); LCMS (electrospray) m/z (M+H) + 413. |
| 284 | 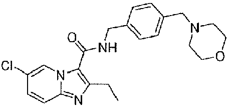 | White solid; mp = 133°C: ¹H-NMR (400 MHz, DMSO-d6): δ 1.24 (t, *J* = 7.6 Hz, 3H), 2.31 (brs, 4H), 2.97 (q, *J* = 7.6 Hz, 2H), 3.42 (s, 2H), 3.54 (brs, 4H), 4.50 (d, *J* = 5.6 Hz, 2H), 7.26 (d, *J* = 8.0 Hz, 2H), 7.31 (d, *J* = 8.0 Hz, 2H), 7.44 (d, *J* = 9.2 Hz, 1H), 7.65 (d, *J* = 9.2 Hz, 1H), 8.46 (t, *J* = 5.6 Hz, 1H, NH), 9.06 (s, 1H) ; LCMS (electrospray) m/z (M+H) + 413. |
| 285 | 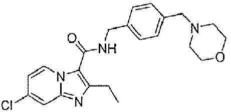 | White solid; mp = 107°C: ¹H-NMR (400 MHz, DMSO-d6): δ 1.25 (t, *J* = 7.6 Hz, 3H), 2.32 (brs, 4H), 2.97 (q, *J* = 7.6 Hz, 2H), 3.43 (s, 2H), 3.55 (brs, 4H), 4.50 (d, *J* = 5.6 Hz, 2H), 7.08 (d, *J* = 7.2 Hz, 1H), 7.26 (d, *J* = 8.0 Hz, 2H), 7.31 (d, *J* = 8.0 Hz, 2H), 7.78 (s, 1H), 8.45 (t, *J* = 5.6 Hz, 1H, NH), 8.95 (d, *J* = 7.2 Hz, 1H); LCMS (electrospray) m/z (M+H) + 413. |
| 286 | 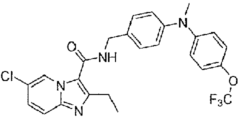 | White solid; mp = 138°C: ¹H-NMR (400 MHz, DMSO-d6): δ 1.26 (t, *J* = 7.6 Hz, 3H), 3.00 (q, *J* = 7.6 Hz, 2H), 3.25 (s, 3H), 4.50 (d, *J* = 6.0 Hz, 2H), 6.92 (d, *J* = 8.4 Hz, 2H), 7.10 (d, *J* = 8.4 Hz, 2H), 7.19 (d, *J* = 8.4 Hz, 2H), 7.35 (d, *J* = 8.4 Hz, 2H), 7.45 (d, *J* = 9.2 Hz, 1H), 7.66 (d, *J* = 9.2 Hz, 1H), 8.46 (t, *J* = 6.0 Hz, 1H, NH), 9.09 (s, 1H); LCMS (electrospray) m/z (M+H) + 503. |
| 287 | 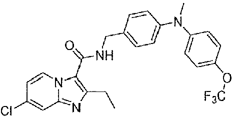 | White solid; mp = 134°C: ¹H-NMR (400 MHz, DMSO-d6): δ 1.26 (t, *J* = 7.6 Hz, 3H), 2.97 (q, *J* = 7.6 Hz, 2H), 3.24 (s, 3H), 4.50 (d, *J* = 6.0 Hz, 2H), 6.92 (d, *J* = 8.8 Hz, 2H), 7.08-7.10 (m, 3H), 7.19 (d, *J* = 8.8 Hz, 2H), 7.35 (d, *J* = 8.8 Hz, 2H), 7.78 (s, 1H), 8.45 (t, *J* = 6.0 Hz, 1H, NH), 8.97 (d, *J* = 7.2 Hz, 1H); LCMS (electrospray) m/z (M+H) + 503. |
| 288 | 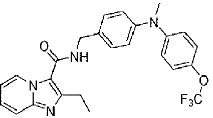 | White solid; mp = 120°C: ¹H-NMR (400 MHz, DMSO-d6): δ 1.27 (t, *J* = 7.6 Hz, 3H), 2.99 (q, *J* = 7.6 Hz, 2H), 3.25 (s, 3H), 4.50 (d, *J* = 5.6 Hz, 2H), 6.92 (d, *J* = 8.4 Hz, 2H), 6.98-7.02 (m, 1H), 7.10 (d, *J* = 8.4 Hz, 2H), 7.19 (d, *J* = 8.4 Hz, 2H), 7.34-7.39 (m, 3H), 7.59 (d, *J* = 6.8 Hz, 1H), 8.38 (t, *J* = 5.6 Hz, 1H, NH), 8.98 (d, *J* = 6.8 Hz, 1H); LCMS (electrospray) m/z (M+H) + 469. |
| 289 | 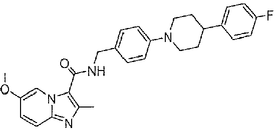 | Ivory solid; mp = 192°C: ¹H-NMR (400 MHz, DMSO-d6): δ 1.69-1.76 (m, 2H), 1.81-1.84 (m, 2H), 2.54 (s, 3H), 2.63-2.74 (m, 3H), 3.74-3.78 (m, 5H), 4.42 (d, *J* = 5.6 Hz, 2H), 6.94 (d, *J* = 8.8 Hz, 2H), 7.07-7.12 (m, 2H), 7.16 (dd, *J* = 2.4 Hz, 9.6 Hz, 1H), 7.22 (d, *J* = 8.8 Hz, 2H), 7.27-7.31 (m, 2H), 7.48 (d, *J* = 9.6 Hz, 1H), 8.17 (t, *J* = 5.6 Hz, 1H, NH), 8.70 (d, *J* = 2.4 Hz, 1H); LCMS (electrospray) m/z (M+H) + 473. |
| 290 | 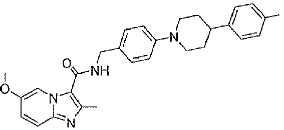 | Ivory solid; mp = 178°C: ¹H-NMR (400 MHz, DMSO-d6): δ 1.68-1.75 (m, 2H), 1.79-1.82 (m, 2H), 2.24 (s, 3H), 2.54 (s, 3H), 2.56-2.62 (m, 1H), 2.67-2.74 (m, 2H), 3.73-3.77 (m, 5H), 4.42 (d, *J* = 6.0 Hz, 2H), 6.94 (d, *J* = 8.8 Hz, 2H), 7.08 (d, *J* = 8.8 Hz, 2H), 7.12 (d, *J* = 8.8 Hz, 2H), 7.16 (dd, *J* = 2.4 Hz, 9.6 Hz, 1H), 7.22 (d, *J* = 8.8 Hz, 2H), 7.48 (d, *J* = 9.6 Hz, 1H), 8.18 (t, *J* = 6.0 Hz, 1H, NH), 8.70 (d, *J* = 2.4 Hz, 1H); LCMS (electrospray) m/z (M+H) + 469. |
| 291 | 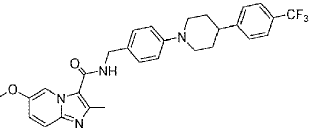 | Ivory solid; mp = 207°C: ¹H-NMR (400 MHz, DMSO-d6): δ 1.74-1.81 (m, 2H), 1.85-1.88 (m, 2H), 2.56 (s, 3H), 2.70-2.78 (m, 3H), 3.77-3.80 (m, 5H), 4.42 (d, *J* = 5.6 Hz, 2H), 6.95 (d, *J* = 8.8 Hz, 2H), 7.16 (dd, *J* = 2.4 Hz, 9.6 Hz, 1H), 7.23 (d, *J* = 8.8 Hz, 2H), 7.47 (d, *J* = 9.6 Hz, 1H), 7.49 (d, *J* = 8.8 Hz, 2H), 7.64 (d, *J* = 8.8 Hz, 2H), 8.18 (t, *J* = 5.6 Hz, 1H, NH), 8.70 (d, *J* = 2.4 Hz, 1H); LCMS (electrospray) m/z (M+H) + 523. |
| 292 | 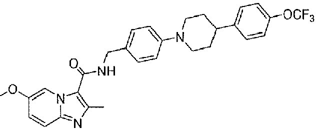 | White solid; mp = 183°C: ¹H-NMR (400 MHz, DMSO-d6): δ 1.71-1.77 (m, 2H), 1.84-1.87 (m, 2H), 2.54 (s, 3H), 2.69-2.75 (m, 3H), 3.75-3.79 (m, 5H), 4.42 (d, *J* = 6.0 Hz, 2H), 6.95 (d, *J* = 8.8 Hz, 2H), 7.16 (dd, *J* = 2.4 Hz, 9.6 Hz, 1H), 7.22 (d, *J* = 8.8 Hz, 2H), 7.27 (d, *J* = 8.8 Hz, 2H), 7.39 (d, *J* = 8.8 Hz, 2H), 7.48 (d, *J* = 9.6 Hz, 1H), 8.17 (t, *J* = 6.0 Hz, 1H, NH), 8.70 (d, *J* = 2.4 Hz, 1H); LCMS (electrospray) m/z (M+H) + 539. |
| 293 | 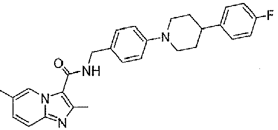 | Ivory solid; mp = 181°C: ¹H-NMR (400 MHz, DMSO-d6): δ 1.69-1.76 (m, 2H), 1.81-1.84 (m, 2H), 2.29 (s, 3H), 2.53 (s, 3H), 2.64-2.74 (m, 3H), 3.74-3.77 (m, 2H), 4.41 (d, *J* = 6.0 Hz, 2H), 6.94 (d, *J* = 8.8 Hz, 2H), 7.07-7.12 (m, 2H), 7.20-7.23 (m, 3H), 7.27-7.30 (m, 2H), 7.45 (d, *J* = 9.2 Hz, 1H), 8.21 (t, *J* = 6.0 Hz, 1H, NH), 8.83 (s, 1H); LCMS (electrospray) m/z (M+H) + 457. |
| 294 | 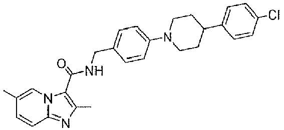 | Ivory solid; mp = 188°C ¹H-NMR (400 MHz, DMSO-d6): δ 1.68-1.75 (m, 2H), 1.81-1.84 (m, 2H), 2.29 (s, 3H), 2.53 (s, 3H), 2.63-2.74 (m, 3H), 3.75-3.78 (m, 2H), 4.41 (d, *J* = 6.0 Hz, 2H), 6.94 (d, *J* = 8.8 Hz, 2H), 7.21-7.24 (m, 3H), 7.28 (d, *J* = 8.8 Hz, 2H), 7.32 (d, *J* = 8.8 Hz, 2H), 7.47 (d, *J* = 8.8 Hz, 1H), 8.21 (t, *J* = 6.0 Hz, 1H, NH), 8.82 (d, *J* = 1.6 Hz, 1H); LCMS (electrospray) m/z (M+H) + 473. |
| 295 | 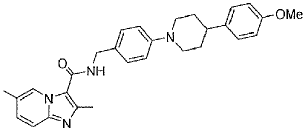 | Ivory solid; mp = 184°C: ¹H-NMR (400 MHz, DMSO-d6): δ 1.67-1.74 (m, 2H), 1.79-1.82 (m, 2H), 2.29 (s, 3H), 2.53 (s, 3H), 2.54-2.59 (m, 1H), 2.67-2.73 (m, 2H), 3.70 (s, 3H), 3.74-3.77 (m, 2H), 4.41 (d, *J* = 6.0 Hz, 2H), 6.84 (d, *J* = 8.8 Hz, 2H), 6.94 (d, *J* = 8.8 Hz, 2H), 7.16 (d, *J* = 8.8 Hz, 2H), 7.21-7.23 (m, 3H), 7.45 (d, *J* = 9.6 Hz, 1H), 8.21 (t, *J* = 6.0 Hz, 1H, NH), 8.82 (d, *J* = 0.8 Hz, 1H); LCMS (electrospray) m/z (M+H) + 469. |
| 296 | 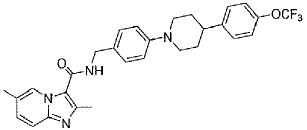 | Ivory solid; mp = 184°C: ¹H-NMR (400 MHz, DMSO-d6): δ 1.71-1.78 (m, 2H), 1.83-1.86 (m, 2H), 2.29 (s, 3H), 2.53 (s, 3H), 2.69-2.75 (m, 3H), 3.76-3.79 (m, 2H), 4.41 (d, *J* = 6.0 Hz, 2H), 6.95 (d, *J* = 8.8 Hz, 2H), 7.21-7.23 (m, 3H), 7.27 (d, *J* = 8.8 Hz, 2H), 7.39 (d, *J* = 8.8 Hz, 2H), 7.47 (d, *J* = 8.8 Hz, 1H), 8.21 (t, *J* = 6.0 Hz, 1H, NH), 8.82 (s, 1H); LCMS (electrospray) m/z (M+H) + 523. |
| 297 | 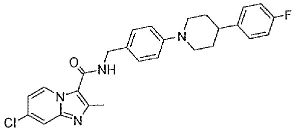 | Pale yellow solid: ¹H-NMR (400 MHz, DMSO-d6): δ 1.69-1.76(m, 2H), 1.82-1.85(m, 2H), 2.56 (s, 3H), 2.61-2.75(m, 3H), 3.75-3.78 (m, 2H), 4.43 (d, *J* = 6.0 Hz, 2H), 6.95 (d, *J* = 8.4 Hz, 2H), 7.08-7.13 (m, 3H), 7.23 (d, *J* = 8.4 Hz, 2H), 7.28-7.32 (m, 2H), 7.74 (d, *J* = 1.2 Hz, 1H), 8.35 (t, *J* = 5.8 Hz, 1H), 9.00 (d, *J* = 7.6 Hz, 1H); LCMS (electrospray) m/z (M+H) + 477. |
| 298 | 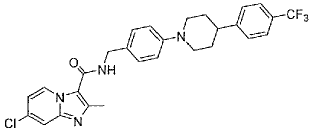 | Pale yellow solid: ¹H-NMR (400 MHz, DMSO-d6): δ 1.75-1.81 (m, 2H), 1.86-1.89 (m, 2H), 2.56 (s, 3H), 2.72-2.82 (m, 3H), 3.79 (d, *J* = 11.6 Hz, 2H), 4.43 (d, *J* = 6.0 Hz, 2H), 6.96 (d, *J* = 8.4 Hz, 2H), 7.09 (dd, *J* = 7.6, 2.0, 1H), 7.24 (d, *J* = 8.4 Hz, 2H), 7.50 (d, *J* = 8.0 Hz, 2H), 7.65 (d, *J* = 8.0 Hz, 2H), 7.73 (d, *J* = 1.6 Hz, 1H), 8.35 (t, *J* = 5.8 Hz), 9.00 (d, *J* = 7.6 Hz, 1H); LCMS (electrospray) m/z (M+H) + 527. |
| 299 | 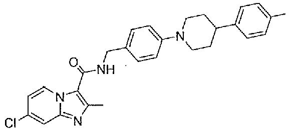 | Yellow solid: ¹H-NMR (400 MHz, DMSO-d6): δ 1.67-1.74 (m, 2H), 1.79-1.81 (m, 2H), 2.24(s, 3H), 2.54 (s, 3H), 2.56-2.62 (m, 1H), 2.67-2.73 (m, 2H), 3.75 (d, *J* =12.4 Hz, 2H), 4.41(d, *J* = 6.0 Hz, 2H), 6.93(d, *J* = 8.8 Hz, 2H), 7.07(dd, *J* = 7.6, 2.0 Hz, 3H), 7.12 (d, *J* = 8.4 Hz, 2H), 7.21 (d, *J* = 8.8 Hz, 2H), 7.72(d, *J* = 2.0 Hz, 1H), 8.33 (t, *J* = 6.0 Hz, 1H), 8.98 (d, *J* = 7.6 Hz, 1H); LCMS (electrospray) m/z (M+H) + 473. |
| 300 | 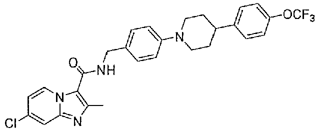 | Pale pink solid: ¹H-NMR (400 MHz, DMSO-d6): δ 1.70-1.74(m, 2H), 1.83-1.86 (m, 2H), 2.55 (s, 3H), 2.68-2.74(m, 3H), 3.76 (d, *J* = 12.4 Hz, 2H), 4.41 (d, *J* = 6.0 Hz, 2H), 6.94 (d, *J* = 8.4 Hz, 2H), 7.07 (dd, *J* = 7.6, 1.2 Hz, 1H), 7.22 (d, *J* = 8.4 Hz, 2H), 7.26 (d, *J* = 8.4 Hz, 2H), 7.38 (d, *J* = 8.4 Hz, 2H), 7.72 (d, *J* = 1.2 Hz, 1H), 8.32(t, *J* = 5.8 Hz, 1H), 8.99 (d, *J* = 7.2 Hz, 1H); LCMS (electrospray) m/z (M+H) + 543 |
| 301 | 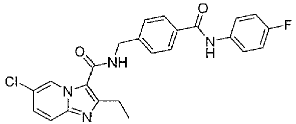 | ¹H-NMR (400 MHz, CDC13 + CD3OD): δ 9.22 -9.23 (m, 1H), 7.88 (d, *J* = 8.4 Hz, 2H), 7.59 -7.63 (m, 2H), 7.50 (dd, *J* = 9.6, 2.0 Hz, 2H), 7.47 (d, *J* = 8.4 Hz, 2H), 7.33 (dd, *J* = 9.6, 2.0 Hz, 1H), 7.02 (dd, *J* = 9.6, 8.4 Hz, 1H), 4.68 (s, 2H), 2.98 (q, *J* = 7.6 Hz, 2H), 1.33 (t, *J* = 7.6 Hz, 3H); LCMS (electrospray) m/z (M+H)+ 451. |
| 302 | 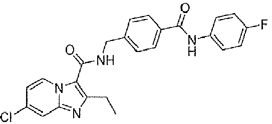 | ¹H-NMR (400 MHz, CDCl3 + CD3OD): δ 9.06 (d, *J* = 7.6 Hz, 1H), 7.88 (d, *J* = 8.0 Hz, 2H), 7.60 -7.62 (m, 2H), 7.54 (d, *J* = 2.0 Hz, 2H), 7.46 (d, *J* = 8.0 Hz, 1H), 7.02 (dd, *J* = 8.8, 8.8 Hz, 2H), 6.93 (dd, *J* = 7.6, 2.0 Hz, 1H), 4.68 (s, 2H), 2.7 (q, *J* = 7.6 Hz, 2H), 1.33 (t, *J* = 7.6 Hz, 3H); LCMS (electrospray) m/z (M+H)+ 451. |
| 303 | 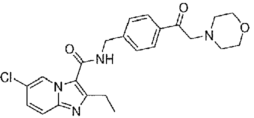 | ¹H-NMR (400 MHz, CDC13): δ 9.51 (d, *J* = 2.0 Hz, 1H), 7.53 (d, *J* = 9.6 Hz, 1H), 7.31 -7.41 (m, 4H), 7.29 (dd, *J* = 9.6, 2.0 Hz, 1H), 6.12 (t, *J* = 5.2 Hz, 1H), 4.68 (d, *J* = 5.6 Hz, 2H), 3.68 -3.80 (m, 4H), 2.97 (q, *J* = 7.6 Hz, 2H), 2.42 -2.52 (m, 4H), 1.39 (t, *J* = 7.6 Hz, 3H); LCMS (electrospray) m/z (M+H)+ 441 |
| 304 | 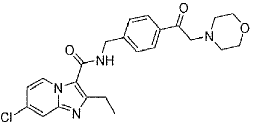 | ¹H-NMR (400 MHz, CDC13): δ 9.35 (d, *J* = 7.2 Hz, 1H), 7.58 (d, *J* = 2.0 Hz, 1H), 7.33 -7.38 (m, 4H), 6.90 (dd, *J* = 7.4, 2.0 Hz, 1H), 6.09 (t, *J* = 5.0 Hz, 1H), 4.68 (d, *J* = 5.6 Hz, 2H), 3.68 -3.80 (m, 4H), 2.96 (q, *J* = 7.6 Hz, 2H), 2.42 -2.52 (m, 4H), 1.39 (t, *J* = 7.6 Hz, 3H); LCMS (electrospray) m/z (M+H)+ 441. |
| 305 | 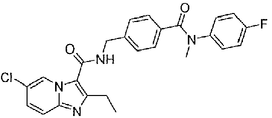 | ¹H-NMR (400 MHz, CDC13): δ 9.48 -9.49 (m, 1H), 7.53 (d, *J* = 8.8 Hz, 1H), 7.28 -7.31 (m, 3H), 7.19 (d, *J* = 8.0 Hz, 2H), 7.00 -7.03 (m, 2H), 6.92 (dd, *J* = 8.8, 8.0 Hz, 2H), 6.07 (m, 1H), 4.62 (d, *J* = 5.6 Hz, 2H), 3.46 (s, 3H), 2.93 (q, *J* = 7.6 Hz, 2H), 1.36 (t, *J* = 7.6 Hz, 3H); LCMS (electrospray) m/z (M+H)+ 465. |
| 306 | 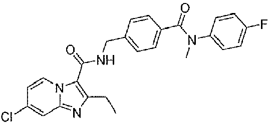 | ¹H-NMR (400 MHz, CDCl3): δ 9.28 (d, *J* = 7.6 Hz, 1H), 7.56 (d, *J* = 2.0 Hz, 1H), 7.26 (d, *J* = 8.0 Hz, 2H), 7.16 (d, *J* = 8.0 Hz, 2H), 6.98 -7.01 (m, 2H), 6.86 -6.93 (m, 3H), 6.92 (dd, *J* = 8.8, 8.0 Hz, 2H), 6.15 (t, *J* = 5.6 Hz, 1H), 4.60 (d, *J* = 5.6 Hz, 2H), 3.43 (s, 3H), 2.89 (q, *J* = 7.6 Hz, 2H), 1.34 (t, *J* = 7.6 Hz, 3H); LCMS (electrospray) m/z (M+H)+ 465. |
| 307 | 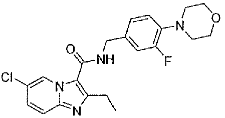 | ¹H-NMR (400 MHz, CDCl3): δ 9.53 (d, *J* = 2.0 Hz, 1H (m, 1H), 7.55 (d, *J* = 9.6 Hz, 1H), 7.31 (dd, *J* = 9.6, 2.0 Hz, 1H), 7.05 -7.10 (m, 2H), 6.93 (dd, *J* = 9.2, 8.0 Hz, 2H), 6.08 (t, *J* = 5.6 Hz, 1H), 4.62 (d, *J* = 5.6 Hz, 2H), 3.85 -3.88 (m, 4H), 3.07 -3.10 (m, 4H), 2.98 (q, *J* = 7.6 Hz, 2H), 1.42 (t, *J* = 7.6 Hz, 3H); LCMS (electrospray) m/z (M+H)+ 417. |
| 308 | 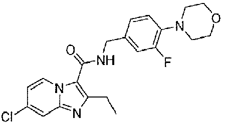 | ¹H-NMR (400 MHz, CDC13): δ 9.36 (d, *J* = 7.6 Hz, 1H), 7.60 (d, *J* = 2.0 Hz, 1H), 7.05 -7.10 (m, 2H), 6.90 -6.95 (m, 2H), 6.07 (t, *J* = 5.6 Hz, 1H), 4.62 (d, *J* = 5.6 Hz, 2H), 3.85 -3.88 (m, 4H), 3.08 -3.09 (m, 4H), 3.00 (q, *J* = 7.6 Hz, 2H), 1.41 (t, *J* = 7.6 Hz, 3H); LCMS (electrospray) m/z (M+H)+ 417. |
| 309 | 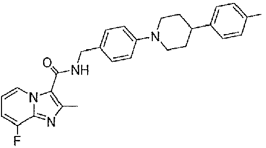 | ¹H-NMR (400 MHz, DMSO-d6): δ 8.80 (d, *J* = 6.8 Hz, 1H), 8.42 (t, *J* = 6.0 Hz, 1H), 7.27 (dd, *J* = 8.0, 7.6 Hz, 1H), 7.22 (d, *J* = 8.8 Hz, 2H), 7.12 (d, *J* = 8.0 Hz, 2H), 7.08 (d, *J* = 8.4 Hz, 2H), 6.95 -6.98 (m, 4H), 6.94 (d, *J* = 8.8 Hz, 2H), 4.41 (d, *J* = 6.0 Hz, 2H), 3.70 -3.80 (m, 2H), 3.40 -3.48 (m, 3H), 2.67 -2.73 (m, 2H), 2.57 (s, 3H), 2.24 (s, 3H), 1.78 -1.84 (m, 2H), 1.50 -1.76 (m, 2H); LCMS (electrospray) m/z (M+H)+ 457. |
| 310 | 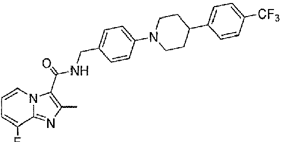 | ¹H-NMR (400 MHz, DMSO-d6): δ 8.80 (d, *J* = 6.8 Hz, 1H), 8.42 (t, *J* = 6.0 Hz, 1H), 7.64 (d, *J* = 8.4 Hz, 2H), 7.49 (d, *J* = 8.4 Hz, 2H), 7.27 (dd, *J* = 7.6, 7.6 Hz, 1H), 7.23 (d, *J* = 8.8 Hz, 2H), 6.96 -6.99 (m, 1H), 6.95 (d, *J* = 8.8 Hz, 2H), 4.42 (d, *J* = 6.0 Hz, 2H), 3.70 -3.80 (m, 2H), 3.30 - 3.40 (m, 3H), 2.70 -2.80 (m, 2H), 2.57 (s, 3H), 1.84 -1.90 (m, 2H), 1.70 - 1.80 (m, 2H); LCMS (electrospray) m/z (M+H)+ 511. |
| 311 | 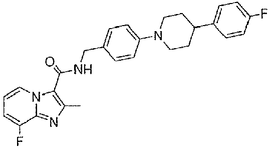 | ¹H-NMR (400 MHz, CDC13): δ 9.24 (d, *J* = 6.8 Hz, 1H), 7.30 (d, *J* = 8.0 Hz, 2H), 7.30 (d, *J* = 8.0 Hz, 2H), 7.15 -7.24 (m, 3H), 6.97 -7.06 (m, 4H), 6.81 -6.97 (m, 1H), 6.05 (br s, 1H), 4.62 (d, *J* = 5.2 Hz, 2H), 3.76 -3.85 (m, 2H), 2.80 -2.90 (m, 2H), 2.69 (s, 3H),2.60 -2.68 (m, 1H), 1.60 -2.00 (m, 4H); LCMS (electrospray) m/z (M+H)+ 461. |
| 312 | 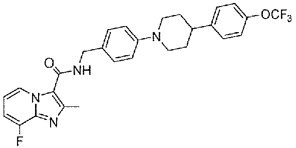 | ¹H-NMR (400 MHz, DMSO-d6): δ 8.80 (d, *J* = 6.8 Hz, 1H), 8.43 (t, *J* = 6.0 Hz, 1H), 7.39 (d, *J* = 8.8 Hz, 2H), 7.24 -7.29 (m, 2H), 7.22 (d, *J* = 8.8 Hz, 2H), 6.96 -6.99 (m, 1H), 6.94 (d, *J* = 8.8 Hz, 2H), 4.42 (d, *J* = 6.0 Hz, 2H), 3.70 -3.80 (m, 2H), 3.30 -3.40 (m, 3H), 2.70 -2.80 (m, 2H), 2.57 (s, 3H), 1.82 -1.90 (m, 2H), 1.68 -1.80 (m, 2H); LCMS (electrospray) m/z (M+H)+ 527. |
| 313 | 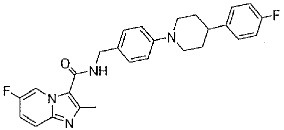 | ¹H-NMR (400 MHz, DMSO-d6): δ-1.68 -1.75 (m, 2H), 1.79 -1.85 (m, 2H), 2.56 (s, 3H), 2.62 -2.73 (m, 3H), 3.74 -3.77 (m, 2H), 4.43 (d, *J* = 5.6 Hz, 2H), 6.95 (d, *J* = 8.4 Hz, 2H), 7.04 -7.11 (m, 2H), 7.23 (d, *J* = 8.4 Hz, 2H), 7.27 -7.30 (m, 2H), 7.44 -7.49 (m, 1H), 7.61 -7.64 (m, 1H), 8.28 (t, *J* = 5.6 Hz, 1H, NH), 9.06 -9.07 (m, 1H), ; LCMS (electrospray) m/z (M+H)+ 461. |
| 314 | 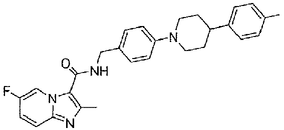 | ¹H-NMR (400 MHz, DMSO-d6): δ-1.68 -1.75 (m, 2H), 1.79 -1.83 (m, 2H), 2.56 (s, 3H), 2.58 -2.62 (m, 1H), 2.68 -2.73 (m, 1H), 3.74 -3.77 (m, 2H), 4.42 (d, *J* = 6.0 Hz, 2H), 6.94 (d, *J* = 8.4 Hz, 2H), 7.07 -7.13 (m, 4H), 7.23 (d, *J* = 8.4 Hz, 2H), 7.44 -7.49 (m, 2H), 7.61 -7.64 (m, 1H), 8.27 (t, *J* = 6.0 Hz, 1H, NH), 9.05 -9.07 (m, 1H) ; LCMS (electrospray) m/z (M+H)+ 456. |
| 315 | 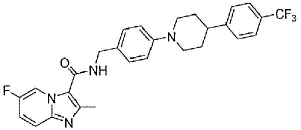 | ¹H-NMR (400 MHz, DMSO-d6): δ-1.71 -1.80 (m, 2H), 1.83 -1.88 (m, 2H), 2.56 (s, 3H), 2.71 -2.80 (m, 3H), 3.77 -3.80 (m, 2H), 4.43 (d, *J* = 6.0 Hz, 2H), 6.96 (d, *J* = 8.4 Hz, 2H), 7.24 (d, *J* = 8.4 Hz, 2H), 7.44 -7.50 (m, 3H), 7.61 -7.65 (m, 3H), 8.28 (t, *J* = 6.0 Hz, 1H, NH), 9.05 -9.07 (m, 1H) ; LCMS (electrospray) m/z (M+H)+ 511. |
| 316 | 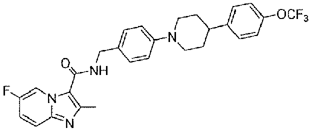 | ¹H-NMR (400 MHz, Acetone-d6): δ-1.82 -1.89 (m, 2H), 1.92 -1.96 (m, 2H), 2.66 (s, 3H), 2.76 -2.84 (m, 3H), 3.82 -3.86 (m, 2H), 4.58 (d, *J* = 5.6 Hz, 2H), 6.99 (d, *J* = 8.4 Hz, 2H), 7.26 -7.31 (m, 4H), 7.37 -7.44 (m, 4H), 7.55 -7.59 (m, 1H), 9.34 -9.36 (m, 1H) ; LCMS (electrospray) m/z (M+H)+ 527. |
| 317 | 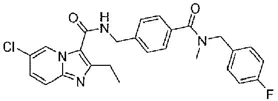 | ¹H-NMR (400 MHz, CDCl3): δ 1.40 (t, *J* = 7.6 Hz, 3H), 2.86 -3.00 (m, 5H), 4.70 -4.47 (m, 4H), 6.20 (brs, 1H), 7.45 -7.01 (m, 9H), 7.53 (d, *J* = 9.6 Hz, 1H), 9.50 (s, 1H). |
| 318 | 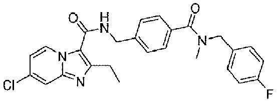 | ¹H-NMR (400 MHz, CDCl3): δ 1.39 (t, *J* = 7.6 Hz, 3H), 2.86 -2.99 (m, 5H), 4.48 (m, 2H), 4.71 -4.69 (m, 2H), 6.16 (brs, 1H), 6.89 (dd, *J* = 7.2Hz, 2.0 Hz, 1H), 7.01 -7.45 (m, 8H), 7.59 (d, *J* = 2.0 Hz, 1H), 9.34 (d, *J* = 7.2 Hz, 1H) |
| 319 | 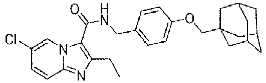 | ¹H-NMR (400 MHz, CDCl3): δ 1.37 (t, *J* = 7.6 Hz, 3H), 2.02 -1.64 (m, 15H), 2.93 (q, *J* = 7.6 Hz, 2H), 3.48 (s, 2H), 4.60 (d, *J* = 5.2 Hz, 2H), 6.05 (brs, 1H), 6.88 (d, *J* = 8.0 Hz, 2H), 7.25 -7.27 (m, 3H), 7.51 (d, *J* = 9.6 Hz, 1H), 9.50 (s, 1H); ); 13C NMR (100 MHz, CDCl3) d 13.4, 23.6, 28.4, 28.5, 34.0, 37.3, 39.7, 43.4, 76.9, 77.2, 77.5, 78.6, 115.1, 117.0, 121.6, 126.3, 128.3, 129.2, 129.7, 144.6, 151.5, 159.6, 161.2, 199.8 |
| 320 | 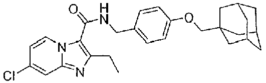 | ¹H-NMR (400 MHz, CDCl3): δ 1.36 (t, *J* = 7.6 Hz, 3H), 2.00 -1.64 (m, 15H), 2.92 (q, *J* = 7.6 Hz, 2H), 3.48 (s, 2H), 4.59 (d, *J* = 5.6 Hz, 2H), 6.05 (brs, 1H), 6.89 -6.85 (m, 3H), 7.25 (d, *J* = 8.4 Hz, 2H), 7.55 (d, *J* = 2.0 Hz, 1H), 9.32 (d, *J* = 7.6 Hz, 1H); 13C NMR (100 MHz, CDCl3) d 13.4, 23.6, 28.4, 28.6, 34.0, 37.3, 39.7, 43.4, 76.9, 77.2, 77.5, 78.6, 114.7, 115.1, 115.8, 128.6, 129.2, 129.7, 133.6, 146.2, 151.7, 159.6, 161.2, 199.8 |
| 321 | 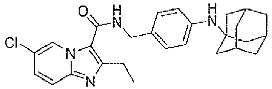 | ¹H-NMR (400 MHz, CDCl3): δ 1.36 (t, *J* = 7.6 Hz, 3H), 2.10 -1.63 (m, 15H), 2.94 (q, *J* = 7.6 Hz, 2H), 4.56 (d, *J* = 5.2 Hz, 2H), 5.99 (brs, 1H), 6.77 (d, *J* = 8.4 Hz, 2H), 7.14 (d, *J* = 8.4 Hz, 2H), 7.20 -7.29 (m, 1H), 7.52 (d, *J* = 9.6 Hz, 1H), 9.51 (d, *J* = 1.6 Hz, 1H); ; LCMS (electrospray) m/z (M+H)+ 463. |
| 322 | 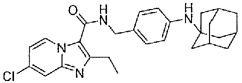 | ¹H-NMR (400 MHz, CDCl3): δ 1.37 (t, *J* = 7.6 Hz, 3H), 2.10 -1.63 (m, 15H), 2.94 (q, *J* = 7.6 Hz, 2H), 4.54 (d, *J* = 5.2 Hz, 2H), 5.95 (brs, 1H), 6.59 (d, *J* = 8.8 Hz, 2H), 6.88 (dd, *J* = 2.4 Hz, 7.6 Hz, 1H), 7.16 (d, *J* = 8.8 Hz, 2H), 7.58(d, *J* = 2.0 Hz, 1H), 9.35 (d, *J* = 7.6 Hz, 1H); LCMS (electrospray) m/z (M+H)+ 463 |
| 323 | 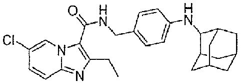 | ¹H-NMR (400 MHz, CDCl3): δ 1.38 (t, *J* = 7.6 Hz, 3H), 2.04 -1.61 (m, 15H), 2.94 (q, *J* = 7.6 Hz, 2H), 4.54 (d, *J* = 5.2 Hz, 2H), 5.98 (brs, 1H), 6.59 (d, *J* = 8.4 Hz, 2H), 7.16 (d, *J* = 8.8, 1H), 7.27 (dd, *J* = 2.4 Hz, 9.6 Hz, 1H), 7.52 (d, *J* = 9.2 Hz, 1H), 9.51 (d, *J* = 1.2 Hz, 1H); LCMS (electrospray) m/z (M+H)+ 463. |
| 324 | 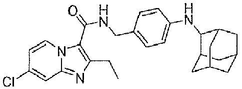 | ¹H-NMR (400 MHz, CDC13): δ 1.37 (t, *J* = 7.6 Hz, 3H), 2.02 -1.61 (m, 15H), 2.93 (q, *J* = 7.6 Hz, 2H), 4.56 (d, *J* = 5.2 Hz, 2H), 5.97 (brs, 1H), 6.78 (d, *J* = 8.4 Hz, 2H), 6.88 (dd, *J* = 2.0 Hz, 7.0 Hz, 1H), 7.14 (d, *J* = 8.4 Hz, 2H), 7.58(d, *J* = 2.0 Hz, 1H), 9.34 (d, *J* = 7.2 Hz, 1H); LCMS (electrospray) m/z (M+H)+ 463. |
| 325 | 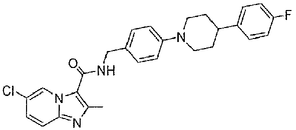 | White solid: ¹H-NMR (400 MHz, DMSO-d6): δ 1.78-1.95 (m, 4H), 2.60 (s, 3H), 2.72-2.82 (m, 1H), 2.92-3.08 (m, 2H), 3.73-3.76 (m, 2H), 4.48 (d, *J* = 6.0 Hz, 2H), 7.11-7.22 (m, 4H), 7.29-7.35 (m, 4H), 7.54 (dd, *J* = 9.6, 1.6 Hz, 1H), 7.68 (d, *J* = 9.6 Hz, 1H), 8.48 (t, *J* = 6.0 Hz, 1H, NH), 9.15 (d, *J* = 1.2 Hz, 1H); LCMS (electrospray) m/z (M+H) + 477. |
| 326 | 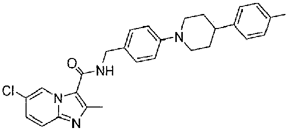 | Beige solid: ¹H-NMR (400 MHz, DMSO-d6): δ 1.66-1.86 (m, 4H), 2.60 (s, 3H), 2.70-2.75 (m, 3H), 3.76-3.79 (m, 2H), 4.43 (d, *J* = 5.6 Hz, 2H), 6.95 (d, *J* = 8.8 Hz, 2H), 7.09-7.15 (m, 4H), 7.24 (d, *J* = 8.8 Hz, 2H), 7.46 (dd, *J* = 9.6, 2.4 Hz, 1H), 7.63 (d, *J* = 9.6 Hz, 1H), 8.36 (t, *J* = 5.6 Hz, 1H, NH), 9.12 (d, *J* = 1.6 Hz, 1H); LCMS (electrospray) m/z (M+H) + 473. |
| 327 | 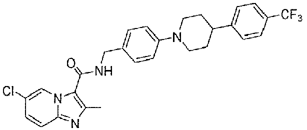 | Beige solid: ¹H-NMR (400 MHz, DMSO-d6): δ 1.72-1.92 (m, 4H), 2.59 (s, 3H), 2.73-2.79 (m, 3H), 3.79-3.82 (m, 2H), 4.44 (d, *J* = 6.0 Hz, 2H), 6.97 (d, *J* = 8.8 Hz, 2H), 7.25 (d, *J* = 8.8 Hz, 2H), 7.46 (dd, *J* = 9.4, 2.2 Hz, 1H), 7.51 (d, *J* = 8.0 Hz, 2H), 7.62-7.67 (m, 3H), 8.37 (t, *J* = 6.0 Hz, 1H, NH), 9.13 (d, *J* = 2.0 Hz, 1H) ); LCMS (electrospray) m/z (M+H) + 527. |
| 328 | 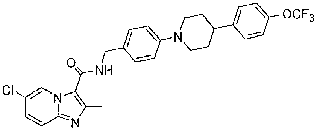 | Beige solid: ¹H-NMR (400 MHz, DMSO-d6): δ 1.78-1.90 (m, 4H), 2.58 (s, 3H), 2.71-2.76 (m, 3H), 3.77-3.81 (m, 2H), 4.44 (d, *J* = 6.0 Hz, 2H), 6.97 (d, *J* = 8.8 Hz, 2H), 7.24 (d, *J* = 8.8 Hz, 2H), 7.29 (d, *J* = 8.8 Hz, 2H), 7.41 (d, *J* = 8.8 Hz, 2H), 7.46 (dd, *J* = 9.6, 2.0 Hz, 1H), 7.63 (d, *J* = 9.6 Hz, 1H), 8.36 (t, *J* = 6.0 Hz, 1H, NH), 9.13 (d, *J* = 1.6 Hz, 1H); LCMS (electrospray) m/z (M+H) + 543. |
| 329 | 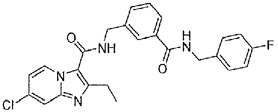 | White solid: ¹H-NMR (400 MHz, CDC13): δ 1.38 (t, *J* = 7.6 Hz, 3H), 2.95 (q, *J* = 7.6 Hz, 2H), 4.61 (d, *J* = 6.0 Hz, 2H), 4.73 (d, *J* = 6.0 Hz, 2H), 6.16 (brs, 1H), 6.37 (brs, 1H), 6.89 (dd, *J* = 2.0, 7.6 Hz, 1H), 6.99 - 7.06 (m, 2H), 7.30 -7.36 (m, 2H), 7.42 (dd, *J* = 7.6, 7.6 Hz, 1H), 7.51 - 7.55 (m, 1H), 7.59 (d, *J* = 2.0 Hz, 1H), 7.66 -7.68 (m, 1H), 7.84 (s, 1H), 9.33 (d, *J* = 7.6 Hz, 1H); LCMS (electrospray) m/z 465, 467 (M+H)+, Cl-isotope pattern. |
| 330 | 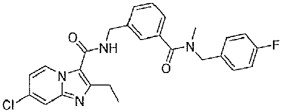 | Pale brown solid: ¹H-NMR (400 MHz, CDC13): δ 1.35 (t, *J* = 7.6Hz, 3H), 2.76 -2.97 (m, 2H), 2.86 & 2.94 (s, 3H), 4.45 & 4.68 (s, 2H), 6.31 (brs, 1H), 6.87 (dd, *J* = 2.0, 7.2 Hz, 1H), 7.00 -7.08 (m, 3H), 7.26 -7.43 (m, 5H), 7.57 (d, *J =* 2.0 Hz, 1H), 9.28 (d, *J* = 7.2 Hz, 1H); LCMS (electrospray) m/z 479, 481 (M+H)+, Cl-isotope pattern. |
| 331 | 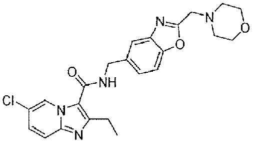 | Pale yellow solid: ¹H-NMR (400 MHz, CDC13): δ 1.37 (t, *J* = 7.6 Hz, 3H), 2.62 -2.64 (m, 4H), 2.94 (q, *J* = 7.6 Hz, 2H), 3.74 -3.76 (m, 4H), 3.85 (s, 2H), 4.78 (d, *J =* 5.6 Hz, 2H), 6.18 (brt, *J* = 5.6 Hz, 1H), 7.28 (dd, *J* = 2.0, 9.6 Hz, 1H), 7.37 (dd, *J* = 1.2, 8.0 Hz, 1H), 7.51 -7.54 (m, 2H), 7.70 (d, *J* = 1.2 Hz, 1H), 9.51 (d, *J* = 2.0 Hz, 1H); LCMS (electrospray) m/z 454, 456 (M+H)+, Cl-isotope pattern. |
| 332 | 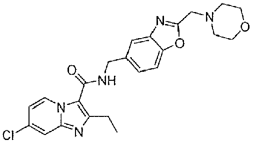 | Pale brown solid: ¹H-NMR (400 MHz, CDC13): δ 1.37 (t, *J* = 7.6 Hz, 3H), 2.63 -2.65 (m, 4H), 2.94 (q, *J* = 7.6 Hz, 2H), 3.75 -3.77 (m, 4H), 3.86 (s, 2H), 4.79 (d, *J* = 6.0 Hz, 2H), 6.13 (brt, *J* = 6.0 Hz, 1H), 6.90 (dd, *J* = 2.0, 7.2 Hz, 1H), 7.37 (dd, *J* = 1.6, 8.4 Hz, 1H), 7.52 (d, *J* = 8.4 Hz, 1H), 7.59 (d, *J* = 2.0 Hz, 1H), 7.72 (d, *J* = 1.6 Hz, 1H), 9.36 (d, *J* = 7.2 Hz, 1H); LCMS (electrospray) m/z 454, 456 (M+H)+, Cl-isotope pattern. |
| 333 | 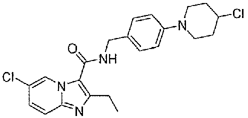 | Pale red solid: ¹H-NMR (400 MHz, CDC13): δ 1.37 (t, *J* = 7.6 Hz, 3H), 1.98 -2.04 (m, 2H), 2.18 -2.29 (m, 2H), 2.93 (q, *J* = 7.6 Hz, 2H), 3.05 - 3.11 (m, 2H), 3.49 -3.55 (m, 2H), 4.19 -4.24 (m, 1H), 4.60 (d, *J* = 5.2 Hz, 2H), 6.01 (brs, 1H), 6.92 (d, *J* = 8.4 Hz, 2H), 7.26 -7.31 (m, 3H), 7.52 (d, *J* = 9.6 Hz, 1H), 9.53 (d, *J* = 2.1 Hz, 1H); LCMS (electrospray) m/z 431 (M+H)+. |
| 334 | 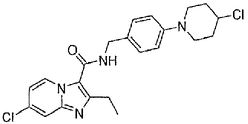 | Pale red solid: ¹H-NMR (400 MHz, CDC13): δ 1.36(t, *J* = 7.2 Hz, 3H), 1.99 -2.04 (m, 2H), 2.17 -2.20 (m, 2H), 2.92 (q, *J* = 7.2 Hz, 2H), 3.05 - 3.10 (m, 2H), 3.50 -3.52 (m, 2H), 4.20 -4.23 (m, 1H), 4.59 (d, *J* = 5.6 Hz, 2H), 5.99 (brs, 1H), 6.89 -6.94 (m, 3H), 7.26 -7.27 (m, 2H), 7.58 (s, 1H), 9.35 (d, *J* = 6.8 Hz, 1H); LCMS (electrospray) m/z 431 (M+H)+. |
| 335 | 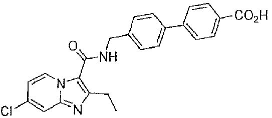 | White solid: ¹H-NMR (400 MHz, DMSO-d6): δ 1.26 (t, *J* = 7.6 Hz, 3H), 2.98 (q, *J* = 7.6 Hz, 2H), 4.58 (d, *J* = 6.0 Hz, 2H), 7.08 (dd, *J* = 2.4, 7.2 Hz, 1H), 7.48 (d, *J* = 8.4 Hz, 2H), 7.72 (d, *J* = 8.0 Hz, 2H), 7.78 -7.80 (m, 3H), 8.00 (d, *J* = 8.4 Hz, 2H), 8.51 (brt, *J* = 5.6 Hz, 1H), 8.97 (d, *J* = 7.2 Hz, 1H); LCMS (electrospray) m/z 434, 436 (M+H)+, Cl-isotope pattern. |
| 336 | 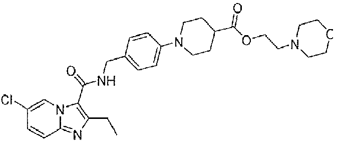 | Pale yellow solid: ¹H-NMR (400 MHz, CDCl3): δ 1.36 (t, *J* = 7.6 Hz, 3H), 1.71 -2.03 (m, 8H), 2.47 -2.55 (m, 2H), 2.62 -2.65 (m, 1H), 2.72 - 2.84 (m, 2H), 2.87 -2.97 (m, 3H), 3.30 -3.40 (m, 2H), 3.62 -3.77 (m, 4H), 4.23 -4.26 (m, 1H), 4.58 (d, *J* = 5.2 Hz, 2H), 6.02 (brs, 1H), 6.91 (d, *J* = 8.4 Hz, 2H), 7.24 -7.29 (m, 3H), 7.51 (d, *J* = 9.6 Hz, 1H), 9.51 (d, *J* = 1.2 Hz, 1H); LCMS (electrospray) m/z 554, 556 (M+H)+, Cl-isotope pattern. |
| 337 | 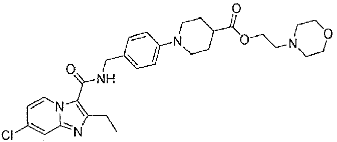 | Pale pink solid: ¹H-NMR (400 MHz, CDC13): δ 1.36 (t, *J* = 7.6 Hz, 3H); 1.82 -1.92 (m, 2H), 2.00 -2.04 (m, 2H), 2.44 -2.51 (m, 5H), 2.61 (t, *J* = 5.6 Hz, 2H), 2.77 -2.84 (m, 2H), 2.91 (t, *J* = 7.6 Hz, 2H), 2.91 -2.97 (m, 1H), 3.62 -3.70 (m, 5H), 4.22 (t, *J* = 5.6 Hz, 2H), 4.58 (d, *J* = 5.6 Hz, 2H), 5.97 (brt, *J* = 5.6 Hz, 1H), 6.88 -6.94 (m, 3H), 7.25 -7.27 (m, 2H), 7.58 -7.59 (m, 1H), 9.35 (d, *J* = 7.6 Hz, 1H); LCMS (electrospray) m/z 554, 556 (M+H)+, Cl- isotope pattern. |
| 338 | 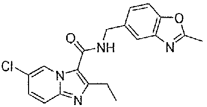 | White solid: ¹H-NMR (400 MHz, CDC13): δ 1.38 (t, *J* = 7.6 Hz, 3H), 2.64 (s, 3H), 2.95 (q, *J* = 7.6 Hz, 2H), 4.78 (d, *J* = 5.6 Hz, 2H), 6.15 (brt, *J* = 5.6 Hz, 1H), 7.31 -7.35 (m, 2H), 7.46 (d*, J* = 8.4 Hz, 1H), 7.55 (d, *J* = 9.2 Hz, 1H), 7.65 (s, 114), 9.54 (s, 1H); LCMS (electrospray) m/z 369, 371 (M+H)+, Cl- isotope pattern. |
| 339 | 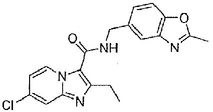 | White solid: ¹H-NMR (400 MHz, CDCl3): δ 1.36 (t, *J* = 7.6 Hz, 3H), 2.64 (s, 3H), 2.93 (q, *J* = 7.6 Hz, 2H), 4.77 (d, *J* = 6.0 Hz, 2H), 6.14 (brt, *J* = 6.0 Hz, 1H), 6.90 (dd, *J* = 2.4, 7.6 Hz, 1H), 7.31 (dd, *J =* 2.0, 8.4 Hz, 1H), 7.45 (d, *J* = 8.4 Hz, 1H), 7.60 (d, *J* = 2.4 Hz, 1H), 7.65 (d, *J* = 2.0 Hz, 1H), 9.36 (d, *J* = 7.6 Hz, 1H); LCMS (electrospray) m/z 369, 371 (M+H)+, Cl- isotope pattern. |
| 340 | 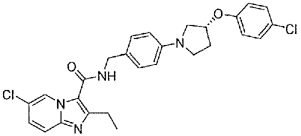 | Pale yellow solid: ¹H-NMR (400 MHz, CDCl3 + CD3OD): δ 1.24 (t, *J* = 7.6 Hz, 3H), 2.19 -2.25 (m, 2H), 2.81 (q, *J* = 7.6 Hz, 2H), 3.33 -3.45 (m, 3H), 3.58 -3.62 (m, 1H), 4.46 (d, *J* = 5.2 Hz, 2H), 4.93 -4.94 (m, 1H), 6.34 (brs, 1H), 6.48 (d, *J* = 8.2 Hz, 2H), 6.72 -6.75 (m, 2H), 7.13 -7.17 (m, 4H), 7.23 -7.26 (m, 1H), 7.42 -7.44 (m, 1H), 9.30 -9.31 (m, 1H); LCMS (electrospray) m/z 509, 511 (M+H)+, Cl- isotope pattern. |
| 341 | 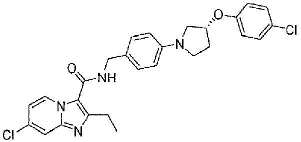 | Pale yellow solid: ¹H-NMR (400 MHz, CDC13): δ 1.35 (t, *J* = 7.6Hz, 3H), 2.27 -2.33 (m, 2H), 2.90 (q, *J* = 7.6 Hz, 2H), 3.41 -3.54 (m, 3H), 3.66 -3.70 (m, 1H), 4.56 (d, *J* = 5.2 Hz, 2H), 4.99 -5.02 (m, 1H), 5.95 (brs, 1H), 6.56 (d, *J* = 8.4 Hz, 2H), 6.79 -6.83 (m, 2H), 6.88 (dd, *J* = 2.0, 7.2 Hz, 1H), 7.21 -7.25 (m, 4H), 7.58 (d, *J* = 2.0 Hz, 1H), 9.34 (d, *J* = 7.2 Hz, 1H); LCMS (electrospray) m/z 509, 511 (M+H)+, Cl- isotope pattern. |
| 342 | 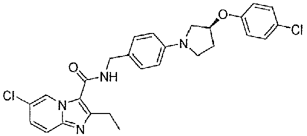 | Pale yellow solid: ¹H-NMR (400 MHz, CDCl3 + CD3OD): δ 1.28 (t, *J* = 7.6 Hz, 3H), 2.23 -2.28 (m, 2H), 2.85 (q, *J* = 7.6 Hz, 2H), 3.37 -3.49 (m, 3H), 3.61 -3.65 (m, 1H), 4.50 (d, *J* = 5.2 Hz, 2H), 4.96 -4.97 (m, 1H), 6.22 (brs, 1H), 6.52 (d, *J* = 8.8 Hz, 2H), 6.75 -6.79 (m, 2H), 7.17 -7.20 (m, 4H), 7.28 (d, *J* = 1.6 Hz, 1H), 7.46 (d, *J* = 9.6 Hz, 1H), 9.37 -9.38 (m, 1H); LCMS (electrospray) m/z 509, 511 (M+H)+, Cl-isotope pattern. |
| 343 | 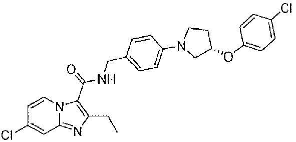 | Pale yellow solid: ¹H-NMR (400 MHz, CDCl3): δ 1.34 (t, *J* = 7.6 Hz, 3H), 2.26 -2.33 (m, 2H), 2.89 (q, *J* = 7.6 Hz, 2H), 3.41 -3.53 (m, 3H), 3.66 -3.70 (m, 1H), 4.56 (d, *J* = 5.2 Hz, 2H), 4.99 -5.01 (m, 1H), 5.97 (bit, *J* = 5.2 Hz, 1H), 6.56 (d, *J* = 8.4 Hz, 2H), 6.78 -6.82 (m, 2H), 6.87 (dd, *J* = 2.0, 7.6 Hz, 1H), 7.21 -7.25 (m, 4H), 7.57 (d, *J* = 2.0 Hz, 1H), 9.34 (d, *J* = 7.6 Hz, 1H); LCMS (electrospray) m/z 509, 511 (M+H)+, Cl-isotope pattern. |
| 344 | 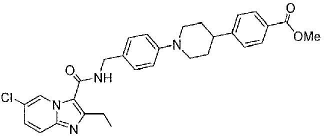 | Pale yellow solid: ¹H-NMR (400 MHz, CDC13): δ 1.39 (t, *J* = 7.6 Hz, 3H), 1.99 -2.19 (m, 4H), 2.77 -2.80 (m, 1H), 2.95 -3.05 (m, 4H), 3.79 - 3.82 (m, 2H), 3.90 (s, 3H), 4.64 (d, *J* = 5.6 Hz, 2H), 6.41 (brs, 1H), 7.31 - 7.39 (m, 7H), 7.61 (d, *J* = 9.6 Hz, 1H), 7.97 -8.00 (m, 2H), 9.49 (d, *J* = 1.2 Hz, 1H); LCMS (electrospray) m/z 531, 533 (M+H)+ , Cl- isotope pattern. |
| 345 | 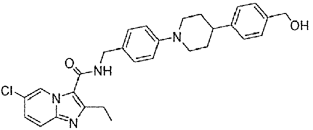 | Yellow solid: ¹H-NMR (400 MHz, CDCl3 + CD3OD): δ 1.32 (t, *J* = 7.6 Hz, 3H), 1.97 -2.03 (m, 2H), 2.65 -2.80 (m, 1H), 2.95 -3.01 (m, 6H), 3.71 -3.74 (m, 2H), 4.57 (s, 2H), 4.57 (d, *J* = 6.4 Hz, 2H), 7.21 -7.28 (m, 6H), 7.40 -7.43 (m, 3H), 7.62 (d, *J* = 9.2 Hz, 1H), 9.28 (d, *J* = 1.6 Hz, 1H); LCMS (electrospray) m/z 503, 505 (M+H)+ , Cl-isotope pattern. |
| 346 | 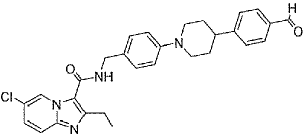 | Violet solid: ¹H-NMR (400 MHz, CDCl3): δ 1.39 (t, *J* = 7.6 Hz, 3H), 1.99 -2.09 (m, 4H), 2.79 -3.02 (m, 4H), 3.47 -3.50 (m, 1H), 3.81 -3.84 (m, 2H), 4.63 (s, 2H), 6.16 (brs, 1H), 7.00 -7.10 (m, 2H), 7.27 -7.45 (m, 5H), 7.57 -7.59 (m, 1H), 7.84 -7.85 (m, 2H), 9.52 (s, 1H), 9.99 (s, 1H); LCMS (electrospray) m/z 501, 503 (M+H)+, Cl- isotope pattern. |
| 347 | 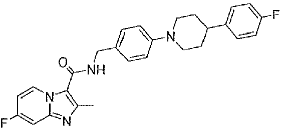 | Pale pink solid: ¹H-NMR (400 MHz, CDCl3): δ 1.94 -2.04 (m, 4H), 2.61 - 2.71 (m, 1H), 2.66 (s, 3H), 3.78 -3.81 (m, 2H), 4.61 (d, *J* = 5.6 Hz, 2H), 6.15 (brs, 1H), 6.79 -6.83 (m, 1H), 6.98 -7.16 (m, 4H), 7.19 -7.24 (m, 3H), 7.31 -7.33 (m, 2H), 9.43 -9.47 (m, 1H); LCMS (electrospray) m/z 461 (M+H)+. |
| 348 | 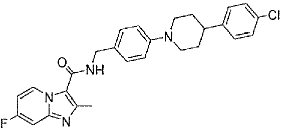 | White solid: ¹H-NMR (400 MHz, CDCl3): δ 1.96 -2.02 (m, 4H), 2.65 - 2.75 (m, 1H), 2.70 (s, 3H), 2.95 -2.99 (m, 2H), 3.77 -3.80 (m, 2H), 4.63 (d, *J* = 5.6 Hz, 2H), 6.42 (brs, 1H), 6.84 -6.88 (m, 1H), 7.19 -7.24 (m, 2H), 7.26 -7.33 (m, 5H), 7.37 -7.41 (m, 2H), 9.42 -9.45 (m, 1H); LCMS (electrospray) m/z 477, 479 (M+H)+, Cl-isotope pattern |
| 349 | 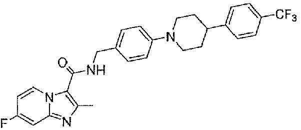 | Pale pink solid: ¹H-NMR (400 MHz, CDCl3): δ 1.97 -2.05 (m, 4H), 2.68 (s, 3H), 2.73 -2.82 (m, 1H), 2.83 -2.96 (m, 2H), 3.80 -3.83 (m, 2H), 4.62 (d, *J* = 5.6 Hz, 2H), 6.25 (brs, 1H), 6.81 -6.85 (m, 1H), 7.00 -7.18 (m, 2H), 7.24 -7.27 (m, 1H), 7.31 -7.41 (m, 4H), 7.56 -7.58 (m, 2H), 9.43 - 9.47 (m, 1H); LCMS (electrospray) m/z 511 (M+H)+. |
| 350 | 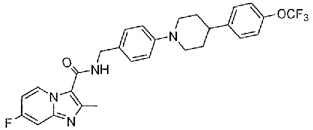 | White solid: ¹H-NMR (400 MHz, CDCl3): δ 1.96 -2.05 (m, 4H), 2.69 (s, 3H), 2.69 -2.74 (m, 1H), 2.85 -2.96 (m, 2H), 3.78 -3.81 (m, 2H), 4.62 (d, *J* = 5.6 Hz, 2H), 6.31 (brs, 1H), 6.82 -6.87 (m, 1H), 7.15 -7.17 (m, 2H), 7.20 -7.30 (m, 5H), 7.35 -7.41 (m, 2H), 9.43 -9.46 (m, 1H); LCMS (electrospray) m/z 527 (M+H)+. |

## Claims

1. A compound having the general formula I: 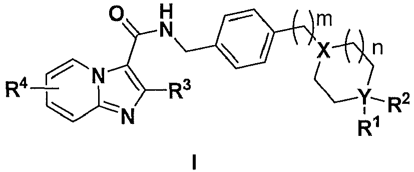 wherein
X is CH or N;
Y is CH, O or N;
m is 0 or 1;
n is 0 or 1;
R¹ is, at each occurrence, independently selected from the group consisting of hydrogen, halogen, methyl, ethyl, t-butyl, phenyl, -NC(O)R⁵, -OR⁵, -C(O)R⁵, -C(O)OR⁵;
R² is, at each occurrence, independently selected from the group consisting of hydrogen and hydroxyl;
R³ is, at each occurrence, independently selected from the group consisting of methyl and ethyl;
R⁴ is, at each occurrence, independently selected from the group consisting of hydrogen, halogen, methyl, -methoxy and -CF₃;
R⁵ is, at each occurrence, independently selected from the group consisting of C₁-C₃ alkylhetorocycle, phenyl and benzyl;
and pharmaceutically acceptable salts thereof;
or
a compound having the general formula VII: 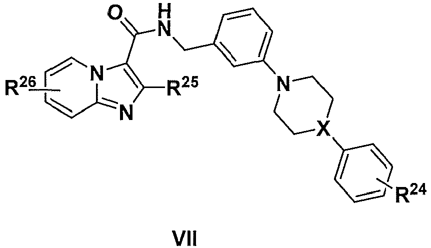 wherein
X is CH or N
R²⁴ is, at each occurrence, independently selected from the group consisting of hydrogen, halogen, C₁-C₂ alkyl, -methoxy, -CF₃ and -OCF₃;
R²⁵ is, at each occurrence, independently selected from the group consisting of methyl and ethyl;
R²⁶ is, at each occurrence, independently selected from the group consisting of hydrogen, halogen, methyl, -methoxy and -CF₃;
and pharmaceutically acceptable salts thereof, or
a compound having the general formula VIII: 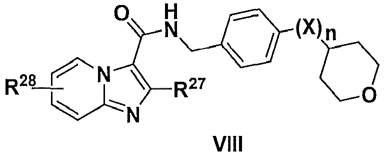 wherein
X is CH₂ or NH
n is 0 or 1
R²⁷ is, at each occurrence, independently selected from the group consisting of methyl and ethyl;
R²⁸ is, at each occurrence, independently selected from the group consisting of hydrogen, halogen, methyl, -methoxy and -CF₃;
and pharmaceutically acceptable salts thereof,
wherein the compound has one of the formulae 1-231, 238, 281-285, 289-300, 309-316, 325-328, 333-337, 340-350, as shown in the following table: 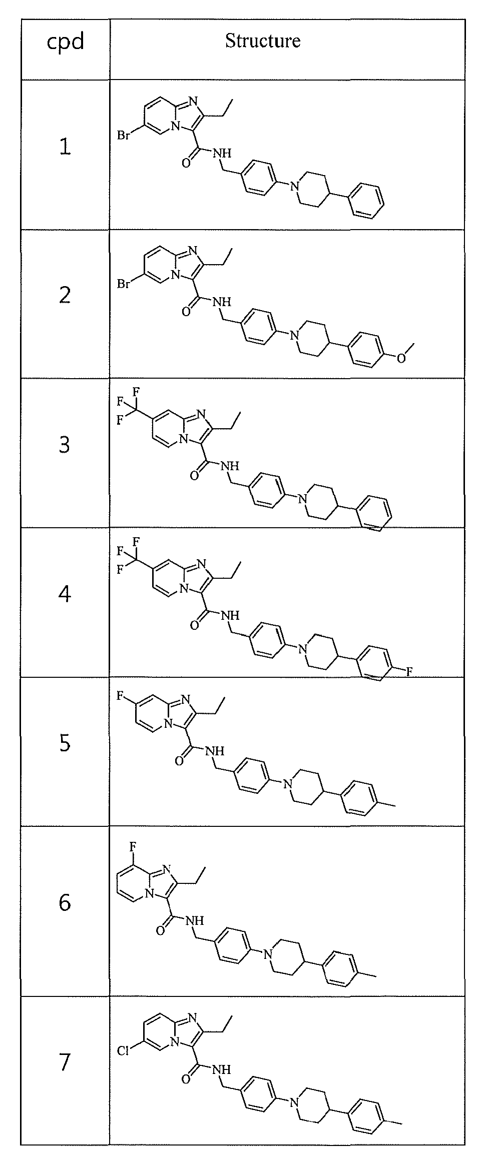 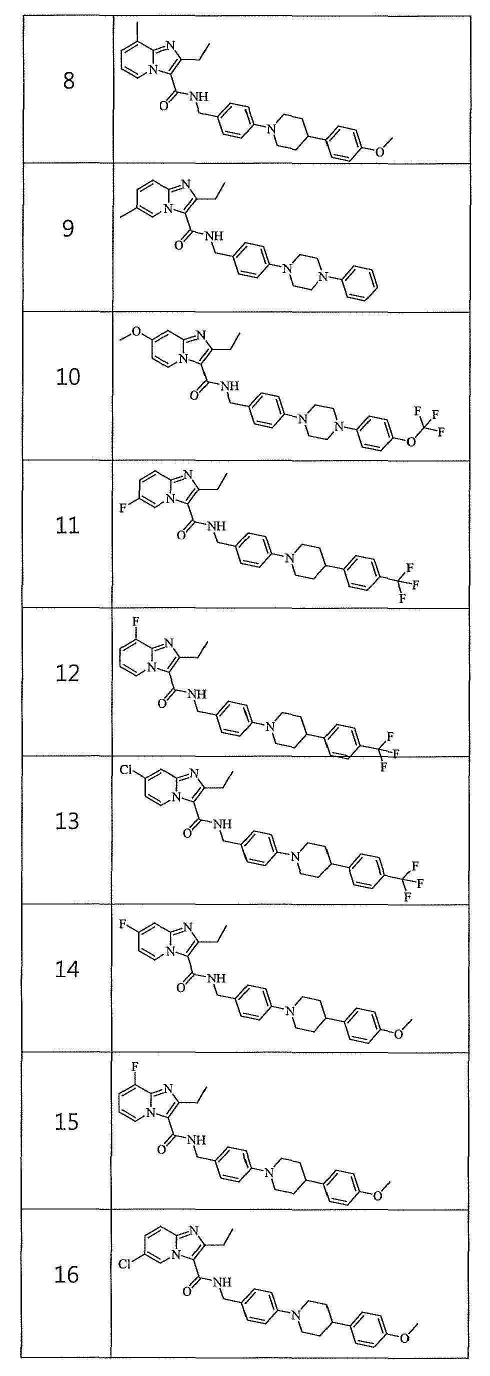 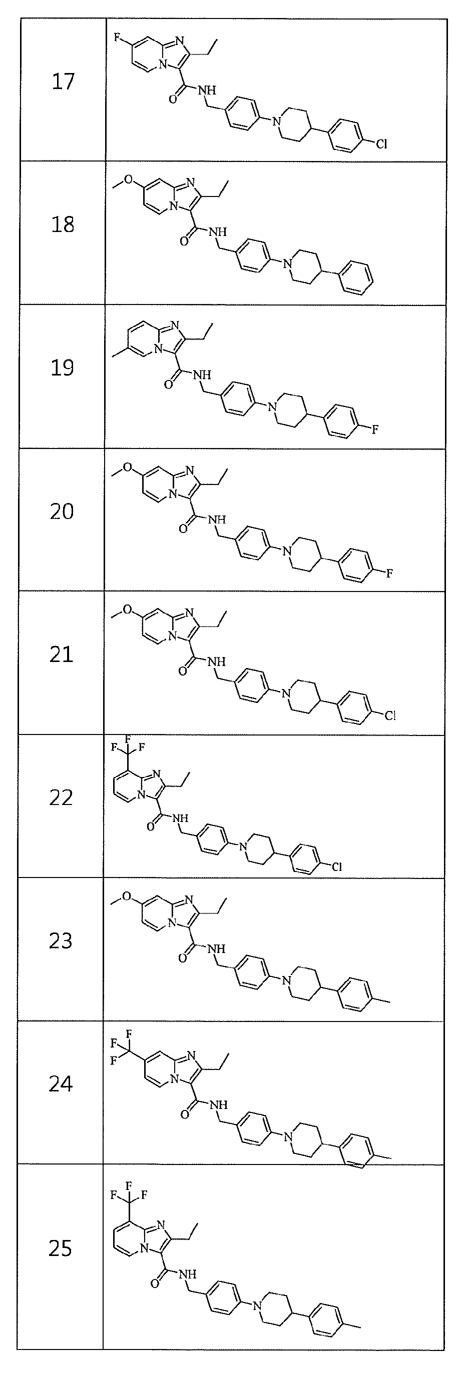 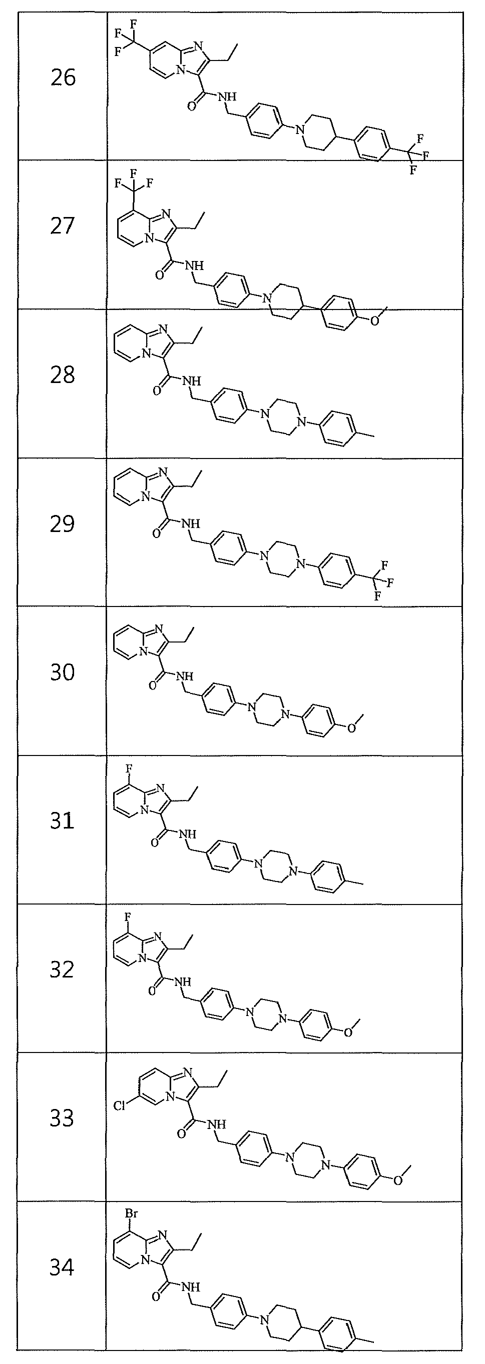 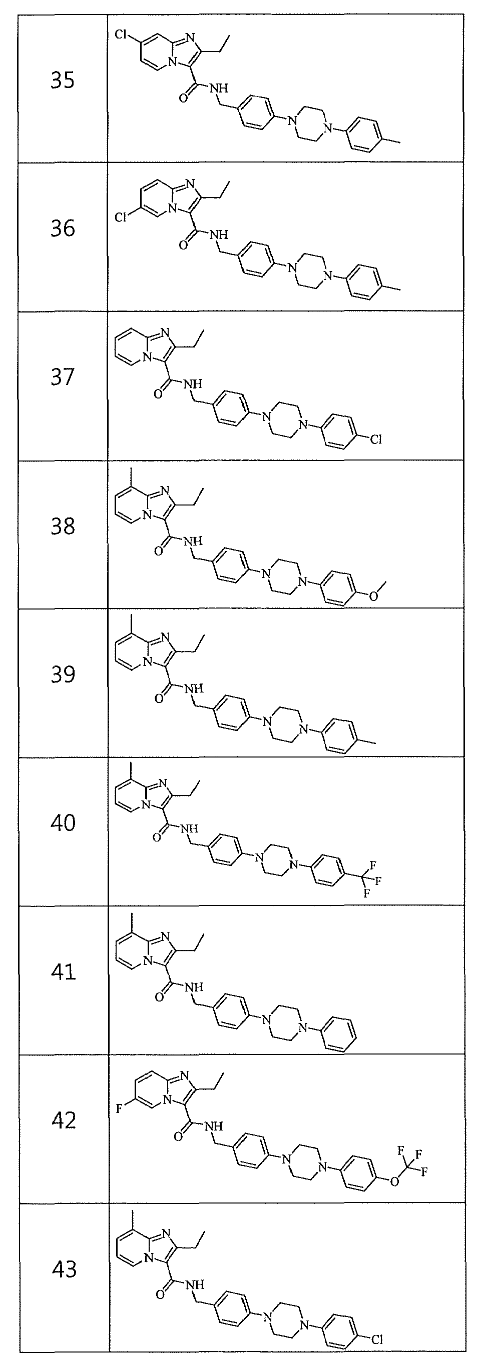 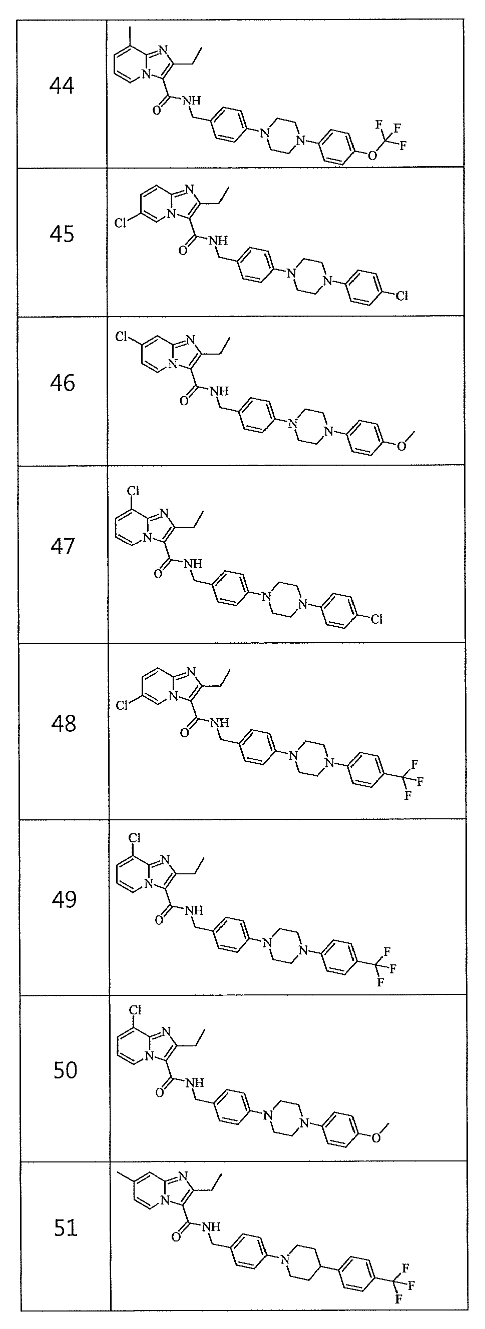 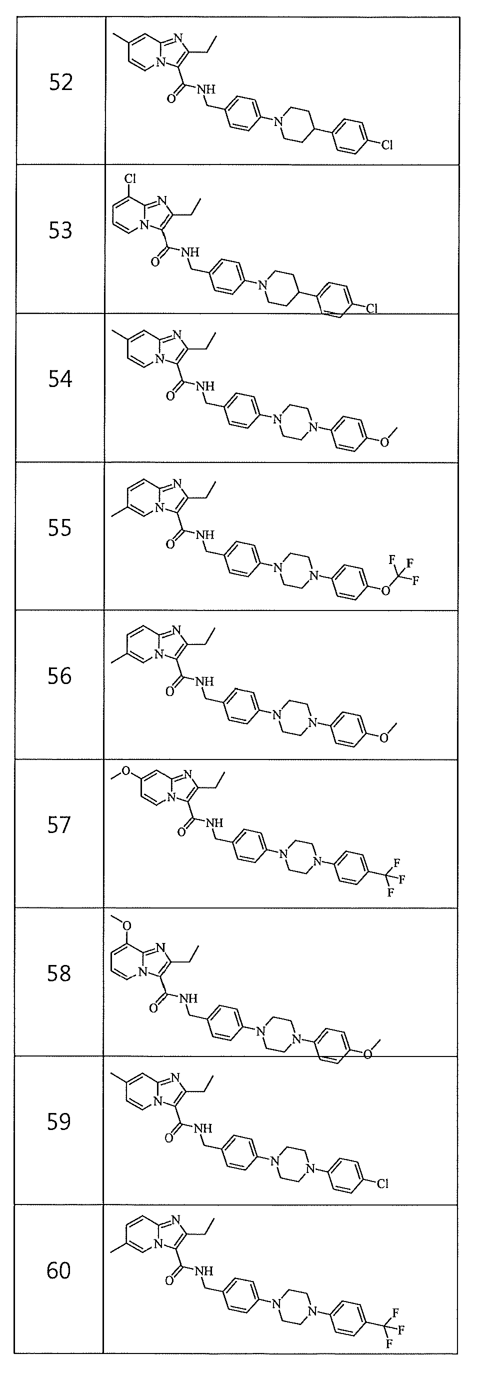 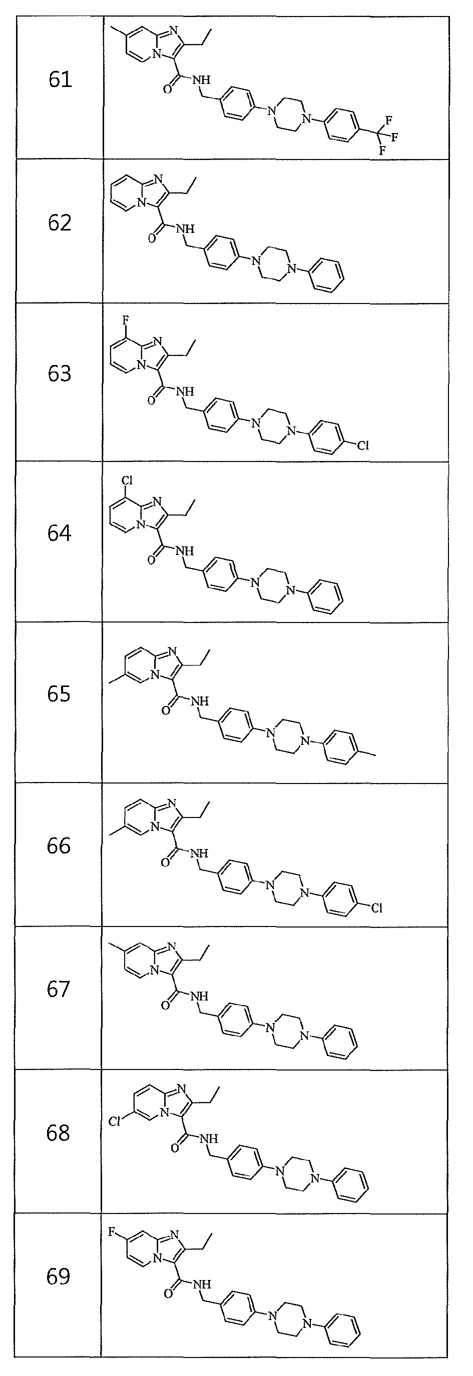 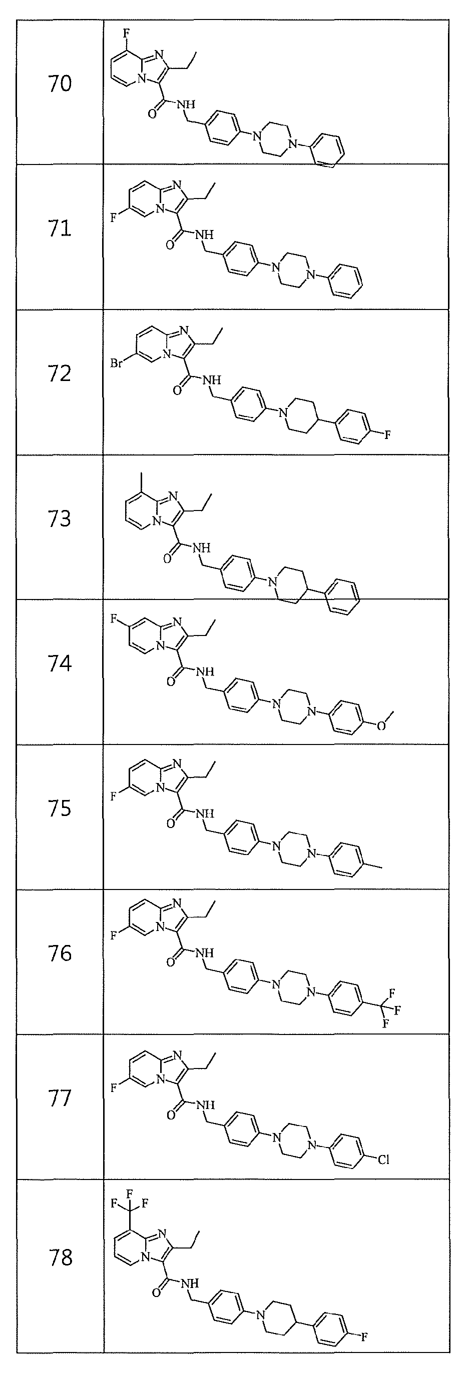 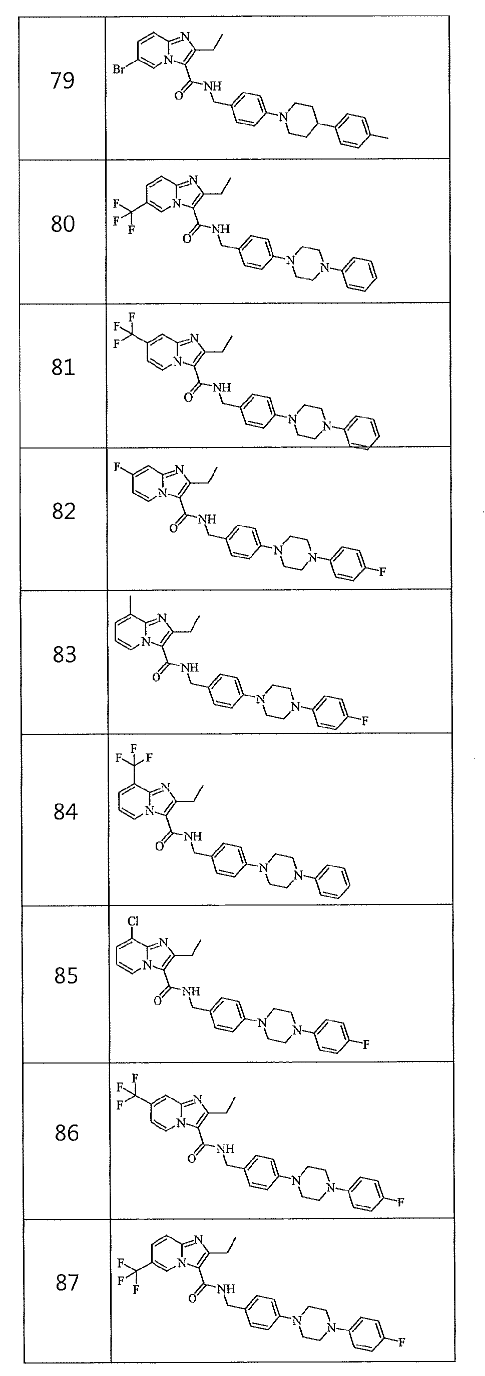 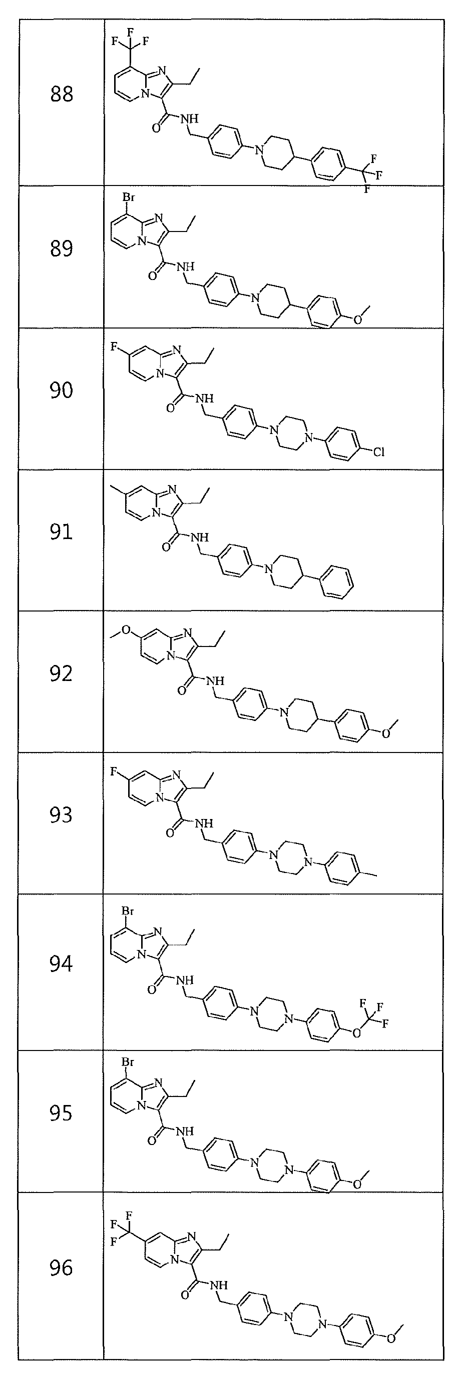 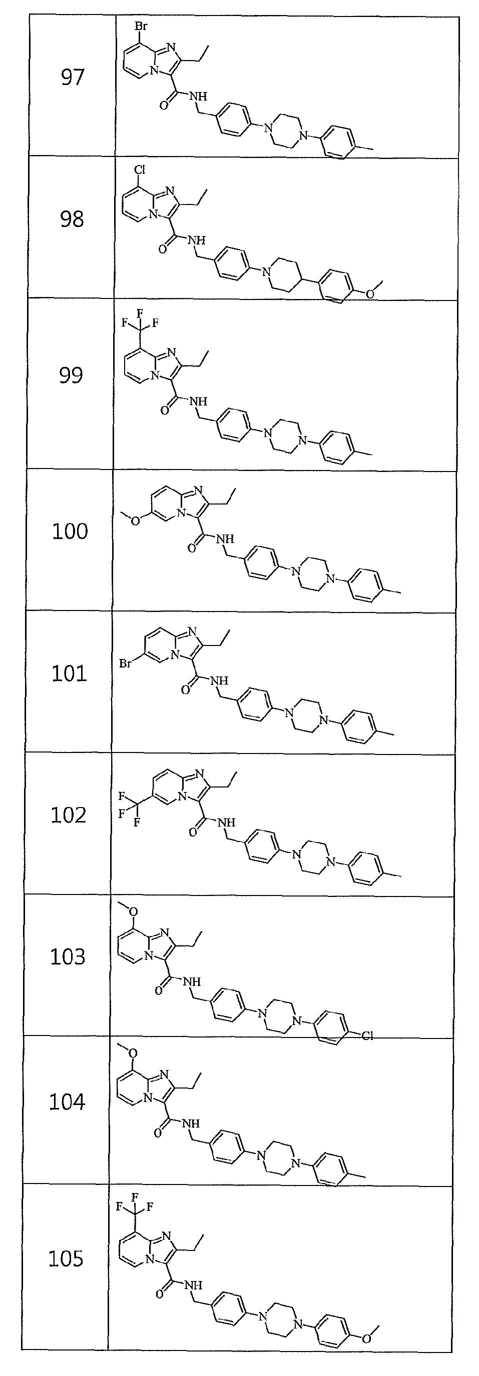 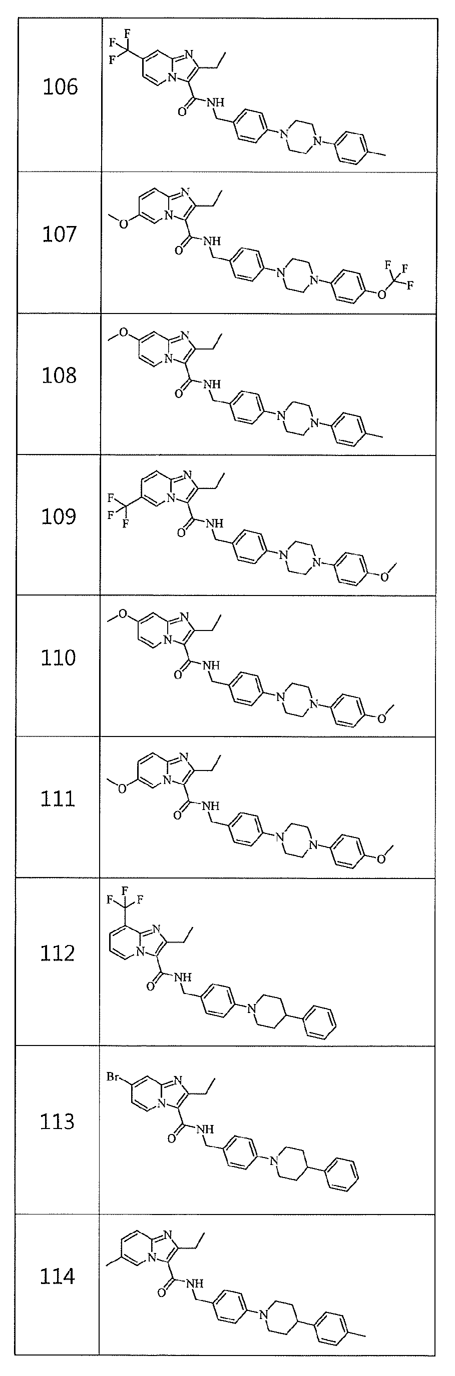 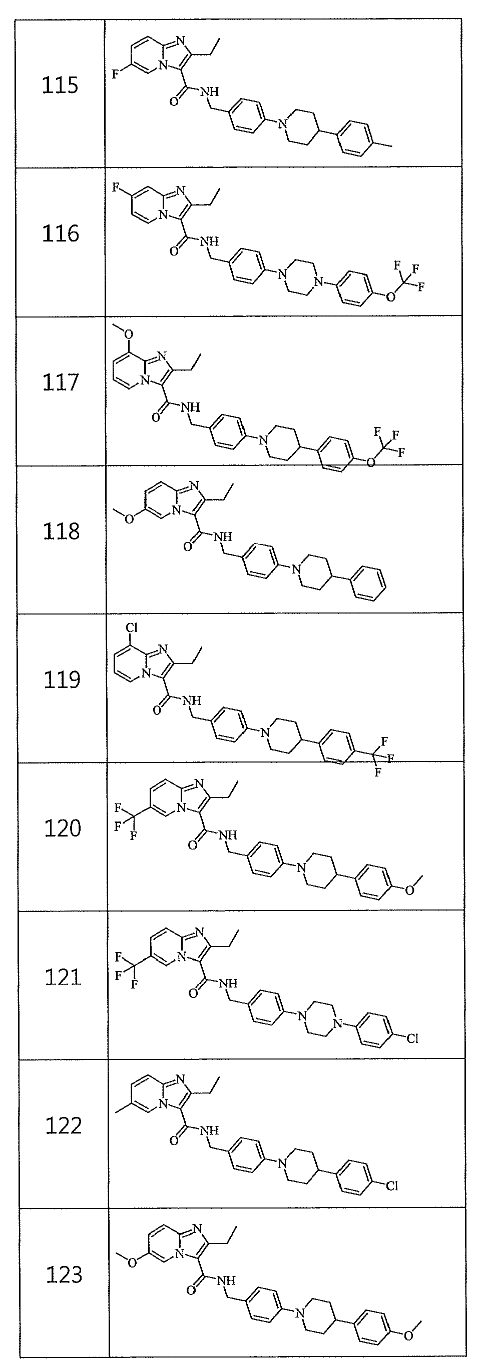 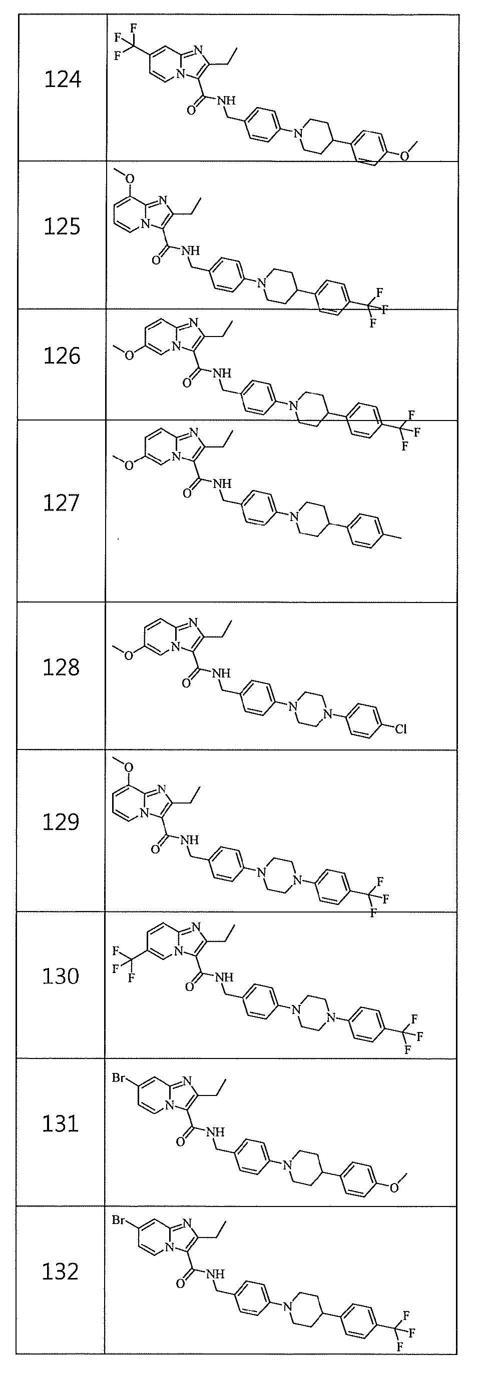 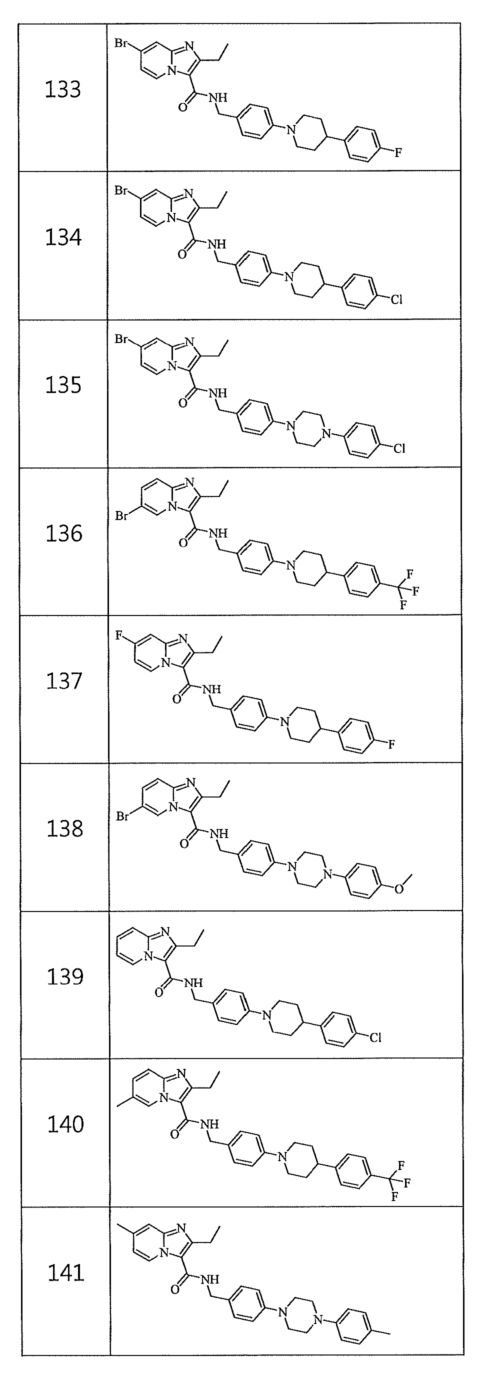 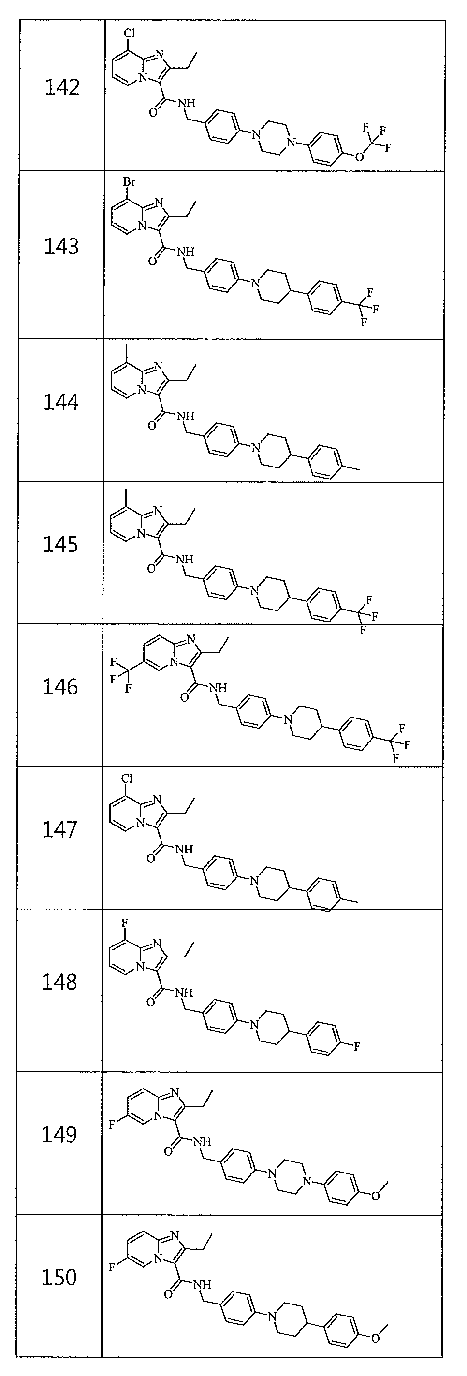 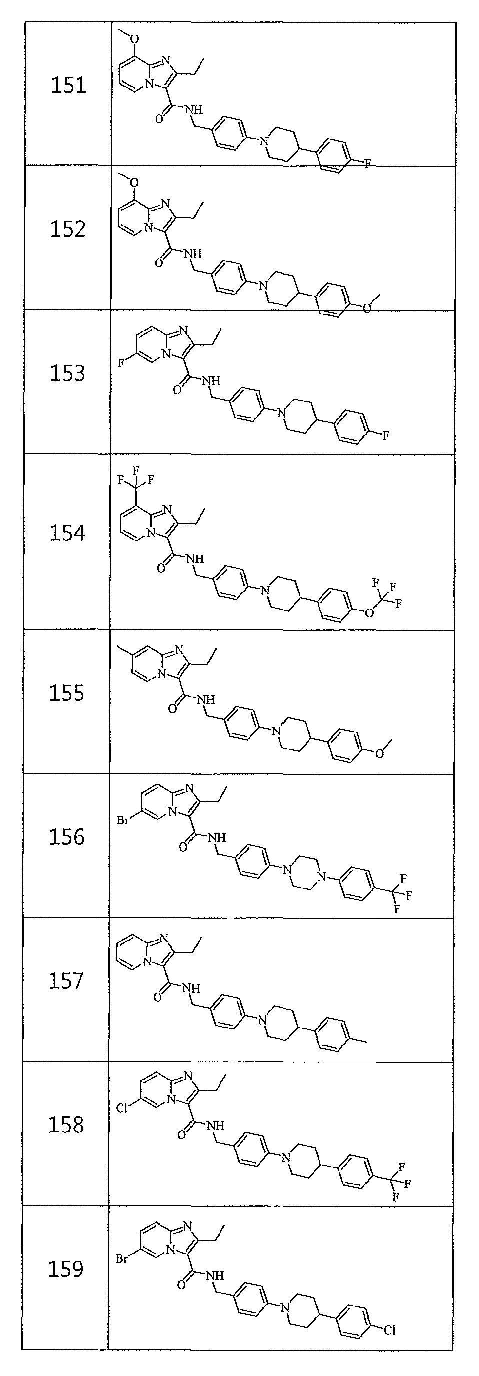 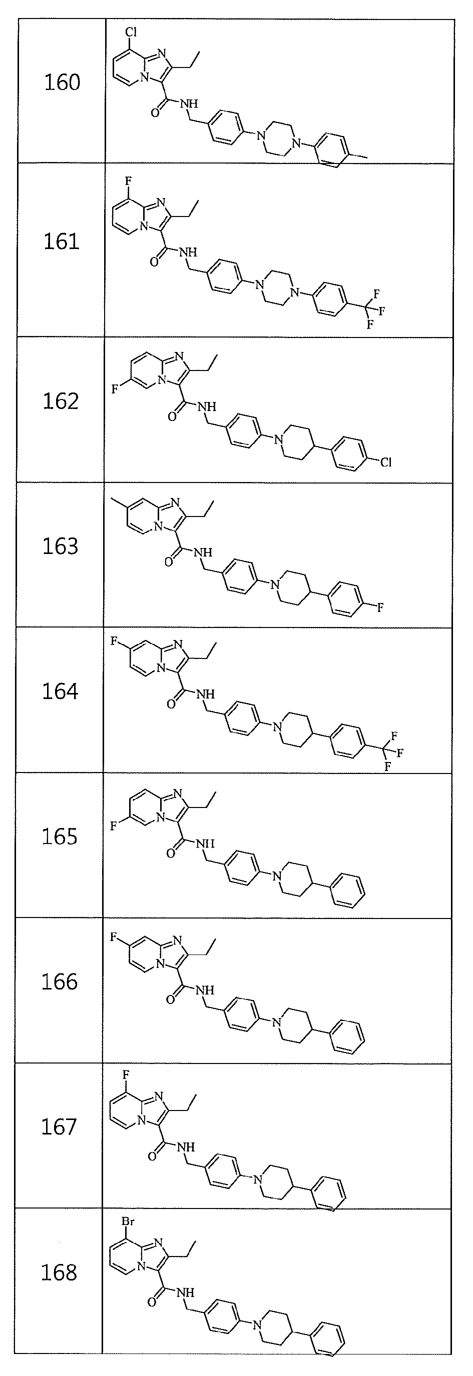 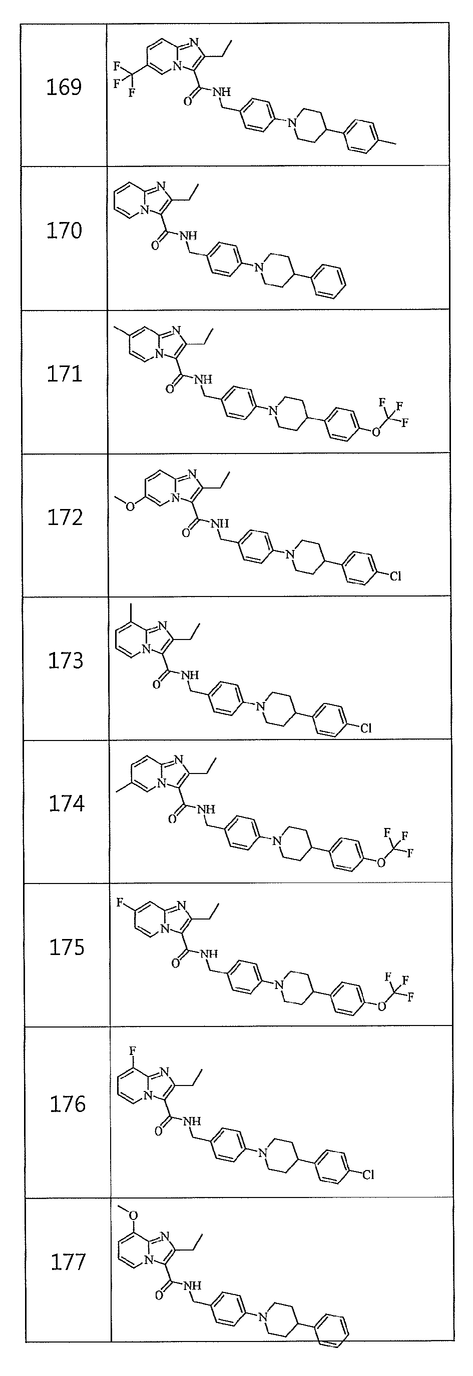 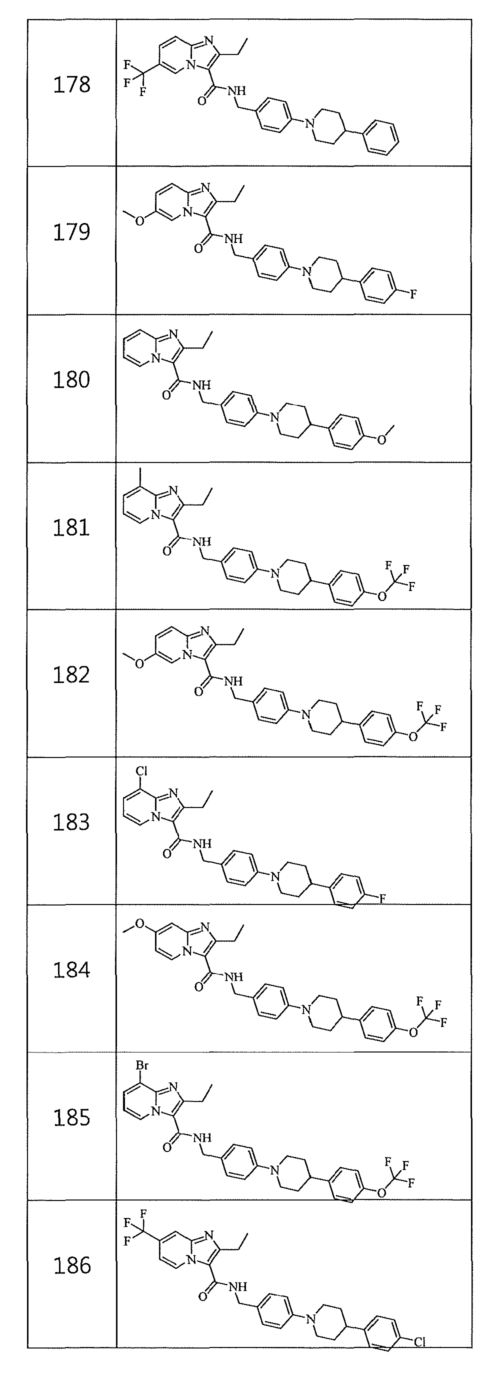 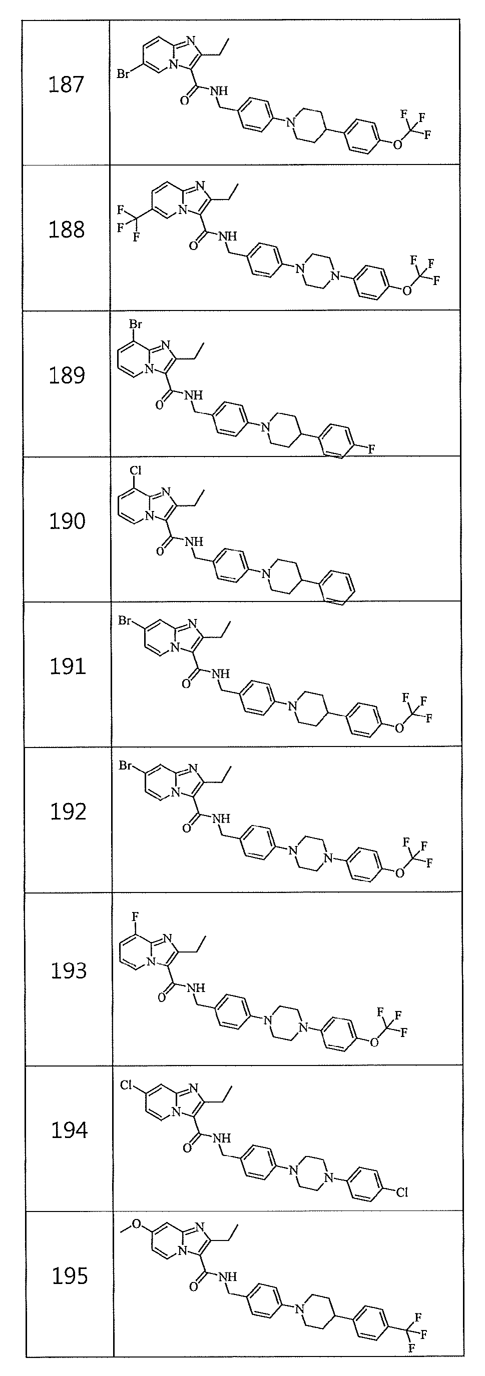 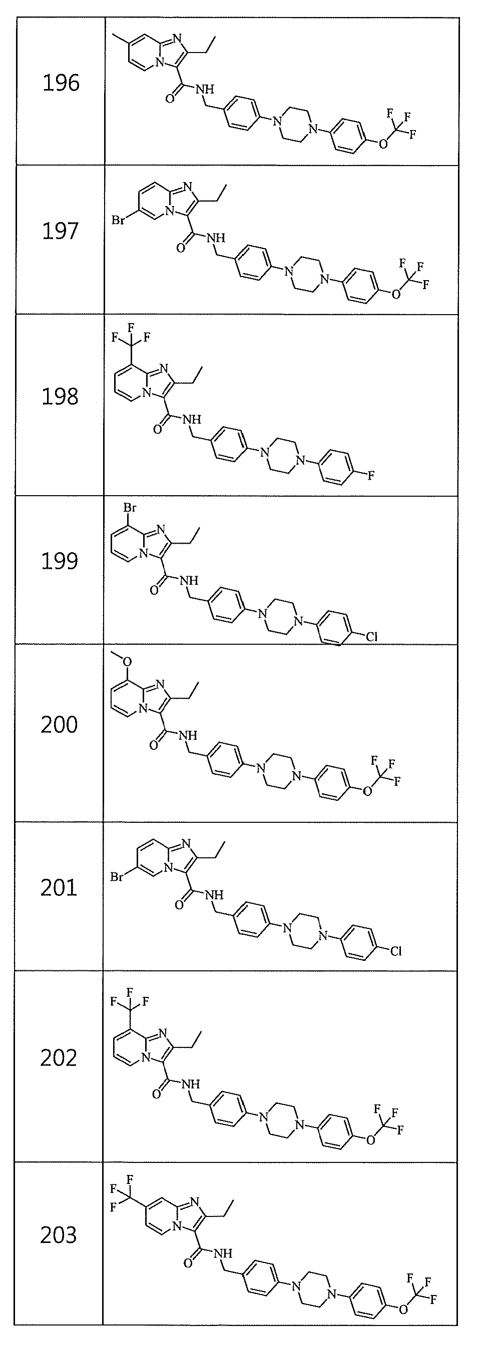 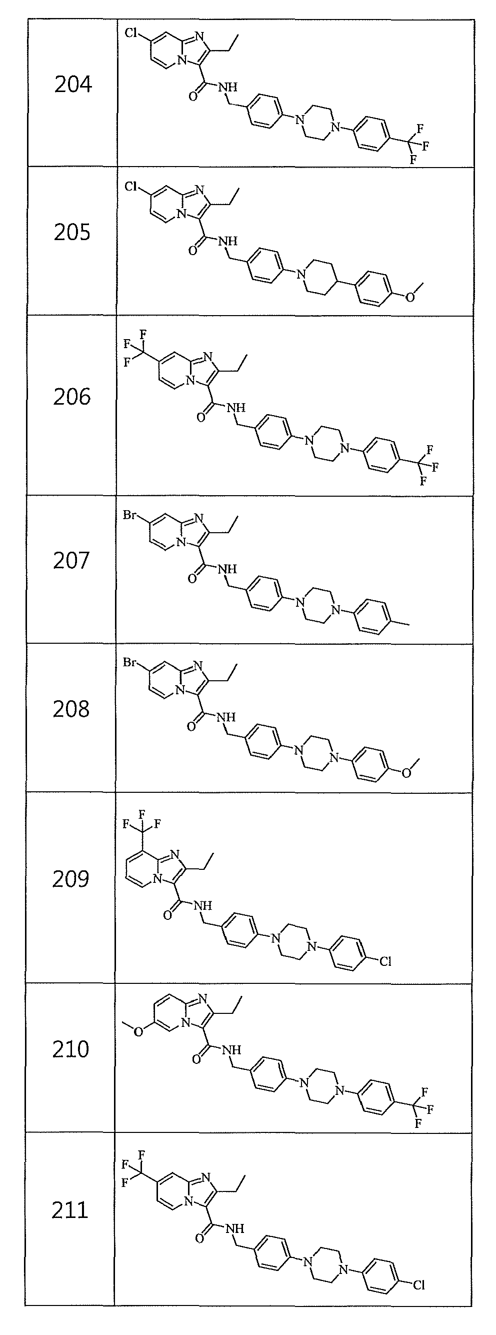 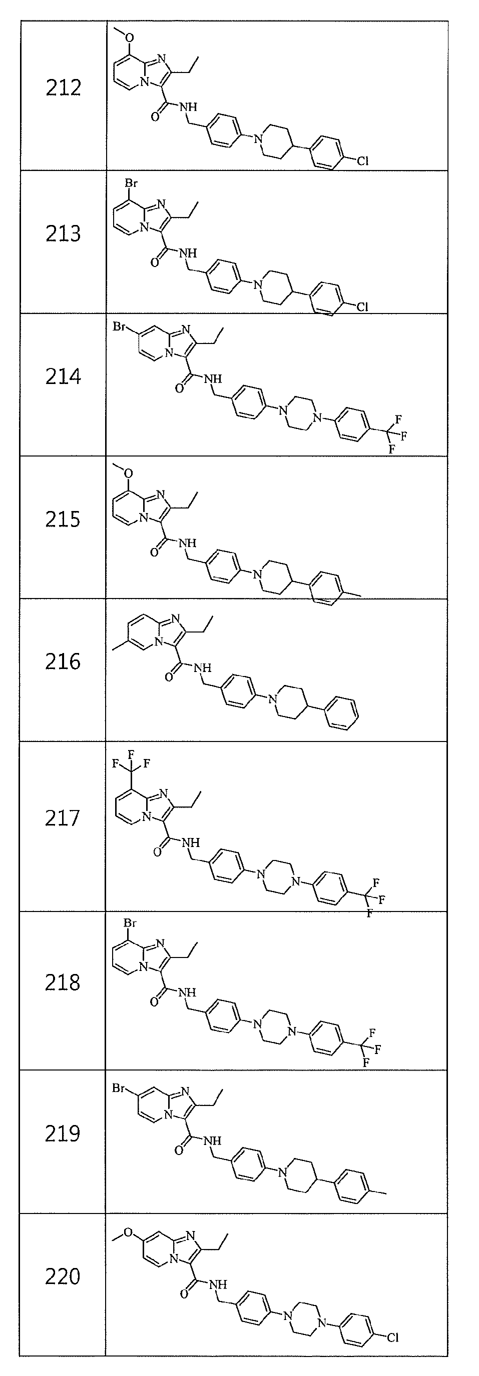 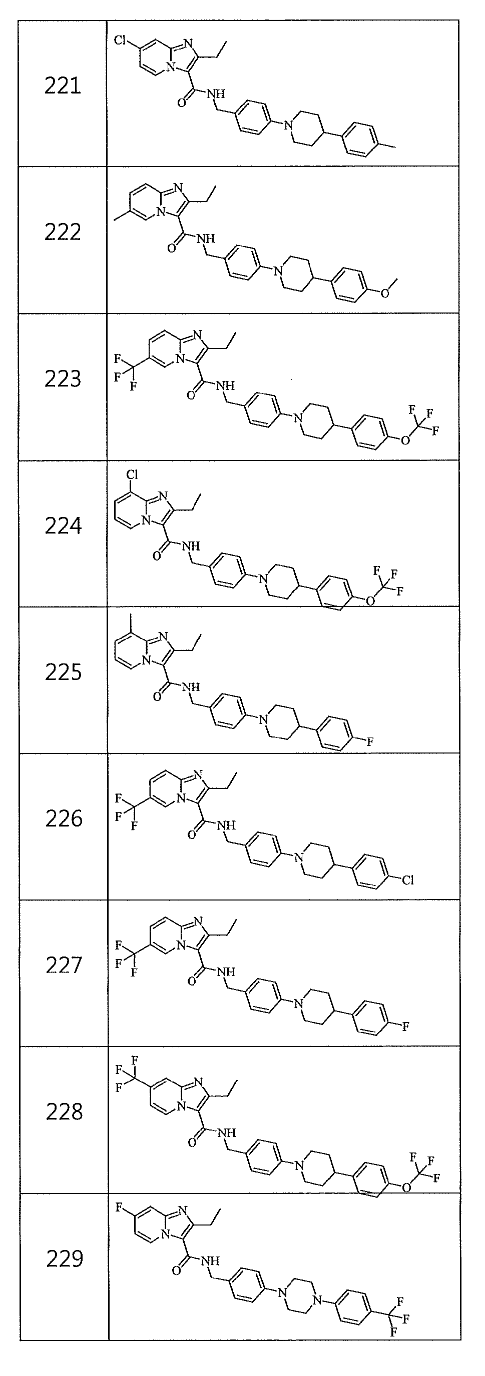 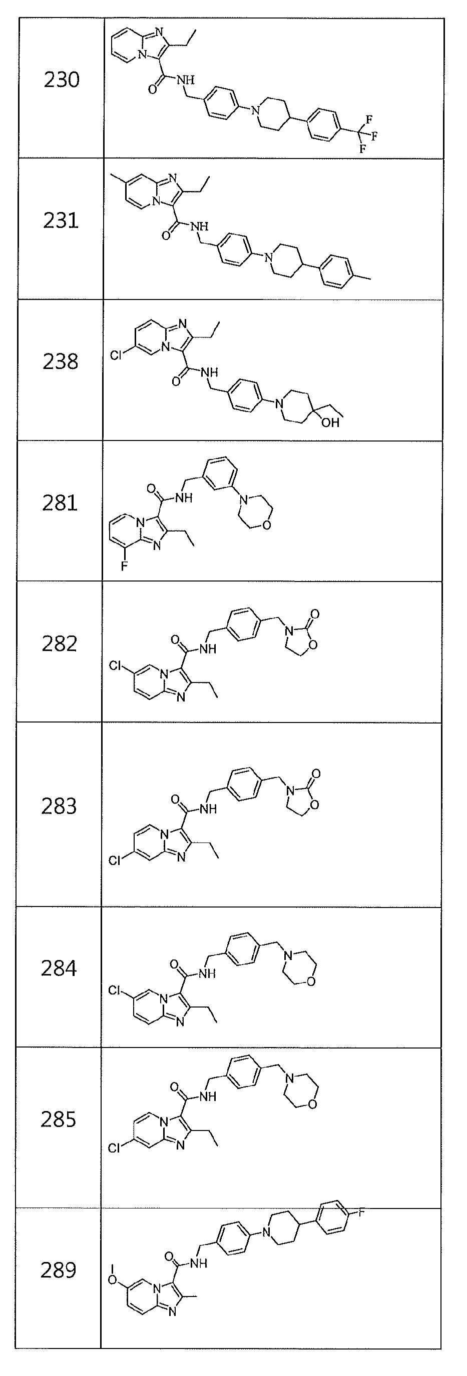 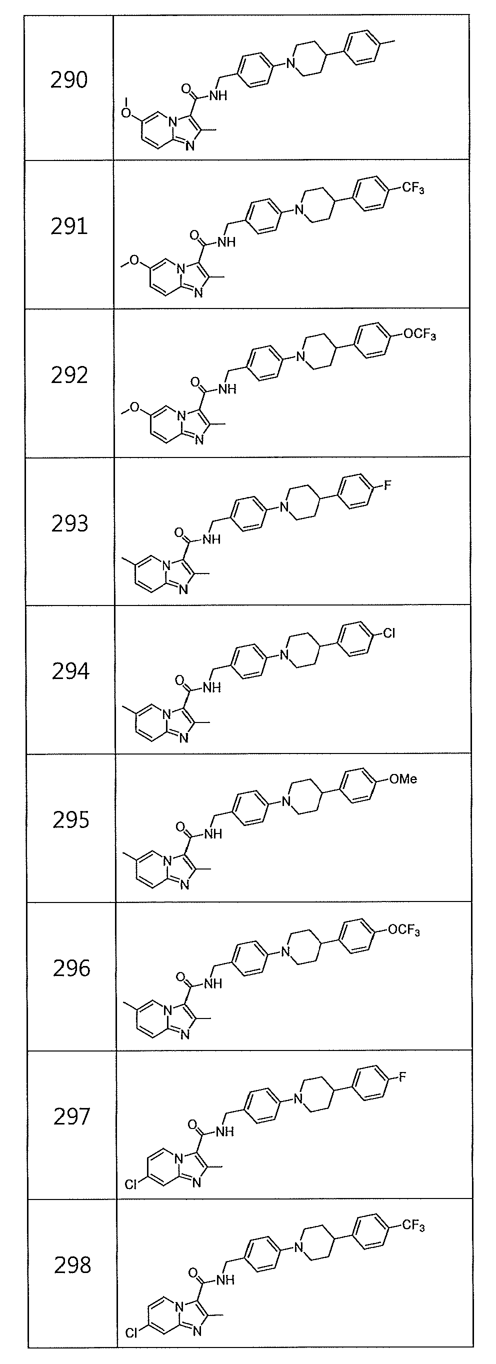 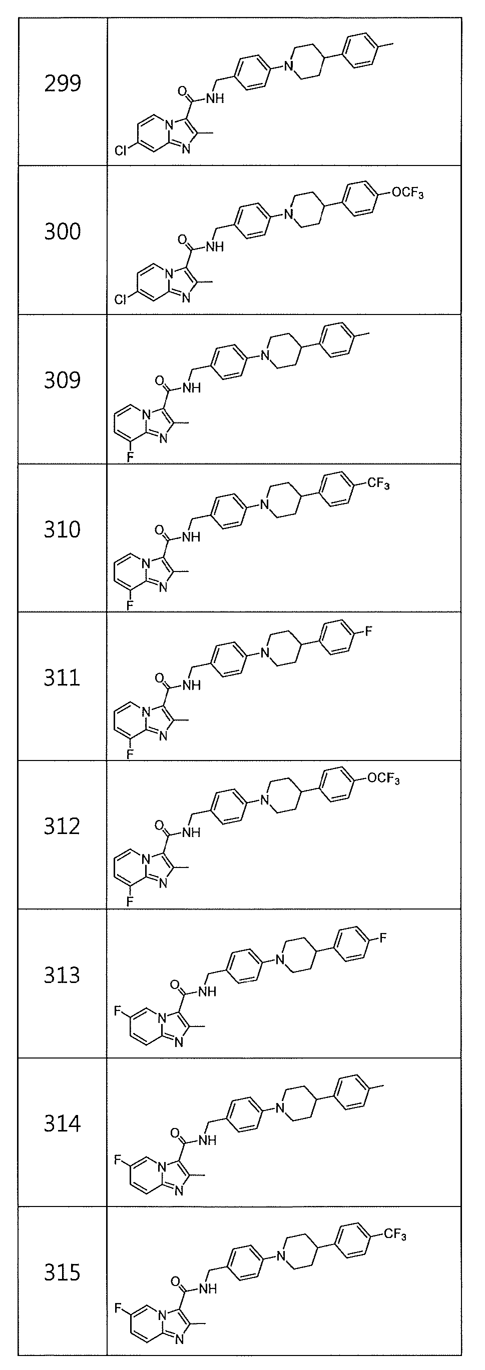 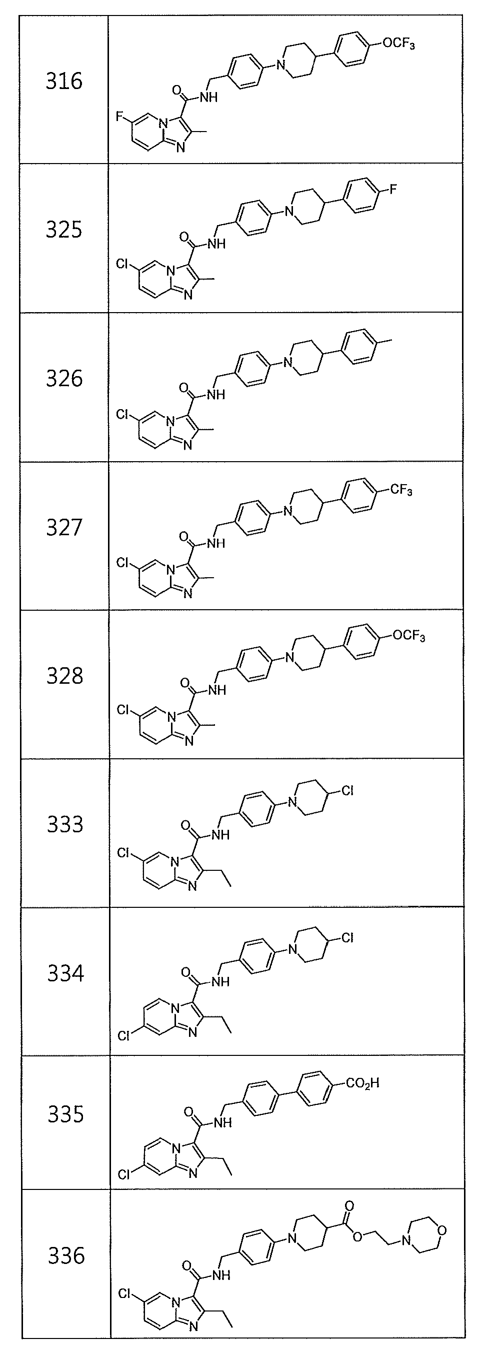 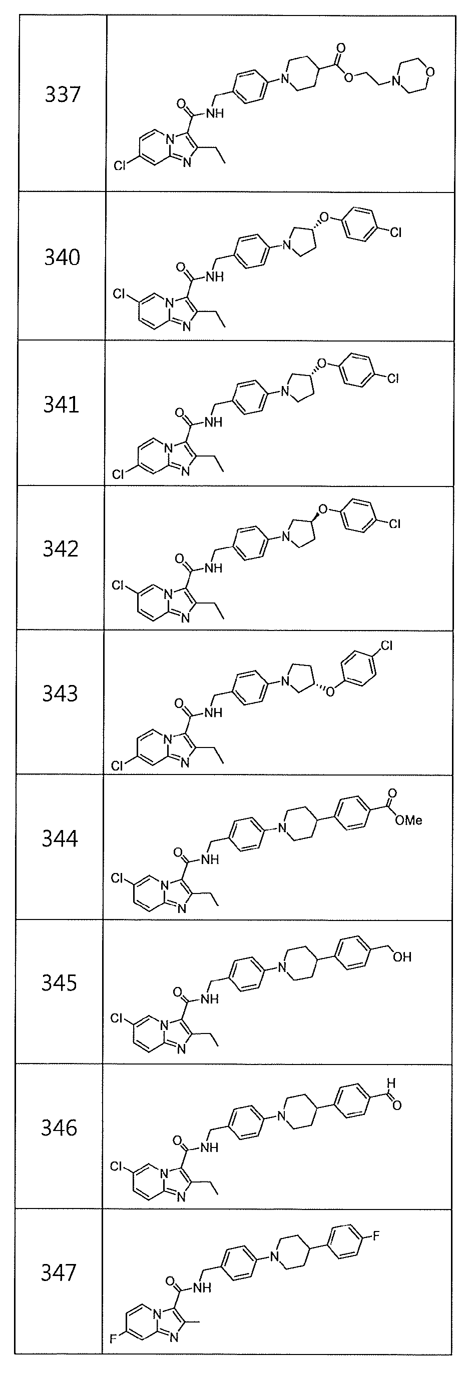 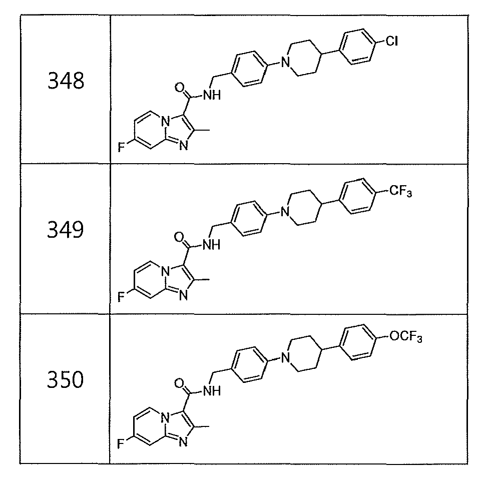 , preferably one of the formulae 1-21, 23-24, 26, 28-33, 35-57, 59-77, 79-83, 85-87, 90-98, 100-102, 106-111, 113-116 118-124, 126-128, 130-142, 144-150, 153, 155-167, 169-184, 186-188, 190-197, 199, 201, 203-208, 210-211, 213-214, 216, 218-231, 238, 281-285, 289-300, 309-316, 325-328, 333-337, 340-350 as shown above.

2. The compound according to claim 1, having one of the formulae 55, 171, 175 and 325 as shown hereafter:
| | |
|---|---|
| 55 | 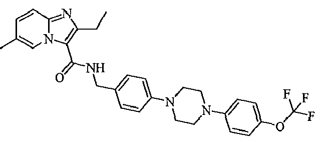 |
| 171 | 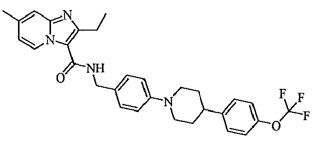 |
| 175 | 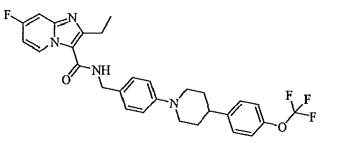 |
| 325 | 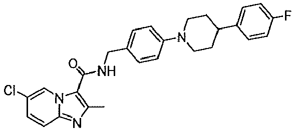 |
and pharmaceutically acceptable salts thereof.

3. The compound according to any of claims 1 - 2, for use in the treatment of a bacterial infection, wherein, preferably, said bacterial infection is Tuberculosis.

4. A pharmaceutical composition comprising a compound according to any of claims 1 - 3, and a pharmaceutically acceptable carrier.

## Patentansprüche

1. Verbindung mit der allgemeinen Formel I: 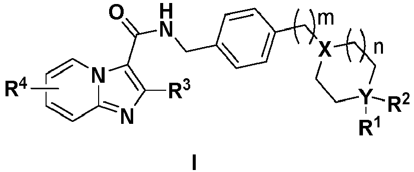 wobei
X CH oder N ist;
Y CH, O oder N ist;
m 0 oder 1 ist;
n 0 oder 1 ist;
R¹, bei jedem Auftreten, unabhängig ausgewählt ist aus der Gruppe, bestehend aus Wasserstoff, Halogen, Methyl, Ethyl, t-Butyl, Phenyl, -NC(O)R⁵, -OR⁵, -C(O)R⁵, -C(O)OR⁵;
R², bei jedem Auftreten, unabhängig ausgewählt ist aus der Gruppe, bestehend aus Wasserstoff und Hydroxyl;
R³, bei jedem Auftreten, unabhängig ausgewählt ist aus der Gruppe, bestehend aus Methyl und Ethyl;
R⁴, bei jedem Auftreten, unabhängig ausgewählt ist aus der Gruppe, bestehend aus Wasserstoff, Halogen, Methyl, -Methoxy und -CF₃;
R⁵, bei jedem Auftreten, unabhängig ausgewählt ist aus der Gruppe, bestehend aus C₁-C₃ Alkylheterocyclus, Phenyl und Benzyl;
und pharmazeutisch akzeptable Salze davon;
oder
eine Verbindung mit der allgemeinen Formel VII: 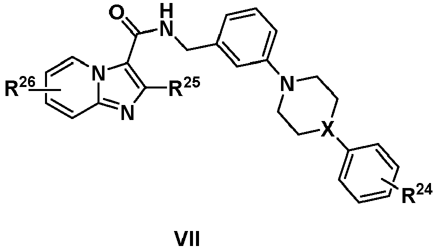 wobei
X CH oder N ist,
R²⁴, bei jedem Auftreten, unabhängig ausgewählt ist aus der Gruppe, bestehend aus Wasserstoff, Halogen, C₁-C₂ Alkyl, -Methoxy, -CF₃ und -OCF₃;
R²⁵, bei jedem Auftreten, unabhängig ausgewählt ist aus der Gruppe, bestehend aus Methyl und Ethyl;
R²⁶, bei jedem Auftreten, unabhängig ausgewählt ist aus der Gruppe, bestehend aus Wasserstoff, Halogen, Methyl, -Methoxy und -CF₃;
und pharmazeutisch akzeptable Salze davon,
oder
eine Verbindung mit der allgemeinen Formel VIII: 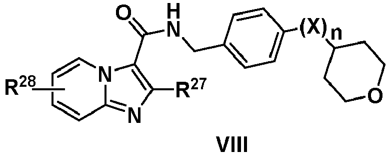 wobei
X CH₂ oder NH ist,
n 0 oder 1 ist,
R²⁷, bei jedem Auftreten, unabhängig ausgewählt ist aus der Gruppe, bestehend aus Methyl und Ethyl;
R²⁸, bei jedem Auftreten, unabhängig ausgewählt ist aus der Gruppe, bestehend aus Wasserstoff, Halogen, Methyl, -Methoxy und -CF₃;
und pharmazeutisch akzeptable Salze davon;
wobei die Verbindung eine der Formeln 1-231, 238, 281-285, 289-300, 309-316, 325-328, 333-337, 340-350 hat, wie in der folgenden Tabelle gezeigt:
| Verbindung | Struktur |
|---|---|
| 1 | 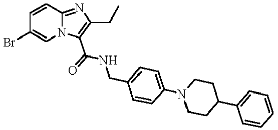 |
| 2 | 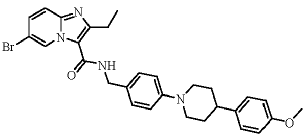 |
| 3 | 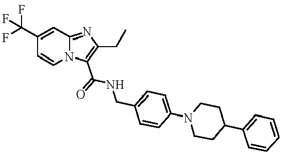 |
| 4 | 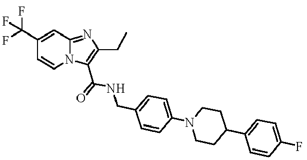 |
| 5 | 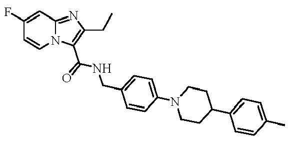 |
| 6 | 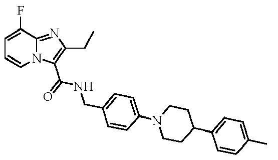 |
| 7 | 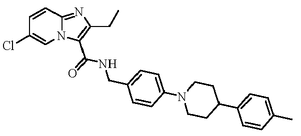 |
| 8 | 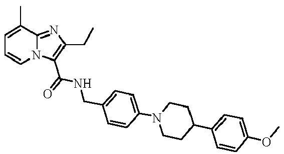 |
| 9 | 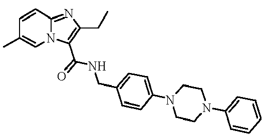 |
| 10 | 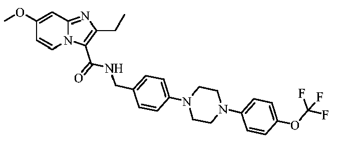 |
| 11 | 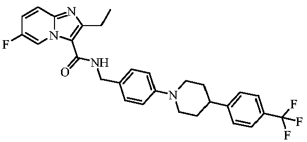 |
| 12 | 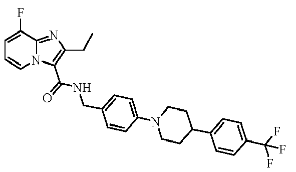 |
| 13 | 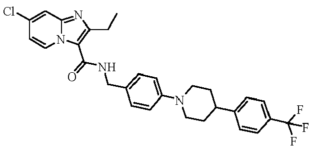 |
| 14 | 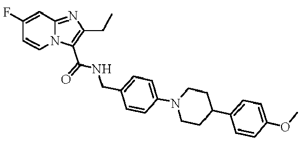 |
| 15 | 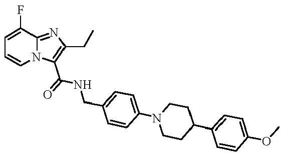 |
| 16 | 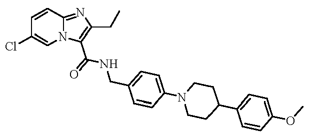 |
| 17 | 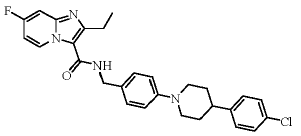 |
| 18 | 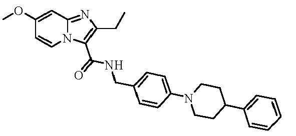 |
| 19 | 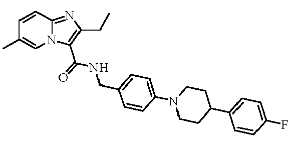 |
| 20 | 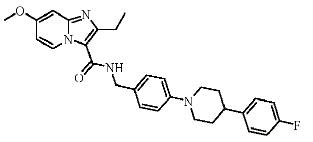 |
| 21 | 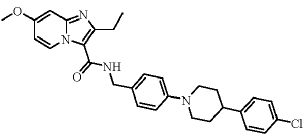 |
| 22 | 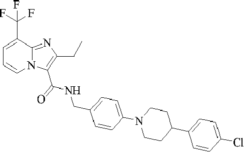 |
| 23 | 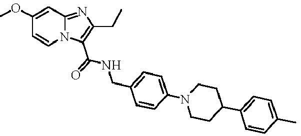 |
| 24 | 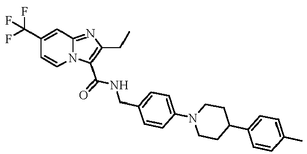 |
| 25 | 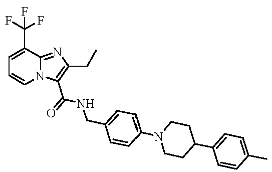 |
| 26 | 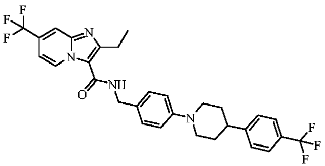 |
| 27 | 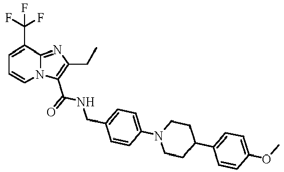 |
| 28 | 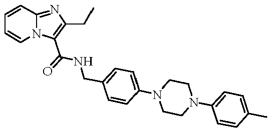 |
| 29 | 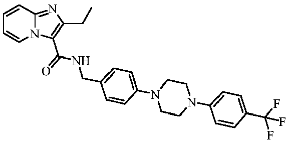 |
| 30 | 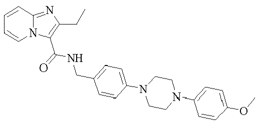 |
| 31 | 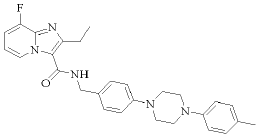 |
| 32 | 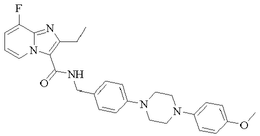 |
| 33 | 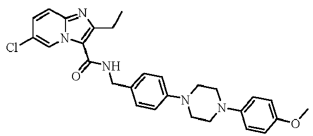 |
| 34 | 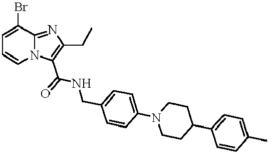 |
| 35 | 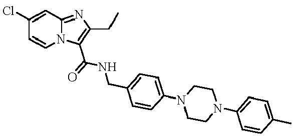 |
| 36 | 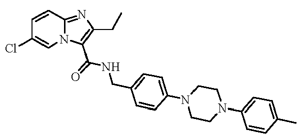 |
| 37 | 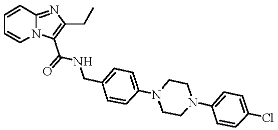 |
| 38 | 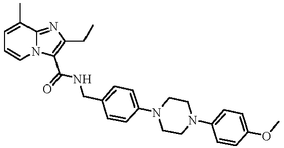 |
| 39 | 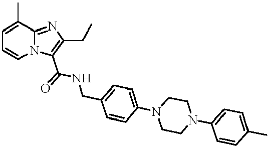 |
| 40 | 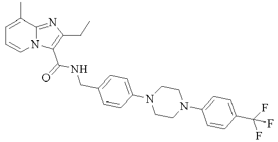 |
| 41 | 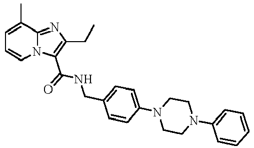 |
| 42 | 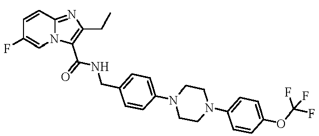 |
| 43 | 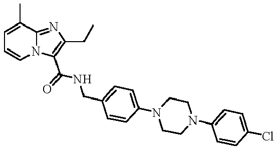 |
| 44 | 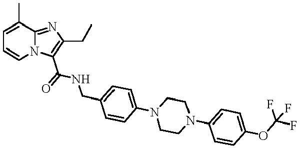 |
| 45 | 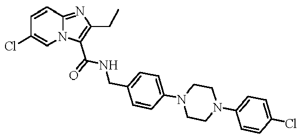 |
| 46 | 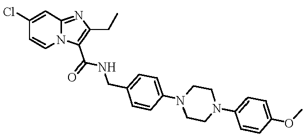 |
| 47 | 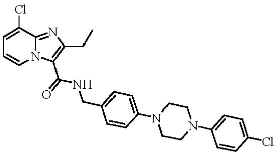 |
| 48 | 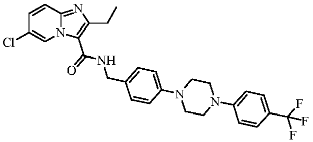 |
| 49 | 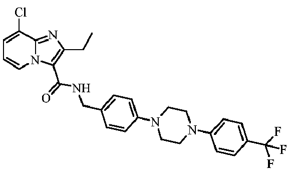 |
| 50 | 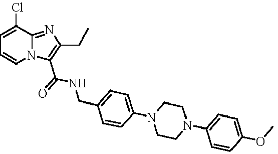 |
| 51 | 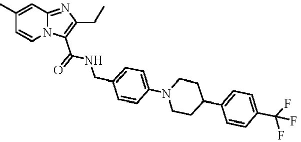 |
| 52 | 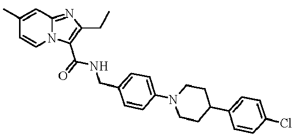 |
| 53 | 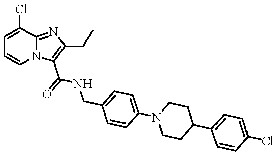 |
| 54 | 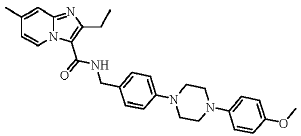 |
| 55 | 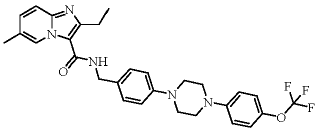 |
| 56 | 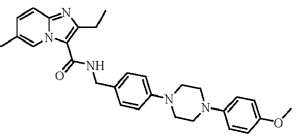 |
| 57 | 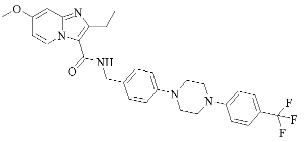 |
| 58 | 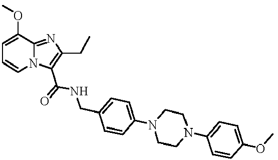 |
| 59 | 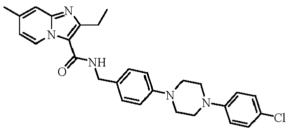 |
| 60 | 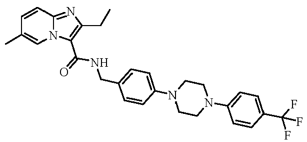 |
| 61 | 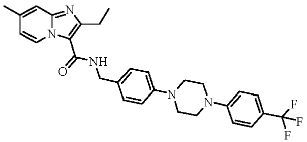 |
| 62 | 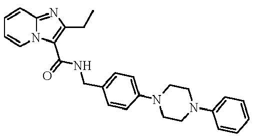 |
| 63 | 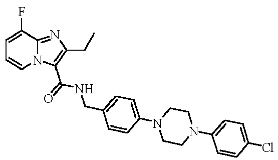 |
| 64 | 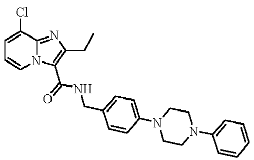 |
| 65 | 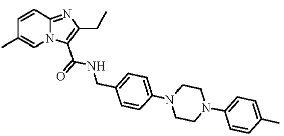 |
| 66 | 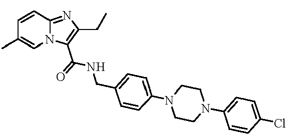 |
| 67 | 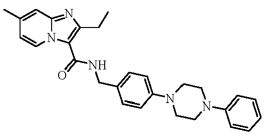 |
| 68 | 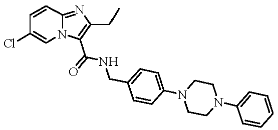 |
| 69 | 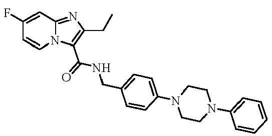 |
| 70 | 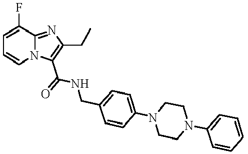 |
| 71 | 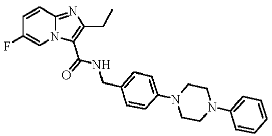 |
| 72 | 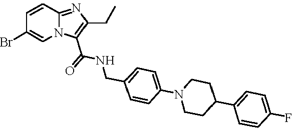 |
| 73 | 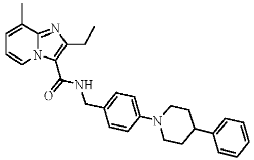 |
| 74 | 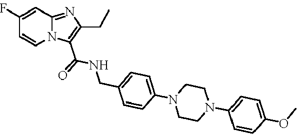 |
| 75 | 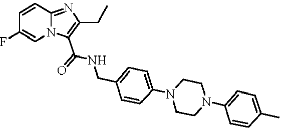 |
| 76 | 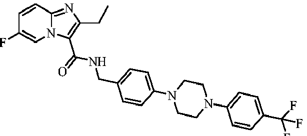 r |
| 77 | 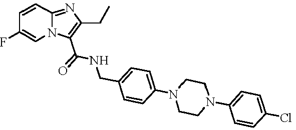 |
| 78 | 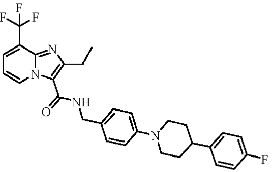 |
| 79 | 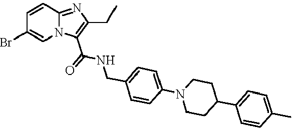 |
| 80 | 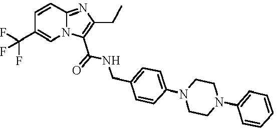 |
| 81 | 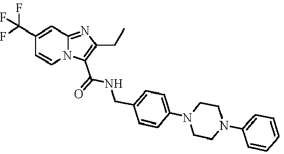 |
| 82 | 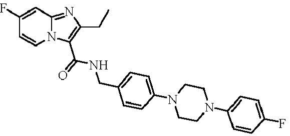 |
| 83 | 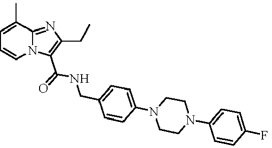 |
| 84 | 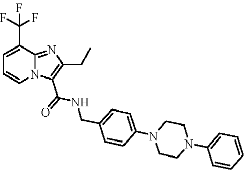 |
| 85 | 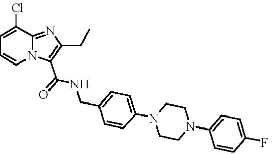 |
| 86 | 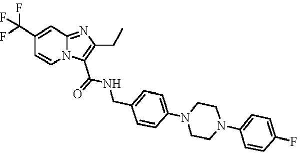 |
| 87 | 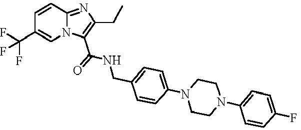 |
| 88 | 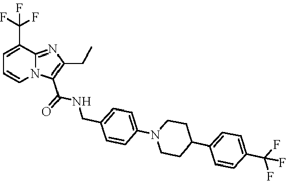 |
| 89 | 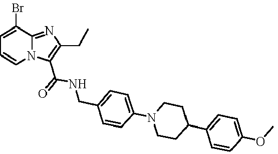 |
| 90 | 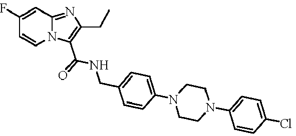 |
| 91 | 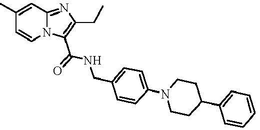 |
| 92 | 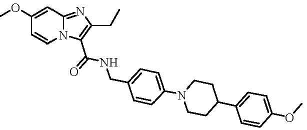 |
| 93 | 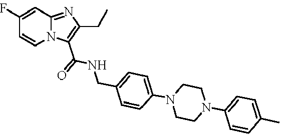 |
| 94 | 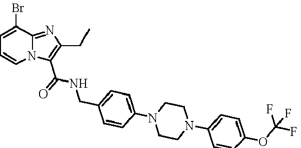 |
| 95 | 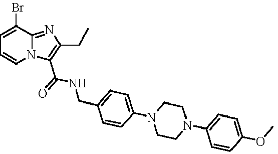 |
| 96 | 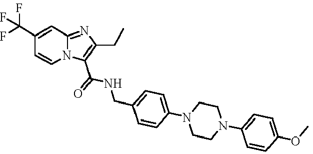 |
| 97 | 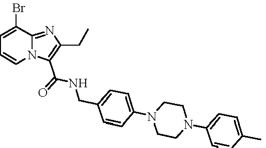 |
| 98 | 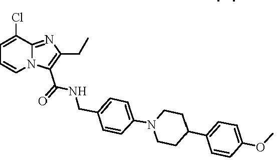 |
| 99 | 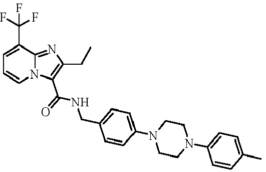 |
| 100 | 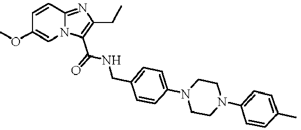 |
| 101 | 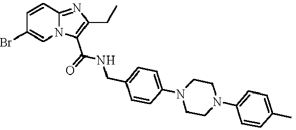 |
| 102 | 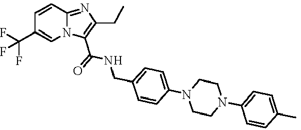 |
| 103 | 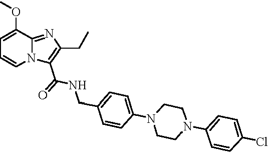 |
| 104 | 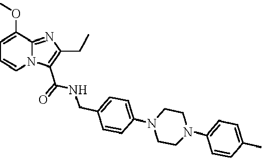 |
| 105 | 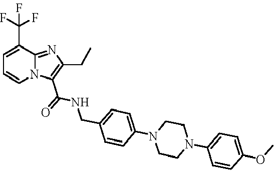 |
| 106 | 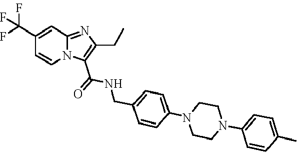 |
| 107 | 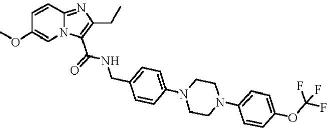 |
| 108 | 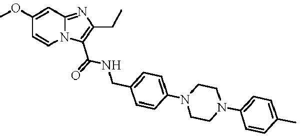 |
| 109 | 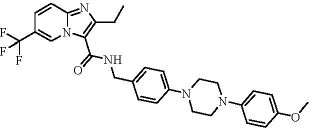 |
| 110 | 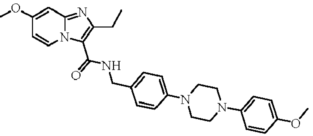 |
| 111 | 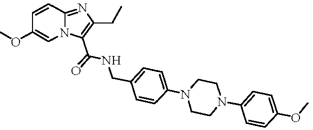 |
| 112 | 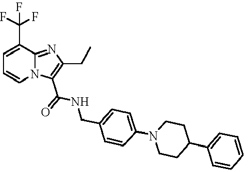 |
| 113 | 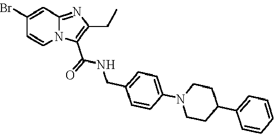 |
| 114 | 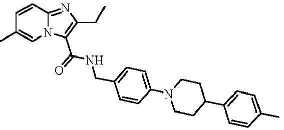 |
| 115 | 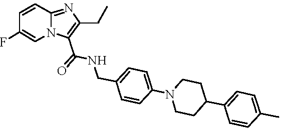 |
| 116 | 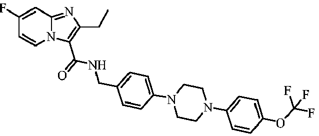 |
| 117 | 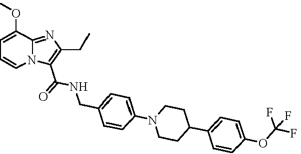 |
| 118 | 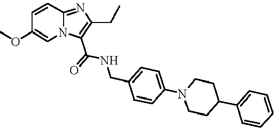 |
| 119 | 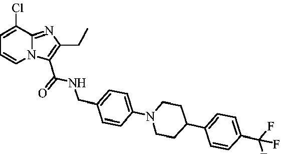 |
| 120 | 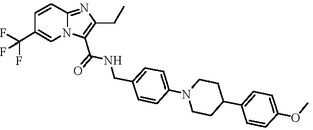 |
| 121 | 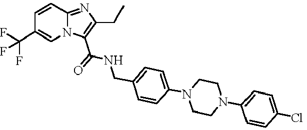 |
| 122 | 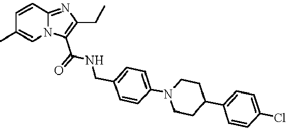 |
| 123 | 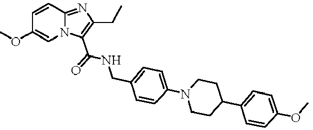 |
| 124 | 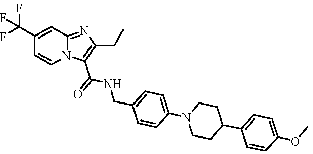 |
| 125 | 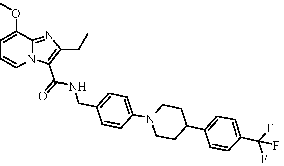 |
| 126 | 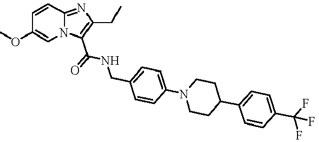 |
| 127 | 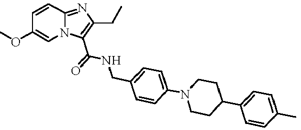 |
| 128 | 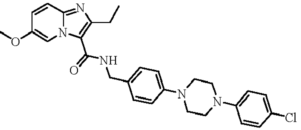 |
| 129 | 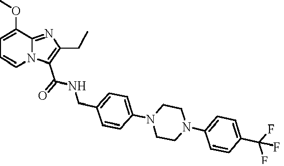 |
| 130 | 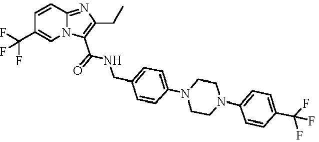 |
| 131 | 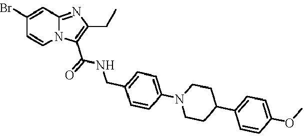 |
| 132 | 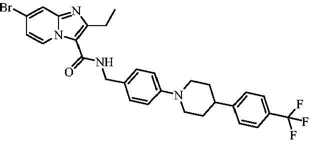 |
| 133 | 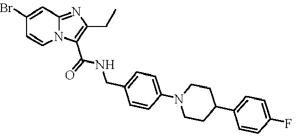 |
| 134 | 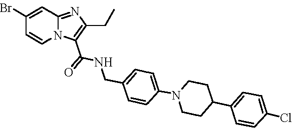 |
| 135 | 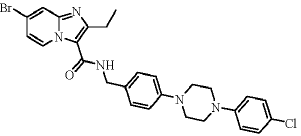 |
| 136 | 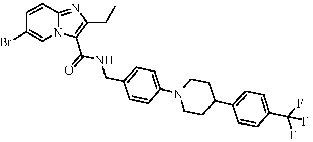 |
| 137 | 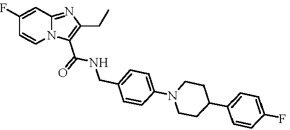 |
| 138 | 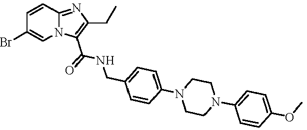 |
| 139 | 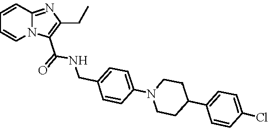 |
| 140 | 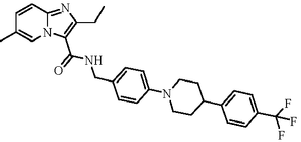 |
| 141 | 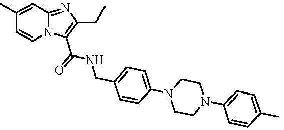 |
| 142 | 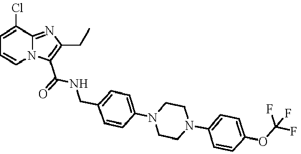 |
| 143 | 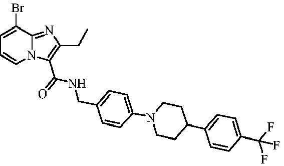 |
| 144 | 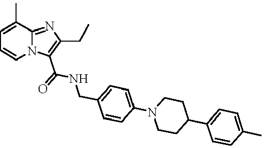 |
| 145 | 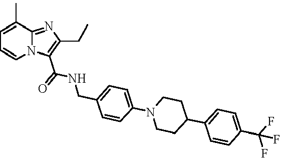 |
| 146 | 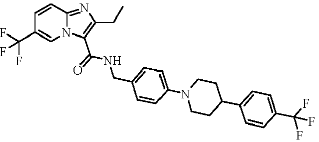 |
| 147 | 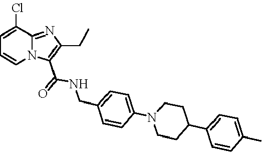 |
| 148 | 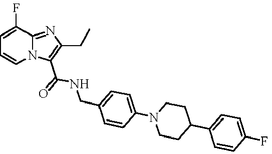 |
| 149 | 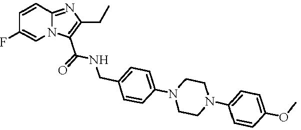 |
| 150 | 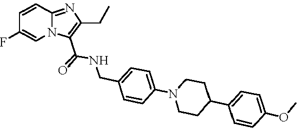 |
| 151 | 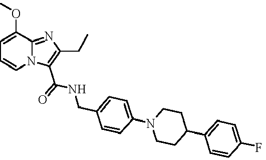 |
| 152 | 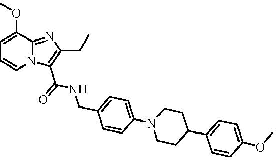 |
| 153 | 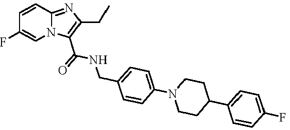 |
| 154 | 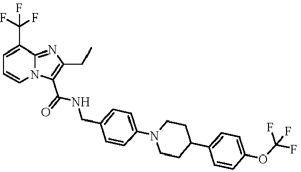 |
| 155 | 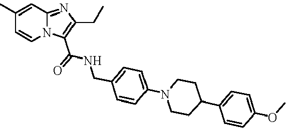 |
| 156 | 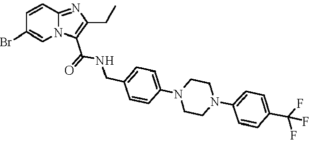 |
| 157 | 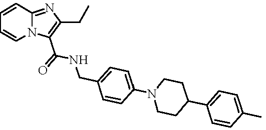 |
| 158 | 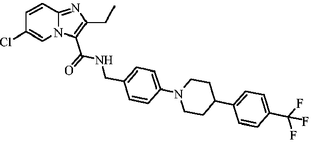 |
| 159 | 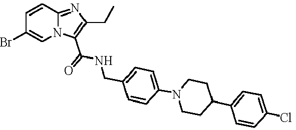 |
| 160 | 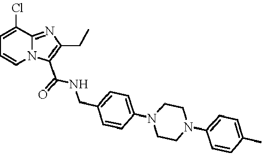 |
| 161 | 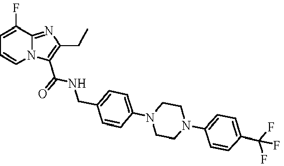 |
| 162 | 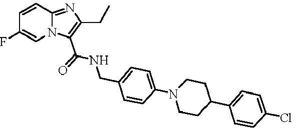 |
| 163 | 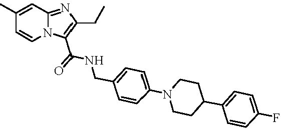 |
| 164 | 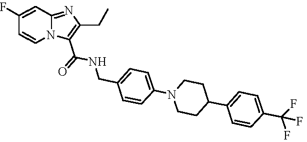 |
| 165 | 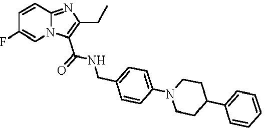 |
| 166 | 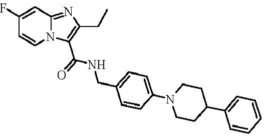 |
| 167 | 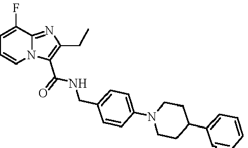 |
| 168 | 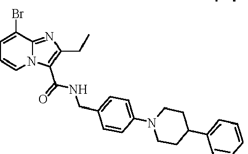 |
| 169 | 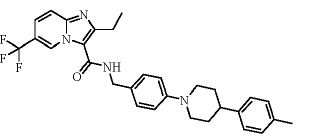 |
| 170 | 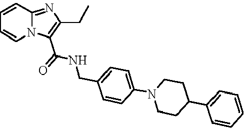 |
| 171 | 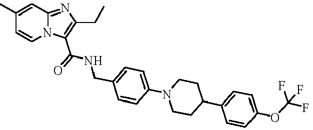 |
| 172 | 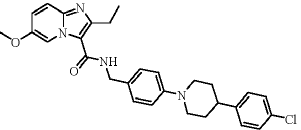 |
| 173 | 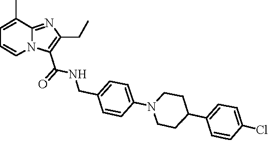 |
| 174 | 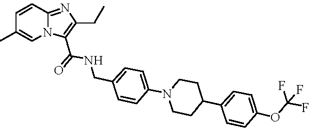 |
| 175 | 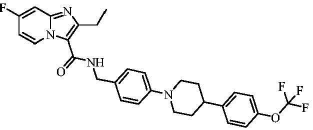 |
| 176 | 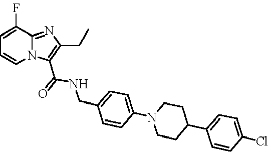 |
| 177 | 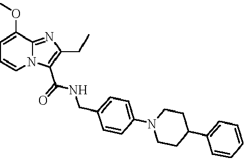 |
| 178 | 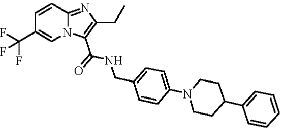 |
| 179 | 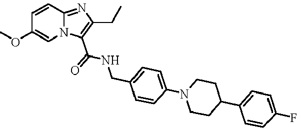 |
| 180 | 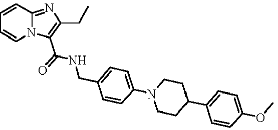 |
| 181 | 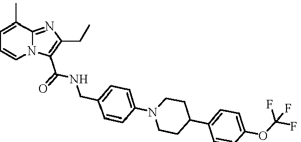 |
| 182 | 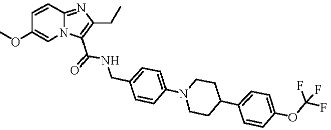 |
| 183 | 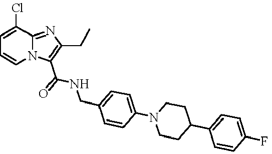 |
| 184 | 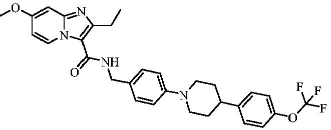 |
| 185 | 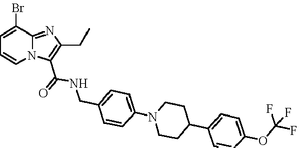 |
| 186 | 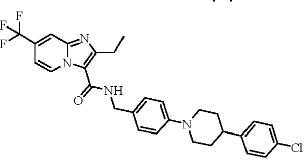 |
| 187 | 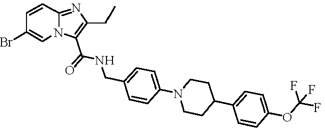 |
| 188 | 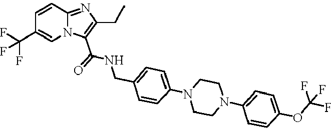 |
| 189 | 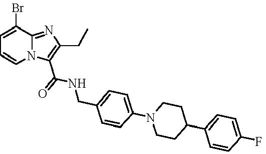 |
| 190 | 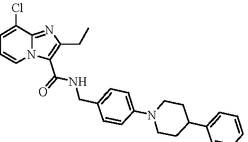 |
| 191 | 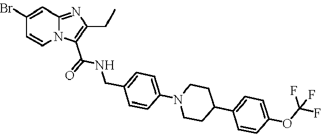 |
| 192 | 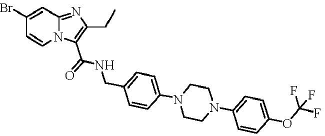 |
| 193 | 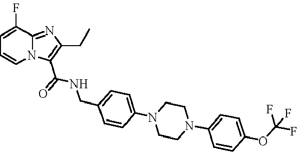 |
| 194 | 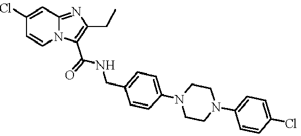 |
| 195 | 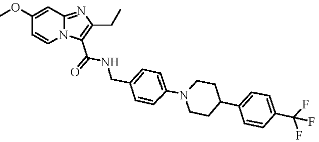 |
| 196 | 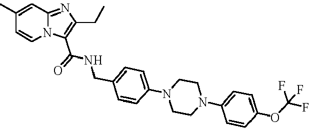 |
| 197 | 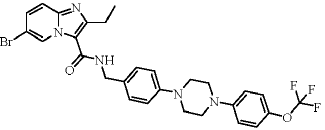 |
| 198 | 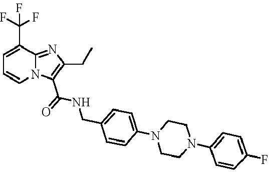 |
| 199 | 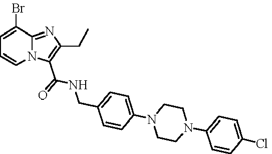 |
| 200 | 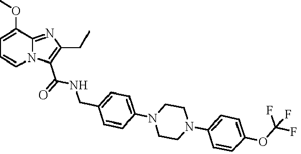 |
| 201 | 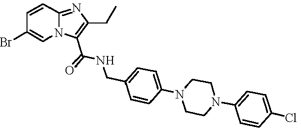 |
| 202 | 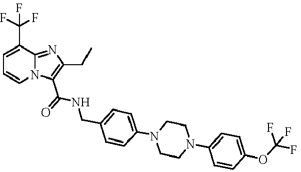 |
| 203 | 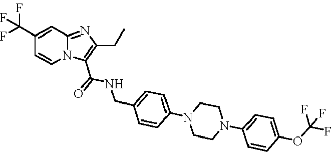 |
| 204 | 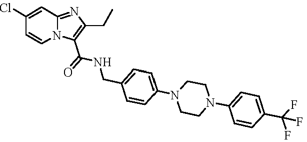 |
| 205 | 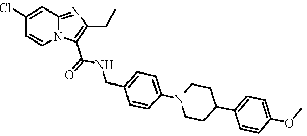 |
| 206 | 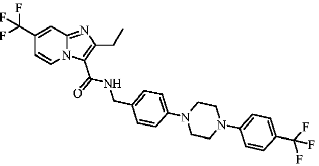 |
| 207 | 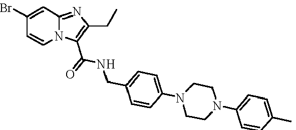 |
| 208 | 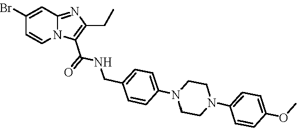 |
| 209 | 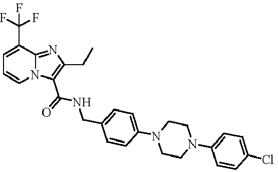 |
| 210 | 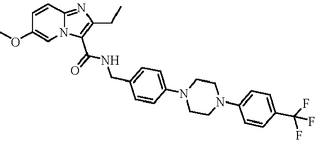 |
| 211 | 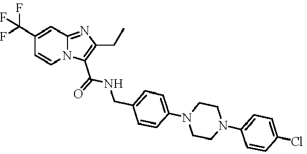 |
| 212 | 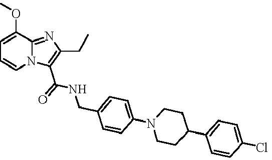 |
| 213 | 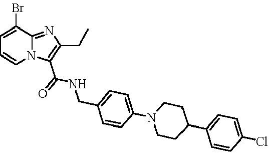 |
| 214 | 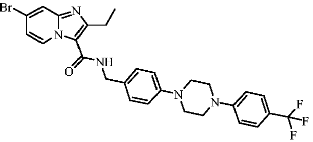 |
| 215 | 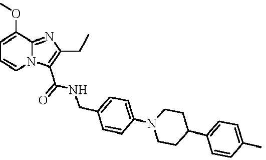 |
| 216 | 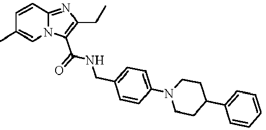 |
| 217 | 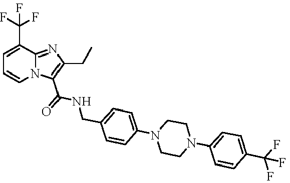 |
| 218 | 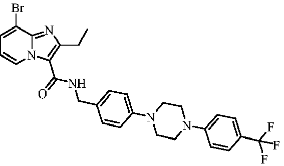 |
| 219 | 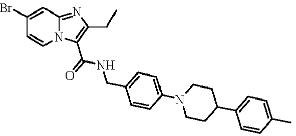 |
| 220 | 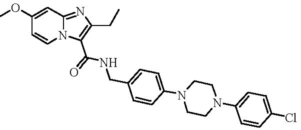 |
| 221 | 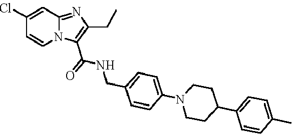 |
| 222 | 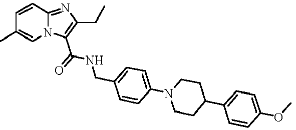 |
| 223 | 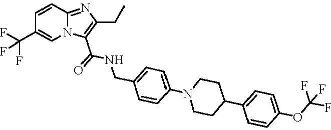 |
| 224 | 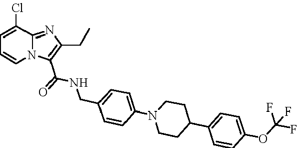 |
| 225 | 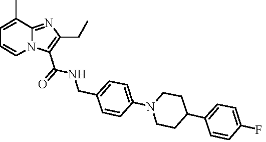 |
| 226 | 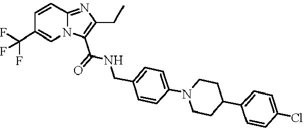 |
| 227 | 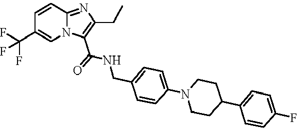 |
| 228 | 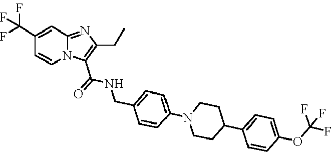 |
| 229 | 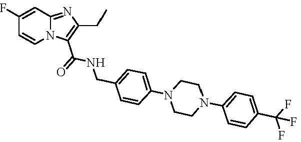 |
| 230 | 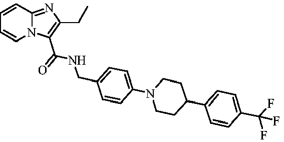 |
| 231 | 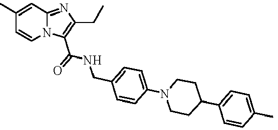 |
| 238 | 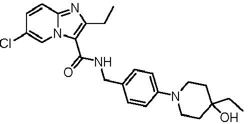 |
| 281 | 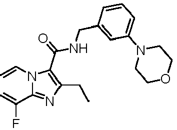 |
| 282 | 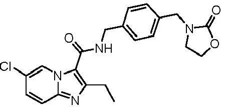 |
| 283 | 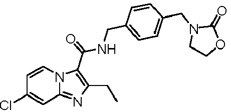 |
| 284 | 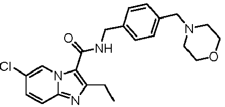 |
| 285 | 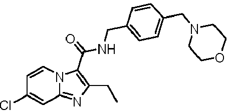 |
| 289 | 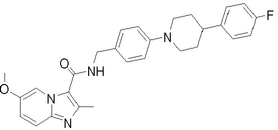 |
| 290 | 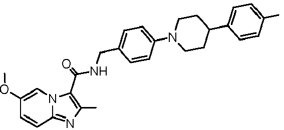 |
| 291 | 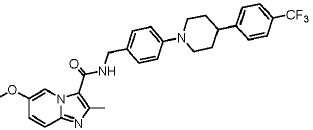 |
| 292 | 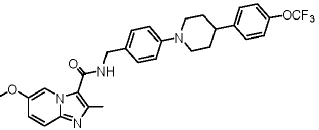 |
| 293 | 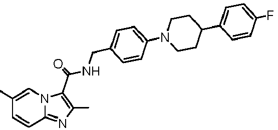 |
| 294 | 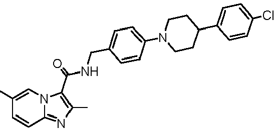 |
| 295 | 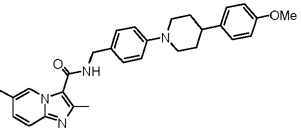 |
| 296 | 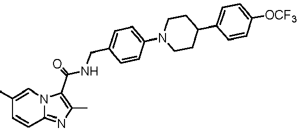 |
| 297 | 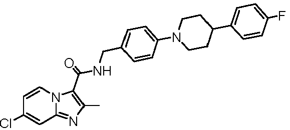 |
| 298 | 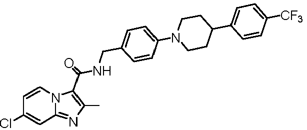 |
| 299 | 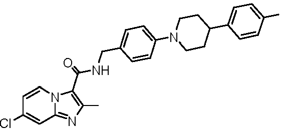 |
| 300 | 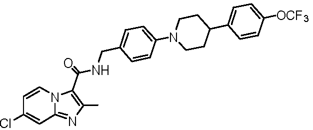 |
| 309 | 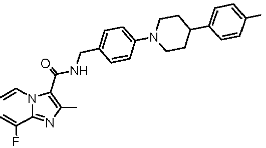 |
| 310 | 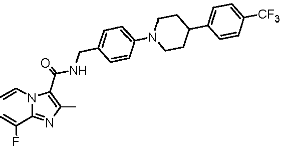 |
| 311 | 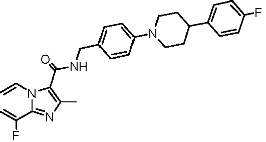 |
| 312 | 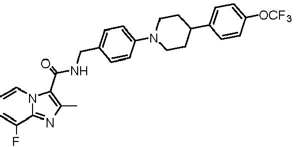 |
| 313 | 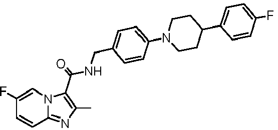 |
| 314 | 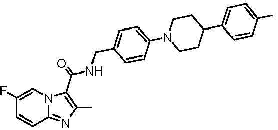 |
| 315 | 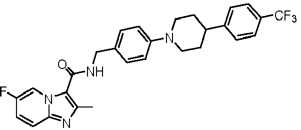 |
| 316 | 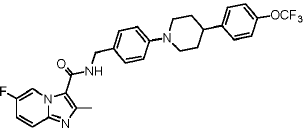 |
| 325 | 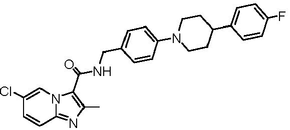 |
| 326 | 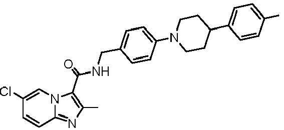 |
| 327 | 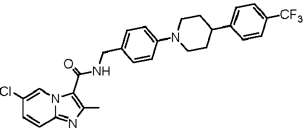 |
| 328 | 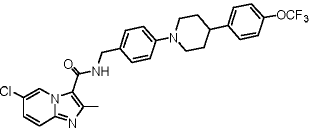 |
| 333 | 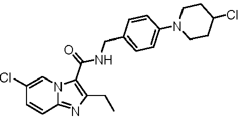 |
| 334 | 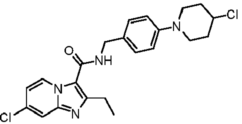 |
| 335 | 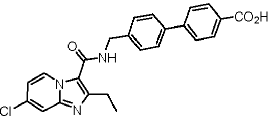 |
| 336 | 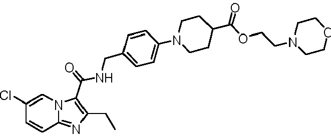 |
| 337 | 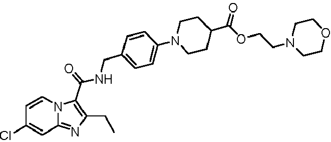 |
| 340 | 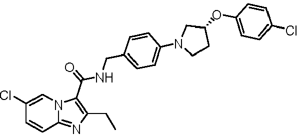 |
| 341 | 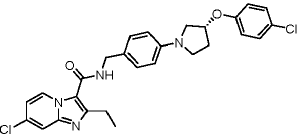 |
| 342 | 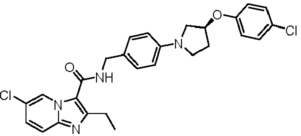 |
| 343 | 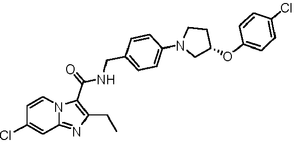 |
| 344 | 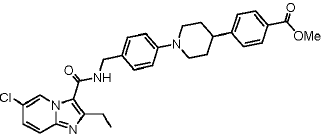 |
| 345 | 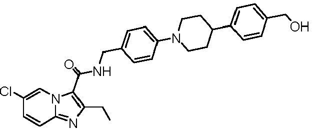 |
| 346 | 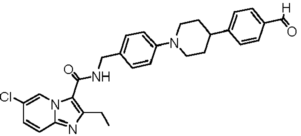 |
| 347 | 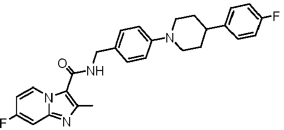 |
| 348 | 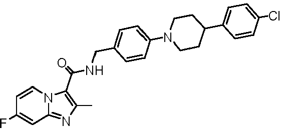 |
| 349 | 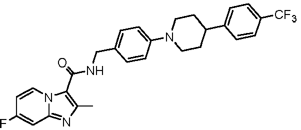 |
| 350 | 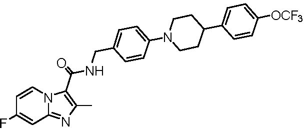 |
bevorzugt eine der Formeln 1-21, 23-24, 26, 28-33, 35-57, 59-77, 79-83, 85-87, 90-98, 100-102, 106-111, 113-116 118-124, 126-128, 130-142, 144-150, 153, 155-167, 169-184, 186-188, 190-197, 199, 201, 203-208, 210-211, 213-214, 216, 218-231, 238, 281-285, 289-300, 309-316, 325-328, 333-337, 340-350, wie oben gezeigt.

2. Verbindung nach Anspruch 1, mit einer der Formeln 55, 171, 175 und 325, wie nachfolgend gezeigt:
| | |
|---|---|
| 55 | 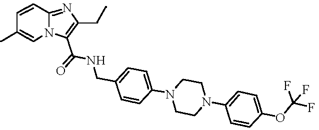 |
| 171 | 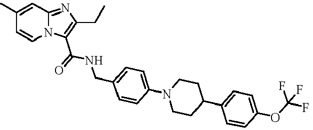 |
| 175 | 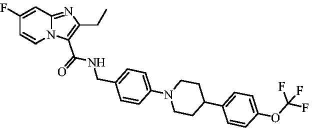 |
| 325 | 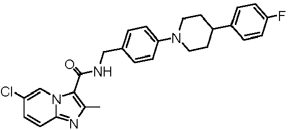 |
und pharmazeutisch akzeptable Salze davon.

3. Verbindung nach einem der Ansprüche 1 - 2, zur Verwendung bei der Behandlung einer bakteriellen Infektion, wobei, bevorzugt, die bakterielle Infektion Tuberkulose ist.

4. Pharmazeutische Zusammensetzung, umfassend eine Verbindung nach einem der Ansprüche 1 - 3, und einen pharmazeutisch akzeptablen Träger.

## Revendications

1. Composé ayant la formule générale I : 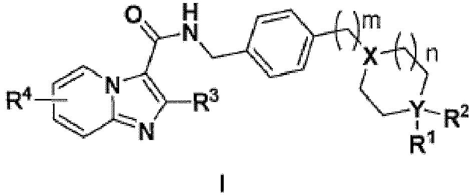 dans laquelle
X est CH ou N ;
Y est CH, O ou N ;
m est 0 ou 1 ;
n est 0 ou 1 ;
R¹ est, à chaque occurrence, sélectionné indépendamment dans le groupe consistant en un hydrogène, un halogène, un méthyle, un éthyle, un t-butyle, un phényle, -NC(O)R⁵, -OR⁵, -C(O)R⁵, -C(O)OR⁵ ;
R² est, à chaque occurrence, sélectionné indépendamment dans le groupe consistant en un hydrogène et un hydroxyle ;
R³ est, à chaque occurrence, sélectionné indépendamment dans le groupe consistant en un méthyle et un éthyle ;
R⁴ est, à chaque occurrence, sélectionné indépendamment dans le groupe consistant en un hydrogène, un halogène, un méthyle, un -méthoxy et -CF₃ ;
R⁵ est, à chaque occurrence, sélectionné indépendamment dans le groupe consistant en un (alkyle en C₁ à C₃)-hétérocycle, un phényle et un benzyle ;
et les sels pharmaceutiquement acceptables de celui-ci ;
ou
un composé ayant la formule générale VII : 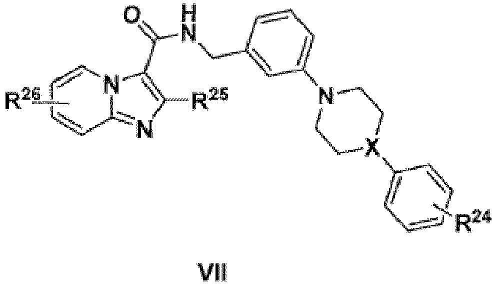 dans laquelle
X est CH ou N
R²⁴ est, à chaque occurrence, sélectionné indépendamment dans le groupe consistant en un hydrogène, un halogène, un alkyle en C₁ à C₂, un -méthoxy, -CF₃ et -OCF₃ ;
R²⁵ est, à chaque occurrence, sélectionné indépendamment dans le groupe consistant en un méthyle et un éthyle ;
R²⁶ est, à chaque occurrence, sélectionné indépendamment dans le groupe consistant en un hydrogène, un halogène, un méthyle, un -méthoxy et -CF₃ ;
et les sels pharmaceutiquement acceptables de celui-ci, ou
un composé ayant la formule générale VIII : 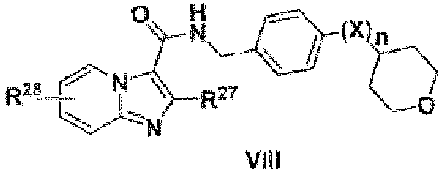 dans laquelle
X est CH₂ ou NH
n est 0 ou 1
R²⁷ est, à chaque occurrence, sélectionné indépendamment dans le groupe consistant en un méthyle et un éthyle ;
R²⁸ est, à chaque occurrence, sélectionné indépendamment dans le groupe consistant en un hydrogène, un halogène, un méthyle, un -méthoxy et -CF₃ ;
et les sels pharmaceutiquement acceptables de celui-ci,
dans lequel le composé présente l'une des formules 1 à 231, 238, 281 à 285, 289 à 300, 309 à 316, 325 à 328, 333 à 337, 340 à 350, telles que représentées dans le tableau suivant : 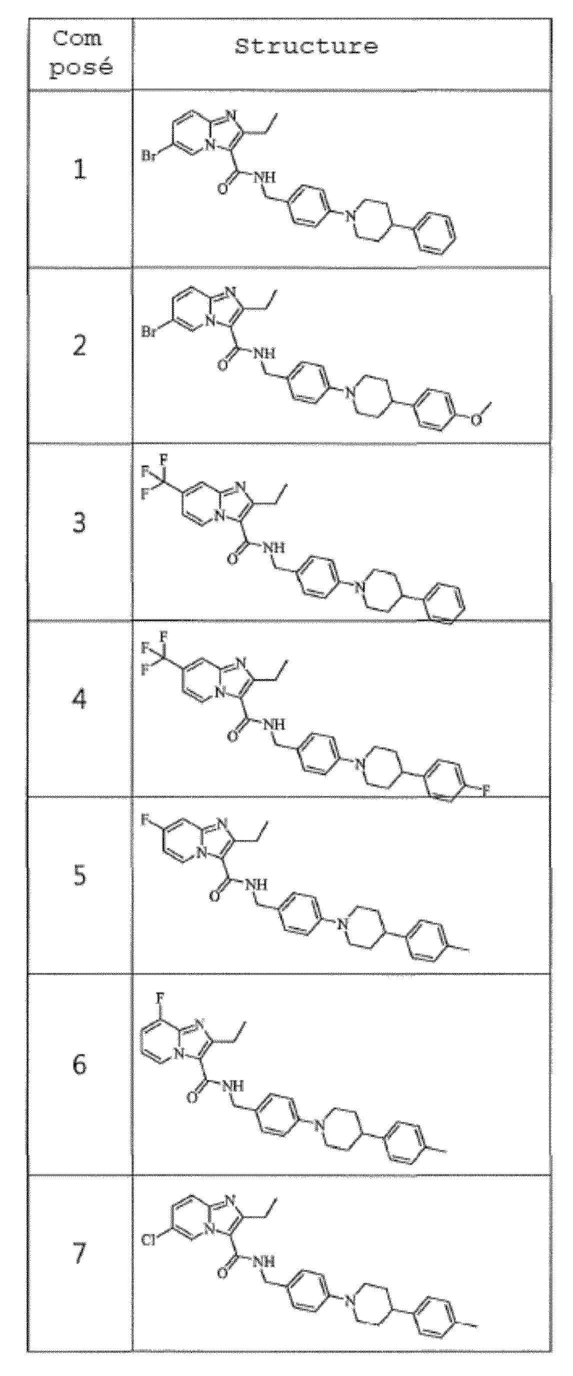 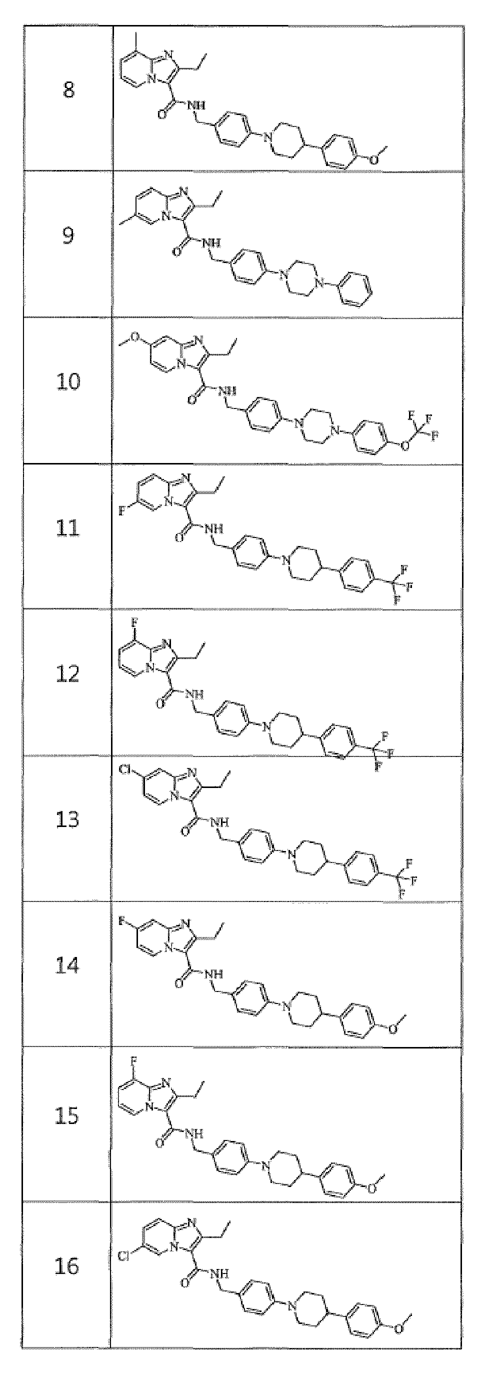 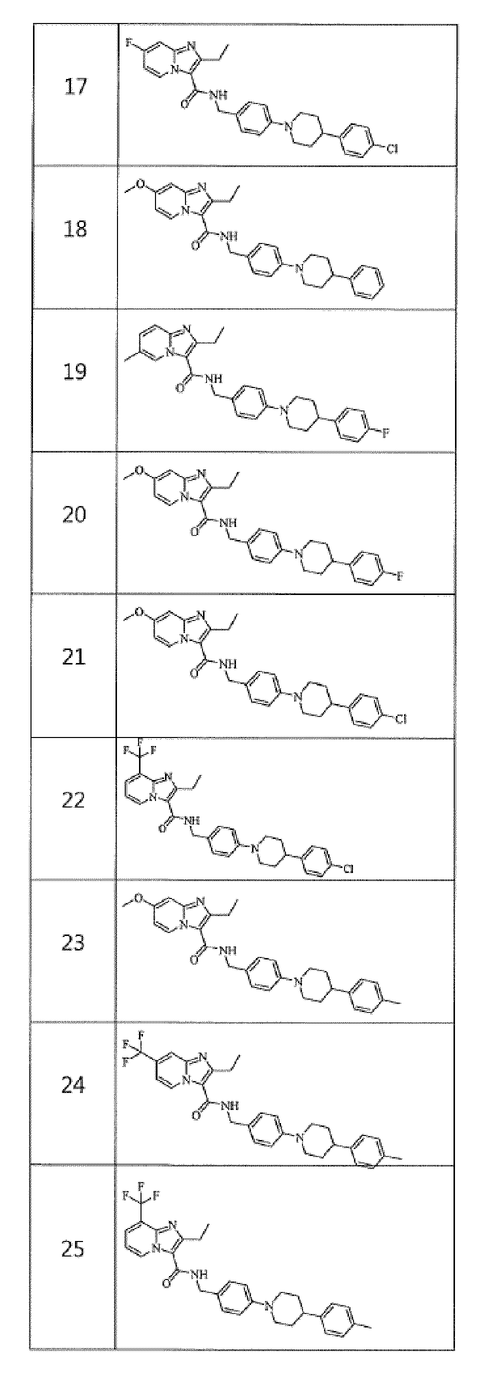 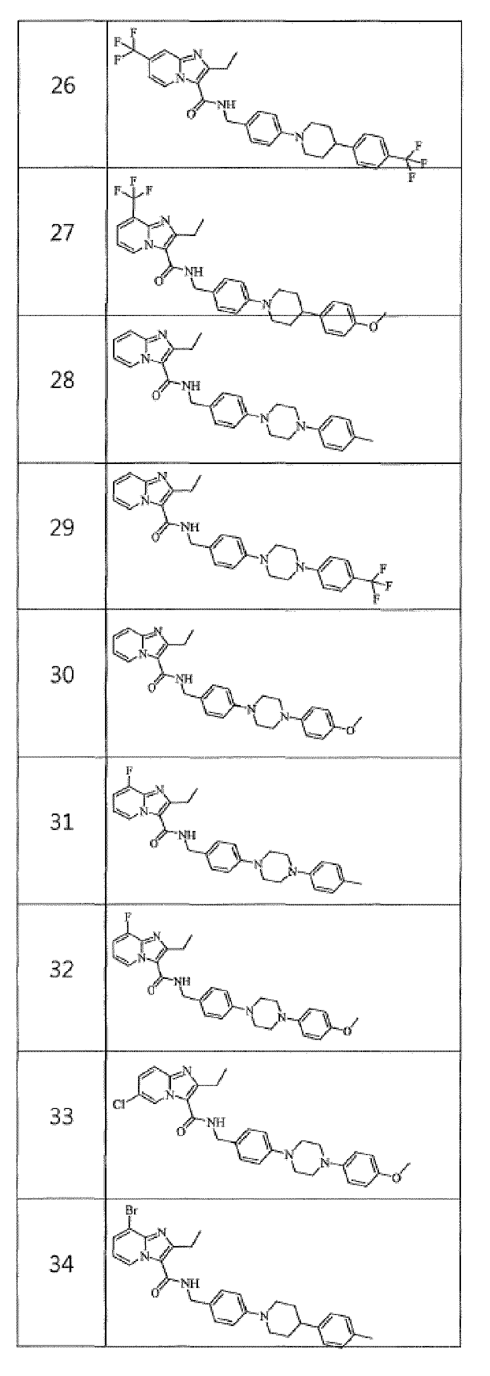 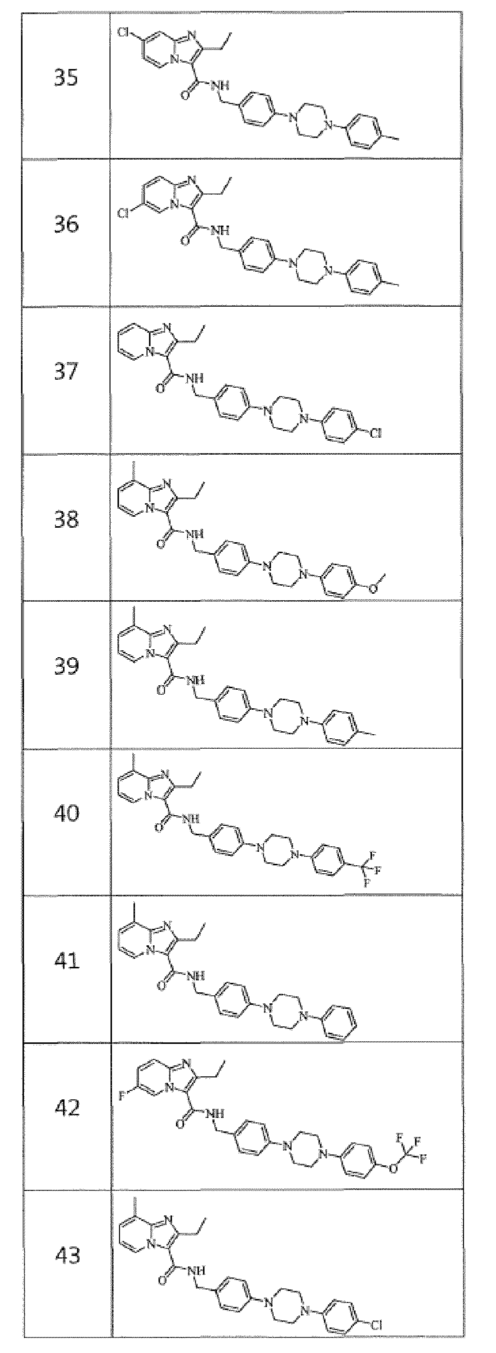 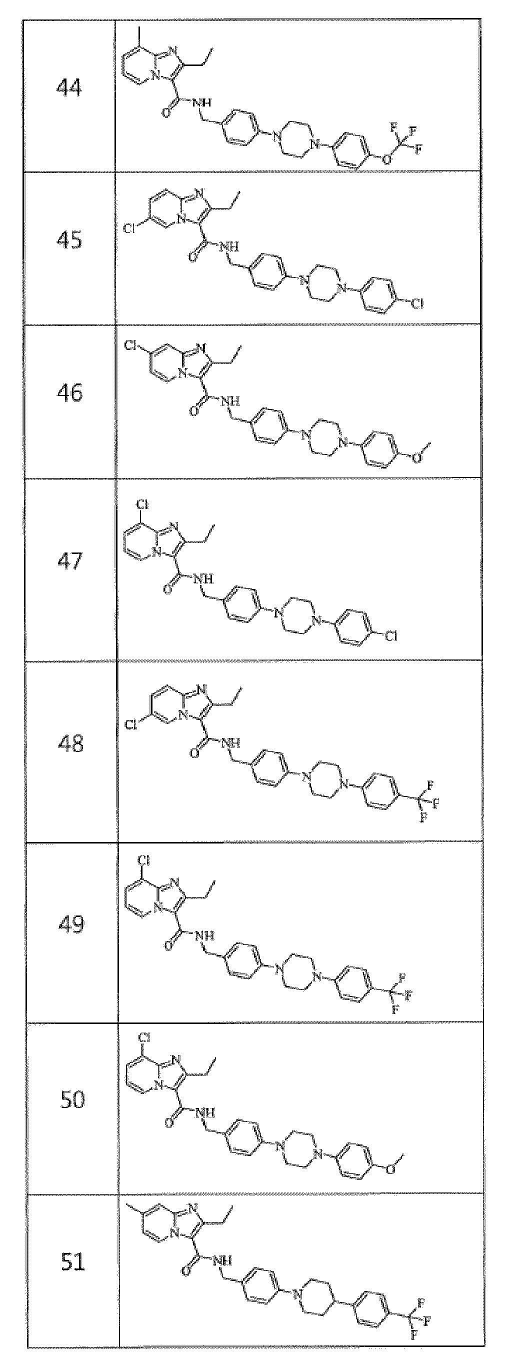 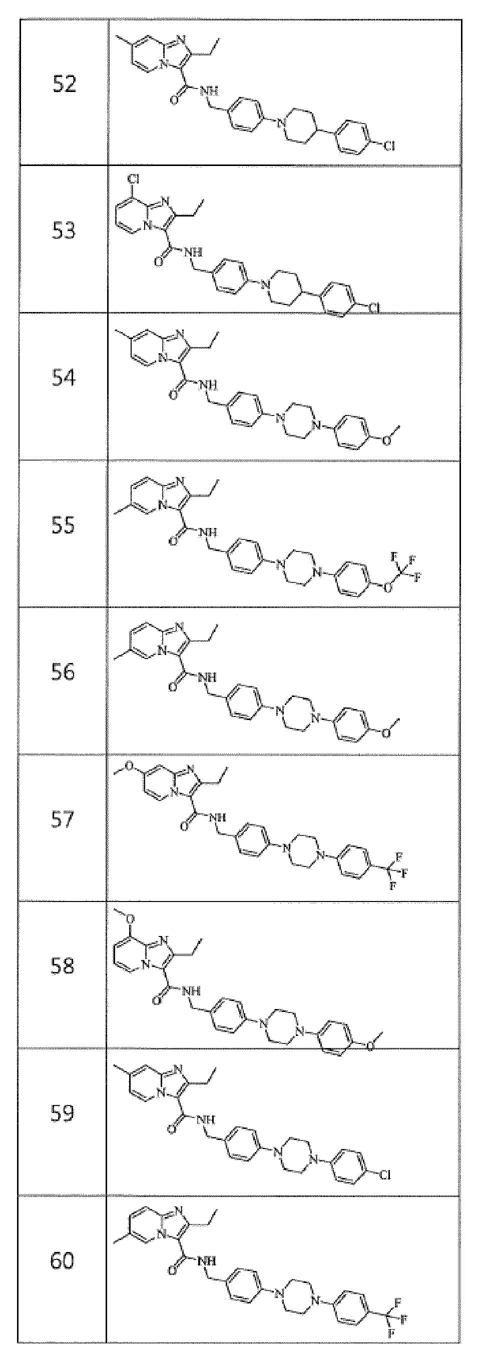 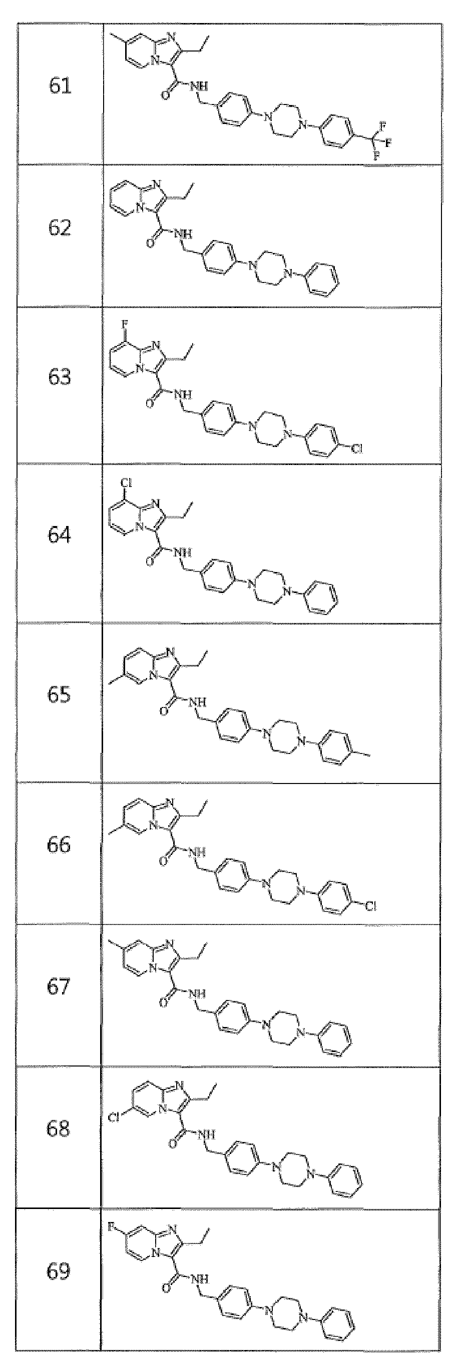 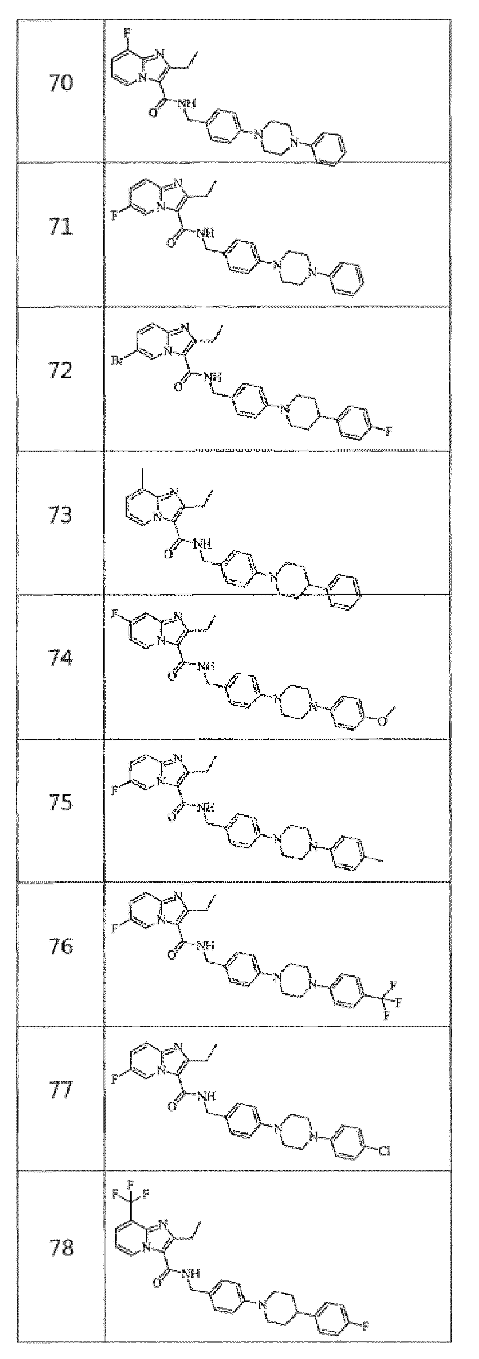 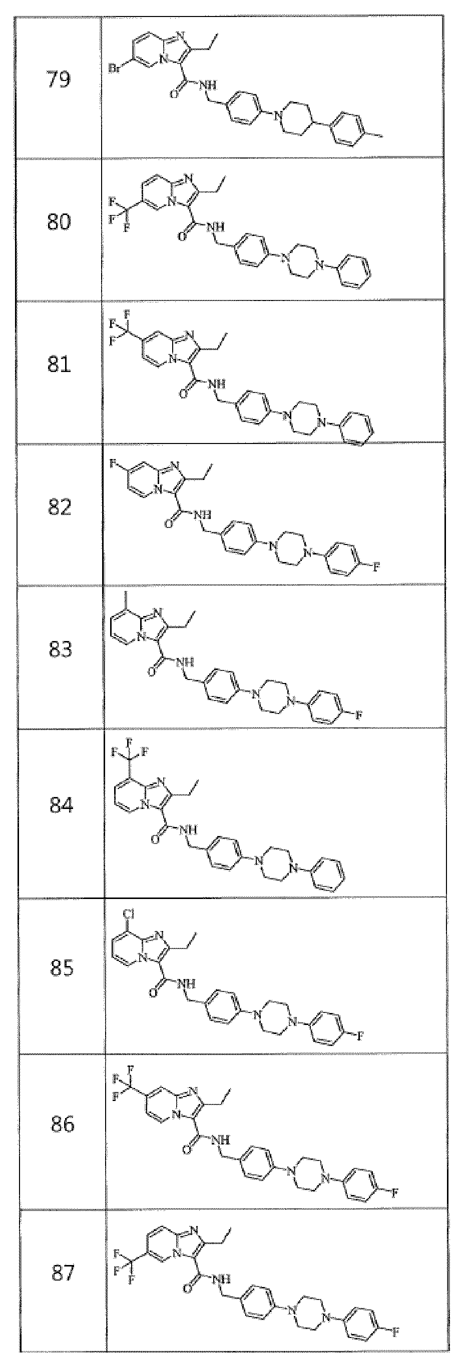 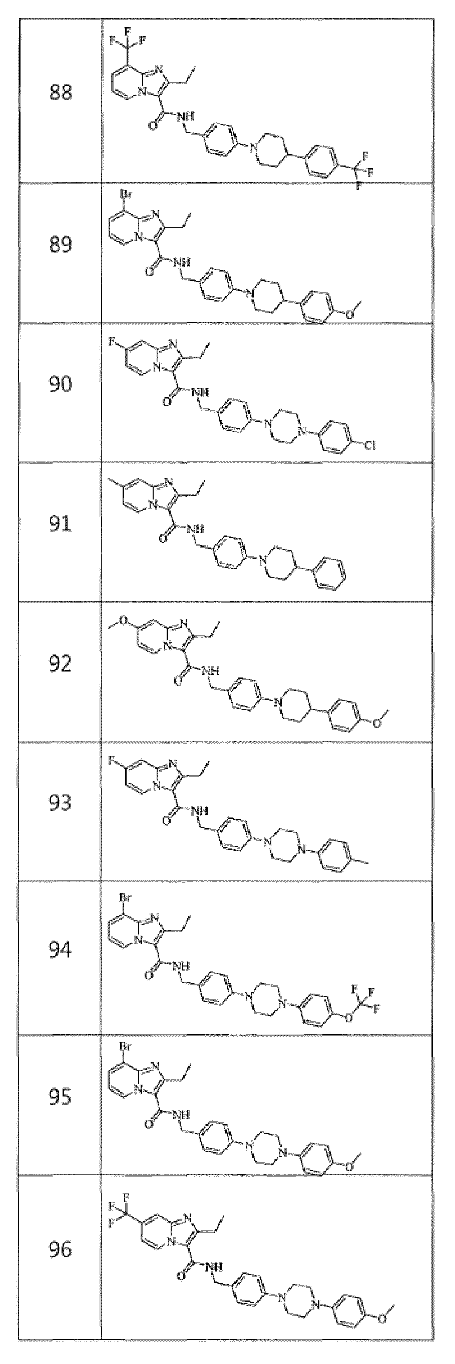 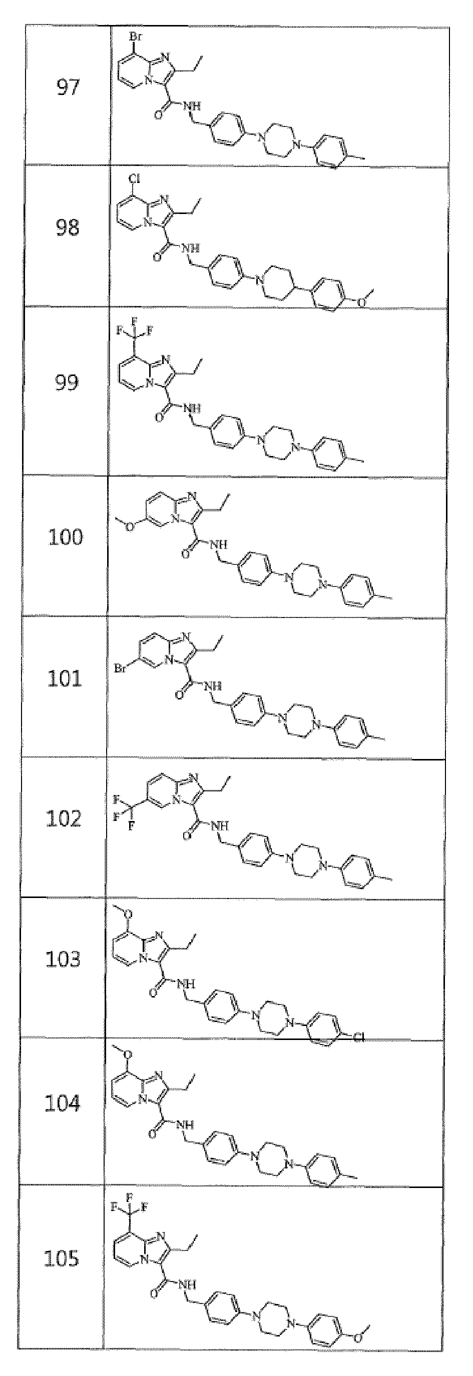 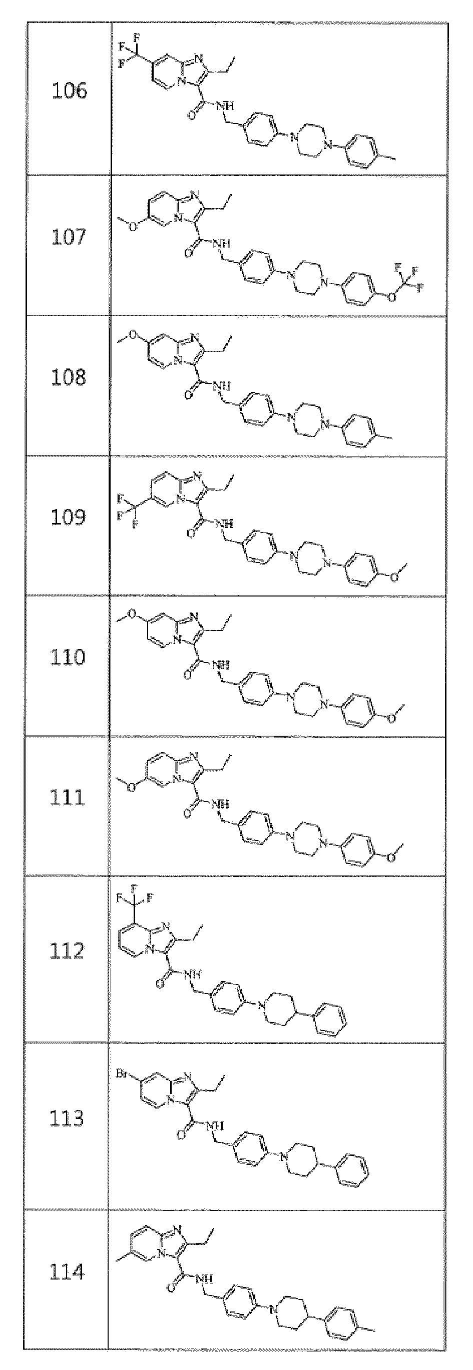 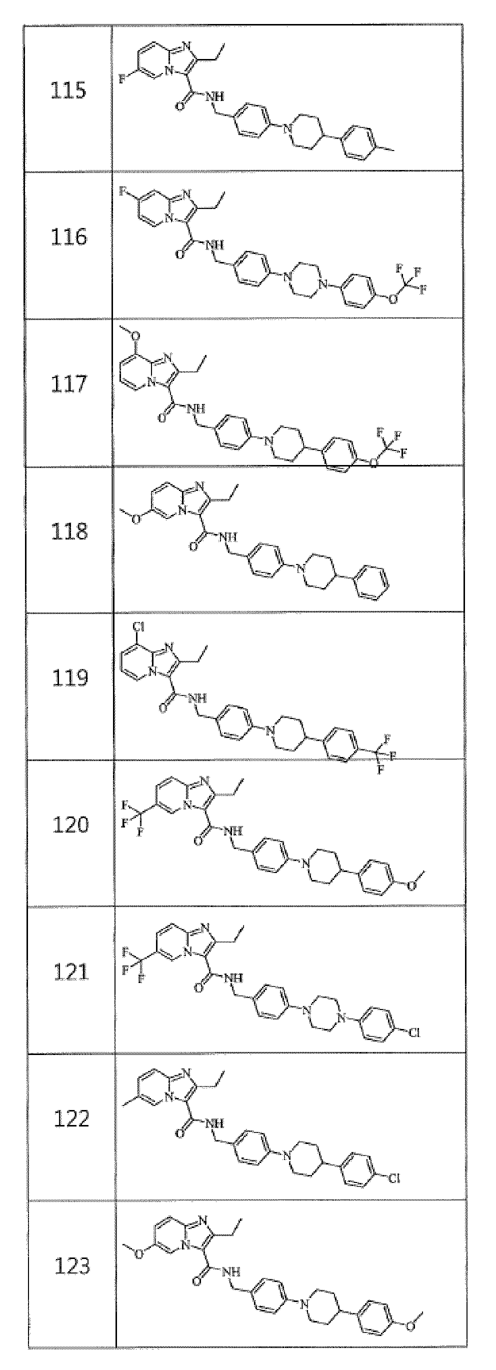 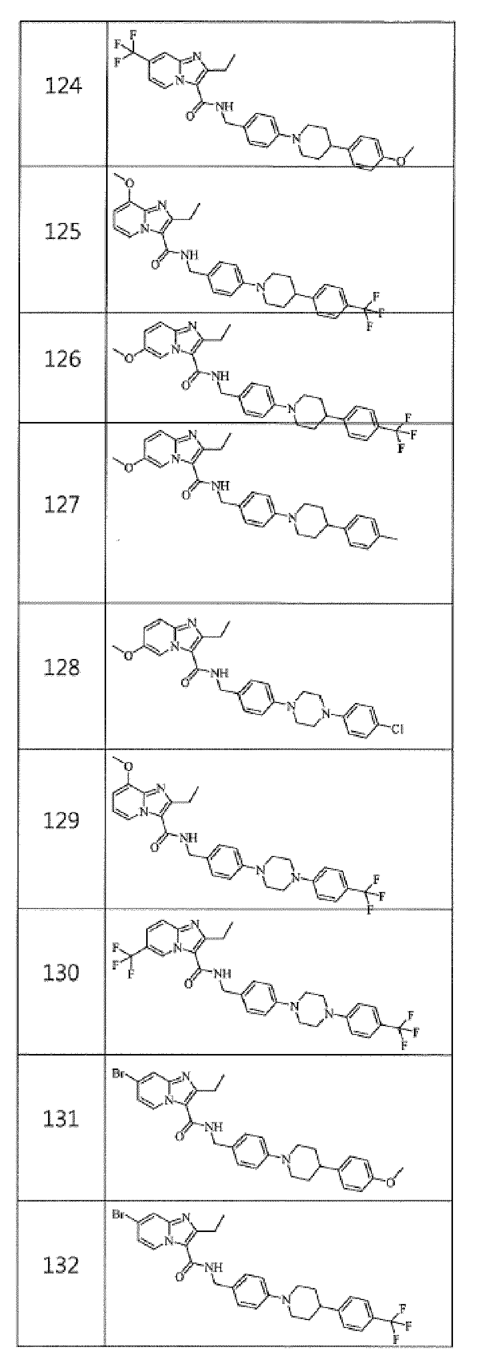 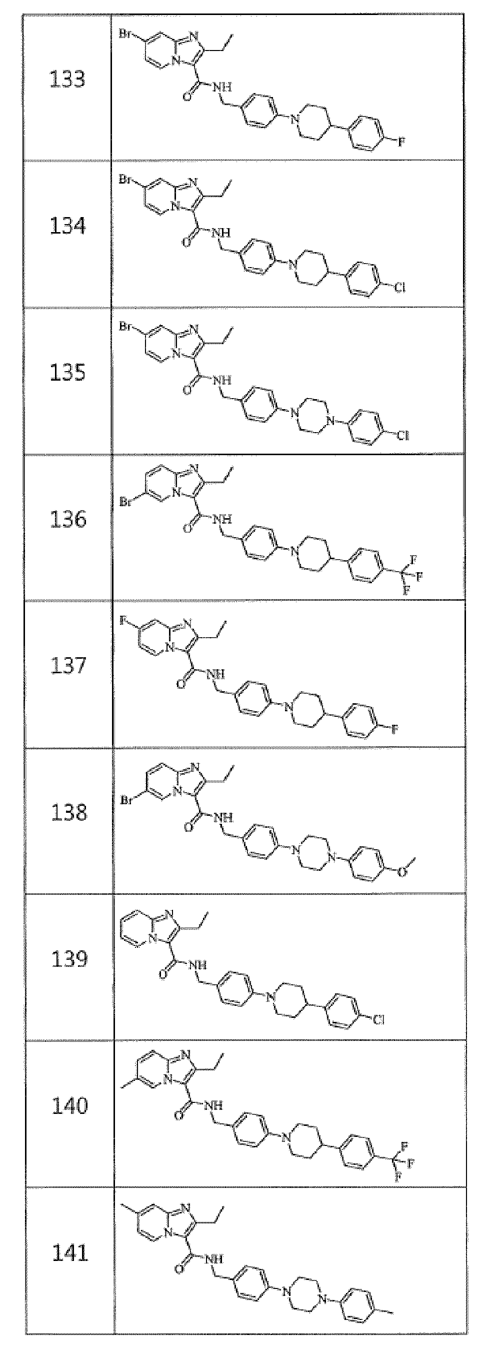 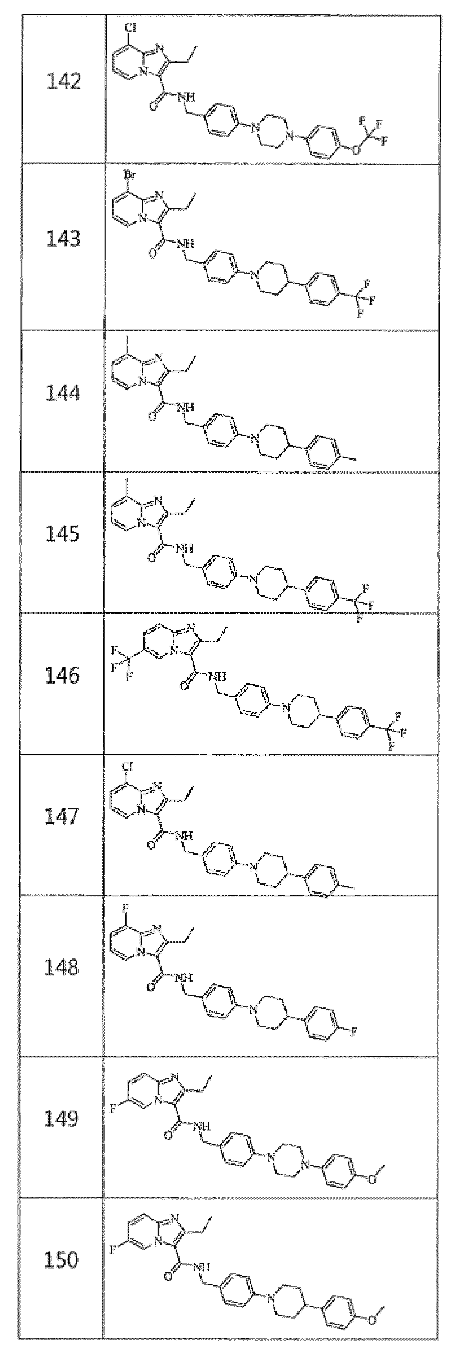 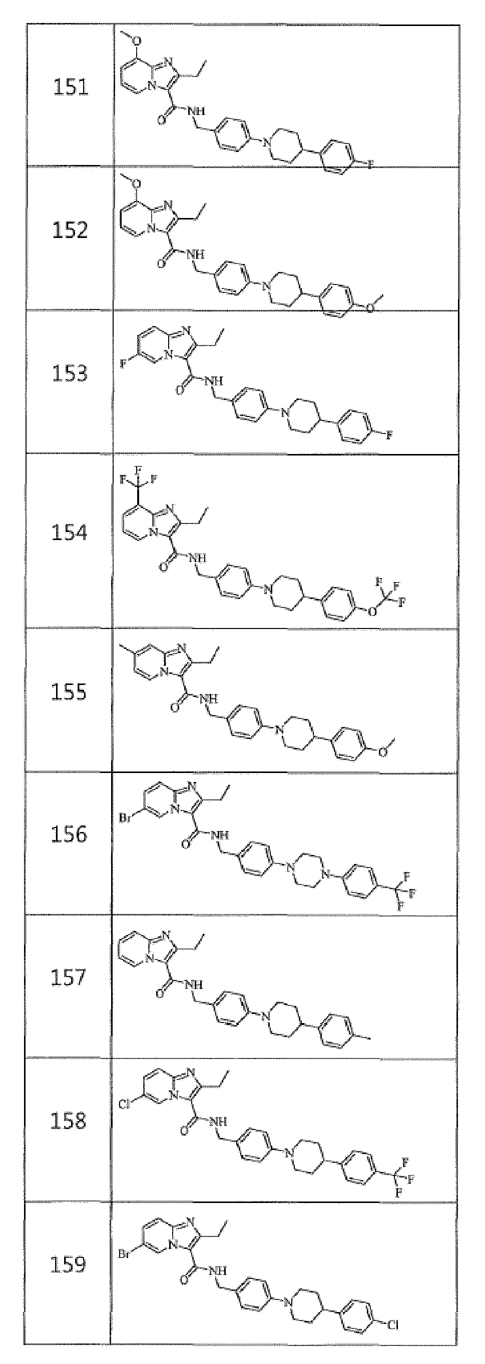 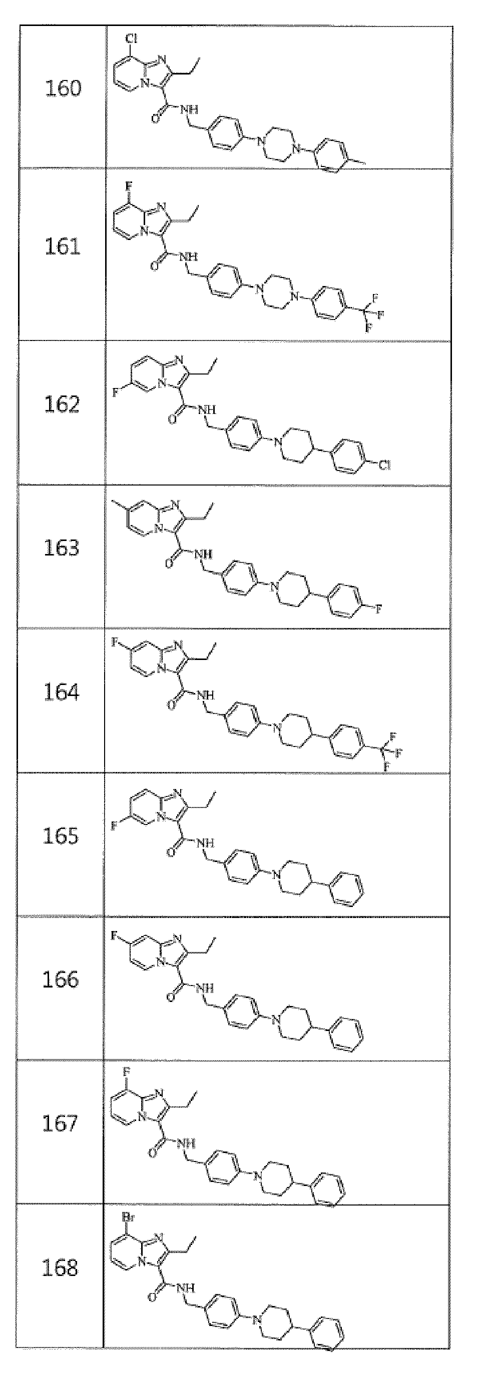 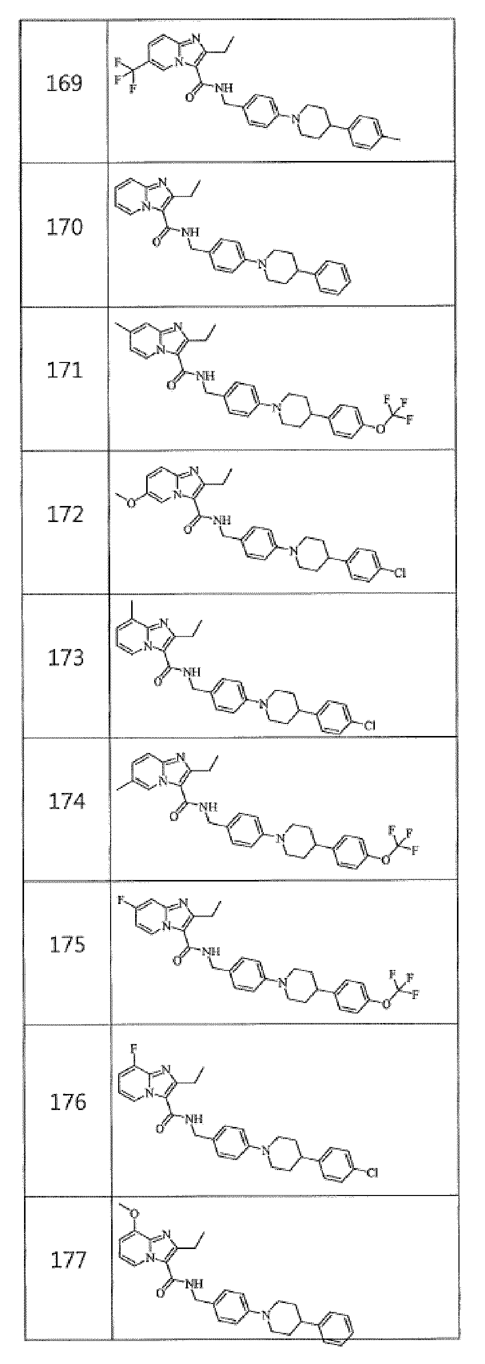 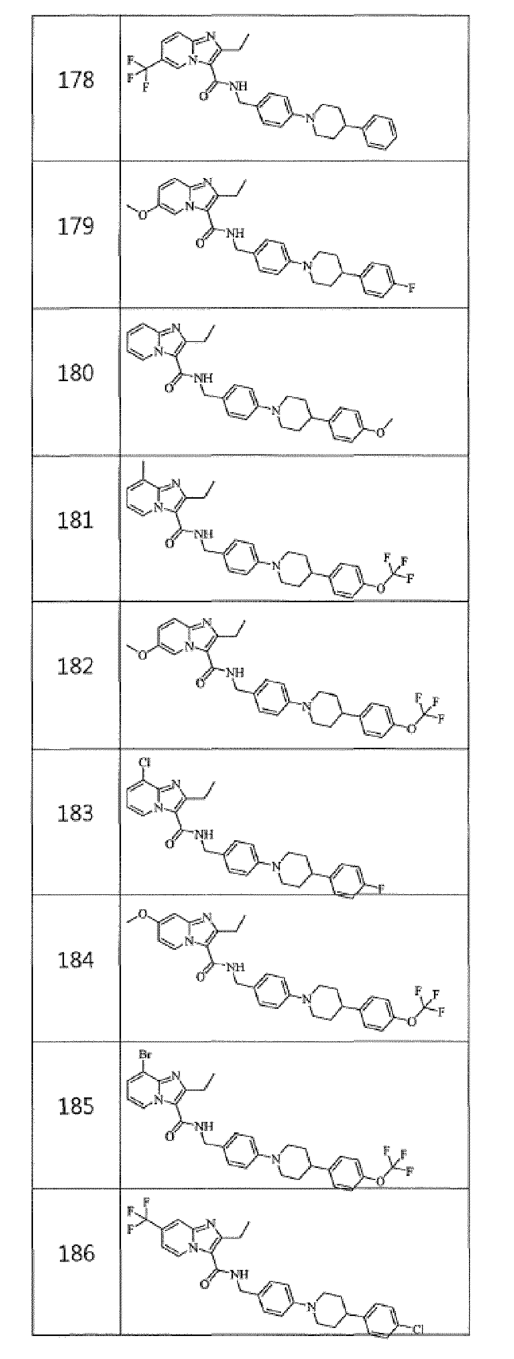 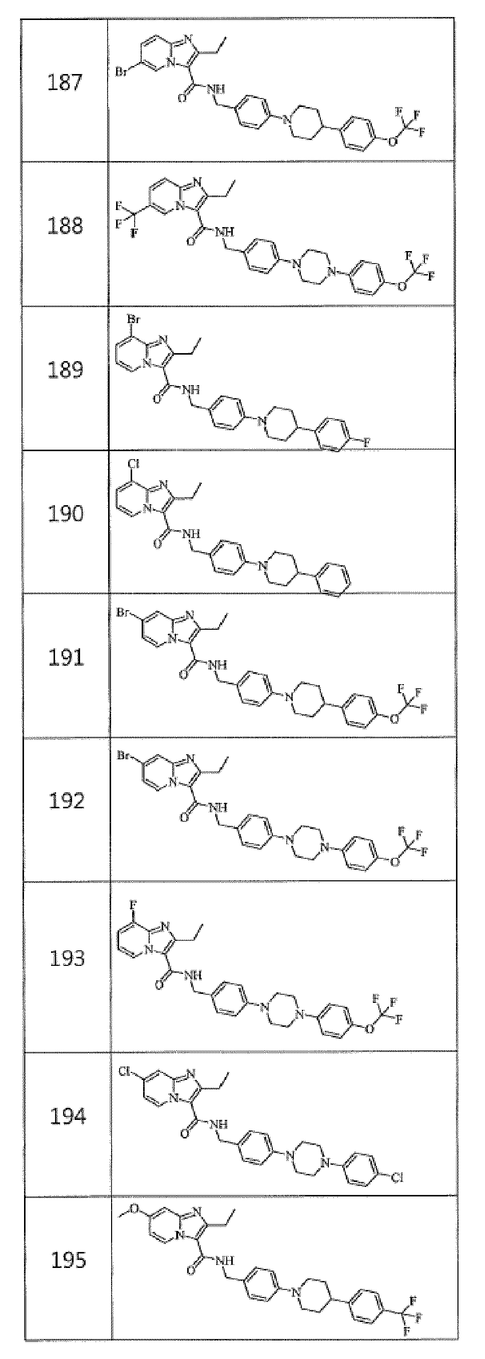 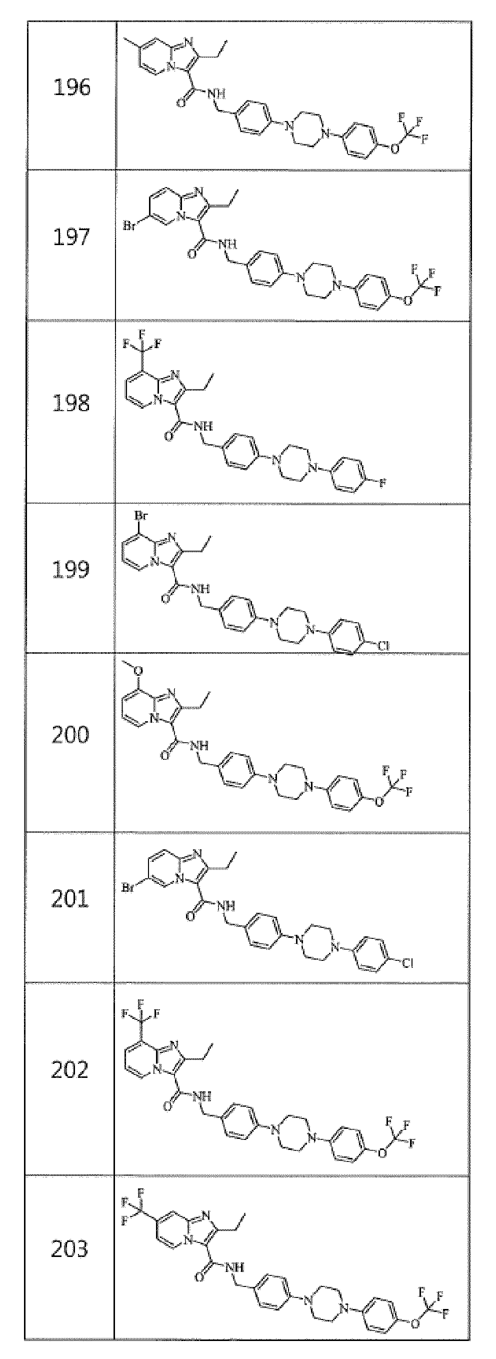 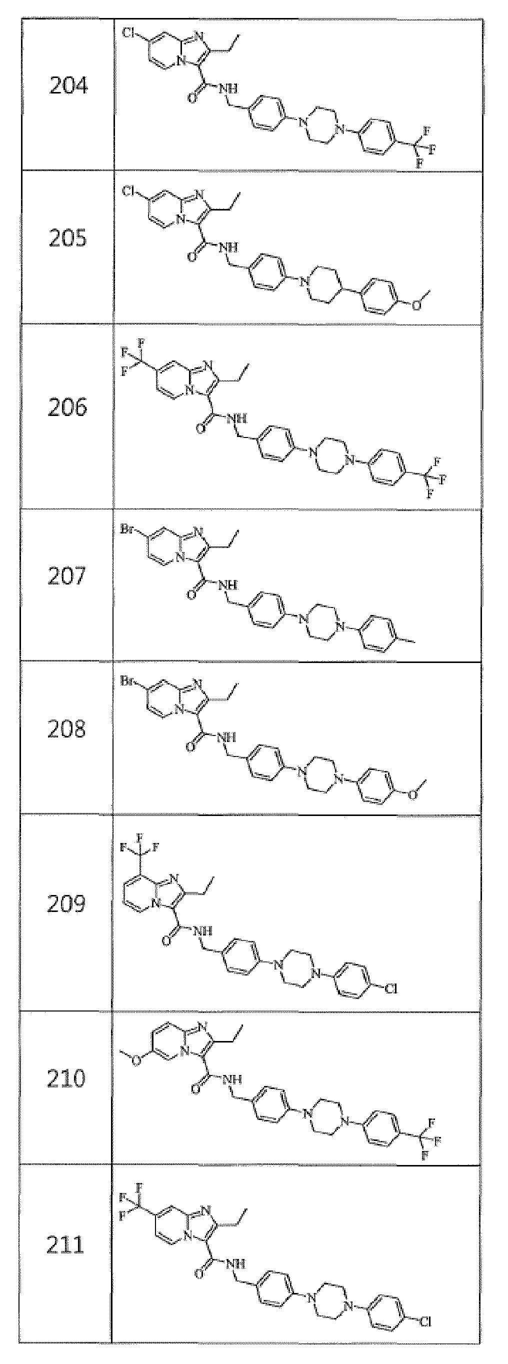 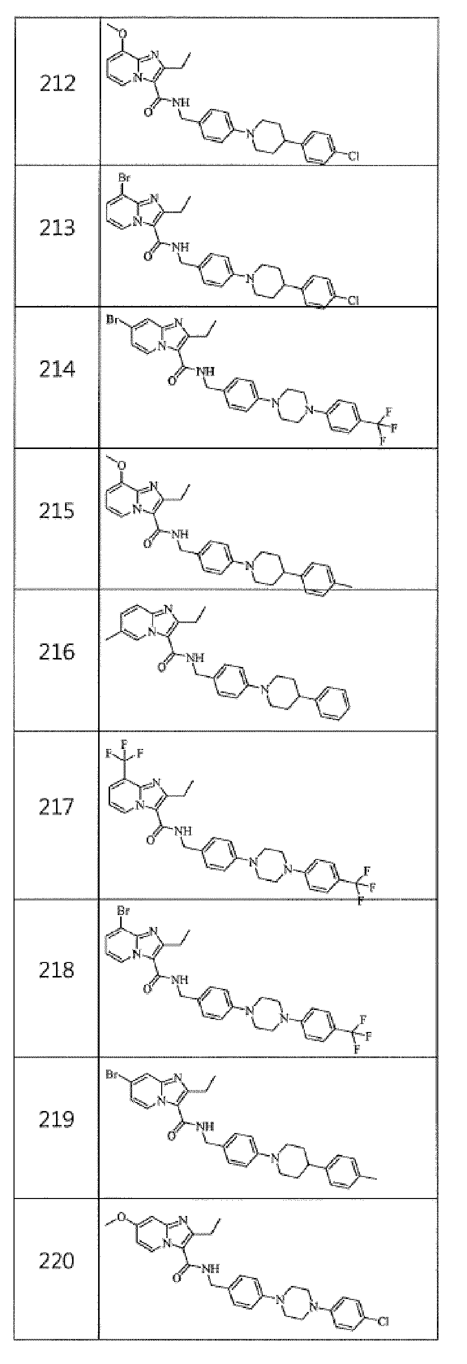 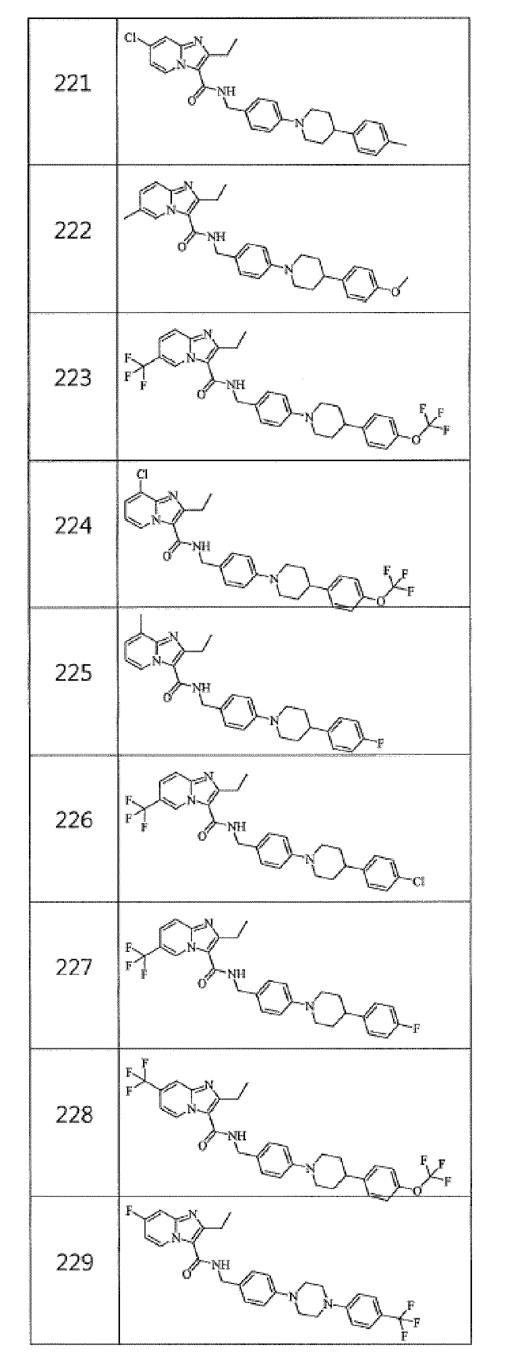 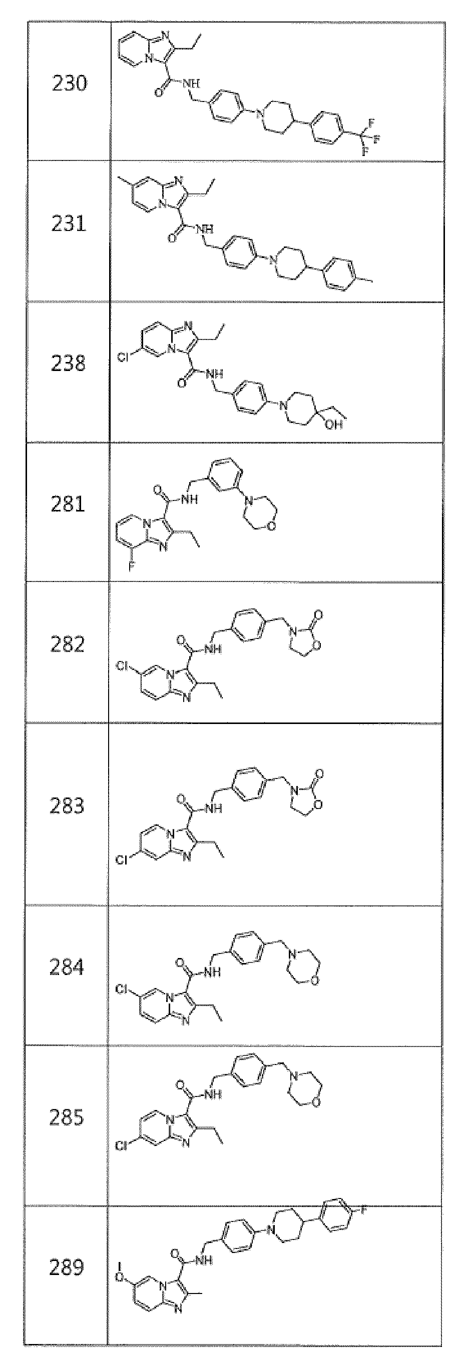 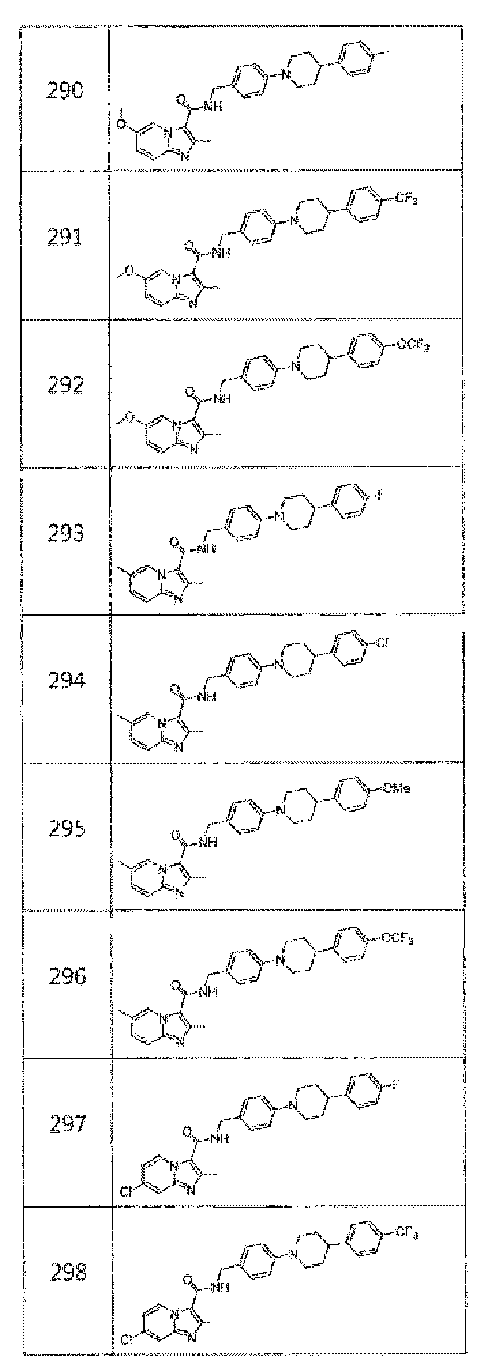 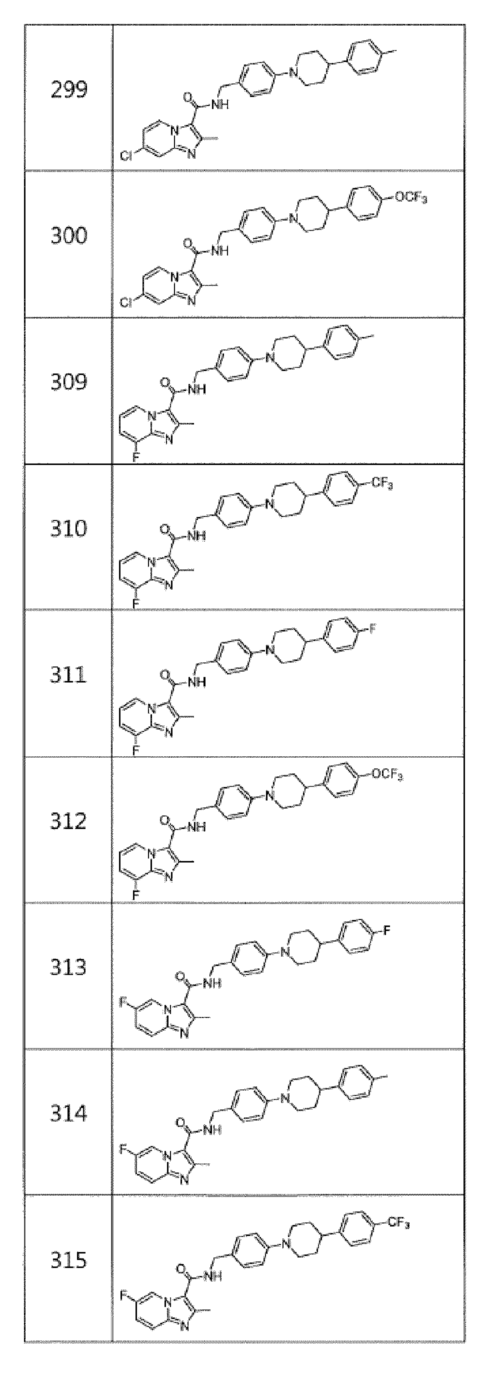 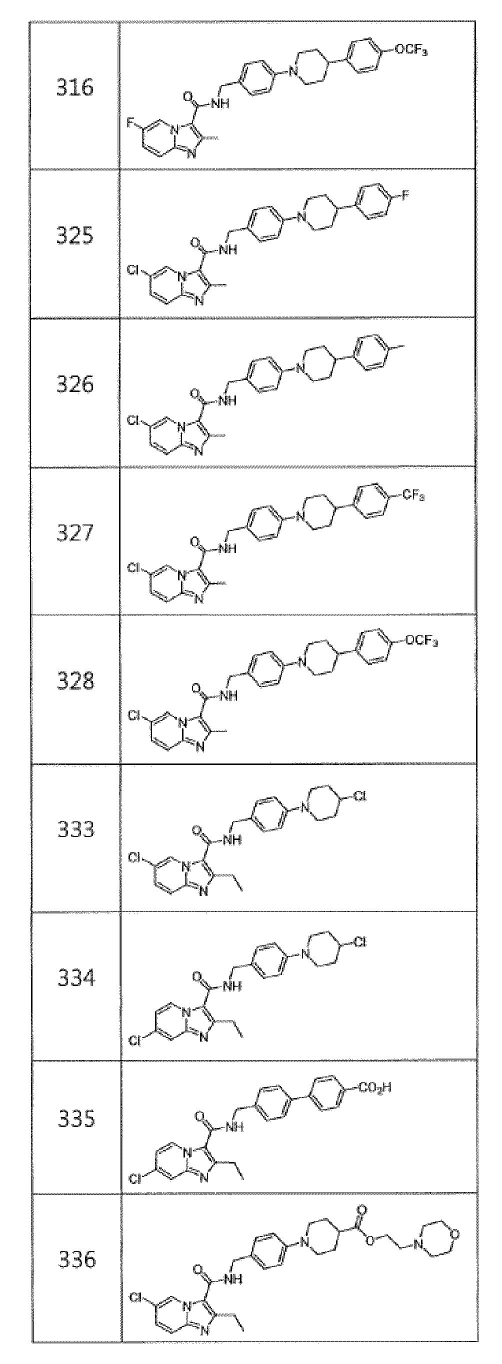 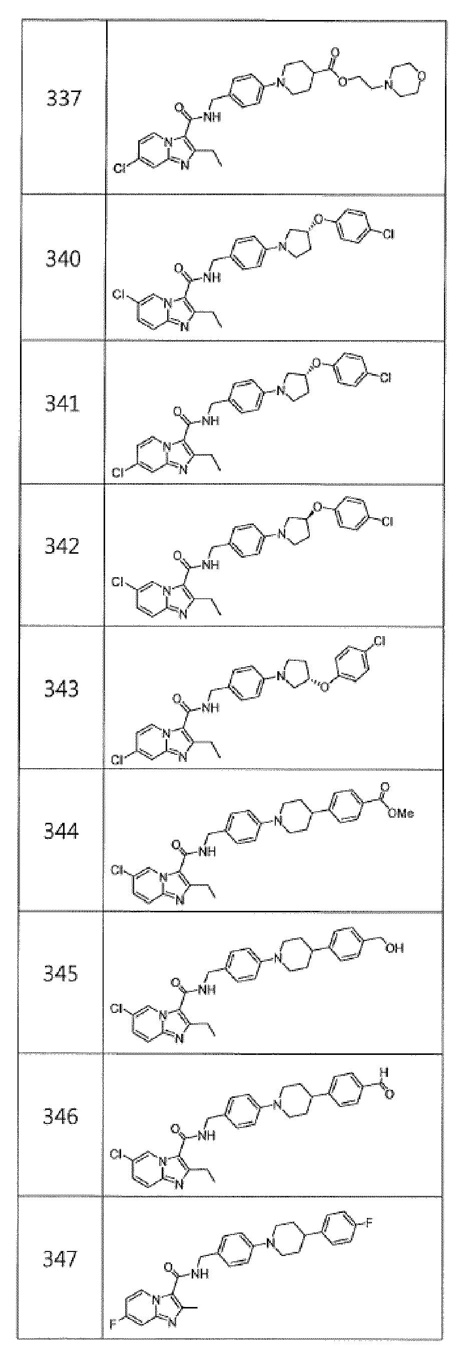 de préférence l'une des formules 1 à 21, 23 et 24, 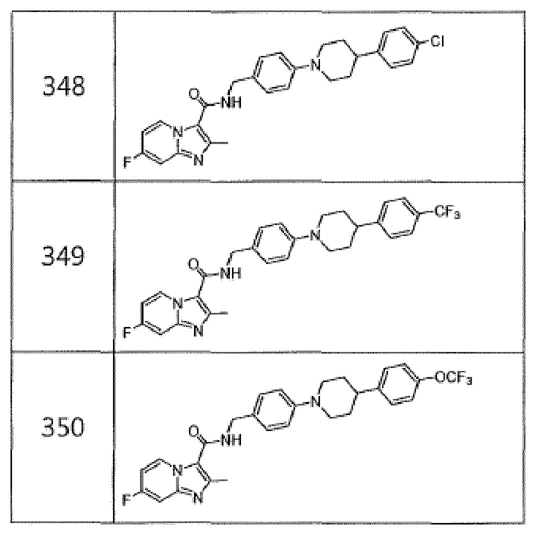 26, 28 à 33, 35 à 57, 59 à 77, 79 à 83, 85 à 87, 90 à 98, 100 à 102, 106 à 111, 113 à 116, 118 à 124, 126 à 128, 130 à 142, 144 à 150, 153, 155 à 167, 169 à 184, 186 à 188, 190 à 197, 199, 201, 203 à 208, 210 et 211, 213 et 214, 216, 218 à 231, 238, 281 à 285, 289 à 300, 309 à 316, 325 à 328, 333 à 337, 340 à 350 telles que représentées ci-dessus.

2. Composé selon la revendication 1, ayant l'une des formules 55, 171, 175 et 325 telles que représentées ci-dessous :
| | |
|---|---|
| 55 | 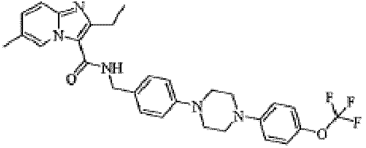 |
| 171 | 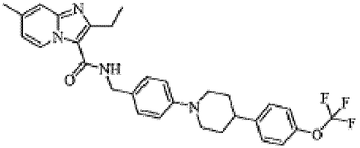 |
| 175 | 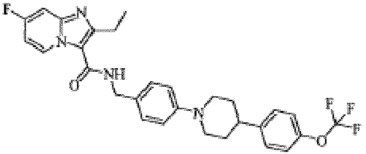 |
| | |
| 325 | 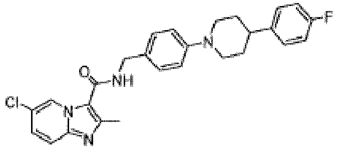 |
et les sels pharmaceutiquement acceptables de ceux-ci.

3. Composé selon l'une quelconque des revendications 1 et 2, pour une utilisation dans le traitement d'une infection bactérienne, dans lequel, de préférence, ladite infection bactérienne est une tuberculose.

4. Composition pharmaceutique comprenant un composé selon l'une quelconque des revendications 1 à 3, et un support pharmaceutiquement acceptable.
